# EUROPEAN PATENT SPECIFICATION

(11) **EP 3 491 024 B1**
(45) Date of publication and mention of the grant of the patent: **18.12.2024**
(21) Application number: 17754531.6
(22) Date of filing: 29.07.2017
(51) Int. Cl.: C07K 16/28, C07K 16/42, G01N 33/00

(54) **ANTI-IDIOTYPIC ANTIBODIES AGAINST ANTI-CD19 ANTIBODIES**
GEGEN ANTI-CD19 ANTIKÖRPER GERICHTETE ANTI-IDIOTYPISCHE ANTIKÖRPER
ANTICOPRS ANTI-IDIOTYPIQUES DIRIGÉS CONTRE ANTICOPRS ANTI-CD19

(30) Priority: 29.07.2016 US 201662369008 P
(43) Date of publication of application: 05.06.2019
(73) Proprietor: Juno Therapeutics, Inc., Seattle, WA 98109 (US)
(72) Inventor: HAUSKINS, Collin, Seattle, WA 98109 (US); HEIPEL, Mark, D., Seattle, WA 98109 (US); SUTHERLAND,Claire, L., Seattle, WA 98109 (US); SATHALIYA, Taher, Seattle, WA 98109 (US); SMITH, Jeff, Seattle, WA 98109 (US)
(74) Representative: Carpmaels & Ransford LLP
(86) International application number: PCT/US2017/044560
(87) International publication number: WO 2018/023100

(56) References cited:
- WO-A1-2014/190273
- BIPULENDU JENA ET AL: "Chimeric Antigen Receptor (CAR)-Specific Monoclonal Antibody to Detect CD19-Specific T Cells in Clinical Trials", PLOS ONE, vol. 8, no. 3, 1 March 2013 (2013-03-01), pages e57838, XP055122892, DOI: 10.1371/journal.pone.0057838
- DATABASE Geneseq [online] 10 October 2013 (2013-10-10), "Anti-IgE mAb light chain variable region, SEQ ID 2.", XP002775184, retrieved from EBI accession no. GSP:BAS38022 Database accession no. BAS38022
- DATABASE Geneseq [online] 13 August 2015 (2015-08-13), "Anti-LINGO1 antibody heavy chain variable region, SEQ ID 63.", XP002775185, retrieved from EBI accession no. GSP:BCB30778 Database accession no. BCB30778
- DATABASE Geneseq [online] 26 February 2015 (2015-02-26), "Mouse anti-tau antibody F11G3 heavy chain variable region, SEQ ID 11.", XP002777043, retrieved from EBI accession no. GSP:BBT47915 Database accession no. BBT47915
- DATABASE Geneseq [online] 18 March 2010 (2010-03-18), "Mouse anti-FGFR3 monoclonal antibody VL sequence, SEQ ID 8.", XP002777044, retrieved from EBI accession no. GSP:AXU66902 Database accession no. AXU66902

## Description

### Field

The present disclosure relates in some aspects to anti-idiotype antibodies that specifically recognize anti-CD19 antibody moieties, in particular, anti-CD19 antibody moieties present in recombinant receptors, including chimeric antigen receptors (CARs). The disclosure further relates to uses of anti-idiotype antibodies for specifically identifying or selecting cells expressing such recombinant receptors, such as anti-CD19 CAR T cells. The disclosure further relates to uses of anti-idiotype antibodies for specifically activating such cells.

### Background

Methods are available for adoptive cell therapy using engineered cells expressing recombinant receptors, such as chimeric antigen receptor (CARs) containing extracellular antibody antigen-binding domains. Various strategies are available to assess activity of such cells either *in vitro* or upon *in vivo* to a subject. Improved methods are needed to specifically assess activity of CAR-expressing cells. Provided are reagents, compositions, and articles of manufacture that meet such needs.

Bipulendu Jena et al., describes chimeric antigen receptor (CAR)-specific monoclonal antibody to detect CD19-specific T cells in clinical trials.

WO2014/190273A1 describes chimeric antigen receptor-targeting monoclonal antibodies.

### Summary

The present invention is set out in the appended set of claims.

The present invention provides an anti-idiotype antibody or antigen-binding fragment thereof comprising:
a CDR-H1 comprising the amino acid sequence of SEQ ID NO: 89 or 90, a CDR-H2 comprising the amino acid sequence of SEQ ID NO: 91, 92 or 93, and a CDR-H3 comprising the amino acid sequence of SEQ ID NO: 94; and
a CDR-L1 comprising the amino acid sequence of SEQ ID NO: 95, a CDR-L2 comprising the amino acid sequence of SEQ ID NO: 96, and a CDR-L3 comprising the amino acid sequence of SEQ ID NO: 97, wherein:
   (i) the anti-idiotype antibody or antigen-binding fragment thereof specifically binds to a target antibody or an antigen-binding fragment thereof, wherein the antibody or antigen-binding fragment thereof comprises the heavy chain variable (VH) region sequence set forth in SEQ ID NO: 30 and the light chain variable (VL) region sequence set forth in SEQ ID NO: 31; and
   (ii) the anti-idiotype antibody or antigen-binding fragment thereof is an agonist antibody of a chimeric antigen receptor (CAR) comprising the target antibody or antigen-binding fragment.

The present invention also provides a conjugate, comprising the anti-idiotype antibody or antigen-binding fragment of the present invention and a heterologous molecule or moiety, optionally wherein the heterologous molecule or moiety is:
i) a label, optionally wherein the label is selected from a fluorescent dye, a fluorescent protein, a radioisotope, a chromophore, a metal ion, a gold particle, a silver particle, a magnetic particle, a polypeptide, an enzyme, a streptavidin, a biotin, a luminescent compound or an oligonucleotide; or
ii) a protein, peptide, nucleic acid or small molecule, which optionally is or comprises a toxin or a Strep-Tag.

The present invention also provides a nucleic acid molecule(s) encoding the heavy chain and light chain of the anti-idiotype antibody or antigen-binding fragment thereof of the present invention.

The present invention also provides a vector, comprising the nucleic acid molecule(s) of the present invention.

The present invention also provides a cell, comprising the anti-idiotype antibody or antigen-binding fragment thereof of the present invention or the nucleic acid molecule of the present invention.

The present invention also provides a method of producing an anti-idiotype antibody or antigen-binding fragment thereof, comprising expressing the heavy and light chain encoded by the nucleic acid molecule of the present invention or the vector of the present invention in a suitable host cell and recovering or isolating the antibody.

The present invention also provides a composition comprising the anti-idiotype antibody or antigen-binding fragment thereof of the present invention, conjugate of the present invention, or the cell of the present invention, optionally further comprising a pharmaceutically acceptable excipient.

The present invention also provides a method of detecting a target antibody or antigen-binding fragment thereof, comprising:
(a) contacting a composition comprising a target antibody or an antigen-binding fragment thereof with the anti-idiotype antibody or antigen-binding fragment thereof of the present invention or the conjugate of the present invention that specifically binds to the target antibody or antigen-binding fragment thereof, wherein the target antibody or antigen-binding fragment thereof comprises the heavy chain variable (VH) region sequence set forth in SEQ ID NO: 30 and the light chain variable (VL) region sequence set forth in SEQ ID NO: 31; and
(b) detecting the anti-idiotype antibody bound to the target antibody or antigen-binding fragment,
optionally wherein the target antibody or antigen-binding fragment is expressed on the surface of a cell, wherein the cell expresses on its surface a CAR comprising the target antibody or antigen-binding fragment, and detecting in (b) comprises detecting cells bound with the anti-idiotype antibody.

The present invention also provides an in vitro or ex vivo method of stimulating cells, comprising incubating an input composition comprising cells expressing a chimeric antigen receptor (CAR) comprising a target antibody or an antigen-binding fragment thereof with the anti-idiotype antibody or antigen-binding fragment thereof of the present invention or the conjugate of the present invention that specifically binds to the target antibody or antigen-binding fragment thereof, thereby generating an output composition comprising stimulated cells, wherein the antibody or antigen-binding fragment thereof comprises the heavy chain variable (VH) region sequence set forth in SEQ ID NO: 30 and the light chain variable (VL) region sequence set forth in SEQ ID NO: 31.

Provided herein are agents that specifically bind to antibodies, including antibody fragments such as scFvs, and chimeric molecules containing the same, such as chimeric antigen receptors. Also provided are compositions and articles of manufacture containing such agents, including those including a surface to which the agent is bound, such as a solid surface, e.g. a plate or bead. Also among the embodiments provided herein are uses and methods of using such agents, compositions and articles, including for detection, use, manipulation and/or stimulation of cells or therapies containing or suspected of containing the antibody or chimeric molecule, such as in the detection, stimulation or use of CAR-expressing cells.

In some embodiments, the agent is an anti-idiotype antibody or antigen-binding fragment thereof that specifically binds to a target antibody that is or contains variable region(s) of the antibody designated FMC63 or an antigen-binding fragment thereof and specifically binds to a chimeric molecule containing such an antibody fragment, in particular a CAR with a binding domain containing antibody variable regions derived from FMC63, such as in the form of an scFv. In some embodiments, the agent, in particular the anti-idiotype antibody or antigen-binding fragment contains a light chain variable (VL) region containing at least 90% sequence identity to the VL region amino acid sequence set forth in SEQ ID NO: 40; and/or a heavy chain variable (VH) region containing at least 90% sequence identity (and/or at least 95 % or 99 % sequence identity, or 100 % identity) to the VH region amino acid sequence set forth in SEQ ID NO: 36. In some embodiments, the antibody or antigen-binding fragment contains a VL region comprising at least 90% sequence identity (and/or at least 95 % or 99 % sequence identity, or 100 % identity) to the VL region amino acid sequence set forth in SEQ ID NO: 40; and/or a VH region comprising at least 90 % sequence identity(and/or at least 95 % or 99 % sequence identity, or 100 % identity) to the VH region amino acid sequence set forth in SEQ ID NO: 36.

In the invention, the complementarity determining region 3 (CDR-H3) contains the amino acid sequence set forth in SEQ ID NO: 94 and contains a CDR-H3 contained within the VH sequence set forth in SEQ ID NO: 36; and the light chain complementarity determining region 3 (CDR-L3) contains the amino acid sequence set forth in SEQ ID NO: 97 and containing a CDR-L3 contained within the VL sequence set forth in SEQ ID NO: 40.

In the invention, the VH region contains a CDR-H1 and a CDR-H2, respectively, including the amino acid sequences of CDR-H1 and CDR-H2 sequences contained within the VH region amino acid sequence set forth in SEQ ID NO: 36; and/or the VL region contains a CDR-L1 and CDR-L2, respectively, including the amino acid sequences of CDR-L1 and CDR-L2 sequences contained within the VL region amino acid sequence set forth in SEQ ID NO: 40.

In the invention, the VH region contains a CDR-H1 set forth in SEQ ID NO: 89 or 90, a CDR-H2 set forth in SEQ ID NO: 91, 92 or 93 and a CDR-H3 set forth in SEQ ID NO: 94; and the VL region contains a CDR-L1 set forth in SEQ ID NO: 95, a CDR-L2 set forth in SEQ ID NO: 96, and a CDR-L3 set forth in SEQ ID NO: 97.

In the invention, the anti-idiotype antibody or antigen-binding fragment thereof contains a CDR-H1, a CDR-H2, and a CDR-H3, respectively, containing the amino acid sequences of CDR-H1, CDR-H2, and CDR-H3 sequences contained within the VH region amino acid sequence set forth in SEQ ID NO: 36; and a CDR-L1, a CDR-L2, and a CDR-L3, respectively, including the amino acid sequences of CDR-L1, CDR-L2, and CDR-L3 sequences contained within the VL region amino acid sequence set forth in SEQ ID NO: 40.

In the invention, the anti-idiotype antibody or antigen-binding fragment thereof comprises a CDR-H1 including the amino acid sequence of SEQ ID NO: 89 or 90, a CDR-H2 including the amino acid sequence of SEQ ID NO: 91, 92 or 93, and a CDR-H3 including the amino acid sequence set forth as SEQ ID NO: 94; and a CDR-L1 including the amino acid sequence of SEQ ID NO: 95, a CDR-L2 including the amino acid sequence of SEQ ID NO: 96, and a CDR-L3 including the amino acid sequence of SEQ ID NO: 97.

In some of any such embodiments, the VH region of the antibody or fragment contains the amino acid sequence of SEQ ID NO: 36; and/or the VL region of the antibody or fragment comprises the amino acid sequence of SEQ ID NO: 40. In some embodiments, the VH region of the antibody or fragment includes the amino acid sequence of SEQ ID NO: 36 and the VL region of the antibody or fragment includes the amino acid sequence of SEQ ID NO: 40.

In the invention, the VH region contains a CDR-H1 set forth in SEQ ID NO: 89, or 90, a CDR-H2 set forth in SEQ ID NO: 91, 92, or 93 and a CDR-H3 set forth in SEQ ID NO: 94; and the VL region contains a CDR-L1 set forth in SEQ ID NO: 95, a CDR-L2 set forth in SEQ ID NO: 96, and a CDR-L3 set forth in SEQ ID NO: 97.

In the invention, the VH region contains a CDR-H1, CDR-H2 and CDR-H3 including the amino acid sequences of CDR-H1, CDR-H2, and CDR-H3 sequences contained within the VH region amino acid sequence set forth in SEQ ID NO: 36; and the VL region contains a CDR-L1, a CDR-L2, and a CDR-L3, respectively, including the amino acid sequences of CDR-L1, CDR-L2, and CDR-L3 sequences contained within the VL region amino acid sequence set forth in SEQ ID NO: 40.

In some of any such embodiments, the VH region of the antibody or fragment comprises the amino acid sequence of SEQ ID NO: 36; and/or the VL region of the antibody or fragment comprises the amino acid sequence of SEQ ID NO: 40.

In some of any such embodiments, the target antibody or antigen-binding fragment is a single chain fragment. In some aspects, the fragment contains antibody variable regions joined by a flexible linker. In some of any such embodiments, the fragment contains an scFv.

In the invention, the target antibody or antigen-binding fragment contains a heavy chain variable region set forth in SEQ ID NO: 30 and a light chain variable region set forth in SEQ ID NO: 31; and optionally is an scFv comprising the sequence of amino acids set forth in SEQ ID NO: 34.

In some of any such embodiments, the anti-idiotype antibody or antigen-binding fragment specifically binds to the same or an overlapping epitope of a target antibody or antigen-binding fragment thereof as the epitope specifically bound by the anti-idiotype antibody or antigen-binding fragment according to any one of the embodiments described herein.

In some of any such embodiments, the target antibody or antigen-binding fragment is within or included in the antigen-binding domain of the extracellular portion of a chimeric antigen receptor (CAR); and/or the anti-idiotype antibody or antigen-binding fragment specifically binds the target antibody or antigen-binding fragment contained within or included in the antigen-binding domain of the extracellular portion of a CAR. In some embodiments, the target antibody or antigen-binding fragment is an scFv and the anti-idiotype antibody or antigen-binding fragment specifically binds to an epitope in the scFv of the CAR.

In some of any such embodiments, the antibody or fragment specifically binds to a single chain variable fragment (scFv) derived from antibody FMC63 comprised in the extracellular portion of a chimeric antigen receptor, wherein the scFv derived from antibody FMC63 contains a heavy chain variable region set forth in SEQ ID NO: 30 and a light chain variable region set forth in SEQ ID NO: 31; and optionally contains the sequence of amino acids set forth in SEQ ID NO: 34.

In some of any such embodiments, the anti-idiotype antibody or antigen-binding fragment specifically binds to an epitope within or including all or a portion of a complementarity determining region (CDR) of the target antibody or antigen-binding fragment.

In some of any such embodiments, the CAR further contains a transmembrane domain linked to the antigen-binding domain via a spacer. In some embodiments, the spacer contains an extracellular portion from CD28, which optionally is human CD28. In some aspects, the extracellular portion from CD28 contains the sequence of amino acids set forth in SEQ ID NO: 27. In some of any such embodiments, the transmembrane domain contains a transmembrane portion of CD28, which optionally is human CD28. In some of any such embodiments, the antibody or fragment does not bind to an epitope in the spacer domain of the CAR.

In some of any such embodiments, the antibody or fragment does not bind or does not specifically bind to CD28 or a portion thereof, which optionally is human CD28, which optionally contains an extracellular portion of CD28, which optionally contains the sequence of amino acids set forth in SEQ ID NO: 27. In some of any such embodiments, the antibody or fragment does not bind to an epitope in an Fc domain, which optionally is a human IgG1 Fc domain.

In some of any such embodiments, the target antibody or antigen-binding fragment specifically binds to human CD19. In some of any such embodiments, the anti-idiotype antibody or fragment does not cross-react with another anti-CD 19 antibody, which optionally is contained in the extracellular antigen-binding domain of another CAR. In some of any such embodiments, the anti-idiotype antibody or fragment does not cross-react with another CAR.

In the invention, the anti-idiotype antibody or fragment is an agonist antibody of a CAR containing the target antibody or antigen-binding fragment.

In some of any such embodiments, the anti-idiotype antibody or antigen-binding fragment thereof is humanized. In some of any such embodiments, the anti-idiotype antibody or antigen-binding fragment thereof is recombinant. In some of any such embodiments, the anti-idiotype antibody or antigen-binding fragment thereof is monoclonal.

In some of any such embodiments, the anti-idiotype antibody or antigen-binding fragment thereof is an antigen-binding fragment. In some aspects, the antigen-binding fragment is selected from among fragment antigen binding (Fab) fragments, F(ab')₂ fragments, Fab' fragments, Fv fragments, a single chain variable fragment (scFv) or a single domain antibody.

In some of any such embodiments, the anti-idiotype antibody or antigen-binding fragment thereof contains at least a portion of an immunoglobulin constant region. In some embodiments, the at least a portion of an immunoglobulin constant region contains an Fc region or a portion of the Fc containing the CH2 and CH3 domains. In some aspects, the constant region is derived from human IgG. In some of any such embodiments, the anti-idiotype antibody or antigen-binding fragment is an intact antibody or full-length antibody.

In some of any such embodiments, provided is a conjugate containing the anti-idiotype antibody or antigen-binding fragment according to any one of the embodiments described above and a heterologous molecule or moiety. In some embodiments, the heterologous molecule or moiety is a label. In some aspects, the label is selected from a fluorescent dye, a fluorescent protein, a radioisotope, a chromophore, a metal ion, a gold particle, a silver particle, a magnetic particle, a polypeptide, an enzyme, a streptavidin, a biotin, a luminescent compound or an oligonucleotide. In some instances, the heterologous molecule or moiety is a protein, peptide, nucleic acid or small molecule, which optionally is or contains a toxin, Strep-Tag.

In some embodiments, provided is a nucleic acid molecule(s) encoding the heavy chain and light chain of the anti-idiotype antibody or antigen-binding fragment thereof according to any one of the embodiments described herein.

In some embodiments, the nucleic acid molecule contains a sequence of nucleotides encoding (i) the heavy chain variable region set forth in SEQ ID NO: 50, (ii) a sequence of nucleotides that has at least 90% sequence identity to the sequence of nucleotides set forth in SEQ ID NO: 50; or (iii) a degenerate sequence of (i) or (ii); and/or a sequence of nucleotides encoding (iv) the light chain variable region set forth in SEQ ID NO: 54, (v) a sequence of nucleotides that has at least 90% sequence identity to the sequence of nucleotides set forth in SEQ ID NO: 54; or (vi) a degenerate sequence of (iv) or (v). In some embodiments, the nucleic acid molecule contains a sequence of nucleotides encoding (i) the heavy chain set forth in SEQ ID NO: 52, (ii) a sequence of nucleotides that has at least 90% sequence identity to the sequence of nucleotides set forth in SEQ ID NO: 52; or (iii) a degenerate sequence of (i) or (ii); and/or a sequence of nucleotides encoding (iv) the light chain set forth in SEQ ID NO: 56, (v) a sequence of nucleotides that has at least 90% sequence identity to the sequence of nucleotides set forth in SEQ ID NO: 56; or (vi) a degenerate sequence of (iv) or (v). In some embodiments, the nucleotide sequence encoding the heavy chain and/or light chain contains a signal sequence.

Provided herein is a vector containing the nucleic acid molecule according to any one of the embodiments described herein. Also provided herein is a cell containing the anti-idiotype antibody or antigen-binding fragment thereof according to any one of the embodiments described herein or the nucleic acid molecule according to any one of the embodiments described herein.

Provided herein is a method of producing an anti-idiotype antibody or antigen-binding fragment thereof, including expressing the heavy and/or light chain encoded by the nucleic acid molecule according to any one of the embodiments described herein or the vector according to any one of the embodiments described herein in a suitable host cell and recovering or isolating the antibody. In some embodiments, the method of producing an anti-idiotype antibody or antigen-binding fragment includes culturing the cell according to any one of the embodiments described herein under conditions in which the heavy chain and/or light chain is expressed and recovering or isolating the antibody. Also provided herein is an anti-idiotype antibody or antigen-binding fragment thereof produced by the method according to any one of the embodiments described herein.

In some embodiments, provided is a composition containing the anti-idiotype antibody or antigen-binding fragment thereof according to any one of the embodiments described herein, the conjugate according to any one of the embodiments described herein, or the cell according to any one of the embodiments described herein. In some of any such embodiments, the composition further contains a pharmaceutically acceptable excipient.

In some embodiments, provided is a kit containing one or more of the anti-idiotype antibody or antigen-binding fragment thereof according to any one of the embodiments described herein, the conjugate according to any one of the embodiments described herein, the nucleic acid according to any one of the embodiments described herein, and, optionally, instructions for use. In some instances, the kit further contains a reagent or support for immobilizing the anti-idiotype antibody or antigen-binding fragment thereof or conjugate, wherein said reagent or support is a bead, a column, a microwell, a stick, a filter, a strip or a soluble oligomeric streptavidin mutein reagent.

Also provided are methods of detection using any of the provided agents, such as the anti-idiotype antibodies. In some embodiments, provided is a method of detecting a target antibody or antigen-binding fragment thereof, such as a CAR containing the same, including (a) contacting a composition containing a target antibody with variable regions derived from an antibody FMC63, or from an antigen-binding fragment thereof, with the anti-idiotype antibody or antigen-binding fragment thereof of the invention; and (b) detecting the anti-idiotype antibody bound to the target antibody or antigen-binding fragment and/or detecting the presence or absence of the target antibody or agent.

In some embodiments, the method includes (a) contacting a composition containing or suspected of containing a target antibody that is the antibody FMC63 or an antigen-binding fragment with the anti-idiotype antibody or antigen-binding fragment thereof of any one of the embodiments described or the conjugate of any of the embodiments described that specifically binds to a target antibody that is antibody FMC63 or an antigen-binding fragment thereof; and (b) detecting the anti-idiotype antibody bound to the target antibody or antigen-binding fragment and/or detecting the presence or absence of the target antibody or agent..

In some aspects, the target antibody or antigen-binding fragment is bound to a cell or expressed on the surface of a cell and detecting in (b) includes detecting cells bound with the anti-idiotype antibody. In some embodiments, the cell expresses on its surface a CAR containing the target antibody or target antigen-binding fragment.

In some embodiments, the provided methods involve detecting a CAR containing a target antibody or antigen-binding fragment thereof of any of the embodiments, such as a CAR containing variable domains derived from FMC63. In some embodiments, the method includes (a) contacting a cell expressing a chimeric antigen receptor (CAR) containing a target antibody that is the antibody FMC63 or an antigen-binding fragment thereof with the anti-idiotype antibody or antigen-binding fragment thereof of any one of the embodiments described or the conjugate of any one of the embodiments described that specifically binds to a target antibody that is antibody FMC63 or an antigen-binding fragment thereof; and (b) detecting cells bound with the anti-idiotype antibody. In some of any such embodiments, the anti-idiotype antibody or antigen-binding fragment thereof is directly or indirectly labeled for detection.

In some the disclosure, provided is a method of selecting cells from a cell population, including (a) contacting a cell population expressing a chimeric antigen receptor (CAR) comprising a target antibody or a cell bound to a target antibody with the anti-idiotype antibody or antigen-binding fragment thereof of any one of the embodiments described or conjugate of any one of the embodiments described that specifically binds to a target antibody that is antibody FMC63 or an antigen-binding fragment thereof, wherein the target antibody is the antibody FMC63 or an antigen-binding fragment thereof; and (b) selecting cells bound with the anti-idiotype antibody.

In some instances, the cells bound with the anti-idiotype antibody are selected by affinity-based separation. In some aspects, the affinity-based separation is immunoaffinity-based separation. In some of any such embodiments, the affinity-based separation is by flow cytometry. In some embodiments, the affinity-based separation is by magnetic activated cell sorting. In some aspects, the affinity-based separation contains affinity chromatography. In some of any such embodiments, the anti-idiotype antibody is reversibly bound or immobilized to a support or a stationary phase.

Also among the provided methods are methods for stimulating cells using the agents, such as stimulating cells containing a molecule such as a CAR that is or contains the target antibody recognized by the anti-idiotype antibody. In some embodiments, the method includes incubating an input composition containing cells expressing a chimeric antigen receptor (CAR) containing a target antibody that is the antibody FMC63 or an antigen-binding fragment thereof with the anti-idiotype antibody or antigen-binding fragment thereof of any one of the embodiments described or the conjugate of any one of the embodiments described that specifically binds to a target antibody that is antibody FMC63 or an antigen-binding fragment thereof, thereby generating an output composition containing stimulated cells.

In some embodiments, the methods result in proliferation, activation, stimulation, cytokine release, or other functional outcome such as upregulation of an activation marker or cytokine release or production, of cells expressing the chimeric receptor such as the CAR recognized by the anti-Id antibody. In some aspects, such proliferation or other functional response or readout is induced in such cells to a degree that is similar to or greater than that induced by incubation of the cells with an agent and/or conditions that stimulates proliferation of T cells, such as anti-CD3/CD28 beads and/or crosslinked anti-CD3. In some aspects, the methods do not involve crosslinking of the anti-idiotype antibody. In some aspects of any of the embodiments, the anti-idiotype agents are capable of inducing the specified proliferation or functional outcome or degree thereof, without crosslinking of the anti-idiotype antibody. In some aspects, anti-idiotype agents herein are advantageous in their ability to stimulate or cause a particular functional outcome of T cells or other immune cells expressing the target receptor, without the need to crosslink the anti-Id antibody or use a secondary agent. In some aspects, the result is achieved with soluble or plate-bound form of the anti-idiotype antibody. In some aspects, the result is achieved with the anti-idiotype antibody coupled to a bead.

In some instances of the disclosure, provided is a method of producing a cell composition, including (a) introducing into cells a nucleic acid molecule encoding a chimeric antigen receptor (CAR), thereby generating an input composition; and (b) incubating the input composition with an anti-idiotype antibody or antigen-binding fragment thereof specific for the antigen receptor of the CAR, thereby producing the cell composition.

In some aspects, the CAR contains a target antibody that specifically binds to CD19. In some cases, the target antibody is the antibody FMC63 or an antigen-binding fragment thereof. In some of any such embodiments, the anti-idiotype antibody or antigen-binding fragment thereof specifically binds to a target antibody that is antibody FMC63 of any one of embodiments described that specifically binds to a target antibody that is antibody FMC63 or an antigen-binding fragment thereof.

In some of any such instances, the introducing in (a) includes introducing the nucleic acid molecule into the cells by viral transduction, transposition, electroporation, or chemical transfection. In some instances, the introducing in (a) includes introducing the nucleic acid molecule in the cells by transduction with a retroviral vector containing the nucleic acid molecule, optionally wherein the viral vector is a retroviral vector or a lentiviral vector. In some aspects, the introducing in (a) includes introducing the nucleic acid molecule in the cells by transposition with a transposon containing the nucleic acid molecule. In some instances, the introducing in (a) includes introducing the nucleic acid molecule in the cells by electroporation or transfection of a vector containing the nucleic acid molecule.

In some of any such instances, the method further includes a step of activating the cells prior to step (a). In some aspects, the step of activating the cells includes contacting the cells with an agonist of CD3 and optionally an agonist of CD28. In some instances, the step of activating the cells includes contacting the cells with a reagent containing agonistic anti-CD3 and anti-CD28 antibodies.

In some of any such embodiments, the incubation is performed under conditions in which the anti-idiotype antibody or antigen-binding fragment thereof binds to the CAR, thereby inducing or modulating a signal in one or more cells in the input composition. In any of such embodiments, the cells contain T cells. In some instances, the T cells contain CD4+ and/or CD8+ T cells.

In some of any such embodiments, the anti-idiotype antibody or antigen-binding fragment thereof is immobilized to a solid support, which optionally contains or is conjugated to a reagent containing a plurality of binding sites capable of reversibly binding to the anti-idiotype antibody or antigen-binding fragment thereof. In some of any such embodiments, the anti-idiotype antibody or antigen-binding fragment thereof is immobilized to a soluble reagent, which optionally is or contains a plurality of binding sites capable of reversibly binding to the anti-idiotype antibody or antigen-binding fragment thereof. In some aspects, the reagent contains a streptavidin mutein.

In some of any such embodiments, the incubation is for at least or about at least 5 minutes, 10 minutes, 30 minutes, 60 minutes, 2 hours, 6 hours, 12 hours, 24 hours, 36, 48 hours, 72 hours or 96 hours.

In some of any such embodiments, the input composition contains less than or less than about 60%, less than or less than about 50%, less than or less than about 40%, less than or less than about 30%, less than or less than about 20% or less than or less than about 10% CAR-expressing cells as a percentage of the total cells in the composition.

In some of any such embodiments, the number of CAR-expressing cells in the output composition is increased by greater than 1.2-fold, 1.5-fold, 2.0-fold, 3.0-fold, 4.0-fold, 5.0-fold, 10-fold or more compared to the number of CAR-expressing cells in the input composition; and/or the percentage of CAR-expressing in the output composition compared to the total cells in the composition is increased by greater than 10 %, 20 %, 40 %, 50 %, 60 %, 70 %, 80 % or more.

In some of any such embodiments, prior to the introducing and/or incubating the cells are not selected or enriched for CAR-expressing cells.

In the invention, the target antibody or antigen-binding fragment contains a heavy chain variable region set forth in SEQ ID NO: 30 and a light chain variable region set forth in SEQ ID NO: 31.

In some instances of the disclosure, provided is a method of purifying an antibody or antigen-binding fragment thereof, including (a) contacting a composition containing a target antibody that is the antibody FMC63 or an antigen-binding fragment thereof with the anti-idiotype antibody or antigen-binding fragment thereof of any one of the embodiments described or the conjugate of any one of the embodiments described that specifically binds to a target antibody that is antibody FMC63 or an antigen-binding fragment thereof; and (b) isolating complexes comprising the anti-idiotype antibody.

In some instances, the complexes containing the anti-idiotype antibody are isolated by affinity-based separation. In some aspects, the affinity-based separation is immunoaffinity-based separation. In some instances, the affinity-based separation is magnetic-based separation. In some instances, the affinity-based separation includes affinity chromatography.

In some instances of the disclosure, provided is a method of identifying an anti-idiotype antibody or antigen-binding fragment including (a) introducing into a subject a soluble immunization reagent containing an antigen-binding fragment of the target antibody fused to a solubilizing moiety; and (b) identifying an antibody from the subject that specifically binds to the target antibody or the antigen-binding fragment thereof.

In some of any such instances, the antigen-binding fragment contains the variable heavy chain region and/or variable light chain region of the target antibody. In some instances, the antigen-binding fragment is a single chain fragment. In some aspects, the antigen-binding fragment is an scFv. In some of any such instances, the antigen-binding fragment is within or included in the antigen-binding domain of the extracellular portion of a chimeric antigen receptor (CAR).

In some of any such instances, the solubilizing moiety is an Fc domain or fragment thereof, which optionally is a human IgG1 Fc. In some aspects, the solubilizing moiety is an Fc domain lacking the hinge region. In some instances, the solubilizing moiety contains the amino acid sequence set forth in SEQ ID NO: 32.

In some of any such instances, identifying the antibody includes (i) isolating B cells from the spleen of the subject and fusing them with immortalized B cells to generate hybridomas; (ii) screening the hybridomas for production of antibodies that specifically bind the target antibody or the antigen-binding fragment thereof or a chimeric antigen receptor containing the antigen-binding fragment; and (iii) sequencing an antibody from a hybridoma producing an antibody that specifically binds, thereby identifying the anti-idiotype antibody.

In some of any such instances, the target antibody binds to CD19. In some instances, the antigen-binding fragment of the target antibody is derived from antibody FMC63, optionally wherein the antigen-binding fragment of the target antibody contains a heavy chain variable region set forth in SEQ ID NO: 30 and/or a light chain variable region set forth in SEQ ID NO: 31. In some instances, the antigen-binding fragment of the target antibody is a single chain variable fragment (scFv) derived from antibody FMC63, optionally wherein the scFv contains the sequence of amino acids set forth in SEQ ID NO: 34.

In some instances of the disclosure, there is provided a method of depleting cells, comprising administering, to a subject, a composition comprising the anti-idiotype antibody or antigen-binding fragment thereof of any one of the embodiments described herein or conjugate of any one of the embodiments described that specifically binds to a target antibody that is antibody FMC63 or an antigen-binding fragment thereof, wherein the subject has been administered a cell expressing a chimeric antigen receptor (CAR) containing a target antibody that is the antibody FMC63 or an antigen-binding fragment thereof. In some instances, the depletion occurs via antibody-dependent cell-mediated cytotoxicity (ADCC).

Disclosed herein is a method of determining the presence or absence of a molecule that binds to a chimeric antigen receptor (CAR), the method including (a) contacting a binding reagent with a sample from a subject having been administered a cell therapy containing cells engineered with a CAR containing a target antibody that is the antibody FMC63 or an antigen-binding fragment thereof under conditions to form a complex comprising the binding reagent and a molecule from the sample that binds to the binding reagent, wherein the binding reagent contains the extracellular domain of the CAR or a portion of the extracellular domain comprising the target antibody or the antigen-binding fragment thereof; and (b) detecting the presence or absence of the complex. In some instances, the method further includes carrying out steps (a) and (b) on a positive control sample and, optionally, determining the presence or absence of the molecule by comparison to the positive control, wherein the positive control sample contains any of the anti-idiotype antibody or antigen-binding fragment thereof as described herein or any of the conjugates described herein that specifically binds to the target antibody or an antigen-binding fragment thereof.

In some of any such instances, the molecule that binds to the binding reagent is or contains an antibody. In some instances, the binding reagent is detectably labeled or is capable of producing a detectable signal. In some instances, the binding reagent is bound to a solid support or is soluble.

In some of any such instances, the complex is detected by an immunoassay. In some example, the immunoassay is an enzyme-linked immunosorbent assay (ELISA), chemiluminescent assay, electrochemiluminescent assay, surface plasmon resonance (SPR)-based biosensor (e.g. , BIAcore), flow cytometry, or Western blot. In some instances, the immunoassay comprises meso scale discovery. In some cases, the immunoassay is a sandwich assay or bridge assay.

In some of any such instances, the binding reagent is a first binding reagent and detecting the presence or absence of the complex includes:(i) contacting the complex formed in step (a) with a second binding reagent, wherein the second binding reagent (1) contains the extracellular domain of the CAR or a portion thereof comprising the target antibody or the antigen-binding fragment thereof, and (2) is detectably labeled or is capable of producing a detectable signal; and (ii) assessing the presence or absence of the detectable signal. In some aspects, the first binding reagent is bound to a solid support, optionally wherein first binding reagent is linked, directly or indirectly, to a biotin and/or bound to a solid support through a streptavidin; and/or the second binding reagent is soluble. In some cases, the extracellular domain of the CAR or portion thereof of the first and second binding reagent is the same.

In some of any such instances, the detectable label is or contains a fluorescent label, a chemiluminescent label, an electroluminescent label, a colorimetric label, a bioluminescent label or a radiolabel; and/or the detectable signal is or contains a fluorescent signal, chemiluminescent signal, electroluminescent signal, colorimetric signal, a bioluminescent signal or a radioactive signal. In some of any such instances, the detectable label is or contains a SULFO-TAG.

In some of any such instances, the antigen-binding fragment of the target antibody contains the variable heavy chain region and/or variable light chain region of the target antibody. In some of any such instances, the antigen-binding fragment of the target antibody is a single chain fragment. In some instances, the antigen-binding fragment of the target antibody is an scFv.

In some of any such instances, the sample comprises whole blood, serum or plasma.

Provided herein is an article of manufacture containing any of the anti-idiotype antibodies or antigen-binding fragment thereof as described herein or any of the conjugates described herein, and instructions for using the anti-idiotype antibody to detect an FMC63 antibody or antigen-binding fragment thereof or a chimeric antigen receptor containing the FMC63 antibody or antigen-binding fragment thereof; to select or enrich, from a population of cells, engineered cells expressing a chimeric antigen receptor (CAR) comprising the antibody FMC63 or an antigen-binding fragment thereof; to stimulate an input composition comprising cells expressing a chimeric antigen receptor containing the FMC63 antibody or antigen-binding fragment thereof.

Disclosed herein is an article of manufacture containing a binding reagent including the extracellular domain of a chimeric antigen receptor (CAR) containing a target antibody that is antibody FMC63 or an antigen-binding fragment thereof, said extracellular domain or portion thereof containing the target antibody or antigen-binding fragment thereof; and an anti-idiotype antibody or antigen-binding fragment described herein or the any of the conjugates described herein. In some instances, the binding reagent is a first binding reagent and the article of manufacture further includes a second binding reagent containing the extracellular domain or portion thereof of the CAR.

In some of any such instances, the extracellular domain of the CAR or portion thereof of the first and second binding reagent is the same.

In some of any such instances, the article of manufacture further includes instructions for using the binding reagent, optionally the first and second binding reagent, for assaying a sample for the presence or absence of a molecule that binds to the binding reagent using an immunoassay, optionally wherein the immunoassay is a bridge or sandwich immunoassay, optionally wherein the sample is from a subject having been administered a cell therapy including cells engineered with a CAR containing a target antibody that is the antibody FMC63 or an antigen-binding fragment thereof.

In some instances, the binding reagent, optionally the first and/or second binding reagent, is detectably labeled or capable of producing a detectable signal. In some cases, one of the first and second binding reagent is attached to a solid support of is capable of being attached to a solid support and the other of the first and second binding reagent is detectable label or is capable of producing a detectable signal. In some instances, the article of manufacture further includes a solid support, optionally wherein the one of the first and second binding reagent is linked, directly or indirectly to biotin, and the solid support comprises a streptavidin-coated surface.

### Brief Description of the Drawings

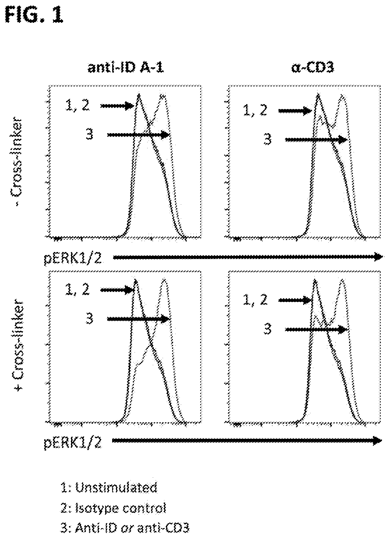

FIG. 1 shows the results of flow cytometry to assess the functional activity of an SJ25C1-derived scFv-specific anti-idiotype antibody clone A-1 (anti-ID A-1) to stimulate Erk1/2 phosphorylation in Jurkat cells engineered with an SJ25C1-derived CAR. Activation with an anti-CD3 antibody was included as a positive control. Unstimulated or isotype control stimulated cells were included as negative controls.

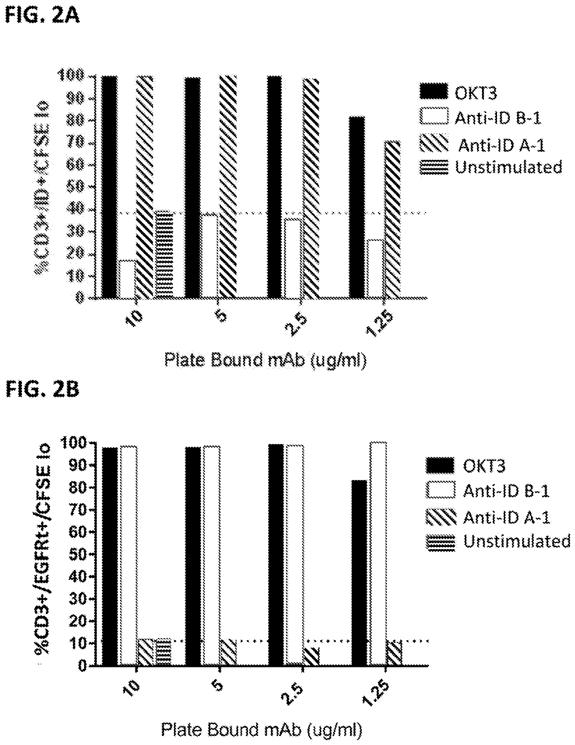

FIG. 2A-B shows results from an assay for the proliferation of T cell expressing a CAR containing either an SJ25C1-derived binding domain (FIG. 2A) or an FMC63-derived binding domain (FIG. 2B), as assessed by dye dilution using flow cytometry following stimulation with an anti-CD3 antibody (OKT3), anti-ID A-1, or anti-ID B-1 anti-idiotype antibody. Unstimulated cells were included as a negative control.

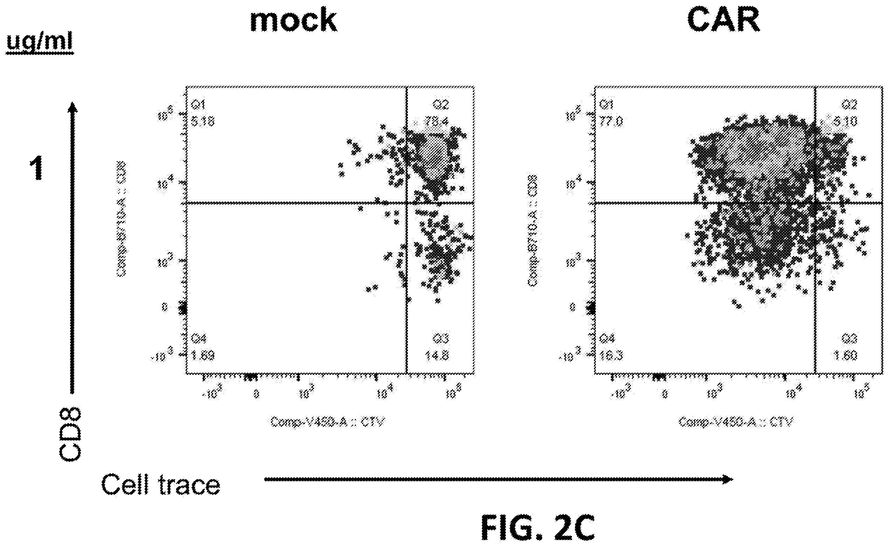

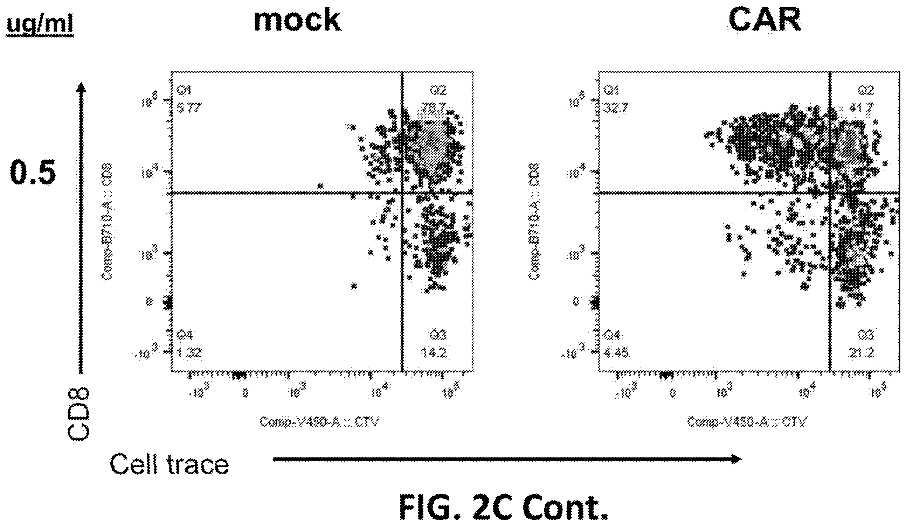

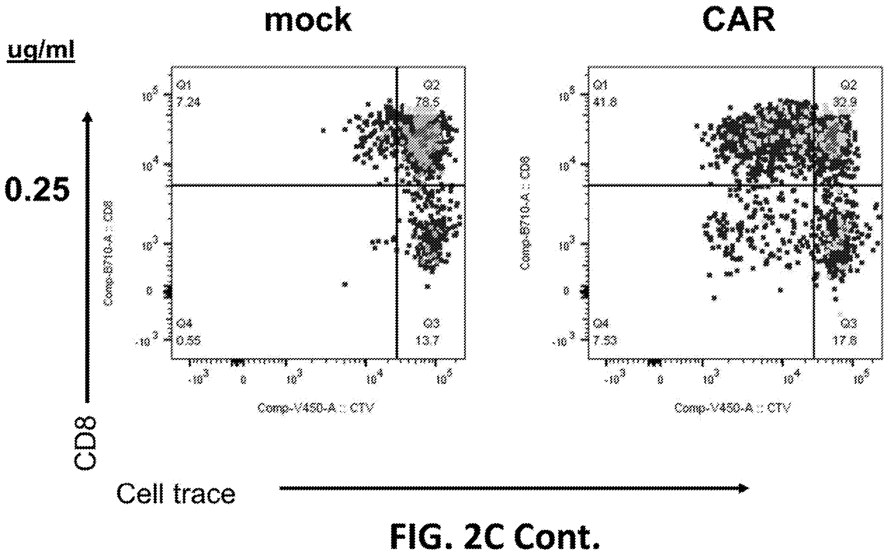

**FIG. 2C** shows results from an assay for the mock transduced and CAR transduced proliferation of T cells (labeled with dye), following culture in the presence of stimulated by plate-bound anti-idiotype antibody recognizing the binding domain of the CAR as assessed by dye dilution using flow cytometry.

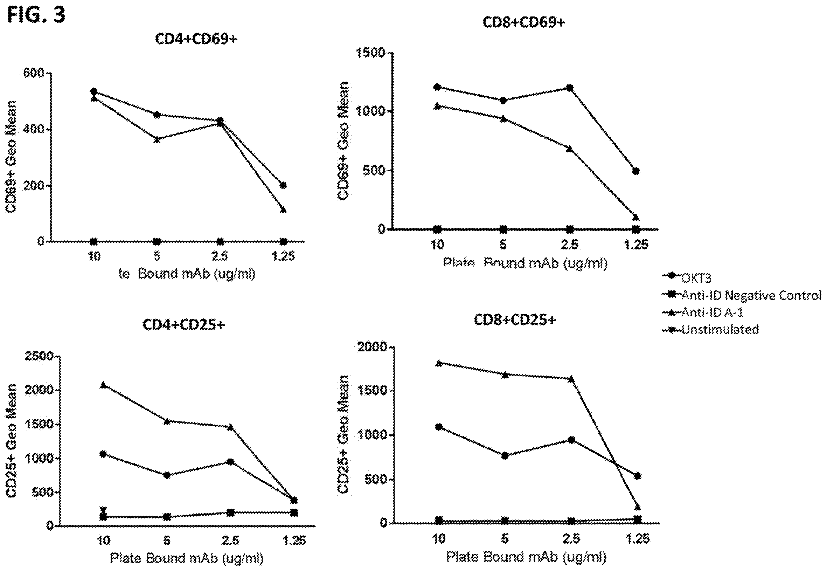

**FIG. 3** shows results for an assessment of the expression of two markers of T cell activation, CD69 and CD25, in CD4⁺ or CD8⁺ T cells expressing a CAR with variable regions derived from SJ25C1 as assessed by flow cytometry following stimulation with plate-bound anti-CD3 antibody (OKT3), anti-ID A-1 or anti-ID B-1.

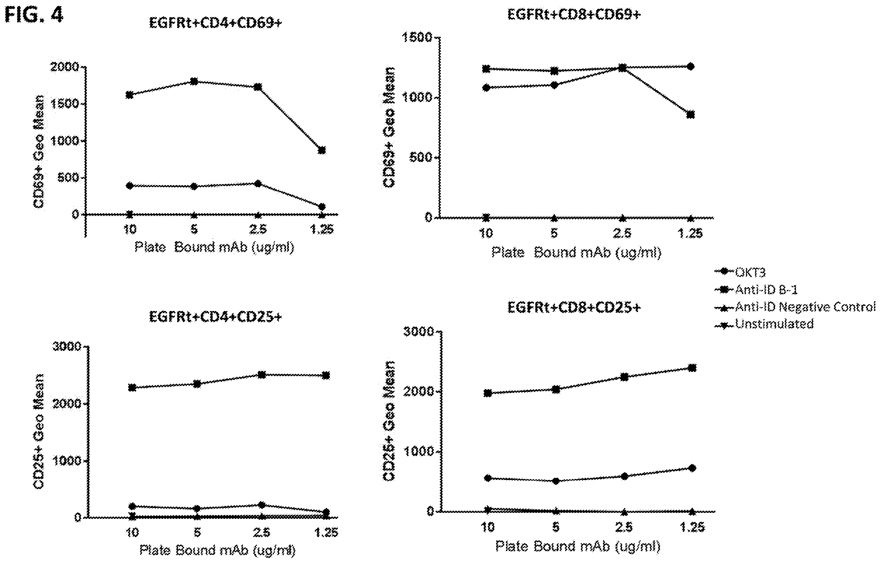

**FIG. 4** shows results for an assessment of the expression of two markers of T cell activation, CD69 and CD25, in CD4⁺ or CD8⁺ T cells expressing a CAR with a binding domain having variable regions derived from FMC63, as assessed by flow cytometry following stimulation with plate-bound anti-CD3 antibody (OKT3), anti-ID A-1 or anti-ID B-1, or a negative control non-target anti-idiotype antibody. Unstimulated cells were included as a negative control.

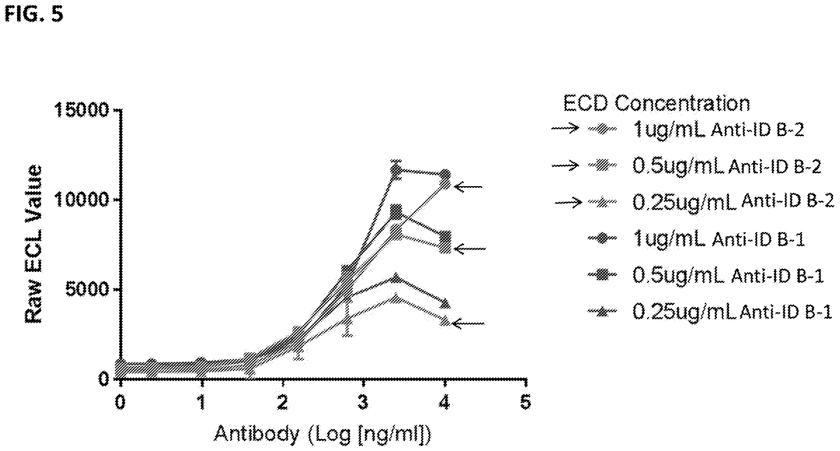

**FIG. 5** shows results from a bridge ELISA for detecting anti-CAR antibodies using anti-ID B-1 and anti-ID B-2 antibodies recognizing the CAR binding domain, at a range of concentrations, as positive controls.

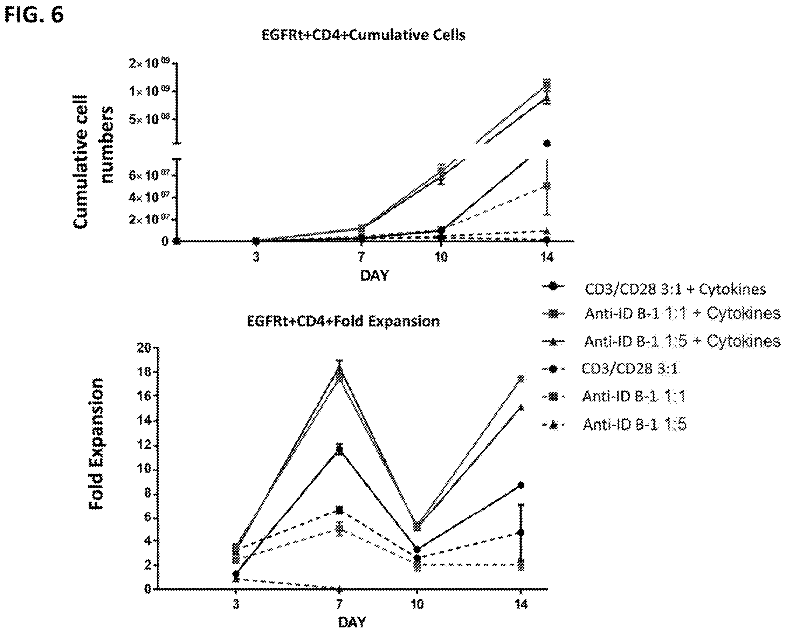

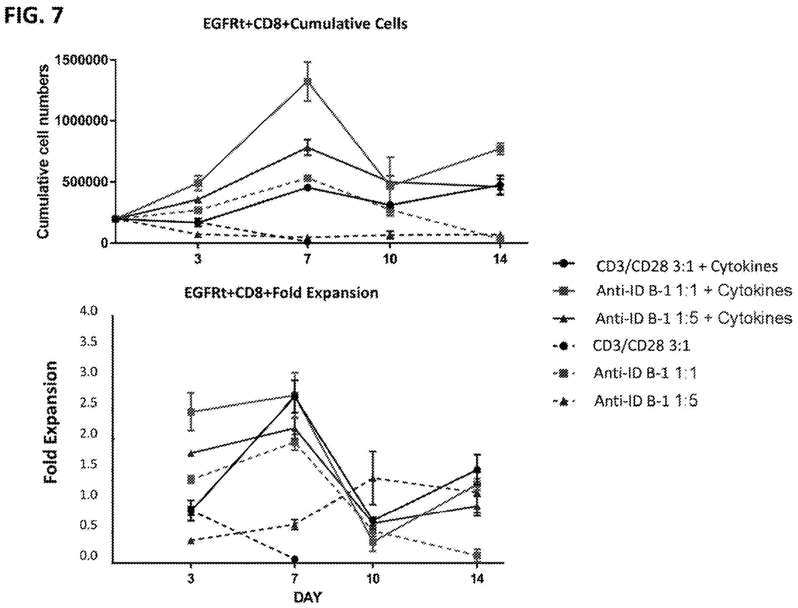

**FIG. 6** and **FIG. 7** shows fold expansion and cumulative cell numbers of EGFRt+/CD4+ T cells or EGFRt+/CD8+ T cells, respectively, stimulated with the indicated ratio of beads coated with anti-idiotype antibody (anti-ID B-1) or control CD3/CD28 antibody coated beads in the presence or absence of cytokines.

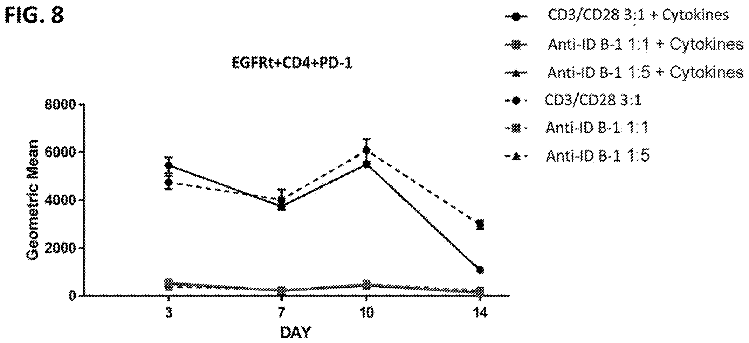

**FIG. 8** shows PD-1 expression levels of CD4+ T cells positive for staining with the anti-EGFR antibody, and thus positive for the transduction marker EGFRt, after stimulation with the indicated ratio of beads coated with anti-idiotype antibody (anti-ID B-1) or control CD3/CD28 antibody coated beads in the presence or absence of cytokines as assessed by flow cytometry at days 3, 7, 10 and 14 of culture.

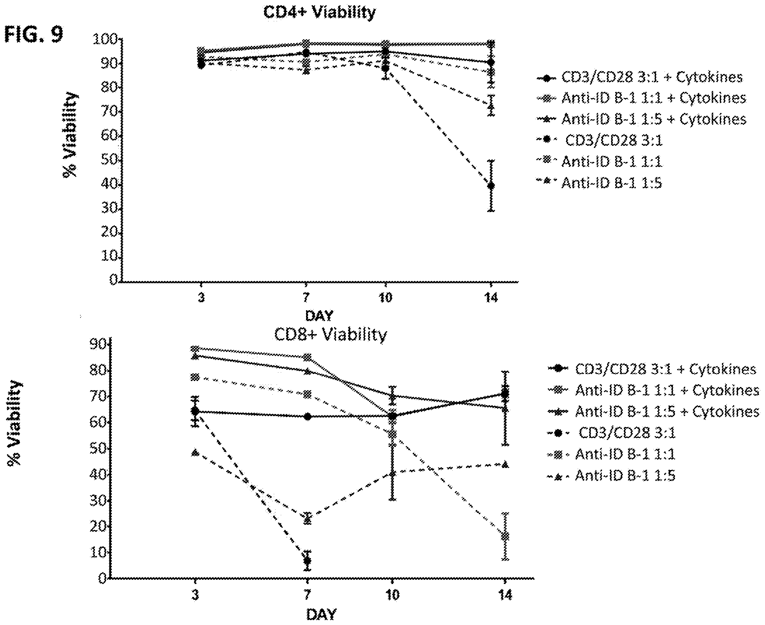

**FIG. 9** shows the viability of CD4⁺ or CD8⁺ T cells expressing an FMC63-derived CAR as assessed by flow cytometry following stimulation with the indicated ratio of beads coated with anti-idiotype antibody (anti-ID B-1) or control CD3/CD28 antibody coated beads in the presence or absence of cytokines as assessed by flow cytometry at days 3, 7, 10 and 14 of culture.

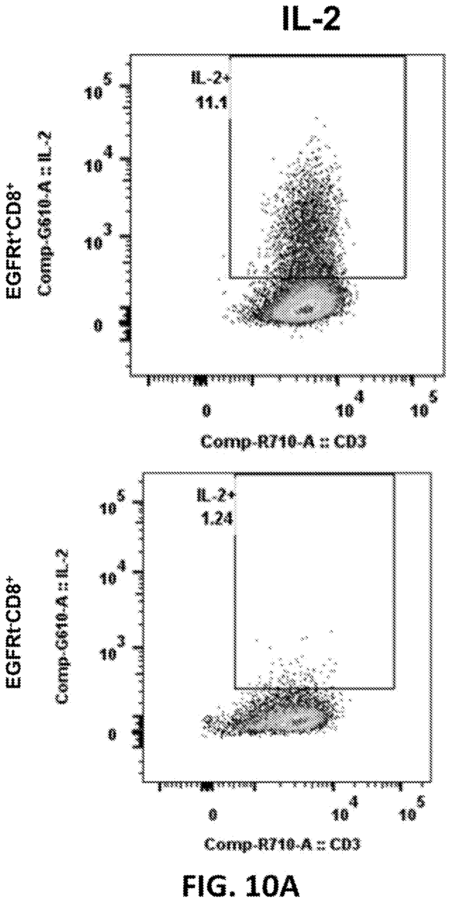

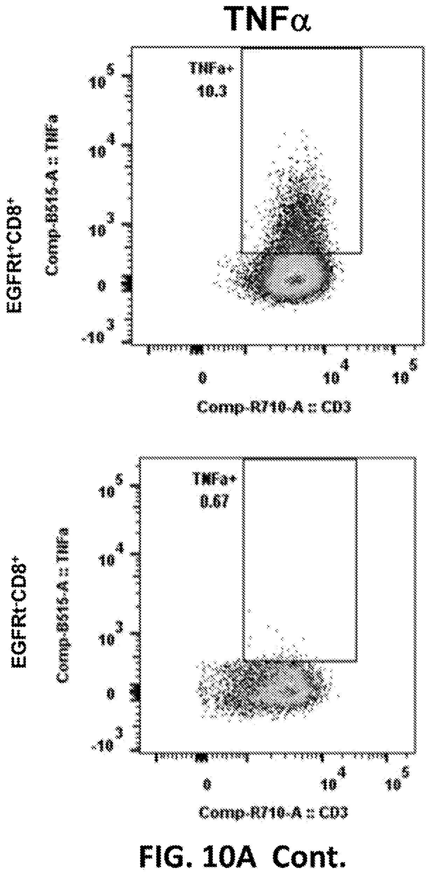

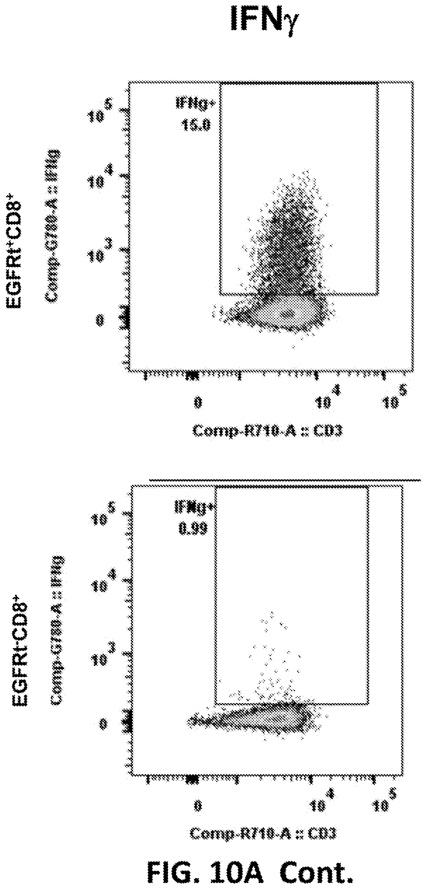

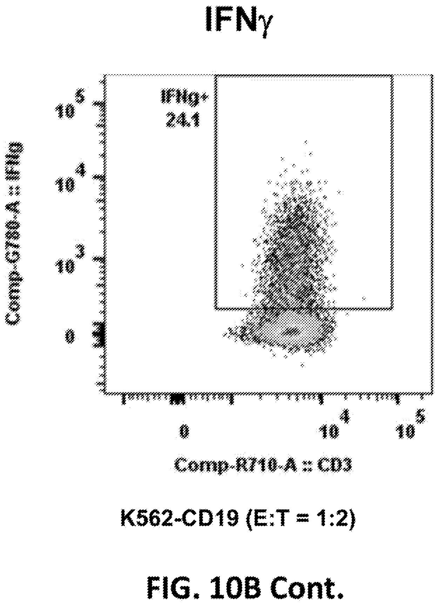

**FIG. 10A** shows intracellular cytokine staining for IL-2, TNFα, and IFNγ of T cells expressing an FMC63-derived CAR following stimulation with FMC63-derived scFv-specific anti-idiotype antibody (anti-ID B-1) coated beads. Shown are results for CD8+ T cells positive or negative for the EGFRt surrogate transduction marker (EGFR+ or EGFRt-).

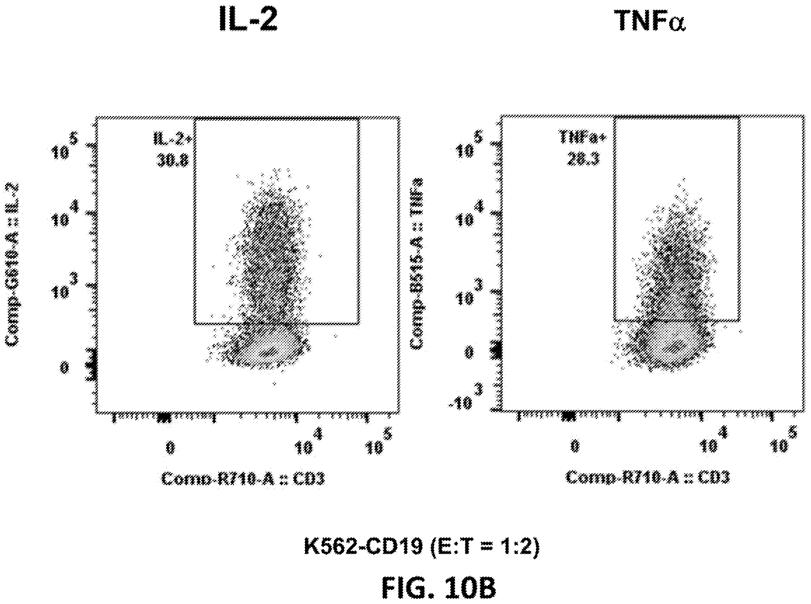

**FIG. 10B** shows intracellular cytokine staining for IL-2, TNFα, and IFNγ of T cells expressing an FMC63-derived CAR following stimulation with antigen-expressing K562-CD19 cells. Shown are results of CD8+ T cells positive for the anti-EGFR antibody as a surrogate for CAR expression.

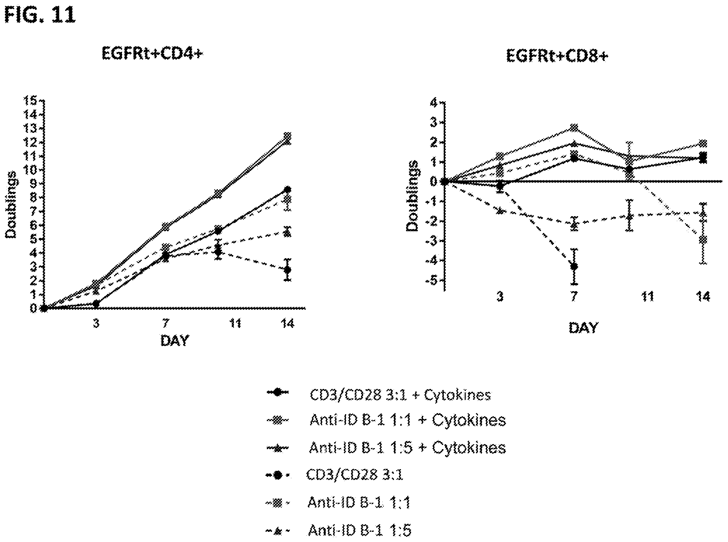

**FIG. 11** shows the number of population doublings in a serial stimulation assay over a 14 day culture period of T cells expressing an FMC63-derived scFv-derived CAR following stimulation with the indicated ratio of beads coated with anti-idiotype antibody (anti-ID B-1) or control anti-CD3/anti-CD28 antibody coated beads in the presence or absence of cytokines. Shown are results for CD4+ T cells positive for the EGFRt surrogate transduction marker (EGFRt+/CD4+) or CD8+ T cells positive for EGFRt (EGFRt+/CD8+).

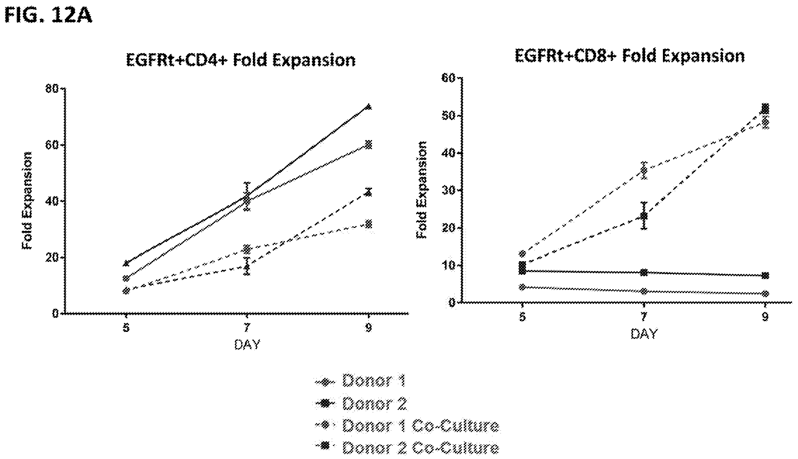

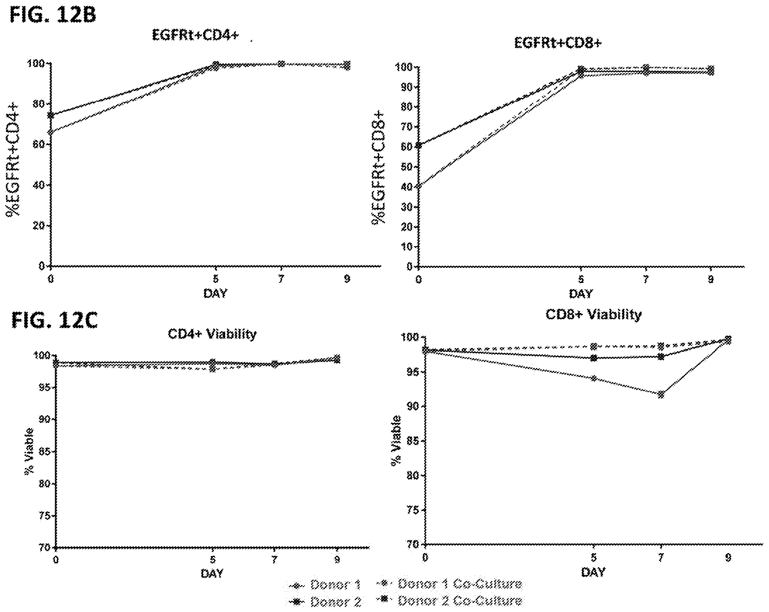

**FIG. 12A-12C** show results following stimulation of CD4+ or CD8+ T cells expressing an FMC63-derived CAR, cultured alone or as a co-culture, with FMC63-derived scFv-specific anti-idiotype antibody (anti-ID B-1) coated beads. Results are shown for two different donors. **FIG. 12A** depicts the fold-expansion of CD4+ T cells or CD8+ T cells in the cultures that were positive for the EGFRt surrogate transduction marker (EGFRt +/CD4+ or EGFRt+/CD8+). **FIG. 12B** shows the frequency of CD4+ T cells or CD8+ T cells in the cultures that were positive for EGFRt (EGFRt+/CD4+ or EGFRt +/CD8+). **FIG. 12C** shows the viability of CD4+ T cells or CD8+ T cells in the cultures.

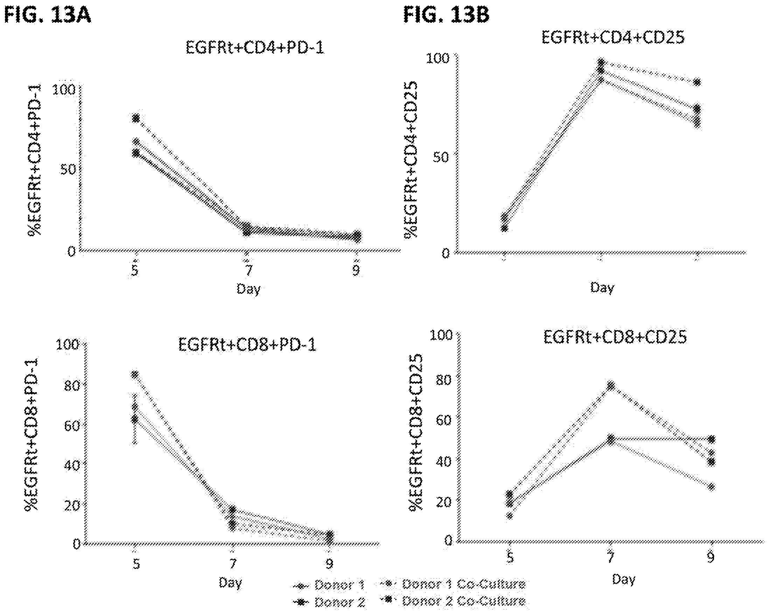

**FIG. 13A** and **13B** show flow cytometry results for T cell surface markers at days 5, 7 and 9 of culture following stimulation of CD4+ or CD8+ T cells expressing an FMC63-derived CAR, cultured alone or as a co-culture, with FMC63-derived scFv-specific anti-idiotype antibody (anti-ID B-1) coated beads. **FIG. 13A** shows surface expression of PD-1 on CD4+ T cells or CD8+ T cells in the cultures that were positive for the EGFRt surrogate transduction marker (EGFRt+/CD4+ or EGFRt+/CD8+). **FIG. 13B** shows surface expression of CD25 on CD4+ T cells or CD8+ T cells in the cultures that were positive for the anti-EGFR antibody as a surrogate for CAR expression (EGFRt+/CD4+ or EGFRt+/CD8+).

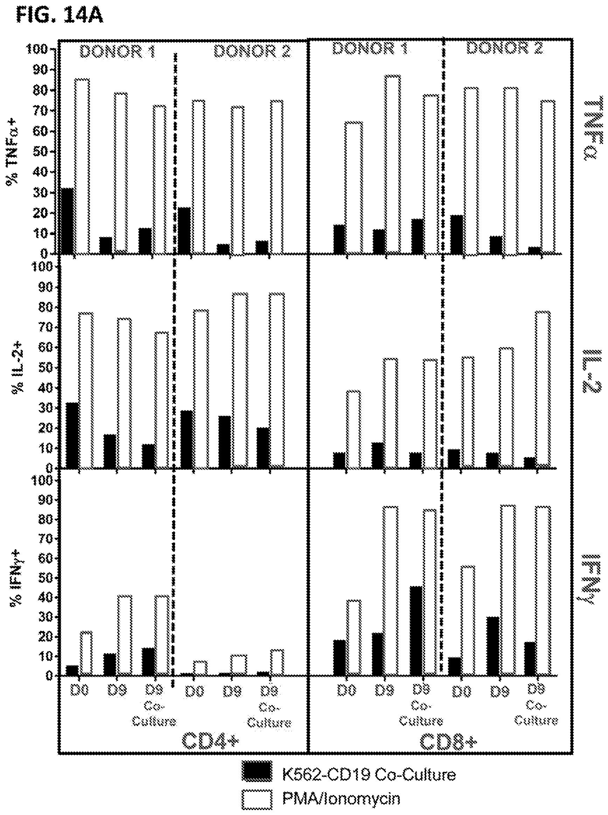

**FIG. 14A** shows intracellular cytokine levels of TNFα, IFNγ, and IL-2 as assessed by flow cytometry of CD4+ or CD8+ T cells present in a thawed composition containing T cells expressing an FMC63-derived CAR that had been expanded in culture either with CD19 expressing K562 cells or with PMA/Ionomycin. Shown are the level of the cytokines in CD4+ and CD8+ T cells, alone or as a co-culture, at thaw (d=0) or after a further culture for an additional 9 days in the presence of anti-ID B-1 conjugated beads.

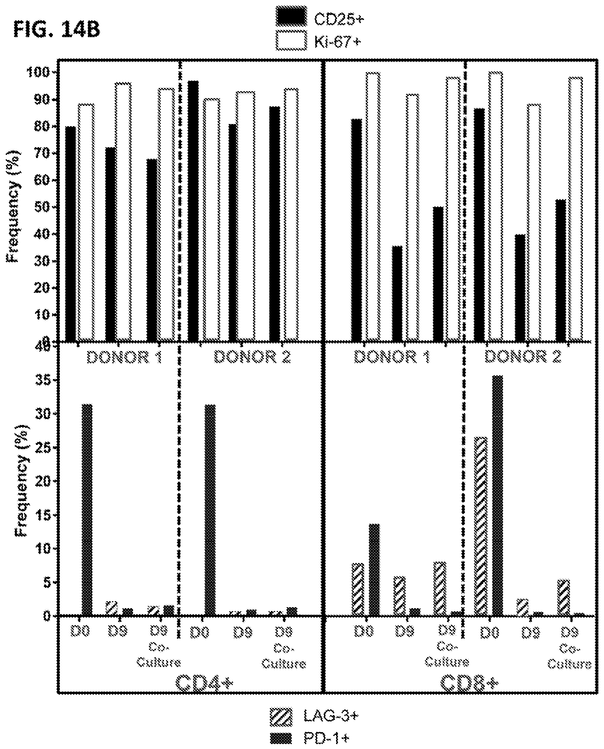

**FIG. 14B** shows the frequency of cells positive for CD25 or Ki67 as assessed by flow cytometry of CD4+ or CD8+ T cells present in a thawed composition containing T cells expressing an FMC63-derived CAR that had been expanded in culture either with CD19 expressing K562 cells or with PMA/Ionomycin. Shown are the level of the markers in CD4+ and CD8+ T cells, alone or as a co-culture, at thaw (d=0) or after a further culture for an additional 9 days in the presence of anti-ID B-1 conjugated beads.

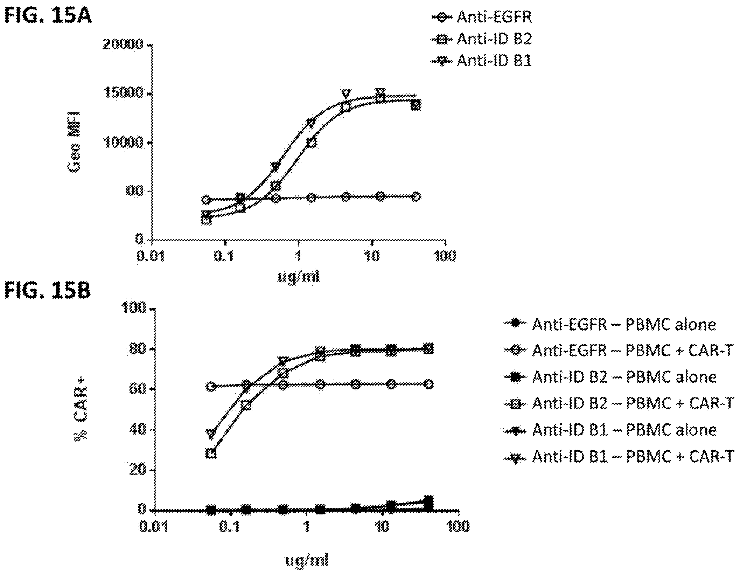

**FIG. 15A** and **15B** show a graph depicting results of staining cells with anti-EGFR antibody or FMC63-derived scFv-specific anti-idiotype antibodies (anti-ID B-1 and anti-ID B-2). **FIG. 15A** shows a graph depicting mean fluorescent intensity of cells that were stained with different concentrations of the antibodies. Cells included a mixture of PBMCs and CAR expressing cells. **FIG. 15B** shows a graph depicting the percentage of CAR expressing cells in de FMC63-derived scFv-specific anti-idiotype antibody (anti-ID B-1) detected in cells that were stained with different concentrations of antibodies. The cells included a mixture of PBMCs and CAR expressing cells and PBMCs alone.

### Detailed Description

Any references in the description to methods of treatment refer to the compounds, pharmaceutical compositions and medicaments of the present invention for use in a method for treatment of the human (or animal) body by therapy (or for diagnosis).

Disclosed herein are agents such as anti-idiotype antibodies and antigen-binding fragments (such as single chain fragments, including scFvs) that specifically recognize anti-CD19 antibody moieties (such as anti-CD19 antibody moieties present in recombinant receptors, including chimeric antigen receptors). Also disclosed are uses and methods of use thereof, and compositions and articles of manufacture including such agents, including for specifically identifying, selecting, and/or stimulating and/or activating cells expressing or including the target antibodies or fragments such as anti-CD19 CAR T cells. In some instances, the disclosed antibodies can be used for specific identification and/or selection of various anti-CD19 CARs, such as CARs bound to or expressed on a cell surface, and can also be used to specifically activate cells expressing target CARs, such as CAR T cells. In some instances, disclosed are antibodies that are specific to the anti-CD19 antibody designated SJ25C1 or FMC63, or an antibody fragment derived therefrom, including antibodies and CARs containing variable regions derived from such antibodies, and/or an antibody containing an idiotope contained therein.

In some aspects, the disclosed anti-idiotype antibodies offer advantages compared to conventional reagents for detecting, identifying, manipulating and/or affecting and/or engineering cells that express a CAR, and in particular a CAR containing an anti-CD19 antibody scFv extracellular domain or one containing the recognized idiotype. In certain available methods detection of the presence or absence or amount of CAR or CAR-expressing cells (and/or stimulation or manipulation of the CAR), in a sample, is carried out by assessing the presence or absence or amount of a surrogate molecule, such as one included in the construct encoding the CAR and thus serving as an indirect or surrogate marker for its expression. In certain available methods, detection is carried out using a generic antibody reagent and/or a reagent that is not specific for the particular CAR assessed, e.g., as compared to other CARs that may have similar or identical domains other than the antigen-binding region; for example, such antibodies may include anti-species antibodies recognizing spacer or other domains from the species from which a CAR domain was derived, and/or antibodies recognizing particular components used in spacer regions of the target and also other chimeric receptors. In certain available methods designed to detect the presence or absence of CARs, detection is carried out using an agent recognizing a CAR constant region. In certain available methods, CAR cells are stimulated through the use of general reagents, such as anti-CD3/CD28 recognizing agents. Certain methods use a recombinant ligand of the CAR (*e.g.,* CD19-Fc). Such methods in certain contexts may not be entirely satisfactory and/or have certain limitations. In some cases, CAR ligands, such as CD19, may not always be entirely effective, e.g., for use in complex flow cytometry panels. Improved methods and agents are needed, including those providing improved sensitivity and/or selectivity. Disclosed herein are instances meeting such needs.

The disclosed anti-idiotype antibodies and antigen-binding fragments in some instances overcome challenges of low binding affinity associated with target antibody ligands and non-specific binding associated with antibody reagents directed to target antibody constant regions, providing a reagent with both high affinity and specificity for its target antibody or antigen binding fragment thereof. In some instances, the disclosed antibodies exhibit greater specificity and binding affinity for their target antibodies or antigen-binding fragments, such as the anti-CD19 antibody designated SJ25C1 or FMC63, compared to CD19-Fc and other reagents currently available for detecting or identifying the CAR.

Furthermore, in certain instances, anti-idiotype antibodies and antigen-binding fragments that may be selected as agonists or antagonists of chimeric receptors comprising their target antibodies or antigen-binding fragments, allowing for selective detection, isolation, ablation and/or depletion (for example, killing via antibody-dependent cell-mediated cytotoxicity, ADCC), and/or stimulation or activation of cells with such chimeric receptors bound to or expressed on their surface. Disclosed herein are anti-idiotype antibody agonists that exhibit activity to stimulate, such as activate, a CAR containing an extracellular binding domain derived from anti-CD19 antibody designated SJ25C1 or FMC63. In some aspects, such antibodies can be used in methods of stimulating and expanding specific CAR-expressing cells, including in processes for generating and preparing the CAR-expressing cells.

Also disclosed herein are nucleic acids encoding the disclosed anti-idiotype antibodies and fragments, and cells, such as recombinant cells, expressing and for production of these anti-idiotype antibodies and fragments. Also disclosed are methods of making and using the anti-idiotype antibodies and fragments, as well as cells expressing or containing the anti-idiotype antibodies and fragments.

The section headings used herein are for organizational purposes only and are not to be construed as limiting the subject matter described.

### I. ANTI-IDIOTYPE ANTIBODIES

Disclosed in some aspects are binding molecules, such as anti-idiotype antibodies or antigen-binding fragments ("anti-IDs") that specifically recognize a target anti-CD19 antibody moiety. In some instances, the disclosed antibodies recognize a target anti-CD 19 antibody that is SJ25C1 or an antigen-binding fragment thereof or is an antibody or antigen-binding fragment derived from SJ25C1. In some instances, the disclosed antibodies recognize a target anti-CD19 antibody that is FMC63 or an antigen-binding fragment thereof is an antibody or antigen-binding fragment derived from FMC63.

SJ25C1 is a mouse monoclonal IgG1 antibody raised against Nalm-1 and -16 cells expressing CD19 of human origin (Ling, N. R., *et al.* (1987). *Leucocyte typing III.* 302). The SJ25C1 antibody comprises CDRH1, H2 and H3 set forth in SEQ ID NOS: 114-116, respectively, and CDRL1, L2 and L3 sequences set forth in SEQ ID NOS: 117-119, respectively. The SJ25C1 antibody comprises the heavy chain variable region (V_{H}) comprising the amino acid sequence of SEQ ID NO: 23 and the light chain variable region (V_{L}) comprising the amino acid sequence of SEQ ID NO: 24.

In some instances, the target antibody is SJ25C1 or an antibody-derived from SJ25C1. In some instances, the antibody derived from SJ25C1is an antibody or antigen-binding fragment that comprises the V_{H} and/or V_{L} of SJ25C1, the idiotype of SJ25C1, the paratope of SJ25C1, or one or more complementarity determining regions (CDRs) of SJ25C1. In some instances, the target antibody that is SJ25C1 or an antibody-derived from SJ25C1 is an antibody or antigen-binding fragment comprising the V_{H} of SJ25C1 set forth in SEQ ID NO:23, or a variant thereof having at least 90% sequence identity to SEQ ID NO:23 , and/or the V_{L} of SJ25C1 set forth in SEQ ID NO:24, or a variant thereof having at least 90% sequence identity to SEQ ID NO:24. In some instances, the target antibody that is SJ25C1 or an antibody-derived from SJ25C1 is an antibody or antigen-binding fragment comprising the V_{H} of SJ25C1 set forth in SEQ ID NO:23, or a variant thereof having at least 90% sequence identity to SEQ ID NO:23, and the V_{L} of SJ25C1 set forth in SEQ ID NO:24, or a variant thereof having at least 90% sequence identity to SEQ ID NO:24. In some instances, the antibody or antigen-binding fragment comprises the V_{H} and V_{L} of SJ25C1 set forth in SEQ ID NO:23 and SEQ ID NO:24, respectively. In some instances, the variant has at least 91%, 92%, 93%, 94%, 95%, 96%, 97%, 98%, 99% or more sequence identity to SEQ ID NO:23 and/or SEQ ID NO:24.

In some instances, the target antibody that is SJ25C1 or an antibody-derived from SJ25C1 is an antibody or antigen-binding fragment comprising one or more heavy chain CDRs (CDR-H) of SJ25C1 V_{H} set forth in SEQ ID NO:23, such as set forth in SEQ ID NOS: 114-116 and/or one or more light chain CDRs (CDR-Ls) of SJ25C1 V_{L} set forth in SEQ ID NO:24, such as set forth in SEQ ID NOS: 117-119. In some instances, the antibody or antigen-binding fragment comprises CDR-H3 of SJ25C1 (e.g. set forth in SEQ ID NO: 116) and/or CDR-L3 of SJ25C1 (e.g. set forth in SEQ ID NO: 119). In some instances, the antibody or antigen-binding fragment comprises CDR-H3 and CDR-L3 of SJ25C1 (e.g. set forth in SEQ ID NO:116 and 119, respectively). In some instances, the target antibody that is SJ25C1 or an antibody-derived from SJ25C1 is an antibody or antigen-binding fragment comprises one or more of CDR-H1, CDR-H2, and CDR-H3 of SJ25C1 (e.g. set forth in SEQ ID NOs: 114, 115, 116, respectively) and/or one or more of CDR-L 1, CDR-L2, and CDR-L3 of SJ25C1 (e.g. set forth in SEQ ID NOs: 117, 118, 119, respectively). In some instances, the target antibody that is SJ25C1 or an antibody-derived from SJ25C1 is an antibody or antigen-binding fragment comprises CDR-H1, CDR-H2, and CDR-H3 of SJ25C1 (e.g. set forth in SEQ ID NOs: 114, 115, 116, respectively) and/or CDR-L1, CDR-L2, and CDR-L3 of SJ25C1 (e.g. set forth in SEQ ID NOs: 117, 118, 119, respectively). In some instances, the target antibody that is SJ25C1 or an antibody-derived from SJ25C1 is an antibody or antigen-binding fragment comprises CDR-H1, CDR-H2, and CDR-H3 of SJ25C1 (e.g. set forth in SEQ ID NOs: 114, 115, 116, respectively) and CDR-L1, CDR-L2, and CDR-L3 of SJ25C1 (e.g. set forth in SEQ ID NOs: 117, 118, 119, respectively). In some instances, the antibody or antigen-binding fragment comprises an antigen-binding fragment, such as a fragment antigen-binding (Fab), a F(ab')2, a Fab', a fragment variable (Fv), or a single chain Fv (scFv). *See* for example Bejcek, B. E., et al. (1995). Cancer research. 55(11): 2346-2351.

FMC63 is a mouse monoclonal IgG1 antibody raised against JVM3 cells expressing CD19 of human origin (Nicholson et al. (1997). Molecular Immunology. 34(16-17):1157-1165). The FMC63 antibody comprises CDRH1, H2 and H3 set forth in SEQ ID NOS: 120-122, respectively, and CDRL1, L2 and L3 sequences set forth in SEQ ID NOS: 123-125, respectively. The FMC63 antibody comprises the heavy chain variable region (V_{H}) comprising the amino acid sequence of SEQ ID NO: 30 and the light chain variable region (V_{L}) comprising the amino acid sequence of SEQ ID NO: 31.

In some instances, the target antibody is FMC63 or an antibody-derived from FMC63. In some instances, the antibody derived from FMC63, is an antibody or antigen-binding fragment that comprises the V_{H} and/or V_{L} of FMC63, the idiotype of FMC63, the paratope of FMC63, or one or more complementarity determining regions (CDRs) of FMC63. In some instances, the target antibody that is FMC63 or an antibody-derived from FMC63 is an antibody or antigen-binding fragment comprising the V_{H} of FMC63 set forth in SEQ ID NO: 30, or a variant thereof having at least 90% sequence identity to SEQ ID NO:30, and/or the V_{L} of FMC63 set forth in SEQ ID NO:31, or a variant thereof having at least 90% sequence identity to SEQ ID NO:31. In some instances, the target antibody that is FMC63 or an antibody-derived from FMC63 is an antibody or antigen-binding fragment comprises the V_{H} of FMC63 set forth in SEQ ID NO:30, or a variant thereof having at least 90% sequence identity to SEQ ID NO:30, and the V_{L} of FMC63 set forth in SEQ ID NO:31, or a variant thereof having at least 90% sequence identity to SEQ ID NO:31. In some instances, the antibody or antigen-binding fragment comprises the V_{H} and V_{L} of FMC63 set forth in SEQ ID NO:30 and SEQ ID NO:31, respectively. In some instances, the variant has at least 91%, 92%, 93%, 94%, 95%, 96%, 97%, 98%, 99% or more sequence identity to SEQ ID NO:30 and/or SEQ ID NO:31.

In some instances, the target antibody that is FMC63 or an antibody-derived from FMC63 is an antibody or antigen-binding fragment comprises one or more heavy chain CDRs (CDR-H) of FMC63 V_{H} set forth in SEQ ID NO:30, such as set forth in SEQ ID NOS: 120-122 and/or one or more light chain CDRs (CDR-Ls) of FMC63 V_{L} set forth in SEQ ID NO:31, such as set forth in SEQ ID NOS: 123-125. In some instances, the antibody or antigen-binding fragment comprises CDR-H3 of FMC63 (e.g. set forth in SEQ ID NO: 122) and/or CDR-L3 of FMC63 (e.g. set forth in SEQ ID NO: 125). In some instances, the antibody or antigen-binding fragment comprises CDR-H3 (e.g. set forth in SEQ ID NO: 122) and CDR-L3 of FMC63 (e.g. set forth in SEQ ID NO: 125). In some instances, the antibody or antigen-binding fragment comprises one or more of CDR-H1, CDR-H2, and CDR-H3 of FMC63 (set forth in SEQ ID NOs: 120, 121, 122, respectively) and/or one or more of CDR-L1, CDR-L2, and CDR-L3 of FMC63 (set forth in SEQ ID NOs: 123, 124, 125, respectively). In some instances, the antibody or antigen-binding fragment comprises CDR-H1, CDR-H2, and CDR-H3 of FMC63 (set forth in SEQ ID NOs: 120, 121, 122, respectively) and/or CDR-L1, CDR-L2, and CDR-L3 of FMC63 (set forth in SEQ ID NOs: 123, 124, 125, respectively). In some instances, the antibody or antigen-binding fragment comprises CDR-H1, CDR-H2, and CDR-H3 of FMC63 (set forth in SEQ ID NOs: 120, 121, 122, respectively) and CDR-L1, CDR-L2, and CDR-L3 of FMC63 (set forth in SEQ ID NOs: 123, 124, 125, respectively). In some instances, the antibody or antigen-binding fragment comprises an antigen-binding fragment, such as a fragment antigen-binding (Fab), a F(ab')2, a Fab', a fragment variable (Fv), or a single chain Fv (scFv).

In some instances, the disclosed anti-idiotype antibodies include antibodies that specifically bind to a variable domain (Fv), such as a single chain Fv (scFv), derived from SJ25C1 or FMC63. In some instances, the anti-idiotype antibodies specifically bind to a particular epitope or region of an Fv, generally an epitope or region comprising one or more complementarity determining regions. In some instances, the anti-idiotype antibodies specifically bind to an epitope or region overlapping an Fv paratope.

In some instances, the disclosed anti-idiotype antibodies include those that specifically bind to an anti-CD 19 moiety derived from SJ25C1 or FMC63 that is contained as part of the extracellular domain of a target chimeric antigen receptor (CAR). In some instances, the target CAR contains an antigen-binding portion that contains the SJ25C1 or FMC63 antibody molecule or antigen-binding fragment or portion of the SJ25C1 or FMC63 antibody. In some instances, the target CAR includes an antigen-binding domain that is an scFv derived from the VH and VL chains of the antibody SJ25C1 or FMC63. In some instances, there is disclosed an anti-idiotype antibody that specifically binds to an anti-CD 19 CAR that contains an scFv derived from antibody SJ25C1 or FMC63. Exemplary features of CARs are described further below.

The term "antibody" herein is used in the broadest sense and includes polyclonal and monoclonal antibodies, including intact antibodies and functional (antigen-binding) antibody fragments, including fragment antigen binding (Fab) fragments, F(ab')₂ fragments, Fab' fragments, Fv fragments, recombinant IgG (rIgG) fragments, single chain antibody fragments, including single chain variable fragments (scFv), and single domain antibodies (e.g., sdAb, sdFv, nanobody) fragments. The term encompasses genetically engineered and/or otherwise modified forms of immunoglobulins, such as intrabodies, peptibodies, chimeric antibodies, fully human antibodies, humanized antibodies, and heteroconjugate antibodies, multispecific, e.g., bispecific, antibodies, diabodies, triabodies, and tetrabodies, tandem di-scFv, tandem tri-scFv. Unless otherwise stated, the term "antibody" should be understood to encompass functional antibody fragments thereof. The term also encompasses intact or full-length antibodies, including antibodies of any class or sub-class, including IgG and sub-classes thereof, IgM, IgE, IgA, and IgD.

The term "anti-idiotype antibody" refers to an antibody, including antigen-binding fragments thereof, that specifically recognizes, is specifically targeted to, and/or specifically binds to an idiotope of an antibody, such as an antigen-binding fragment. The idiotopes of an antibody may include, but are not necessarily limited to, residues within one or more of complementarity determining region(s) (CDRs) of the antibody, variable regions of the antibody, and/or partial portions or portions of such variable regions and/or of such CDRs, and/or any combination of the foregoing. The CDR may be one or more selected from the group consisting of CDR-H1, CDR-H2, CDR-H3, CDR-L1, CDR-L2, and CDR-L3. The variable regions of the antibody may be heavy chain variable regions, light chain variable regions, or a combination of the heavy chain variable regions and the light chain variable regions. The partial fragments or portions of the heavy chain variable regions and/or the light chain variable regions of the antibody may be fragments including 2 or more, 5 or more, or 10 or more contiguous amino acids, for example, from about 2 to about 100, from about 5 to about 100, from about 10 to about 100, from about 2 to about 50, from about 5 to about 50, or from about 10 to about 50 contiguous amino acids within the heavy chain variable regions or the light chain variable regions of the antibody; the idiotope may include multiple non-contiguous stretches of amino acids. The partial fragments of the heavy chain variable regions and the light chain variable regions of the antibody may be fragments including 2 or more, 5 or more, or 10 or more contiguous amino acids, for example, from about 2 to about 100, from about 5 to about 100, from about 10 to about 100, from about 2 to about 50, from about 5 to about 50, or from about 10 to about 50 contiguous amino acids within the variable regions, and in some instances contain one or more CDRs or CDR fragments. The CDR fragments may be consecutive or nonconsecutive 2 or more, or 5 or more amino acids within the CDR. Therefore, the idiotopes of the antibody may be from about 2 to about 100, from about 5 to about 100, from about 10 to about 100, from about 2 to about 50, from about 5 to about 50, or from about 10 to about 50 contiguous amino acids containing one or more CDR or one or more CDR fragments within the heavy chain variable regions or the light chain variable regions of the antibody. In another instance, the idiotopes may be a single amino acid which is located at the variable regions of the antibody, for example, CDR sites.

In some instances, the idiotope is any single antigenic determinant or epitope within the variable portion of an antibody. In some cases it can overlap the actual antigen-binding site of the antibody, and in some cases it may comprise variable region sequences outside of the antigen-binding site of the antibody. The set of individual idiotopes of an antibody is in some instances referred to as the "idiotype" of such antibody.

The terms "complementarity determining region," and "CDR," synonymous with "hypervariable region" or "HVR," are known in the art to refer to non-contiguous sequences of amino acids within antibody variable regions, which confer antigen specificity and/or binding affinity. In general, there are three CDRs in each heavy chain variable region (CDR-H1, CDR-H2, CDR-H3) and three CDRs in each light chain variable region (CDR-L1, CDR-L2, CDR-L3). "Framework regions" and "FR" are known in the art to refer to the non-CDR portions of the variable regions of the heavy and light chains. In general, there are four FRs in each full-length heavy chain variable region (FR-H1, FR-H2, FR-H3, and FR-H4), and four FRs in each full-length light chain variable region (FR-L1, FR-L2, FR-L3, and FR-L4).

The precise amino acid sequence boundaries of a given CDR or FR can be readily determined using any of a number of well-known schemes, including those described by Kabat et al. (1991), "Sequences of Proteins of Immunological Interest," 5th Ed. Public Health Service, National Institutes of Health, Bethesda, MD ("Kabat" numbering scheme), Al-Lazikani et al., (1997) JMB 273,927-948 ("Chothia" numbering scheme), MacCallum et al., J. Mol. Biol. 262: 732-745 (1996), "Antibody-antigen interactions: Contact analysis and binding site topography," J. Mol. Biol. 262, 732-745." ("Contact" numbering scheme), Lefranc MP et al., "IMGT unique numbering for immunoglobulin and T cell receptor variable domains and Ig superfamily V-like domains," Dev Comp Immunol, 2003 Jan;27(1): 55-77 ("IMGT" numbering scheme), and Honegger A and Plückthun A, "Yet another numbering scheme for immunoglobulin variable domains: an automatic modeling and analysis tool," J Mol Biol, 2001 Jun 8;309(3): 657-70, ("Aho" numbering scheme).

The boundaries of a given CDR or FR may vary depending on the scheme used for identification. For example, the Kabat scheme is based structural alignments, while the Chothia scheme is based on structural information. Numbering for both the Kabat and Chothia schemes is based upon the most common antibody region sequence lengths, with insertions accommodated by insertion letters, for example, "30a," and deletions appearing in some antibodies. The two schemes place certain insertions and deletions ("indels") at different positions, resulting in differential numbering. The Contact scheme is based on analysis of complex crystal structures and is similar in many respects to the Chothia numbering scheme.

Table 1, below, lists exemplary position boundaries of CDR-L1, CDR-L2, CDR-L3 and CDR-H1, CDR-H2, CDR-H3 as identified by Kabat, Chothia, and Contact schemes, respectively. For CDR-H1, residue numbering is listed using both the Kabat and Chothia numbering schemes. FRs are located between CDRs, for example, with FR-L1 located between CDR-L1 and CDR-L2, and so forth. It is noted that because the shown Kabat numbering scheme places insertions at H35A and H35B, the end of the Chothia CDR-H1 loop when numbered using the shown Kabat numbering convention varies between H32 and H34, depending on the length of the loop.

**Table 1**

| **CDR** | **Kabat** | **Chothia** | **Contact** |
|---|---|---|---|
| CDR-L1 | L24--L34 | L24--L34 | L30--L36 |
| CDR-L2 | L50--L56 | L50--L56 | L46--L55 |
| CDR-L3 | L89--L97 | L89--L97 | L89--L96 |
| CDR-H1 (Kabat Numbering¹) | H31--H35B | H26--H32..34 | H30--H35B |
| CDR-H1 (Chothia Numbering²) | H31--H35 | H26--H32 | H3 0--H35 |
| CDR-H2 | H50--H65 | H52--H56 | H47--H58 |
| CDR-H3 | H95--H102 | H95--H102 | H93--H101 |

| | | | |
|---|---|---|---|
| 1 - Kabat et al. (1991), "Sequences of Proteins of Immunological Interest," 5th Ed. Public Health Service, National Institutes of Health, Bethesda, MD 2 - Al-Lazikani et al., (1997) JMB 273,927-948 | | | |

Thus, unless otherwise specified, a "CDR" or "complementary determining region," or individual specified CDRs (e.g., "CDR-H1, CDR-H2), of a given antibody or region thereof, such as a variable region thereof, should be understood to encompass a (or the specific) complementary determining region as defined by any of the aforementioned schemes. For example, where it is stated that a particular CDR (e.g., a CDR-H3) contains the amino acid sequence of a corresponding CDR in a given V_{H} or V_{L} amino acid sequence, it is understood that such a CDR has a sequence of the corresponding CDR (e.g., CDR-H3) within the variable region, as defined by any of the aforementioned schemes. In some instances, specified CDR sequences are specified.

Likewise, unless otherwise specified, a FR or individual specified FR(s) (e.g., FR-H1, FR-H2), of a given antibody or region thereof, such as a variable region thereof, should be understood to encompass a (or the specific) framework region as defined by any of the known schemes. In some instances, the scheme for identification of a particular CDR, FR, or FRs or CDRs is specified, such as the CDR as defined by the Kabat, Chothia, or Contact method. In other cases, the particular amino acid sequence of a CDR or FR is given.

The term "variable region" or "variable domain" refers to the domain of an antibody heavy or light chain that is involved in binding the antibody to antigen. The variable domains of the heavy chain and light chain (V_{H} and V_{L}, respectively) of a native antibody generally have similar structures, with each domain comprising four conserved framework regions (FRs) and three CDRs. (See, e.g., Kindt et al. Kuby Immunology, 6th ed., W.H. Freeman and Co., page 91 (2007). A single V_{H} or V_{L} domain may be sufficient to confer antigen-binding specificity. Furthermore, antibodies that bind a particular antigen may be isolated using a V_{H} or V_{L} domain from an antibody that binds the antigen to screen a library of complementary V_{L} or V_{H} domains, respectively. See, e.g., Portolano et al., J. Immunol. 150: 880-887 (1993); Clarkson et al., Nature 352: 624-628 (1991).

Among the disclosed antibodies are antibody fragments. An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies; linear antibodies; single-chain antibody molecules (e.g. scFv); and multispecific antibodies formed from antibody fragments. In particular instances, the antibodies are single-chain antibody fragments comprising a variable heavy chain region and/or a variable light chain region, such as scFvs.

Single-domain antibodies are antibody fragments comprising all or a portion of the heavy chain variable domain or all or a portion of the light chain variable domain of an antibody. In certain instances, a single-domain antibody is a human single-domain antibody.

Antibody fragments can be made by various techniques, including but not limited to proteolytic digestion of an intact antibody as well as production by recombinant host cells. In some instances, the antibodies are recombinantly produced fragments, such as fragments comprising arrangements that do not occur naturally, such as those with two or more antibody regions or chains joined by synthetic linkers, e.g., peptide linkers, and/or that are may not be produced by enzyme digestion of a naturally-occurring intact antibody. In some aspects, the antibody fragments are scFvs.

A "humanized" antibody is an antibody in which all or substantially all CDR amino acid residues are derived from non-human CDRs and all or substantially all framework regions (FRs) amino acid residues are derived from human FRs. In some instances, the humanized forms of a non-human antibody, e.g., a murine antibody, are chimeric antibodies that contain minimal sequences derived from non-human immunoglobulin. In certain instances, the humanized antibodies are antibodies from non-human species having one or more complementarily determining regions (CDRs) from the non-human species and a framework region (FR) from a human immunoglobulin molecule. In some instances, a humanized antibody optionally may include at least a portion of an antibody constant region derived from a human antibody. A "humanized form" of a non-human antibody, refers to a variant of the non-human antibody that has undergone humanization, typically to reduce immunogenicity to humans, while retaining the specificity and affinity of the parental non-human antibody. In some instances, some FR residues in a humanized antibody are substituted with corresponding residues from a non-human antibody (e.g., the antibody from which the CDR residues are derived), e.g., to restore or improve antibody specificity or affinity. (See, e.g., Queen, U.S. Pat. No. 5,585,089 and Winter, U.S. Pat. No. 5,225,539.) Such chimeric and humanized monoclonal antibodies can be produced by recombinant DNA techniques known in the art.

In certain instances, a humanized antibody is a human immunoglobulin (recipient antibody) in which residues from a heavy chain variable region of the recipient are replaced by residues from a heavy chain variable region of a non-human species (donor antibody) such as mouse, rat, rabbit, or non-human primate having the desired specificity, affinity, and/or capacity. In some instances, FR residues of the human immunoglobulin are replaced by corresponding non-human residues. Furthermore, humanized antibodies may comprise residues that are not found in the recipient antibody or in the donor antibody. In some instances, a nucleic acid sequences encoding human variable heavy chains and variable light chains are altered to replace one or more CDR sequences of the human (acceptor) sequence by sequence encoding the respective CDR in the nonhuman antibody sequence(donor sequence). In some instances, the human acceptor sequence may comprise FR derived from different genes. In particular instances, a humanized antibody will contain substantially all of at least one, and typically two, variable domains, in which all or substantially all of the hypervariable loops correspond to those of a non-human immunoglobulin, and all or substantially all of the FRs are those of a human immunoglobulin sequence. In some instances, the humanized antibody optionally will also comprise at least a portion of an immunoglobulin constant region (Fc), typically that of a human immunoglobulin. For further details, see, e.g., Jones et al., Nature 321:522-525 (1986); Riechmann et al., Nature 332:323-329 (1988); and Presta, Curr. Op. Struct. Biol. 2:593-596 (1992). See also, e.g., Vaswani and Hamilton, Ann. Allergy, Asthma & Immunol. 1:105-115 (1998); Harris, Biochem. Soc. Transactions 23:1035-1038 (1995); Hurle and Gross, Curr. Op. Biotech. 5:428-433 (1994); and U.S. Pat. Nos. 6,982,321 and 7,087,409. In some instances, disclosed herein are humanized anti-idiotype antibodies.

In particular instances, an antibody, e.g., an anti-idiotype antibody, is humanized. In certain instances, the antibody is humanized by any suitable known means. For example, in some instances, a humanized antibody can have one or more amino acid residues introduced into it from a source which is non-human. These non-human amino acid residues are often referred to as "import" residues, which are typically taken from an "import" variable domain. In particular instances, humanization can be essentially performed by following the method of Winter and co-workers (Jones et al. (1986) Nature 321:522-525; Riechmann et al. (1988) Nature 332:323-327; Verhoeyen et al. (1988) Science 239:1534-1536), such as by substituting hypervariable region sequences for the corresponding sequences of a human antibody. Accordingly, such "humanized" antibodies are chimeric antibodies (U.S. Pat. No. 4,816,567) wherein substantially less than an intact human variable domain has been substituted by the corresponding sequence from a non-human species. In certain instances, the humanized antibody is a human antibody in which some hypervariable region residues and possibly some FR residues are substituted by residues from analogous sites in rodent antibodies.

Sequences encoding full length antibodies can be subsequently obtained by joining the rendered variable heavy and variable light chain sequences to human constant heavy chain and constant light chain regions. Suitable human constant light chain sequences include kappa and lambda constant light chain sequences. Suitable human constant heavy chain sequences include IgG1, IgG2 and sequences encoding IgG1 mutants which have rendered immune-stimulating properties. Such mutants may have a reduced ability to activate complement and/or antibody dependent cellular cytotoxicity and are described in U.S. Pat. No. 5,624,821; WO 99/58572, U.S. Pat. No. 6,737,056. A suitable constant heavy chain also includes an IgG 1 comprising the substitutions E233P, L234V, L235A, A327G, A330S, P331S and a deletion of residue 236. In another instance, the full length antibody comprises an IgA, IgD, IgE, IgM, IgY or IgW sequence.

Suitable human donor sequences can be determined by sequence comparison of the peptide sequences encoded by the mouse donor sequences to a group of human sequences, preferably to sequences encoded by human germ line immunoglobulin genes or mature antibody genes. A human sequence with a high sequence homology, preferably with the highest homology determined may serve as the acceptor sequence to for the humanization process.

In addition to the exchange of human CDRs for mouse CDRs, further manipulations in the human donor sequence may be carried out to obtain a sequence encoding a humanized antibody with optimized properties (such as affinity of the antigen).

Furthermore the altered human acceptor antibody variable domain sequences may also be rendered to encode one or more amino acids (according to the Kabat numbering system) of position 4, 35, 38, 43, 44, 46, 58, 62, 64, 65, 66, 67, 68, 69, 73, 85, 98 of the light variable region and 2, 4, 36, 39, 43, 45, 69, 70, 74, 75, 76, 78, 92 of the heavy variable region corresponding to the non-human donor sequence (Carter and Presta, U.S. Pat. No. 6,407,213)

In particular instances, it is generally desirable that antibodies be humanized with retention of high affinity for the antigen and other favorable biological properties. To achieve this goal, in some instances, the humanized antibodies are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, i.e., the analysis of residues that influence the ability of the candidate immunoglobulin to bind its antigen. In this way, FR residues can be selected and combined from the recipient and imported sequences so that the desired antibody characteristic, such as increased affinity for the target antigen(s), is achieved. In general, the hypervariable region residues are directly and most substantially involved in influencing antigen binding.

In particular instances, choice of human variable domains, both light and heavy, to be used in making the humanized antibodies can be important to reduce antigenicity. According to the so-called "best-fit" method, the sequence of the variable domain of a rodent antibody is screened against the entire library of known human variable-domain sequences. The human sequence which is closest to that of the rodent is then accepted as the human framework for the humanized antibody. See, e.g., Sims et al. (1993) J. Immunol. 151:2296; Chothia et al. (1987) J. Mol. Biol. 196:901. Another method uses a particular framework derived from the consensus sequence of all human antibodies of a particular subgroup of light or heavy chains. The same framework may be used for several different humanized antibodies. See, e.g., Carter et al. (1992) Proc. Natl. Acad. Sci. USA, 89:4285; Presta et al. (1993) J. Immunol., 151:2623.

Among the disclosed antibodies are human antibodies. A "human antibody" is an antibody with an amino acid sequence corresponding to that of an antibody produced by a human or a human cell, or non-human source that utilizes human antibody repertoires or other human antibody-encoding sequences, including human antibody libraries. The term excludes humanized forms of non-human antibodies comprising non-human antigen-binding regions, such as those in which all or substantially all CDRs are non-human.

Human antibodies may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic animals, the endogenous immunoglobulin loci have generally been inactivated. Human antibodies also may be derived from human antibody libraries, including phage display and cell-free libraries, containing antibody-encoding sequences derived from a human repertoire.

Among the disclosed antibodies are monoclonal antibodies, including monoclonal antibody fragments. The term "monoclonal antibody" as used herein refers to an antibody obtained from or within a population of substantially homogeneous antibodies, i.e., the individual antibodies comprising the population are identical, except for possible variants containing naturally occurring mutations or arising during production of a monoclonal antibody preparation, such variants generally being present in minor amounts. In contrast to polyclonal antibody preparations, which typically include different antibodies directed against different epitopes, each monoclonal antibody of a monoclonal antibody preparation is directed against a single epitope on an antigen. The term is not to be construed as requiring production of the antibody by any particular method. A monoclonal antibody may be made by a variety of techniques, including but not limited to generation from a hybridoma, recombinant DNA methods, phage-display and other antibody display methods.

### A. SJ25C1-derived Antibodies

In some instances, disclosed are anti-idiotype antibodies specific to a target anti-CD19 antibody that is or is derived from antibody SJ25C1 or an antigen-binding fragment thereof. In some instances, the disclosed antibodies or antigen-binding fragments are specific to an SJ25C1-derived scFv.

In some instances, disclosed are anti-idiotype antibodies or antigen-binding fragments thereof that include a heavy chain variable (V_{H}) region comprising at least 90% sequence identity to the V_{H} region amino acid sequence set forth in SEQ ID NO: 1, such as at least 91%, 92%, 93%, 94%, 95%, 96%, 97%, 98%, or 99% sequence identity thereto.

In some instances, disclosed are anti-idiotype antibodies or antigen-binding fragments thereof that include a heavy chain variable (VH) region containing a heavy chain complementarity determining region 3 (CDR-H3) having the amino acid sequence set forth in SEQ ID NO: 11 or 84 and/or a CDR-H3 contained within the heavy chain variable (V_{H}) sequence set forth in SEQ ID NO: 1.

In some of any such instances, the V_{H} region includes a heavy chain complementarity determining region 1 (CDR-H1) comprising the amino acid sequence set forth in SEQ ID NO: 9, 78, 79, or 80, and/or a CDR-H1 contained within the V_{H} sequence set forth in SEQ ID NO: 1; and/or a heavy chain complementarity determining region 2 (CDR-H2) comprising the amino acid sequence set forth in SEQ ID NO: 10, 81, 82, or 83 and/or a CDR-H2 contained within the V_{H} sequence set forth in SEQ ID NO: 1.

Disclosed are anti-idiotype antibodies or antigen-binding fragments thereof that include a heavy chain variable (VH) region comprising a heavy chain complementarity determining region 1 (CDR-H1), CDR-H2, and CDR-H3, wherein the CDR-H1 comprises the amino acid sequence set forth in SEQ ID NO: 9, 78, 79, or 80; the CDR-H2 comprises the amino acid sequence set forth in SEQ ID NO: 10, 81, 82, or 83; and/or the CDR-H3 comprises the amino acid sequence set forth in SEQ ID NO: 11 or 84. In some instances, disclosed are antibodies or antigen-binding fragments thereof that include a CDR-H1 having the amino acid sequence set forth in SEQ ID NO: 9, 78, 79, or 80; a CDR-H2 having the amino acid sequence set forth in SEQ ID NO: 10, 81, 82, or 83; and a CDR-H3 having the amino acid sequence set forth in SEQ ID NO: 11 or 84.

Disclosed are anti-idiotype antibodies or antigen-binding fragments thereof that includes a heavy chain complementarity determining region 1 (CDR-H1), a CDR-H2, and a CDR-H3, respectively, comprising the amino acid sequences of a CDR-H1, a CDR-H2, and a CDR-H3 contained within the V_{H} region amino acid sequence set forth in SEQ ID NO: 1.

In some of any such instances, the V_{H} region contains a framework region 1 (FR1), a FR2, a FR3, and/or a FR4 sequence having at least 90% sequence identity, respectively, to a FR1, FR2, FR3, and/or FR4 of the amino acid sequence set forth in SEQ ID NO: 1. In some instances, the V_{H} region contains a framework region 1 (FR1), a FR2, a FR3, and/or a FR4 sequence having at least 91%, 92%, 93%, 94%, 95%, 96%, 97%, 98%, 98%, 99% sequence identity, respectively, to a FR1, FR2, FR3, and/or FR4 of the amino acid sequence set forth in SEQ ID NO: 1. In some instances, the V_{H} region contains a framework region 1 (FR1), a FR2, a FR3, and a FR4 sequence having at least 90%, 91%, 92%, 93%, 94%, 95%, 96%, 97%, 98%, 98%, 99% sequence identity, respectively, to a FR1, FR2, FR3, and FR4 of the amino acid sequence set forth in SEQ ID NO: 1.

In some of any of such instances, the V_{H} region has the sequence of amino acids set forth in SEQ ID NO: 1.

In some of any such instances, the anti-idiotype antibody or antigen-binding fragment is a heavy chain only, a VH-only, and/or does not include a VL or antigen-binding portion thereof and/or the antigen-binding site of the anti-idiotype antibody or fragment includes residues from the heavy chain only and/or does not include residues from a light chain.

In some of any such instances, the anti-idiotype antibody or fragment does not contain a light chain variable (V_{L}) region, does not contain a CDR-L1, CDR-L2, and/or CDR-L3, and/or is a single-domain antibody (sdAb) containing only the V_{H} region. In some instances, the antibody or fragment is a sdAb that only contains a VH region from any as described.

In some instances of any of the anti-idiotype antibodies or fragments containing any of the above VH region sequences, the anti-idiotype antibody or fragment further comprises a light chain variable (V_{L}) region. In some such instances, the V_{L} region has at least 90% sequence identity to the V_{L} region amino acid sequence set forth in SEQ ID NO: 5, such as at least 91%, 92%, 93%, 94%, 95%, 96%, 97%, 98%, or 99% sequence identity to the V_{L} region amino acid sequence set forth in SEQ ID NO: 5.

In some of any such instances, the V_{L} region comprises a light chain complementarity determining region 3 (CDR-L3) comprising the amino acid sequence set forth in SEQ ID NO: 14 or 87. In some of any such instances, the V_{L} region comprises a light chain complementarity determining region 3 (CDR-L3) having the amino acid sequence set forth in SEQ ID NO: 14 or 87.

In some of any such instances, the V_{L} region comprises a light chain complementarity determining region 1 (CDR-L1) comprising the amino acid sequence set forth in SEQ ID NO: 12 or 85, and/or a CDR-L1 contained within the V_{L} sequence set forth in SEQ ID NO: 5; and/or a light chain complementarity determining region 2 (CDR-L2) comprising the amino acid sequence set forth in SEQ ID NO: 13 or 86, and/or a CDR-L2 contained within the V_{L} sequence set forth in SEQ ID NO: 5. In some of any such instances, the V_{L} region comprises a light chain complementarity determining region 1 (CDR-L1) having the amino acid sequence set forth in SEQ ID NO: 12 or 85, and/or a CDR-L1 contained within the V_{L} sequence set forth in SEQ ID NO: 5; and/or a light chain complementarity determining region 2 (CDR-L2) having the amino acid sequence set forth in SEQ ID NO: 13 or 86, and/or a CDR-L2 contained within the V_{L} sequence set forth in SEQ ID NO: 5.

In some of any such instances, the V_{L} region comprises a CDR-L1 containing the amino acid sequence set forth in SEQ ID NO: 12 or 85; a CDR-L2 containing the amino acid sequence set forth in SEQ ID NO: 13 or 86; and a CDR-L3 containing the amino acid sequence set forth in SEQ ID NO: 14 or 57.

In some of any such instances, the V_{L} region comprises the CDR-L1, CDR-L2, and CDR-L3, respectively, comprising the amino acid sequences of a CDR-L1, a CDR-L2, and a CDR-L3 contained within the V_{L} region amino acid sequence set forth in SEQ ID NO: 5.

In some of any such instances, the V_{L} region comprises a framework region 1 (FR1), a FR2, a FR3, and/or a FR4 having at least 90% sequence identity, respectively, to the FR1, FR2, FR3, and/or FR4 of the amino acid sequence set forth in SEQ ID NO: 5. In some instances, the V_{L} region comprises a framework region 1 (FR1), a FR2, a FR3, and/or a FR4 sequence having at least 91%, 92%, 93%, 94%, 95%, 96%, 97%, 98%, 98%, 99% sequence identity, respectively, to a FR1, FR2, FR3, and/or FR4 of the amino acid sequence set forth in SEQ ID NO: 5. In some instances, the V_{L} region comprises a framework region 1 (FR1), a FR2, a FR3, and a FR4 sequence having at least 90%, 91%, 92%, 93%, 94%, 95%, 96%, 97%, 98%, 98%, 99% sequence identity, respectively, to a FR1, FR2, FR3, and FR4 of the amino acid sequence set forth in SEQ ID NO: 5.

In some of any such instances, the V_{L} region has the amino acid sequence set forth in SEQ ID NO: 5.

Disclosed are anti-idiotype antibodies or antigen-binding fragments thereof that comprise the amino acid sequences of CDR-H1, CDR-H2, and CDR-H3 sequences contained within the V_{H} region amino acid sequence set forth in SEQ ID NO: 1; and/or comprise the amino acid sequences of CDR-L1, CDR-L2, and CDR-L3 sequences contained within the light chain variable (VL) region amino acid sequence set forth in SEQ ID NO: 5.

Disclosed are anti-idiotype antibodies or antigen-binding fragments thereof that include the V_{H} and V_{L} regions having amino acid sequences having at least 90 %, 91%, 92%, 93%, 94%, 95%, 96%, 97%, 98% or 99% identity to SEQ ID NOs: 1 and 5, respectively.

In some instances, disclosed are anti-idiotype antibodies or antigen-binding fragments thereof that include the V_{H} and V_{L} regions having amino acid sequences set forth in SEQ ID NOs: 1 and 5, respectively.

In some of any such instances, the V_{H} and V_{L} regions include the amino acid sequences of SEQ ID NOs: 1 and 5, respectively.

In some instances, the anti-idiotype antibody specific to antibody SJ25C1 or an antigen-binding fragment thereof is a single-chain antibody fragment, such as an scFv or diabody. In some instances, the single-chain antibody includes one or more linkers joining two antibody domains or regions, such as a variable heavy chain (V_{H}) region and a variable light chain (V_{L}). The linker typically is a peptide linker, e.g., a flexible and/or soluble peptide linker. Among the linkers are those rich in glycine and serine and/or in some cases threonine. In some instances, the linkers further include charged residues such as lysine and/or glutamate, which can improve solubility. In some instances, the linkers further include one or more proline.

In some instances, the anti-idiotype antibody is an intact antibody or full-length antibody. In some instances, the anti-ID may contain at least a portion of an immunoglobulin constant region, such as one or more constant region domains. In some instances, the constant regions include a light chain constant region (CL) and/or a heavy chain constant region 1 (CH1). In some instances, the anti-ID includes a CH2 and/or CH3 domain, such as an Fc region. In some instances, the Fc region is an Fc region of a human IgG, such as IgG1 or IgG4. In some instances, the anti-idiotype antibody contains the CH domain set forth in SEQ ID NO:2 or a portion thereof or a sequence of amino acids that exhibits at least 85%, 86%, 87%, 88%, 89%, 90%, 91%, 92%, 93%, 94%, 95%, 96%, 97%, 98%, or 99% sequence identity to SEQ ID NO:2 or a portion thereof. In some instances, the anti-idiotype antibody contains the CL domain set forth in SEQ ID NO:6 or a portion thereof or a sequence of amino acids that exhibits at least 85%, 86%, 87%, 88%, 89%, 90%, 91%, 92%, 93%, 94%, 95%, 96%, 97%, 98%, or 99% sequence identity to SEQ ID NO:6 or a portion thereof.

In some instances, the anti-idiotype antibody specific for SJ25C1 comprises the heavy chain sequence set forth in SEQ ID NO:3 or a sequence that exhibits at least 85%, 86%, 87%, 88%, 89%, 90%, 91%, 92%, 93%, 94%, 95%, 96%, 97%, 98%, or 99% sequence identity to SEQ ID NO:3 and/or comprises the light chain sequence set forth in SEQ ID NO:7 or a sequence that exhibits at least 85%, 86%, 87%, 88%, 89%, 90%, 91%, 92%, 93%, 94%, 95%, 96%, 97%, 98%, or 99% sequence identity to SEQ ID NO:7. In some instances, the anti-idiotype antibody specific for SJ25C1 comprises the heavy chain sequence set forth in SEQ ID NO:3 and/or the light chain sequence set forth in SEQ ID NO:7. In some instances, the heavy chain and/or light chain of the anti-idiotype antibody further comprises a signal peptide. In some cases, the signal peptide has the sequence set forth in SEQ ID NO:4 or SEQ ID NO: 8.

In some instances, the anti-idiotype antibody is an antigen-binding fragment. In some instances, the antigen-binding fragment is selected from the group consisting of fragment antigen binding (Fab) fragments, F(ab')₂ fragments, Fab' fragments, Fv fragments, a single chain variable fragment (scFv) or a single domain antibody.

Accordingly, disclosed are single-chain antibody fragments, such as scFvs and diabodies, particularly human single-chain fragments, typically comprising linker(s) joining two anti-idiotype antibody domains or regions, such V_{H} and V_{L} domains. The linker typically is a peptide linker, e.g., a flexible and/or soluble peptide linker, such as one rich in glycine and serine.

In some aspects, the linkers rich in glycine and serine (and/or threonine) include at least 80, 85, 90, 91, 92, 93, 94, 95, 96, 97, 98, or 99% such amino acid(s). In some instances, they include at least at or about 50%, 55%, 60%, 70%, or 75%, glycine, serine, and/or threonine. In some instances, the linker is comprised substantially entirely of glycine, serine, and/or threonine. The linkers generally are between about 5 and about 50 amino acids in length, typically between at or about 10 and at or about 30, e.g., 10, 11, 12, 13, 14, 15, 16, 17, 18, 19, 20, 21, 22, 23, 24, 25, 26, 27, 28, 29, or 30, and in some examples between 10 and 25 amino acids in length. Exemplary linkers include linkers having various numbers of repeats of the sequence GGGS (3GS; SEQ ID NO: 29) or GGGGS (4GS; SEQ ID NO: 26), such as between 2, 3, 4, and 5 repeats of such a sequence. Exemplary linkers include those having or consisting of a sequence set forth in SEQ ID NO: 25 (GGGGSGGGGSGGGGS). Exemplary linkers further include those having or consisting of the sequence set forth in SEQ ID NO: 33 (GSTSGSGKPGSGEGSTKG).

In some instances, the anti-idiotype antibodies include isolated antibodies. In some instances, the anti-ID is humanized, recombinant, and/or monoclonal. In some instances, the anti-ID is human.

In some instances, the anti-idiotype antibody specific for SJ25C1 is humanized. In particular instances, all or substantially all CDR amino acid residues of the humanized anti-idiotype antibody specific for SJ25C1 are derived from anti SJ25C1non-human CDRs. In some instances, the humanized anti-idiotype antibody specific for SJ25C1 includes at least a portion of an antibody constant region derived from a human antibody.

In certain instances, the humanized anti-idiotype antibody specific for SJ25C1 includes a human immunoglobulin (recipient antibody) in which residues from the heavy chain variable region of the recipient are replaced by residues from a heavy chain variable region of the nonhuman anti-idiotype antibody specific for SJ25C1. In some instances, FR residues of the human immunoglobulin are replaced by corresponding non-human residues. In some instances, the humanized antibody contains FR derived from different genes. In some instances, the humanized anti-idiotype antibody specific for SJ25C1 contains at least a portion of an immunoglobulin constant region (Fc), typically that of a human immunoglobulin.

In some instances, the humanized anti-idiotype antibody specific for SJ25C1 contains an altered human acceptor antibody variable domain sequences that have been rendered to encode one or more amino acid residues of position 4, 35, 38, 43, 44, 46, 58, 62, 64, 65, 66, 67, 68, 69, 73, 85, 98 (Kabat) of the light variable region and 2, 4, 36, 39, 43, 45, 69, 70, 74, 75, 76, 78, 92(Kabat) of the heavy variable region corresponding to the non-human donor sequence.

In certain instances, the anti-idiotype antibody specific for SJ25C1 is humanized. In particular instances, the humanized anti-idiotype antibody specific for SJ25C1 contains one or more of a CDR-L1, CDR-L2, CDR-L3 and CDR-H1, CDR-H2, CDR-H3 region of a non-human anti-idiotype antibody that is specific for SJ25C1. In some instances, some or all of the CDR-L1, CDR-L2, CDR-L3 and CDR-H1, CDR-H2, CDR-H3 region of contain one or more amino acid modifications. In some instances, the modifications replacing a nonhuman amino acid residue with a human residue. In particular instances, the one or more of the CDR-L1, CDR-L2, CDR-L3 and CDR-H1, CDR-H2, CDR-H3 are inserted into the FR regions of a human antibody. In particular instances, the CDR-L1, CDR-L2, CDR-L3 and CDR-H1, CDR-H2, CDR-H3 of the nonhuman anti-idiotype antibody are the CDRs of the VH and VL regions having amino acid sequences set forth in SEQ ID NOs: 1 and 5, respectively. In some instances, all of the CDR-L1, CDR-L2, CDR-L3 and CDR-H1, CDR-H2, CDR-H3 of the anti-idiotype antibody specific for SJ25C1 are inserted into the FRs of the human antibody. In particular instances, the CDR and FR regions are the regions as identified by Kabat, Chothia, AbM, and/or and Contact schemes.

In particular instances, one or more or all of the CDR-L1, CDR-L2, CDR-L3 and CDR-H1, CDR-H2, CDR-H3 of the nonhuman anti-idiotype antibody specific for SJ25C1 are inserted into framework regions of a human antibody. In certain instances, the human antibody is an IgA, IgD, IgE, IgG, and IgM antibody. In particular instances, the human antibody is one of a subclass of human IgA, IgD, IgE, IgG, and IgM, e.g., human IgG1, IgG₂, IgG₃, IgG₄, IgAi, or IgA₂. In some instances, one or more or all of the CDR-L1, CDR-L2, CDR-L3 and CDR-H1, CDR-H2, CDR-H3 of the nonhuman anti-idiotype antibody specific for SJ25C1 are inserted into framework regions of an antigen binding region that is from and/or is derived from a human antibody. In certain instances, the antigen binding fragment is from and/or is derived from a human IgA, IgD, IgE, IgG, and IgM antibody. The subunit structures and three-dimensional configurations of different classes of human immunoglobulins are well known and described generally in, for example, Abbas et al. Cellular and Mol. Immunology, 4th ed. (W.B. Saunders, Co., 2000). In some instances, the human antibody or antigen binding fragment thereof may be part of a larger fusion molecule, formed by covalent or non-covalent association of the human antibody with one or more other proteins or peptides.

In some instances, one or more or all of the CDR-L1, CDR-L2, CDR-L3 and CDR-H1, CDR-H2, CDR-H3 of the nonhuman anti-idiotype antibody specific for SJ25C1 are inserted into framework regions of a human antibody or antigen-binding fragment thereof having all or a portion of an Fc region. In certain instances, the humanized anti-idiotype antibody specific for SJ25C1 contains all or a portion of an Fc region. In some instances, the Fc region has one or more modifications, such as an amino acid modification (e.g. a substitution, insertion, or deletion) at one or more amino acid positions. Such modifications can be made, for example, to improve half-life, alter binding to one or more types of Fc receptors, and/or alter effector functions. In some instances, modified Fc regions have altered (e.g., decreased) binding to FcαRs, relative to that of an unmodified Fc region. In certain instances, the humanized anti-idiotype antibody contains all or a portion of a modified Fc region having an altered (e.g., decreased) binding to Fc receptor relative to that of an unmodified Fc region. Non-limiting examples of Fc modifications that alter its binding to the Fc receptors are described, for example, in U.S. Pat. Nos. 7,217,797 and 7,732,570; and U.S. Application Nos. US 2010/0143254 and 2010/0143254.

In certain instances, a humanized anti-idiotype antibody specific for SJ25C1 has a heavy chain containing the amino acid sequence set forth in SEQ ID NO: 132 and a light chain containing the amino acid sequence set forth in SEQ ID NO: 133. In particular instances, a humanized anti-idiotype antibody specific for SJ25C1 has a heavy chain containing the amino acid sequence set forth in SEQ ID NO: 134 and a light chain containing the amino acid sequence set forth in SEQ ID NO: 135. In some instances, a humanized anti-idiotype antibody specific for SJ25C1 has a heavy chain containing the amino acid sequence set forth in SEQ ID NO: 136 and a light chain containing the amino acid sequence set forth in SEQ ID NO: 137.

### B. FMC63-derived Antibodies

In some instances, disclosed are anti-idiotype antibodies specific to a target anti-CD19 antibody that is or is derived from antibody FMC63 or an antigen-binding fragment thereof. In some instances, the disclosed antibodies or antigen-binding fragments are specific to an FMC63-derived scFv.

In some instances, disclosed are anti-idiotype antibodies or antigen-binding fragments thereof that include a heavy chain variable (V_{H}) region comprising at least 90% sequence identity to the V_{H} region amino acid sequence set forth in SEQ ID NO: 36 or 58, such as at least 91%, 92%, 93%, 94%, 95%, 96%, 97%, 98%, or 99% sequence identity thereto.

In some instances, disclosed are antibodies or antigen-binding fragments thereof that include a VH region having a heavy chain complementarity determining region 1 (CDR-H1) containing the amino acid sequence of GYX₃FX₅X₆YX₈MX₁₀ (SEQ ID NO: 108), wherein X₃ is T or S, X₅ is T or S, X₆ is D or R, X₈ is Y or W, and X₁₀ is K or N; and/or a heavy chain complementarity determining region 2 (CDR-H2) containing the amino acid sequence of WIGX₄IX₆PX₈X₉X₁₀X₁₁TX₁₃X₁₄NQX₁₇FKX₂₀ (SEQ ID NO: 109), wherein X₄ is D or M, X₆ is Nor H, X₈ is N or S, X₉ is N or D, X₁₀ is G or S, X₁₁ is G or E, X₁₃ is D or R, X₁₄ is Y or L, X₁₇ is N or K, and X₂₀ is G or D; and/or a heavy chain complementarity determining region 3 (CDR-H3) containing the amino acid sequence of AX₂X₃X₄X₅X₆X₇X₈X₉X₁₀X₁₁X₁₂X₁₃X₁₄X₁₅ (SEQ ID NO: 110), wherein X₂ is R or S, X₃ is E or I, X₄ is G or Y, X₅ is N or Y, X₆ is N or E, X₇ is Y or null, X₈ is G or null, X₉ is S or null, X₁₀ is R or null, X₁₁ is D or null, X₁₂ is A or null, X₁₃ is M or null, X₁₄ is D or E, and X₁₅ is Y or A.

In some instances, disclosed are anti-idiotype antibodies or antigen-binding fragments thereof that include a heavy chain variable (VH) region containing a heavy chain complementarity determining region 3 (CDR-H3) having the amino acid sequence set forth in SEQ ID NO: 46, 67, 94 or 104 and/or a CDR-H3 contained within the heavy chain variable (V_{H}) sequence set forth in SEQ ID NO: 36 or 58.

In some of any such instances, the V_{H} region includes a heavy chain complementarity determining region 1 (CDR-H1) comprising the amino acid sequence set forth in SEQ ID NO: 44, 65, 88, 89, 90, 98, 99, or 100, and/or a CDR-H1 contained within the V_{H} sequence set forth in SEQ ID NO: 36 or 58; and/or a heavy chain complementarity determining region 2 (CDR-H2) comprising the amino acid sequence set forth in SEQ ID NO: 45, 66, 91, 92, 93, 101, 102, or 103, and/or a CDR-H2 contained within the V_{H} sequence set forth in SEQ ID NO: 36 or 58.

Disclosed are anti-idiotype antibodies or antigen-binding fragments thereof that include a heavy chain variable (VH) region comprising a heavy chain complementarity determining region 1 (CDR-H1), CDR-H2, and CDR-H3, wherein the CDR-H1 comprises the amino acid sequence set forth in SEQ ID NO: 44, 65, 88, 89, 90, 98, 99, or 100; the CDR-H2 comprises the amino acid sequence set forth in SEQ ID NO: 45, 66, 91, 92, 93, 101, 102, or 103; and/or the CDR-H3 comprises the amino acid sequence set forth in SEQ ID NO: 46, 67, 94 or 104. In some instances, disclosed are antibodies or antigen-binding fragments thereof that include a CDR-H1 having the amino acid sequence set forth in SEQ ID NO: 44, 65, 88, 89, 90, 98, 99, or 100; a CDR-H2 having the amino acid sequence set forth in SEQ ID NO: 45, 66, 91, 92, 93, 101, 102, or 103; and a CDR-H3 having the amino acid sequence set forth in SEQ ID NO: 46, 67, 94 or 104.

Disclosed are anti-idiotype antibodies or antigen-binding fragments thereof that includes a heavy chain complementarity determining region 1 (CDR-H1), a CDR-H2, and a CDR-H3, respectively, comprising the amino acid sequences of a CDR-H1, a CDR-H2, and a CDR-H3 contained within the V_{H} region amino acid sequence set forth in SEQ ID NO: 36 or 58.

In some instances, the anti-idiotype antibody or antigen-binding fragment thereof includes a CDR-H1 set forth in SEQ ID NOS: 44, 88, 89 or 90; a CDR-H2 set forth in SEQ ID NOS: 45, 91, 92 or 93; and/or a CDR-H3 set forth in SEQ ID NO: 46 or 94. In some instances, the anti-idiotype antibody or antigen-binding fragment thereof includes a CDR-H1 set forth in SEQ ID NO: 65, 98, 99 or 100; a CDR-H2 set forth in SEQ ID NO: 66, 101, 102 or 103; and/or a CDR-H3 set forth in SEQ ID NO: 67 or 104, respectively.

In some of any such instances, the V_{H} region contains a framework region 1 (FR1), a FR2, a FR3, and/or a FR4 sequence having at least 90% sequence identity, respectively, to a FR1, FR2, FR3, and/or FR4 of the amino acid sequence set forth in SEQ ID NO: 36 or 58. In some instances, the V_{H} region contains a framework region 1 (FR1), a FR2, a FR3, and/or a FR4 sequence having at least 91%, 92%, 93%, 94%, 95%, 96%, 97%, 98%, 98%, 99% sequence identity, respectively, to a FR1, FR2, FR3, and/or FR4 of the amino acid sequence set forth in SEQ ID NO: 36 or 58. In some instances, the V_{H} region contains a framework region 1 (FR1), a FR2, a FR3, and a FR4 sequence having at least 90%, 91%, 92%, 93%, 94%, 95%, 96%, 97%, 98%, 98%, 99% sequence identity, respectively, to a FR1, FR2, FR3, and FR4 of the amino acid sequence set forth in SEQ ID NO: 36 or 58.

In some of any of such instances, the V_{H} region has the sequence of amino acids set forth in SEQ ID NO: 36 or 58.

In some of any such instances, the anti-idiotype antibody or antigen-binding fragment is a heavy chain only, a VH-only, and/or does not include a VL or antigen-binding portion thereof and/or the antigen-binding site of the anti-idiotype antibody or fragment includes residues from the heavy chain only and/or does not include residues from a light chain.

In some of any such instances, the anti-idiotype antibody or fragment does not contain a light chain variable (V_{L}) region, does not contain a CDR-L1, CDR-L2, and/or CDR-L3, and/or is a single-domain antibody (sdAb) containing only the V_{H} region. In some instances, the antibody or fragment is a sdAb that only contains a VH region from any as described.

In some instances of any of the anti-idiotype antibodies or fragments containing any of the above VH region sequences, the anti-idiotype antibody or fragment further comprises a light chain variable (V_{L}) region. In some such instances, the V_{L} region has at least 90% sequence identity to the V_{L} region amino acid sequence set forth in SEQ ID NO: 40 or 62, such as at least 91%, 92%, 93%, 94%, 95%, 96%, 97%, 98%, or 99% sequence identity to the V_{L} region amino acid sequence set forth in SEQ ID NO: 40 or 62.

In some instances, disclosed are antibodies or antigen-binding fragments thereof that include a VH region having a light chain complementarity determining region 1 (CDR-L1) containing the amino acid sequence of X₁AX₃X₄X₅X₆X₇X₈YX₁₀X₁₁WY (SEQ ID NO: 111), wherein X₁ is S or R, X₃ is S or R, X₄ is S or G, X₅ is G or N, X₆ is V or I, X₇ is I or H, X₈ is N or null, X₁₀ is M or L, and X₁₁ is Y or A; and/or a light chain complementarity determining region 2 (CDR-L2) containing the amino acid sequence of X₁X₂X₃YX₅X₆X₇X₈LAX₁₁ (SEQ ID NO: 112), wherein X₁ is P or L, X₂ is W or L, X₃ is I or V, X₅ is L or N, X₆ is T or A, X₇ is S or K, X₈ is N or T, and X₁₁ is S or D; and/or a light chain complementarity determining region 3 (CDR-L3) containing the amino acid sequence of QX₂X₃X₄X₅X₆PX₈T (SEQ ID NO: 113), wherein X₂ is Q or H, X₃ is W or F, X₄ is S or W, X₅ is S or W, X₆ is N or T, and X₈ is L or Y.

In some of any such instances, the V_{L} region comprises a light chain complementarity determining region 3 (CDR-L3) comprising the amino acid sequence set forth in SEQ ID NO: 49, 97, 70, or 107. In some of any such instances, the V_{L} region comprises a light chain complementarity determining region 3 (CDR-L3) having the amino acid sequence set forth in SEQ ID NO: 49, 97, 70, or 107.

In some of any such instances, the V_{L} region comprises a light chain complementarity determining region 1 (CDR-L1) comprising the amino acid sequence set forth in SEQ ID NO: 47, 68, 95, or 105, and/or a CDR-L1 contained within the V_{L} sequence set forth in SEQ ID NO: 40 or 62; and/or a light chain complementarity determining region 2 (CDR-L2) comprising the amino acid sequence set forth in SEQ ID NO: 48, 69, 96, or 106, and/or a CDR-L2 contained within the V_{L} sequence set forth in SEQ ID NO: 40 or 62. In some of any such instances, the V_{L} region comprises a light chain complementarity determining region 1 (CDR-L1) having the amino acid sequence set forth in SEQ ID NO: 47, 68, 95, or 105, and/or a CDR-L1 contained within the V_{L} sequence set forth in SEQ ID NO: 40 or 62; and/or a light chain complementarity determining region 2 (CDR-L2) having the amino acid sequence set forth in SEQ ID NO: 48, 69, 96, or 106, and/or a CDR-L2 contained within the V_{L} sequence set forth in SEQ ID NO: 40 or 62.

In some of any such instances, the V_{L} region comprises a CDR-L1 containing the amino acid sequence set forth in SEQ ID NO: 47, 68, 95, or 105; a CDR-L2 containing the amino acid sequence set forth in SEQ ID NO: 48, 69, 96, or 106; and a CDR-L3 containing the amino acid sequence set forth in SEQ ID NO: 49, 97, 70, or 107.

In some of any such instances, the V_{L} region comprises the CDR-L1, CDR-L2, and CDR-L3, respectively, comprising the amino acid sequences of a CDR-L1, a CDR-L2, and a CDR-L3 contained within the V_{L} region amino acid sequence set forth in SEQ ID NO: 40 or 62.

In some instances, the anti-idiotype antibody or antigen-binding fragment thereof includes a CDR-L1 set forth in SEQ ID NOS: 47 or 95; a CDR-L2 set forth in SEQ ID NOS: 48 or 96; and/or a CDR-L3 set forth in SEQ ID NO: 49 or 97. In some instances, the anti-idiotype antibody or antigen-binding fragment thereof includes a CDR-L1 set forth in SEQ ID NO: 68 or 105; a CDR-L2 set forth in SEQ ID NO: 69 or 106; and/or a CDR-L3 set forth in SEQ ID NO: 70 or 107, respectively.

In some of any such instances, the V_{L} region comprises a framework region 1 (FR1), a FR2, a FR3, and/or a FR4 having at least 90% sequence identity, respectively, to the FR1, FR2, FR3, and/or FR4 of the amino acid sequence set forth in SEQ ID NO: 40 or 62. In some instances, the V_{L} region comprises a framework region 1 (FR1), a FR2, a FR3, and/or a FR4 sequence having at least 91%, 92%, 93%, 94%, 95%, 96%, 97%, 98%, 98%, 99% sequence identity, respectively, to a FR1, FR2, FR3, and/or FR4 of the amino acid sequence set forth in SEQ ID NO: 40 or 62. In some instances, the V_{L} region comprises a framework region 1 (FR1), a FR2, a FR3, and a FR4 sequence having at least 90%, 91%, 92%, 93%, 94%, 95%, 96%, 97%, 98%, 98%, 99% sequence identity, respectively, to a FR1, FR2, FR3, and FR4 of the amino acid sequence set forth in SEQ ID NO: 40 or 62.

In some of any such instances, the V_{L} region has the amino acid sequence set forth in SEQ ID NO: 40 or 62.

Disclosed are anti-idiotype antibodies or antigen-binding fragments thereof that comprise the amino acid sequences of CDR-H1, CDR-H2, and CDR-H3 sequences contained within the V_{H} region amino acid sequence set forth in SEQ ID NO: 36 or 58; and/or comprise the amino acid sequences of CDR-L1, CDR-L2, and CDR-L3 sequences contained within the light chain variable (VL) region amino acid sequence set forth in SEQ ID NO: 40 or 62.

Disclosed are anti-idiotype antibodies or antigen-binding fragments thereof that include the V_{H} region having amino acid sequences having at least 90 %, 91%, 92%, 93%, 94%, 95%, 96%, 97%, 98% or 99% identity to SEQ ID NOs: 36 or 58 and V_{L} region having amino acid sequences having at least 90 %, 91%, 92%, 93%, 94%, 95%, 96%, 97%, 98% or 99% identity to SEQ ID NOs: 40 or 62.

In some instances, disclosed are anti-idiotype antibodies or antigen-binding fragments thereof that include the V_{H} region having amino acid sequences set forth in SEQ ID NOs: 36 or 58 and V_{L} region having amino acid sequences set forth in SEQ ID NOs: 40 or 62. In some of instances, the disclosed antibody contains the V_{H} region set forth in SEQ ID NO: 36 and the V_{L} region set forth in SEQ ID NOs:40. In some of instances, the disclosed antibody contains the V_{H} region set forth in SEQ ID NO: 58 and the V_{L} region set forth in SEQ ID NOs: 62.

In some instances, the anti-idiotype antibody is a single-chain antibody fragment, such as an scFv or diabody. In some instances, the single-chain antibody includes one or more linkers joining two antibody domains or regions, such as a variable heavy chain (V_{H}) region and a variable light chain (V_{L}). The linker typically is a peptide linker, e.g., a flexible and/or soluble peptide linker. Among the linkers are those rich in glycine and serine and/or in some cases threonine. In some instances, the linkers further include charged residues such as lysine and/or glutamate, which can improve solubility. In some instances, the linkers further include one or more proline.

In some instances, the anti-idiotype antibody is an intact antibody or full-length antibody. In some instances, the anti-ID may contain at least a portion of an immunoglobulin constant region, such as one or more constant region domains. In some instances, the constant regions include a light chain constant region (CL) and/or a heavy chain constant region 1 (CH1). In some instances, the anti-ID includes a CH2 and/or CH3 domain, such as an Fc region. In some instances, the Fc region is an Fc region of a human IgG, such as IgG1 or IgG4. In some instances, the anti-idiotype antibody contains the CH domain set forth in SEQ ID NO:37 or 59 or a portion thereof or a sequence of amino acids that exhibits at least 85%, 86%, 87%, 88%, 89%, 90%, 91%, 92%, 93%, 94%, 95%, 96%, 97%, 98%, or 99% sequence identity to SEQ ID NO:37 or 59 or a portion thereof. In some instances, the anti-idiotype antibody contains the CL domain set forth in SEQ ID NO:41 or 63 or a portion thereof or a sequence of amino acids that exhibits at least 85%, 86%, 87%, 88%, 89%, 90%, 91%, 92%, 93%, 94%, 95%, 96%, 97%, 98%, or 99% sequence identity to SEQ ID NO:41 or 63 or a portion thereof.

In some instances, the anti-idiotype antibody specific for FMC63 comprises the heavy chain sequence set forth in SEQ ID NO:38 or 60 or a sequence that exhibits at least 85%, 86%, 87%, 88%, 89%, 90%, 91%, 92%, 93%, 94%, 95%, 96%, 97%, 98%, or 99% sequence identity to SEQ ID NO:38 or 60 and/or comprises the light chain sequence set forth in SEQ ID NO:42 or 63 or a sequence that exhibits at least 85%, 86%, 87%, 88%, 89%, 90%, 91%, 92%, 93%, 94%, 95%, 96%, 97%, 98%, or 99% sequence identity to SEQ ID NO:42 or 63. In some instances, the anti-idiotype antibody specific for FMC63 comprises the heavy chain sequence set forth in SEQ ID NO:38 and/or the light chain sequence set forth in SEQ ID NO:42. In some instances, the anti-idiotype antibody specific for FMC63 comprises the heavy chain sequence set forth in SEQ ID NO:60 and/or the light chain sequence set forth in SEQ ID NO:63. In some instances, the heavy chain and/or light chain of the anti-idiotype antibody further comprises a signal peptide. In some cases, the signal peptide has the sequence set forth in SEQ ID NO:39, 43, 61 or 64.

In some instances, the anti-idiotype antibody is an antigen-binding fragment. In some instances, the antigen-binding fragment is selected from the group consisting of fragment antigen binding (Fab) fragments, F(ab')₂ fragments, Fab' fragments, Fv fragments, a single chain variable fragment (scFv) or a single domain antibody.

Accordingly, disclosed are single-chain antibody fragments, such as scFvs and diabodies, particularly human single-chain fragments, typically comprising linker(s) joining two anti-idiotype antibody domains or regions, such V_{H} and V_{L} domains. The linker typically is a peptide linker, e.g., a flexible and/or soluble peptide linker, such as one rich in glycine and serine.

In some aspects, the linkers rich in glycine and serine (and/or threonine) include at least 80, 85, 90, 91, 92, 93, 94, 95, 96, 97, 98, or 99% such amino acid(s). In some instances, they include at least at or about 50%, 55%, 60%, 70%, or 75%, glycine, serine, and/or threonine. In some instances, the linker is comprised substantially entirely of glycine, serine, and/or threonine. The linkers generally are between about 5 and about 50 amino acids in length, typically between at or about 10 and at or about 30, e.g., 10, 11, 12, 13, 14, 15, 16, 17, 18, 19, 20, 21, 22, 23, 24, 25, 26, 27, 28, 29, or 30, and in some examples between 10 and 25 amino acids in length. Exemplary linkers include linkers having various numbers of repeats of the sequence GGGS (3GS; SEQ ID NO: 29) or GGGGS (4GS; SEQ ID NO: 26), such as between 2, 3, 4, and 5 repeats of such a sequence. Exemplary linkers include those having or consisting of a sequence set forth in SEQ ID NO: 25 (GGGGSGGGGSGGGGS). Exemplary linkers further include those having or consisting of the sequence set forth in SEQ ID NO: 33 (GSTSGSGKPGSGEGSTKG).

In some instances, the anti-idiotype antibodies include isolated antibodies. In some instances, the anti-ID is humanized, recombinant, and/or monoclonal. In some instances, the anti-ID is human.

In some instances, the anti-idiotype antibody specific for FMC63 is humanized. In particular instances, all or substantially all CDR amino acid residues of the humanized anti-idiotype antibody specific for FMC63 are derived from non-human, anti-FMC63 CDRs. In some instances, the humanized anti-idiotype antibody specific for FMC63 includes at least a portion of an antibody constant region derived from a human antibody.

In certain instances, the humanized anti-idiotype antibody specific for FMC63 includes a human immunoglobulin (recipient antibody) in which residues from the heavy chain variable region of the recipient are replaced by residues from a heavy chain variable region of the nonhuman anti-idiotype antibody specific for FMC63. In some instances, FR residues of the human immunoglobulin are replaced by corresponding non-human residues. In some instances, the humanized antibody contains FR derived from different genes. In some instances, the humanized anti-idiotype antibody specific for FMC63 contains at least a portion of an immunoglobulin constant region (Fc), typically that of a human immunoglobulin.

In some instances, the humanized anti-idiotype antibody specific for FMC63 contains an altered human acceptor antibody variable domain sequences that have been rendered to encode one or more amino acid residues of position 4, 35, 38, 43, 44, 46, 58, 62, 64, 65, 66, 67, 68, 69, 73, 85, 98 (Kabat) of the light variable region and 2, 4, 36, 39, 43, 45, 69, 70, 74, 75, 76, 78, 92(Kabat) of the heavy variable region corresponding to the non-human donor sequence .

In certain instances, the anti-idiotype antibody specific for FMC63 is humanized. In particular instances, the humanized anti-idiotype antibody specific for FMC63 contains one or more of a CDR-L1, CDR-L2, CDR-L3 and CDR-H1, CDR-H2, CDR-H3 of a non-human anti-idiotype antibody specific for FMC63. In some instances, some or all of the CDR-L1, CDR-L2, CDR-L3 and CDR-H1, CDR-H2, CDR-H3 region of contain one or more amino acid modifications. In some instances, the modifications replacing a nonhuman amino acid residue with a human residue. In particular instances, the one or more of the CDR-L1, CDR-L2, CDR-L3 and CDR-H1, CDR-H2, CDR-H3 are inserted into human FR regions. In particular instances, the CDR-L1, CDR-L2, CDR-L3 and CDR-H1, CDR-H2, CDR-H3 of the nonhuman anti-idiotype antibody are the CDRs of the VH regions having amino acid sequences set forth in SEQ ID NOs: 36 or 58. In certain instances, the CDRs of the nonhuman anti-idiotype VH region are or include a CDR-H1 containing with the amino acid sequence set forth in SEQ ID NO: 44, 65, 88, 89, 90, 98, 99, or 100; the CDR-H2 containing the amino acid sequence set forth in SEQ ID NO: 45, 66, 91, 92, 93, 101, 102, or 103; and/or the CDR-H3 containing the amino acid sequence set forth in SEQ ID NO: 46, 67, 94 or 104. In some instances, the CDR-L1, CDR-L2, CDR-L3 and CDR-H1, CDR-H2, CDR-H3 of the nonhuman anti-idiotype antibody are the CDRs of the VL regions having amino acid sequences set forth in SEQ ID NOs: 40 or 62. In some instances, the CDRs of the nonhuman anti-idiotype VL region are or include a CDR-L1 containing the amino acid sequence set forth in SEQ ID NO: 47, 68, 95, or 105; a CDR-L2 containing the amino acid sequence set forth in SEQ ID NO: 48, 69, 96, or 106; and a CDR-L3 containing the amino acid sequence set forth in SEQ ID NO: 49, 97, 70, or 107. In some instances, all of the CDR-L1, CDR-L2, CDR-L3 and CDR-H1, CDR-H2, CDR-H3 regions of the anti-idiotype antibody specific for FMC63 are inserted into the FRs of the human antibody. In particular instances, the CDR and FR regions are the regions as identified by Kabat, Chothia, AbM, and/or and Contact schemes.

In particular instances, one or more or all of the CDR-L1, CDR-L2, CDR-L3 and CDR-H1, CDR-H2, CDR-H3 of the nonhuman anti-idiotype antibody specific for FMC63 are inserted into framework regions of a human antibody. In certain instances, the human antibody is an IgA, IgD, IgE, IgG, and IgM antibody. In particular instances, the human antibody is one of a subclass of human IgA, IgD, IgE, IgG, and IgM, e.g., human IgG₁, IgG₂, IgG₃, IgG₄, IgAi, or IgA₂. In some instances, one or more or all of the CDR-L1, CDR-L2, CDR-L3 and CDR-H1, CDR-H2, CDR-H3 of the nonhuman anti-idiotype antibody specific for FMC63 are inserted into framework regions of an antigen binding region that is from and/or is derived from a human antibody. In certain instances, the antigen binding fragment is from and/or is derived from a human IgA, IgD, IgE, IgG, and IgM antibody. The subunit structures and three-dimensional configurations of different classes of human immunoglobulins are well known and described generally in, for example, Abbas et al. Cellular and Mol. Immunology, 4th ed. (W.B. Saunders, Co., 2000). In some instances, the human antibody or antigen binding fragment thereof may be part of a larger fusion molecule, formed by covalent or non-covalent association of the human antibody with one or more other proteins or peptides.

In some instances, one or more or all of the CDR-L1, CDR-L2, CDR-L3 and CDR-H1, CDR-H2, CDR-H3 of the nonhuman anti-idiotype antibody specific for FMC63 are inserted into framework regions of a human antibody or antigen-binding fragment thereof having all or a portion of an Fc region. In certain instances, the humanized anti-idiotype antibody specific for FMC63 contains all or a portion of an Fc region. In some instances, the Fc region has one or more modifications, such as an amino acid modification (e.g. a substitution, insertion, or deletion) at one or more amino acid positions. Such modifications can be made, for example, to improve half-life, alter binding to one or more types of Fc receptors, and/or alter effector functions. In some instances, modified Fc regions have altered (e.g., decreased) binding to FcαRs, relative to that of an unmodified Fc region. In certain instances, the humanized anti-idiotype antibody contains all or a portion of a modified Fc region having an altered (e.g., decreased) binding to Fc receptor relative to that of an unmodified Fc region. Non-limiting examples of Fc modifications that alter its binding to the Fc receptors are described, for example, in U.S. Pat. Nos. 7,217,797 and 7,732,570; and U.S. Application Nos. US 2010/0143254 and 2010/0143254.

In certain instances, a humanized anti-idiotype antibody specific for FMC63 has a heavy chain containing the amino acid sequence set forth in SEQ ID NO: 138 and a light chain containing the amino acid sequence set forth in SEQ ID NO: 139. In particular instances, a humanized anti-idiotype antibody specific for FMC63 has a heavy chain containing the amino acid sequence set forth in SEQ ID NO: 140 and a light chain containing the amino acid sequence set forth in SEQ ID NO: 141. In some instances, a humanized anti-idiotype antibody specific for FMC63 has a heavy chain containing the amino acid sequence set forth in SEQ ID NO: 142 and a light chain containing the amino acid sequence set forth in SEQ ID NO: 143. In certain instances, a humanized anti-idiotype antibody specific for FMC63 has a heavy chain containing the amino acid sequence set forth in SEQ ID NO: 144 and a light chain containing the amino acid sequence set forth in SEQ ID NO: 145. In particular instances, a humanized anti-idiotype antibody specific for FMC63 has a heavy chain containing the amino acid sequence set forth in SEQ ID NO: 146 and a light chain containing the amino acid sequence set forth in SEQ ID NO: 147. In some instances, a humanized anti-idiotype antibody specific for FMC63 has a heavy chain containing the amino acid sequence set forth in SEQ ID NO: 148 and a light chain containing the amino acid sequence set forth in SEQ ID NO: 149.

### C. Exemplary Features

Anti-idiotype antibodies disclosed herein may be identified, screened for, or characterized for their physical/chemical properties and/or biological activities by various known assays. In one aspect, the anti-idiotype antibody is tested for its antigen binding activity, e.g., by known methods such as ELISA, Western blotting, and/or flow cytometric assays, including cell-based binding assays, for example, assessing binding of the anti-idiotype antibody (e.g., conjugated to a fluorescent marker or tagged) to a cell presenting the target anti-CD19 antibody moiety, in some cases compared to results using cells that do not express the target anti-CD19 antibody moiety. Binding affinity may be measured as Kd or EC50.

In some instances of any of the disclosed antibodies, e.g. any of the disclosed antibodies in Section A above, the target anti-CD19 antibody moiety is SJ25C1 or is an antibody derived from SJ25C1.

In some instances of any of the disclosed antibodies, e.g. any of the disclosed antibodies in Section B above, the target anti-CD19 antibody moiety is FMC63 or is an antibody derived from FMC63.

Competition assays may be used to identify an antibody that competes with any of the anti-idiotype antibodies described herein. Assays for mapping epitopes bound by the anti-idiotype antibodies and reference antibodies also may be used and are known.

In some instances, the anti-idiotype antibody does not cross-react with an anti-CD19 antibody moiety different from the target anti-CD19 antibody moiety. In some instances, the target anti-CD19 antibody moiety is derived from the SJ25C1 antibody. In some instances, the target anti-CD19 antibody moiety is derived from the SJ25C1 antibody, and the anti-idiotype antibody does not cross-react with an anti-CD19 antibody moiety derived from the FMC63 antibody, an anti-CD19 antibody comprising a V_{H} comprising the amino acid sequence of SEQ ID NO: 30 and a V_{L} comprising the amino acid sequence of SEQ ID NO: 31.In some instances, the target anti-CD19 antibody moiety is derived from the FMC63 antibody. In some instances, the target anti-CD19 antibody moiety is derived from the FMC63 antibody, and the anti-idiotype antibody does not cross-react with an anti-CD19 antibody moiety derived from the SJ25C1 antibody, an anti-CD19 antibody comprising a V_{H} comprising the amino acid sequence of SEQ ID NO: 23 and a V_{L} comprising the amino acid sequence of SEQ ID NO: 24.

In some instances, the anti-idiotype antibody specifically binds to a target anti-CD19 antibody moiety that is part of a fusion protein, such as a recombinant receptor. In some instances, the anti-idiotype antibody does not bind to any epitope in the fusion protein outside of the target anti-CD 19 antibody moiety. For example, in some instances, the target anti-CD 19 antibody moiety is, or is part of, the antigen-binding domain of a chimeric antigen receptor (CAR), and the anti-idiotype antibody does not bind any epitope outside of the antigen-binding domain. In some instances, the CAR antigen-binding domain comprises or consists of an scFv.

In some instances, the anti-idiotype antibody specifically binds to a target anti-CD19 antibody moiety that is an scFv contained in a CAR. In some instances, the anti-idiotype antibody specifically binds to an epitope overlapping one or more complementarity determining regions (CDRs) of the target anti-CD19 scFv. In some instances, the anti-idiotype antibody does not bind any epitopes in the CAR outside of the scFv; in some instances, it does not bind to a reference antibody. In some instances, the reference antibody specifically binds to the same antigen as the target antibody, e.g., to the CD19 and/or comprises one or more variable heavy and/or variable light framework region(s) having at least 90, 95, 96, 97, 98, or 99 % identity to the corresponding framework region(s) of the target antibody (in some aspects, the one or more framework regions comprise an FR1, FR2, FR3, and/or FR4 of the heavy and/or the light chain); and/or contains the same heavy and/or light chain v-gene (or v-gene usage) as the target antibody and/or is derived from the same v-gene sequence as the target antibody. In some aspects, the reference antibody is FMC63. In some aspects, the reference antibody is SJ25C1. In some instances, the CAR comprises a spacer linking the scFv to its transmembrane domain, and the anti-idiotype antibody does not bind any epitope in the spacer. In some instances, the spacer is a sequence derived from CD28, such as an extracellular portion from CD28. In some instances, the spacer comprises the amino acid sequence of SEQ ID NO: 27. In some instances, the anti-idiotype antibody does not bind any epitope in an Fc domain, such as the Fc domain of IgG1. In some instances, the Fc domain is an IgG1 Fc domain lacking the hinge region. In some instances, the Fc domain comprises the amino acid sequence of SEQ ID NO: 32.

In some instances, the anti-idiotype antibody does not cross-react with a different CAR. In some instances, the anti-idiotype antibody does not cross-react with a different anti-CD19 CAR. In some instances, the anti-idiotype antibody does not cross-react with an anti-CD19 antibody moiety, e.g., of a reference antibody, having one or more different idiotopes compared to the target anti-CD19 scFv. In some instances, the anti-idiotype antibody is specific for a target anti-CD19 scFv of a CAR derived from the SJ25C1 antibody. In some instances, the target anti-CD19 antibody moiety is derived from the SJ25C1 antibody, and the anti-idiotype antibody does not cross-react with a CAR containing an anti-CD19 antibody moiety derived from the FMC63 antibody. In some instances, the anti-idiotype antibody is specific for a target anti-CD19 scFv of a CAR derived from the FMC63 antibody. In some instances, the target anti-CD19 antibody moiety is derived from the FMC63 antibody, and the anti-idiotype antibody does not cross-react with a CAR containing anti-CD19 antibody moiety derived from the SJ25C1 antibody.

In some instances, the anti-idiotype antibody is an agonist of the CAR. In some instances, the anti-idiotype antibody is an antagonist of the CAR.

In some instances, the disclosed anti-idiotype antibodies are capable of binding a target anti-CD19 moiety, such as antibody SJ25C1 or FMC63, with at least a certain affinity, as measured by any of a number of known methods. In some instances, the affinity is represented by an equilibrium dissociation constant (K_{D}); in some instances, the affinity is represented by EC₅₀. In certain instances, the binding affinity (EC50) and/or the dissociation constant of the anti-idiotype antibody to the anti-CD19 moiety is at or about or less than at or about 100 nM, 50 nM, 40 nM, 30 nM, 25 nM, 20 nM, 19, 18, 17, 16, 15, 14, 13, 12, 11, 10, 9, 8, 7, 6, 5, 4, 3, 2, or 1 nM, such as between at or about 1 nM and at or about 15 nM, e.g., between at or about 5 and at or about 10 nM. In one instance, the extent of binding of an anti-idiotype antibody to a moiety unrelated to the target anti-CD19 moiety is less than, at, or about 10% of the binding of the antibody to the target anti-CD19 moiety as measured, e.g., by a radioimmunoassay (RIA).

### D. Nucleic Acids

Also disclosed are nucleic acids encoding the antibodies and/or portions, e.g., chains, thereof. Among the disclosed nucleic acids are those encoding the anti-idiotype antibodies described herein. The nucleic acids may include those encompassing natural and/or non-naturally occurring nucleotides and bases, e.g., including those with backbone modifications. The terms "nucleic acid molecule", "nucleic acid" and "polynucleotide" may be used interchangeably, and refer to a polymer of nucleotides. Such polymers of nucleotides may contain natural and/or non-natural nucleotides, and include, but are not limited to, DNA, RNA, and PNA. "Nucleic acid sequence" refers to the linear sequence of nucleotides that comprise the nucleic acid molecule or polynucleotide. Exemplary nucleic acids and vectors are those having the sequences set forth as SEQ ID NOs: 15-22, 50-57, 71-77 and CDR-encoding portions thereof, as well as sequences containing at least at or about 90, 91, 92, 93, 94, 95, 96, 97, 98, or 99% identity thereto. The nucleic acid may encode an amino acid sequence comprising the V_{L} and/or an amino acid sequence comprising the V_{H} of the anti-idiotype antibody (e.g., the light and/or heavy chains of the antibody).

Also disclosed are vectors containing the nucleic acids, host cells containing the vectors, e.g., for producing the antibodies. Also disclosed are methods for producing the antibodies. In a further instance, one or more vectors (e.g., expression vectors) comprising such nucleic acid are disclosed. In a further instance, a host cell comprising such nucleic acid is disclosed. In one such instance, a host cell comprises (e.g., has been transformed with): (1) a vector comprising a nucleic acid that encodes an amino acid sequence comprising the V_{L} of the antibody and an amino acid sequence comprising the V_{H} of the antibody, or (2) a first vector comprising a nucleic acid that encodes an amino acid sequence comprising the V_{L} of the antibody and a second vector comprising a nucleic acid that encodes an amino acid sequence comprising the V_{H} of the antibody. In some instances, a method of making the anti-idiotype antibody is disclosed, wherein the method comprises culturing a host cell comprising a nucleic acid encoding the antibody, as disclosed above, under conditions suitable for expression of the antibody, and optionally recovering the antibody from the host cell (or host cell culture medium).

### E. Methods of Producing the Antibodies

Also disclosed are methods of making an anti-idiotype antibody, such as of any of the disclosed instances. In some instances, for recombinant production of the anti-idiotype antibody, nucleic acid encoding an antibody, e.g., as described above, may be isolated and inserted into one or more vectors for further cloning and/or expression in a host cell. Such nucleic acid may be readily isolated and sequenced using conventional procedures (e.g., by using oligonucleotide probes that are capable of binding specifically to genes encoding the heavy and light chains of the antibody).

In addition to prokaryotes, eukaryotic microbes such as filamentous fungi or yeast are suitable cloning or expression hosts for antibody-encoding vectors, including fungi and yeast strains whose glycosylation pathways have been modified to mimic or approximate those in human cells, resulting in the production of an antibody with a partially or fully human glycosylation pattern. See Gerngross, Nat. Biotech. 22: 1409-1414 (2004), and Li et al., Nat. Biotech. 24: 210-215 (2006).

Exemplary eukaryotic cells that may be used to express polypeptides include, but are not limited to, COS cells, including COS 7 cells; 293 cells, including 293-6E cells; CHO cells, including CHO-S, DG44. Lec13 CHO cells, and FUT8 CHO cells; PER.C6^{®} cells; and NSO cells. In some instances, the antibody heavy chains and/or light chains may be expressed in yeast. See, *e.g.,* U.S. Publication No. US 2006/0270045 A1. In some instances, a particular eukaryotic host cell is selected based on its ability to make desired post-translational modifications to the heavy chains and/or light chains. For example, in some instances, CHO cells produce polypeptides that have a higher level of sialylation than the same polypeptide produced in 293 cells.

In some instances, the anti-idiotype antibody is produced in a cell-free system. Exemplary cell-free systems are described, *e.g.,* in Sitaraman et al., Methods Mol. Biol. 498: 229-44 (2009); Spirin, Trends Biotechnol. 22: 538-45 (2004); Endo et al., Biotechnol. Adv. 21: 695-713 (2003).

The disclosed instances further include vectors and host cells and other expression systems for expressing and producing the antibodies and other binding proteins, including eukaryotic and prokaryotic host cells, including bacteria, filamentous fungi, and yeast, as well as mammalian cells such as human cells, as well as cell-free expression systems.

The anti-idiotype antibodies or antibody moieties can be humanized antibodies or human antibodies. A "humanized" antibody is an antibody in which all or substantially all CDR amino acid residues are derived from non-human CDRs and all or substantially all FR amino acid residues are derived from human FRs. A humanized antibody optionally may include at least a portion of an antibody constant region derived from a human antibody. A "humanized form" of a non-human antibody, refers to a variant of the non-human antibody that has undergone humanization, typically to reduce immunogenicity to humans, while retaining the specificity and affinity of the parental non-human antibody. In some instances, some FR residues in a humanized antibody are substituted with corresponding residues from a non-human antibody (e.g., the antibody from which the CDR residues are derived), e.g., to restore or improve antibody specificity or affinity.

Among the disclosed anti-idiotype antibodies or antibody moieties are human antibodies. A "human antibody" is an antibody with an amino acid sequence corresponding to that of an antibody produced by a human or a human cell, or non-human source that utilizes human antibody repertoires or other human antibody-encoding sequences, including human antibody libraries. The term excludes humanized forms of non-human antibodies comprising non-human antigen-binding regions, such as those in which all or substantially all CDRs are non-human.

Human antibodies may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic animals, the endogenous immunoglobulin loci have generally been inactivated. Human antibodies also may be derived from human antibody libraries, including phage display and cell-free libraries, containing antibody-encoding sequences derived from a human repertoire.

### F. Immunoconjugates

In some instances, the anti-idiotype antibody is or is part of an immunoconjugate (anti-idiotype antibody immunoconjugate), in which the anti-idiotype antibody is conjugated to one or more heterologous molecule(s), such as, but not limited to, a cytotoxic or an imaging agent. Cytotoxic agents include, but are not limited to, radioactive isotopes (e.g., At211, 1131, I125, Y90, Re186, Re188, Sm153, Bi212, P32, Pb212 and radioactive isotopes of Lu); chemotherapeutic agents (e.g., maytansinoids, taxanes, methotrexate, adriamicin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; toxins such as small molecule toxins or enzymatically active toxins. In some instances, the antibody is conjugated to one or more cytotoxic agents, such as chemotherapeutic agents or drugs, growth inhibitory agents, toxins (e.g., protein toxins, enzymatically active toxins of bacterial, fungal, plant, or animal origin, or fragments thereof), or radioactive isotopes.

Among the anti-idiotype antibody immunoconjugates are antibody-drug conjugates (ADCs), in which an anti-idiotype antibody is conjugated to one or more drugs, including but not limited to a maytansinoid (see U.S. Patent Nos. 5,208,020, 5,416,064 and European Patent EP 0 425 235 B1); an auristatin such as monomethylauristatin drug moieties DE and DF (MMAE and MMAF) (see U.S. Patent Nos. 5,635,483 and 5,780,588, and 7,498,298); a dolastatin; a calicheamicin or derivative thereof (see U.S. Patent Nos. 5,712,374, 5,714,586, 5,739,116, 5,767,285, 5,770,701, 5,770,710, 5,773,001, and 5,877,296; Hinman etal., Cancer Res. 53: 3336-3342 (1993); and Lode et al., Cancer Res. 58: 2925-2928 (1998)); an anthracycline such as daunomycin or doxorubicin (see Kratz et al., Current Med. Chem. 13: 477-523 (2006); Jeffrey et al., Bioorganic & Med. Chem. Letters 16: 358-362 (2006); Torgov et al., Bioconj. Chem. 16: 717-721 (2005); Nagy et al., Proc. Natl. Acad. Sci. USA 97: 829-834 (2000); Dubowchik et al., Bioorg. & Med. Chem. Letters 12: 1529-1532 (2002); King et al., J. Med. Chem. 45: 4336-4343 (2002); and U.S. Patent No. 6,630,579); methotrexate; vindesine; a taxane such as docetaxel, paclitaxel, larotaxel, tesetaxel, and ortataxel; a trichothecene; and CC1065.

Also among the anti-idiotype antibody immunoconjugates are those in which the antibody is conjugated to an enzymatically active toxin or fragment thereof, including but not limited to diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes.

Also among the anti-idiotype antibody immunoconjugates are those in which the anti-idiotype antibody is conjugated to a radioactive atom to form a radioconjugate. Exemplary radioactive isotopes include At²¹¹, I¹³¹, I¹²⁵, Y⁹⁰, Re¹⁸⁶, Re¹⁸⁸, Sm¹⁵³, Bi²¹², P³², Pb²¹² and radioactive isotopes of Lu.

Conjugates of an anti-idiotype antibody and cytotoxic agent may be made using any of a number of known protein coupling agents, e.g., linkers, (see Vitetta et al., Science 238: 1098 (1987)), WO94/11026. The linker may be a "cleavable linker" facilitating release of a cytotoxic drug in the cell, such as acid-labile linkers, peptidase-sensitive linkers, photolabile linkers, dimethyl linkers, and disulfide-containing linkers (Chari et al., Cancer Res. 52: 127-131 (1992); U.S. Patent No. 5,208,020).

Also disclosed are anti-idiotype antibody immunoconjugates comprising an anti-idiotype antibody attached to a label,e.g., a detectable label, which can generate a detectable signal, indirectly or directly. These anti-idiotype antibody immunoconjugates can be used for research or diagnostic applications. The label is preferably capable of producing, either directly or indirectly, a detectable signal. For example, the label may be radio-opaque or a radioisotope, such as ³H, ¹⁴C, ³²P, ³⁵S, ¹²³I, ¹²⁵I, ¹³¹I; a fluorescent (fluorophore) or chemiluminescent (chromophore) compound, such as fluorescein isothiocyanate, rhodamine or luciferin; an enzyme, such as alkaline phosphatase,β-galactosidase or horseradish peroxidase; an imaging agent; or a metal ion. In some instances, the label is a radioactive atom for scintigraphic studies, for example ⁹⁹Tc or ¹²³I, or a spin label for nuclear magnetic resonance (NMR) imaging (also known as magnetic resonance imaging, MRI), such as zirconium-89, iodine-123, iodine-131, indium-111, fluorine-19, carbon-13, nitrogen-15, oxygen-17, gadolinium, manganese or iron. Zirconium-89 may be complexed to various metal chelating agents and conjugated to antibodies, *e.g.,* for PET imaging (WO 2011/056983).

Examples of detectable labels include but are not limited to radionucleotides, enzymes, coenzymes, fluorescers, chemiluminescers, chromogens, enzyme substrates or cofactors, enzyme inhibitors, prosthetic group complexes, free radicals, particles, dyes, and the like. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin, coumarin, Alexa488, Oregon green 488, rhodamine green, Alexa 532, Cy3, Bodipy 588/586, Alexa586, TAMRA, Rox, Alexa 594, Texas red, Bodipy 630/650, Cy5, Alexa647, IR Dye 680, IR Dye 680, IR Dye 700 DX, Cy5.5, Alexa 750, IR Dye 800CW, IR Dye 800, Atto 532, and Atto 465.

In some instances, the anti-idiotype antibody immunoconjugate is detectable indirectly. For example, a secondary antibody that is specific for the anti-idiotype antibody immunoconjugate and contains a detectable label can be used to detect the anti-idiotype antibody immunoconjugate.

### Multispecific Antibodies

In certain instances, the anti-idiotype antibodies are multispecific. Among the multispecific binding molecules are multispecific antibodies, including, e.g. bispecific. Multispecific binding partners, e.g., antibodies, have binding specificities for at least two different sites, which may be in the same or different antigens. In certain instances, one of the binding specificities is for an anti-CD19 antibody moiety and the other is for another antigen. In certain instances, bispecific antibodies may bind to two different epitopes of an anti-CD19 antibody moiety. Bispecific antibodies may also be used to localize cytotoxic agents to cells which express an anti-CD19 antibody moiety on their surface, such as anti-CD19 CAR T cells. Bispecific antibodies can be prepared as full length antibodies or antibody fragments. Among the bispecific antibodies are multispecific single-chain antibodies, e.g., diabodies, triabodies, and tetrabodies, tandem di-scFvs, and tandem tri-scFvs.

### G. Variants

In certain instances, the anti-idiotype antibodies include one or more amino acid variations, e.g., substitutions, deletions, insertions, and/or mutations, compared to the sequence of an anti-idiotype antibody described herein. Exemplary variants include those designed to improve the binding affinity and/or other biological properties of the anti-idiotype antibody. Amino acid sequence variants of an anti-idiotype antibody may be prepared by introducing appropriate modifications into the nucleotide sequence encoding the anti-idiotype antibody, or by peptide synthesis. Such modifications include, for example, deletions from, and/or insertions into and/or substitutions of residues within the amino acid sequences of the anti-idiotype antibody. Any combination of deletion, insertion, and substitution can be made to arrive at the final construct, disclosed that the final construct possesses the desired characteristics, e.g., antigen-binding.

In certain instances, the anti-idiotype antibodies include one or more amino acid substitutions, e.g., as compared to an anti-idiotype antibody sequence described herein. Sites of interest for substitutional mutagenesis include the CDRs and FRs. Amino acid substitutions may be introduced into an anti-idiotype antibody of interest and the products screened for a desired activity, e.g., retained/improved antigen binding, decreased immunogenicity, improved half-life, and/or improved effector function, such as the ability to promote antibody-dependent cellular cytotoxicity (ADCC) or complement-dependent cytotoxicity (CDC). In some instances, the variant anti-idiotype antibody exhibits retained or improved binding to a target anti-CD19 antibody or fragment thereof. For example, in some instances, the variant anti-idiotype antibody exhibits an increase in binding affinity to the target anti-CD19 antibody of at least about 10% (such as at least about any of 20, 30, 40, 50, 60, 70, 80, 90, 100, 200, 300, 400, 500, 1000% or more) as compared to the unmodified anti-idiotype antibody.

In some instances, one or more residues within a CDR of a parent antibody (e.g. a humanized or human antibody) is/are substituted. In some instances, the substitution is made to revert a sequence or position in the sequence to a germline sequence, such as an antibody sequence found in the germline (e.g., human germline), for example, to reduce the likelihood of immunogenicity, e.g., upon administration to a human individual.

In some instances, alterations are made in CDR "hotspots," residues encoded by codons that undergo mutation at high frequency during the somatic maturation process (see, e.g., Chowdhury, Methods Mol. Biol. 207: 179-196 (2008)), and/or residues that contact antigen, with the resulting variant V_{H} or V_{L} being tested for binding affinity. Affinity maturation by constructing and reselecting from secondary libraries has been described, e.g., in Hoogenboom et al. in Methods in Molecular Biology 178: 1-37 (O'Brien et al., ed., Human Press, Totowa, NJ, (2001)). In some instances of affinity maturation, diversity is introduced into the variable genes chosen for maturation by any of a variety of methods (e.g., error-prone PCR, chain shuffling, or oligonucleotide-directed mutagenesis). A secondary library is then created. The library is then screened to identify any antibody variants with the desired affinity. Another method to introduce diversity involves CDR-directed approaches, in which several CDR residues (e.g., 4-6 residues at a time) are randomized. CDR residues involved in antigen binding may be specifically identified, e.g., using alanine scanning mutagenesis or modeling. CDR-H3 and CDR-L3 in particular are often targeted.

In certain instances, substitutions, insertions, or deletions may occur within one or more CDRs so long as such alterations do not substantially reduce the ability of the antibody to bind antigen. For example, conservative alterations (e.g., conservative substitutions as disclosed herein) that do not substantially reduce binding affinity may be made in CDRs. Such alterations may, for example, be outside of antigen contacting residues in the CDRs. In certain instances of the variant V_{H} and V_{L} sequences disclosed above, each CDR either is unaltered, or contains no more than one, two or three amino acid substitutions.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion to the N- or C-terminus of the antibody to an enzyme or a polypeptide which increases the serum half-life of the antibody.

### Modifications

In certain instances, the antibody is altered to increase or decrease the extent to which the antibody is glycosylated, for example, by removing or inserting one or more glycosylation sites by altering the amino acid sequence and/or by modifying the oligosaccharide(s) attached to the glycosylation sites, e.g., using certain cell lines.

Exemplary modifications, variants, and cell lines are described, e.g., in Patent Publication Nos. US 2003/0157108, US 2004/0093621, US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki et al. J. Mol. Biol. 336: 1239-1249 (2004); Yamane-Ohnuki et al. Biotech. Bioeng. 87: 614 (2004). Ripka et al. Arch. Biochem. Biophys. 249: 533-545 (1986); US Pat Appl No US 2003/0157108 A1, Presta, L; and WO 2004/056312 A1, Yamane-Ohnuki et al. Biotech. Bioeng. 87: 614 (2004); Kanda, Y. et al., Biotechnol. Bioeng., 94(4): 680-688 (2006); and WO2003/085107); WO 2003/011878 (Jean-Mairet et al.); U.S. Patent No. 6,602,684 (Umana et al.); and US 2005/0123546 (Umana et al.); WO 1997/30087 (Patel et al.); WO 1998/58964 (Raju, S.); and WO 1999/22764 (Raju, S.).

Among the modified antibodies are those having one or more amino acid modifications in the Fc region, such as those having a human Fc region sequence or other portion of a constant region (e.g., a human IgG1, IgG2, IgG3 or IgG4 Fc region) comprising an amino acid modification (e.g. a substitution) at one or more amino acid positions.

Such modifications can be made, e.g., to improve half-life, alter binding to one or more types of Fc receptors, and/or alter effector functions.

Also among the variants are cysteine engineered antibodies such as "thioMAbs" and other cysteine engineered variants, in which one or more residues of an antibody are substituted with cysteine residues, in order to generate reactive thiol groups at accessible sites, e.g., for use in conjugation of agents and linker-agents, to produce immunoconjugates. Cysteine engineered antibodies are described, e.g., in U.S. Patent Nos. 7,855,275 and 7,521,541.

In some instances, the antibodies are modified to contain additional nonproteinaceous moieties, including water soluble polymers. Exemplary polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer is attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

### II. METHODS OF IDENTIFYING ANTI-IDIOTYPE ANTIBODIES

In some instances, there is disclosed a method of identifying an anti-idiotype antibody or antigen-binding fragment that specifically binds to a target antibody or antigen-binding fragment thereof, such as by using hybridoma methods with an immunogen comprising the target antibody or fragment thereof (*see* for example Kohler and Milstein, Nature, 256: 495 (1975) and Sergeeva et al., Blood, 117(16): 4262-4272). The immunogen may comprise an immunogenicity-enhancing moiety fused to the target antibody or fragment. Such immunogenicity-enhancing moiety may have properties that include, without limitation, increasing solubility and half-life of the immunogen. Exemplary immunogenicity-enhancing moieties include Fc domains or fragments thereof. In some instances, the immunogenicity-enhancing moiety is an Fc domain (such as from IgG1, optionally human). In some instances, the immunogenicity-enhancing moiety is an Fc domain (such as from IgG1, optionally human) lacking all or a portion of the hinge region.

In some instances, there is disclosed a method of identifying an anti-idiotype antibody or antigen-binding fragment that specifically binds to a target antibody or antigen-binding fragment thereof, comprising: (a) introducing into a subject a soluble immunogen comprising an antigen-binding fragment of the target antibody fused to an immunogenicity-enhancing moiety; and (b) identifying an antibody from the subject that specifically binds to the target antibody or antigen-binding fragment thereof. In some instances, the antigen-binding fragment comprises the variable heavy chain region and/or variable light chain region of the target antibody. In some instances, the antigen-binding fragment is a single chain fragment. In some instances, the antigen-binding fragment is an scFv. In some instances, the antigen-binding fragment is within or included in the antigen-binding domain of the extracellular portion of a chimeric antigen receptor (CAR).

In some instances, the immunogenicity-enhancing moiety is an Fc domain or fragment thereof, which optionally is a human IgG1 Fc. In some instances, the immunogenicity-enhancing moiety is an Fc domain lacking the hinge region. In some instances, the immunogenicity-enhancing moiety comprises the amino acid sequence set forth in SEQ ID NO: 32.

In some instances, identifying the antibody comprises using hybridoma techniques to identify individual antibody clones produced by the subject and screening the clones for binding to the target antibody or antigen-binding fragment thereof. In some instances, identifying the antibody comprises: (i) isolating B cells from the spleen of the subject and fusing them with immortalized B cells to generate hybridomas; (ii) screening the hybridomas for production of antibodies that specifically bind the target antibody or the antigen-binding fragment thereof or a chimeric antigen receptor comprising the antigen-binding fragment; and (iii) sequencing an antibody from a hybridoma producing an antibody that specifically binds the target antibody or antigen-binding fragment, thereby identifying the anti-idiotype antibody. In some instances, screening a hybridoma comprises determining the binding affinity of the hybridoma antibody for a target molecule comprising the target antibody or an idiotope of the target antibody, such as an scFv or a CAR or fragment thereof comprising the variable heavy chain region and/or variable light chain region of the target antibody, or portions thereof, such as one or more of the CDRs of the VH and/or VL regions. In some instances, screening a hybridoma further comprises determining the binding affinity of the hybridoma antibody for a non-target molecule that does not comprise an idiotope of the target antibody, such as an Fc or fragment thereof or another antibody or fragment thereof that do not comprise the variable heavy chain region and/or variable light chain region of the target antibody, or portions thereof, such as one or more of the CDRs of the VH and/or VL regions, wherein binding of the hybridoma antibody to the target molecule but not the non-target molecule indicates that the hybridoma antibody specifically binds the target antibody.

In some of any such instances, the method comprises: (a) introducing into a subject a soluble immunogen comprising an scFv of the target antibody fused to an Fc domain or fragment thereof; and (b) identifying an antibody from the subject that specifically binds to a molecule comprising an idiotope of the target antibody, such as the immunogen. In some instances, the scFv is within or included in the antigen-binding domain of the extracellular portion of a chimeric antigen receptor (CAR). In some instances, the Fc domain or fragment thereof is a human IgG1 Fc or fragment thereof. In some instances, the Fc domain or fragment thereof is an Fc domain lacking the hinge region. In some instances, the Fc domain or fragment thereof comprises the amino acid sequence set forth in SEQ ID NO: 32. In some instances, identifying the antibody comprises using hybridoma techniques to identify individual antibody clones produced by the subject and screening the antibody clones for binding to a molecule comprising an idiotope of the target antibody, such as the immunogen. In some instances, identifying the antibody comprises: (i) isolating B cells from the spleen of the subject and fusing them with immortalized B cells to generate hybridomas; (ii) screening the hybridomas for production of antibodies that specifically bind a molecule comprising an idiotope of the target antibody, such as the immunogen; and (iii) sequencing an antibody from a hybridoma producing an antibody that specifically binds a molecule comprising an idiotope of the target antibody, such as the immunogen, thereby identifying the anti-idiotype antibody. In some instances, screening a hybridoma comprises determining the binding affinity of the hybridoma antibody for a target molecule comprising an idiotope of the target antibody, such as the immunogen, an scFv, or a CAR or fragment thereof comprising the variable heavy chain region and/or variable light chain region of the target antibody, or portions thereof, such as one or more of the CDRs of the VH and/or VL regions. In some instances, screening a hybridoma further comprises determining the binding affinity of the hybridoma antibody for a non-target molecule that does not comprise an idiotope of the target antibody, such as an Fc or fragment thereof or another antibody or fragment thereof that does not comprise the variable heavy chain region and/or variable light chain region of the target antibody, or portions thereof, such as one or more of the CDRs of the VH and/or VL regions, wherein binding of the hybridoma antibody to the target molecule but not the non-target molecule indicates that the hybridoma antibody specifically binds the target antibody.

In some of any such instances, the method comprises: (a) introducing into a subject a soluble immunogen comprising an scFv of the target antibody fused to an Fc domain or fragment thereof; and (b) identifying an antibody from the subject that (i) binds to a target molecule comprising an idiotope of the target antibody, such as the immunogen, an scFv, or a CAR or fragment thereof comprising the variable heavy chain region and/or variable light chain region of the target antibody, or portions thereof, such as one or more of the CDRs of the VH and/or VL regions; and (ii) does not bind to a non-target molecule that does not comprise an idiotope of the target antibody, such as an Fc or fragment thereof or another antibody or fragment thereof that does not comprise the variable heavy chain region and/or variable light chain region of the target antibody, or portions thereof, such as one or more of the CDRs of the VH and/or VL regions. In some instances, the scFv is within or included in the antigen-binding domain of the extracellular portion of a chimeric antigen receptor (CAR). In some instances, the Fc domain or fragment thereof is a human IgG1 Fc or fragment thereof. In some instances, the Fc domain or fragment thereof is an Fc domain lacking the hinge region. In some instances, the Fc domain or fragment thereof comprises the amino acid sequence set forth in SEQ ID NO: 32. In some instances, identifying the antibody comprises using hybridoma techniques to identify individual antibody clones produced by the subject and screening the antibody clones for binding to the target and non-target molecules. In some instances, identifying the antibody comprises: (i) isolating B cells from the spleen of the subject and fusing them with immortalized B cells to generate hybridomas; (ii) screening the hybridomas for production of antibodies that bind to the target molecule but not to the non-target molecule; and (iii) sequencing an antibody from a hybridoma producing an antibody that binds to the target molecule but not to the non-target molecule, thereby identifying the anti-idiotype antibody. In some instances, screening a hybridoma comprises determining the binding affinity of the hybridoma antibody for the target molecule and the non-target molecule.

In some of any such instances, the method comprises: (a) introducing into a subject a soluble immunogen comprising an scFv derived from the FMC63 antibody (such as an scFv comprising the amino acid sequence of SEQ ID NO: 34) fused to an Fc domain or fragment thereof; and (b) identifying an antibody from the subject that (i) binds to a target molecule comprising an idiotope of the FMC63 antibody, such as the immunogen, an scFv, or a CAR or fragment thereof comprising the variable heavy chain region and/or variable light chain region of the FMC63 antibody, or portions thereof, such as one or more of the CDRs of the VH and/or VL regions; and (ii) does not bind to a non-target molecule that does not comprise an idiotope of the FMC63 antibody, such as an Fc or fragment thereof or another antibody or fragment thereof that does not comprise the variable heavy chain region and/or variable light chain region of the FMC63 antibody, or portions thereof, such as one or more of the CDRs of the VH and/or VL regions. In some instances, the immunogen comprises the amino acid sequence set forth in SEQ ID NO:34 and SEQ ID NO:32, optionally separated by a linker, e.g. set forth in SEQ ID NO:33. In some instances, the immunogen comprises the amino acid sequence of SEQ ID NO: 35. In some instances, the antibody identified in (b) binds to the immunogen and does not bind to either an Fc domain or fragment thereof or a molecule comprising an scFv derived from the SJ25C1 antibody (such as an scFv comprising the amino acid sequence of SEQ ID NO: 28). In some instances, the antibody identified in (b) binds to a target molecule comprising the amino acid sequence of SEQ ID NO: 34 or 35 and does not bind to either a non-target molecule comprising the amino acid sequence of SEQ ID NO: 32 or a non-target molecule comprising the amino acid sequence of SEQ ID NO: 28.

In some of any such instances, the method comprises: (a) introducing into a subject a soluble immunogen comprising an scFv derived from the SJ25C1 antibody (such as an scFv comprising the amino acid sequence of SEQ ID NO: 28) fused to an Fc domain or fragment thereof; and (b) identifying an antibody from the subject that (i) binds to a target molecule comprising an idiotope of the SJ25C1 antibody, such as the immunogen, an scFv, or a CAR or fragment thereof comprising the variable heavy chain region and/or variable light chain region of the SJ25C1 antibody, or portions thereof, such as one or more of the CDRs of the VH and/or VL regions; and (ii) does not bind to a non-target molecule that does not comprise an idiotope of the SJ25C1 antibody, such as an Fc or fragment thereof or another antibody or fragment thereof that does not comprise the variable heavy chain region and/or variable light chain region of the SJ25C1 antibody, or portions thereof, such as one or more of the CDRs of the VH and/or VL regions. In some instances, the immunogen comprises the amino acid sequence set forth in SEQ ID NO:28 and SEQ ID NO:32, optionally separated by a linker, e.g. set forth in SEQ ID NO:33. In some instances, the antibody identified in (b) binds to the immunogen and does not bind to either an Fc domain or fragment thereof or a molecule comprising an scFv derived from the FMC63 antibody (such as an scFv comprising the amino acid sequence of SEQ ID NO: 34). In some instances, the antibody identified in (b) binds to a target molecule comprising the amino acid sequence of SEQ ID NO: 28 and does not bind to either a non-target molecule comprising the amino acid sequence of SEQ ID NO: 32 or a non-target molecule comprising the amino acid sequence of SEQ ID NO: 34.

In some instances, identifying the antibody comprises using hybridoma techniques to identify individual antibody clones produced by the subject and screening the antibody clones for binding to target and non-target molecules. In some instances, identifying the antibody comprises: (i) isolating B cells from the spleen of the subject and fusing them with immortalized B cells to generate hybridomas; (ii) screening the hybridomas for production of antibodies that bind to the target molecule but not to the non-target molecule; and (iii) sequencing an antibody from a hybridoma producing an antibody that binds to the target molecule but not to the non-target molecule, thereby identifying the anti-idiotype antibody. In some instances, screening a hybridoma comprises determining the binding affinity of the hybridoma antibody for the target molecule and the non-target molecule.

In an exemplary instance, the identification of anti-idiotype antibodies recognizing the scFv portion of an exemplary anti-CD19 chimeric antigen receptor (CAR) containing an anti-CD19 scFv derived from SJ25C1 may be generated using an immunogen comprising the sequence set forth as follows:
EVKLQQSGAELVRPGSSVKISCKASGYAFSSYWMNWVKQRPGQGLEWIGQIYPGDG DTNYNGKFKGQATLTADKSSSTAYMQLSGLTSEDSAVYFCARKTISSVVDFYFDYW GQGTTVTVSSGGGGSGGGGSGGGGSDIELTQSPKFMSTSVGDRVSVTCKASQNVGT NVAWYQQKPGQSPKPLIYSATYRNSGVPDRFTGSGSGTDFTLTITNVQSKDLADYFC QQYNRYPYTSGGGTKLEIKR (SEQ ID NO: 28). In some instances, the immunogen may further contain an Fc domain or fragment thereof, e.g. as set forth in SEQ ID NO:32.

In an exemplary instance, the identification of anti-idiotype antibodies recognizing the scFv portion of an exemplary anti-CD19 chimeric antigen receptor (CAR) containing an anti-CD19 scFv derived from FMC63 may be generated using an immunogen comprising the sequence set forth as follows:

DIQMTQTTSSLSASLGDRVTISCRASQDISKYLNWYQQKPDGTVKLLIYHTSRLHSGV
PSRFSGSGSGTDYSLTISNLEQEDIATYFCQQGNTLPYTFGGGTKLEITGSTSGSGKPG
SGEGSTKGEVKLQESGPGLVAPSQSLSVTCTVSGVSLPDYGVSWIRQPPRKGLEWLG
VIWGSETTYYNSALKSRLTIIKDNSKSQVFLKMNSLQTDDTAIYYCAKHYYYGGSYA
MDYWGQGTSVTVSS (SEQ ID NO:34).

In some instances, the immunogen may further contain an Fc domain or fragment thereof, e.g. as set forth in SEQ ID NO:32. In some instances, the identification of anti-idiotype antibodies recognizing the scFv portion of an exemplary anti-CD19 chimeric antigen receptor (CAR) containing an anti-CD19 scFv derived from FMC63 may be generated using an immunogen comprising the sequence set forth as follows:

DIQMTQTTSSLSASLGDRVTISCRASQDISKYLNWYQQKPDGTVKLLIYHTSRLHSGVPSRFS
GSGSGTDYSLTISNLEQEDIATYFCQQGNTLPYTFGGGTKLEITGSTSGSGKPGSGEGSTKGEV
KLQESGPGLVAPSQSLSVTCTVSGVSLPDYGVSWIRQPPRKGLEWLGVIWGSETTYYNSALK
SRLTIIKDNSKSQVFLKMNSLQTDDTAIYYCAKHYYYGGSYAMDYWGQGTSVTVSSPCPAPE
LLGGPSVFLFPPKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQVYTLPPSREE
MTKNQVSLTCLVKGFYPSDIAVEWESNGQPENNYKTTPPVLDSDGSFFLYSKLTVDKSRWQ
QGNVFSCSVMHEALHNHYTQKSLSLSPGK (SEQ ID NO: 35).

### III. CHIMERIC ANTIGEN RECEPTORS (CARS) AND GENETICALLY ENGINEERED CELLS

In some instances, the disclosed anti-idiotypic antibodies specifically bind to an antigen-binding portion of a chimeric antigen receptor (CAR), such as an anti-CD 19 CAR containing an antigen-binding portion derived from antibody SJ25C1 or FMC63. In some instances, the disclosed anti-idiotype antibodies bind to such CARs expressed on a cell, such as cells used in connection with adoptive cell therapy. In some instances, the cells include one or more nucleic acids introduced via genetic engineering, and thereby express the recombinant or genetically engineered CAR products of such nucleic acids. In some instances, chimeric receptors when genetically engineered into immune cells can modulate T cell activity, and, in some cases, can modulate T cell differentiation or homeostasis, thereby resulting in genetically engineered cells with improved longevity, survival and/or persistence *in vivo,* such as for use in adoptive cell therapy methods. In some instances, the disclosed anti-idiotypic antibodies can be used in methods to modulate one or more of these activities, including to activate, stimulate and/or expand engineered cells expressing the target CAR.

In some instances, the cells include one or more nucleic acids introduced via genetic engineering in accord with the disclosed methods, and thereby express recombinant or genetically engineered products of such nucleic acids. In some instances, the nucleic acids are heterologous, i.e., normally not present in a cell or sample obtained from the cell, such as one obtained from another organism or cell, which for example, is not ordinarily found in the cell being engineered and/or an organism from which such cell is derived. In some instances, the nucleic acids are not naturally occurring, such as a nucleic acid not found in nature, including one comprising chimeric combinations of nucleic acids encoding various domains from multiple different cell types. In particular instances, the nucleic acids contain a gene that encodes a CAR.

In some instances, the disclosed methods may be carried out simultaneously, sequentially or concurrently with one or more processing steps for manufacturing or preparing genetically engineered cells. The processing steps of the methods may include any one or more of a number of cell processing steps, alone or in combination. In particular instances, the processing steps include transduction or transfection of the cells with one or more nucleic acids, e.g., a heterologous polynucleotide comprising a gene encoding a recombinant receptor. In certain instances, cells are transduced with viral vector particles containing a retroviral vector, such as one encoding a recombinant product for expression in the cells. In certain instances, the cells are transfected with one or more non-viral nucleic acids, e.g., an episomal plasmid or a transposon. The methods may further and/or alternatively include other processing steps, such as steps for the isolation, separation, selection, washing, suspension, dilution, concentration, and/or formulation of the cells. In some cases, the methods also can include an ex vivo step for cultivation, stimulation or expansion of cells (e.g., stimulation of the cells, for example, to induce their proliferation and/or activation), which, in some cases, can be carried out in accord with the disclosed methods. In some instances, the methods include isolating cells from the subject, preparing, processing, culturing, and/or engineering them, and re-introducing them into the same subject, before or after cryopreservation.

In some instances, the method includes processing steps carried out in an order in which: cells, e.g., primary cells, are first isolated, such as selected or separated, from a biological sample; selected cells are incubated with viral vector particles for transduction; and transduced cells are formulated in a composition. In some cases, transduced cells are activated, expanded or propagated ex vivo, such as by stimulation in the presence of a stimulation reagent, such as in accord with the disclosed methods. In some instances, the method can include one or more processing steps from among washing, suspending, diluting and/or concentrating cells, which can occur prior to, during, or simultaneous with or subsequent to one or more of the isolation, such as separation or selection, transduction, stimulation, and/or formulation steps.

In particular instances, the cells to be transfected or transduced are not isolated, selected, or enriched prior to contact with the one or more nucleic acids. In some instances, the cells are not selected prior to contacting the cells with the one or more nucleic acids. In some instances, the cells to be transfected or transduced are not enriched prior to contacting the cells with the one or more nucleic acids.

In some instances, one or more of the cell processing steps in connection with preparing, processing and/or incubating cells in connection with the disclosed method, including in connection with preparing a composition containing genetically engineered cells, can be carried out in the internal cavity of a centrifugal chamber, such as a substantially rigid chamber that is generally cylindrical in shape and rotatable around an axis of rotation, which can provide certain advantages compared to other available methods. In some instances, all processing steps are carried out in the same centrifugal chamber. In some instances, one or more processing steps are carried out in different centrifugal chambers, such as multiple centrifugal chambers of the same type. Such methods include any of those as described in International Publication Number WO2016/073602.

Exemplary centrifugal chambers include those produced and sold by Biosafe SA, including those for use with the Sepax^{®} and Sepax^{®} 2 system, including an A-200/F and A-200 centrifugal chambers and various kits for use with such systems. Exemplary chambers, systems, and processing instrumentation and cabinets are described, for example, in US Patent No. 6,123,655, US Patent No. 6,733,433 and Published U.S. Patent Application, Publication No.: US 2008/0171951, and published international patent application, publication no. WO 00/38762. Depending on the particular process (e.g., dilution, wash, transduction, formulation), it is within the level of a skilled artisan to choose a particular kit that is appropriate for the process. Exemplary kits for use with such systems include, but are not limited to, single-use kits sold by BioSafe SA under product names CS-430.1, CS-490.1, CS-600.1 or CS-900.2.

In some instances, the system is included with and/or placed into association with other instrumentation, including instrumentation to operate, automate, control and/or monitor aspects of the various processing steps performed in the system. This instrumentation in some instances is contained within a cabinet. In some instances, the instrumentation includes a cabinet, which includes a housing containing control circuitry, a centrifuge, a cover, motors, pumps, sensors, displays, and a user interface. An exemplary device is described in US Patent No. 6,123,655, US Patent No. 6,733,433 and US 2008/0171951.

In some instances, the system comprises a series of containers, e.g., bags, tubing, stopcocks, clamps, connectors, and a centrifuge chamber. In some instances, the containers, such as bags, include one or more containers, such as bags, containing the cells to be transduced or transfected and the vector particles, e.g., viral vector particles or non-viral plasmids, in the same container or separate containers, such as the same bag or separate bags. In some instances, the system further includes one or more containers, such as bags, containing medium, such as diluent and/or wash solution, which is pulled into the chamber and/or other components to dilute, resuspend, and/or wash components and/or compositions during the methods. The containers can be connected at one or more positions in the system, such as at a position corresponding to an input line, diluent line, wash line, waste line and/or output line.

In some instances, the system, such as a closed system, is sterile. In some instances, all connections of components of the system, such as between tubing line and a container via a connector, are made under sterile conditions. In some instances, connections are made under laminar flow. In some instances, connections are made using a sterile connection device that produces sterile connections, such as sterile welds, between a tubing and a container. In some instances, a sterile connection device effects connection under thermal condition high enough to maintain sterility, such as temperatures of at least 200 °C, such as at least 260 °C or 300 ° C.

In some instances, the system may be disposable, such as a single-use kit. In some instances, a single-use kit can be utilized in a plurality of cycles of a process or processes, such as at least 2, 3, 4, 5 or more times, for example, in processes that occur in a continuous or a semi-continuous manner. In some instances, the system, such as a single-use kit, is employed for processing of cells from a single patient. In aspects of the methods, the processes need not be performed in the same closed system, such as in the same centrifugal chamber, but can be performed under a different closed system, such as in a different centrifugal chamber; in some instances, such different centrifugal chambers are at the respective points in the methods placed in association with the same system, such as placed in association with the same centrifuge. In some instances, all processing steps are performed in a closed system, in which all or a subset of each one or more processing step is performed in the same or a different centrifugal chamber.

### A. Target Chimeric Antigen Receptors (CARs)

In some instances, the disclosed anti-idiotypic antibodies specifically bind the extracellular domain of a target CAR that contains an antigen binding domain of an antibody or antibody fragment that provides specificity for a desired antigen (e.g., tumor antigen) and which is operably linked or connected to an intracellular signaling domain. In some instances, the antigen binding domain includes the antibody SJ25C1 or an antibody fragment of portion derived from SJ25C1. In some instances, the antigen binding domain includes the antibody FMC63 or an antibody fragment of portion derived from FMC63. In some instances, the intracellular signaling domain is an activating intracellular domain portion, such as a T cell activating domain, providing a primary activation signal. In some instances, the intracellular signaling domain contains or additionally contains a costimulatory signaling domain to facilitate effector functions.

In some instances, engineered cells, such as T cells, are disclosed that express a CAR with specificity for a particular antigen (or marker or ligand), such as an antigen expressed on the surface of a particular cell type. In some instances, the antigen is a polypeptide. In some instances, it is a carbohydrate or other molecule. In some instances, the antigen is selectively expressed or overexpressed on cells of the disease or condition, e.g., the tumor or pathogenic cells, as compared to normal or non-targeted cells or tissues. In other instances, the antigen is expressed on normal cells and/or is expressed on the engineered cells.

In particular instances, the recombinant receptor, such as a chimeric receptor, contains an intracellular signaling region, which includes a cytoplasmic signaling domain (also interchangeably called an intracellular signaling domain), such as a cytoplasmic (intracellular) region capable of inducing a primary activation signal in a T cell, for example, a cytoplasmic signaling domain of a T cell receptor (TCR) component (e.g. a cytoplasmic signaling domain of a zeta chain of a CD3-zeta (CD3ζ) chain or a functional variant or signaling portion thereof) that comprises an immunoreceptor tyrosine-based activation motif (ITAM).

In some instances, the chimeric receptor further contains an extracellular ligand-binding domain that specifically binds to a ligand (e.g. antigen). In some instances, the chimeric receptor is a CAR that contains an extracellular antigen-recognition domain that specifically binds to an antigen. In some instances, the ligand, such as an antigen, is a protein expressed on the surface of cells. In some instances, the CAR is a TCR-like CAR and the antigen is a processed peptide antigen, such as a peptide antigen of an intracellular protein, which, like a TCR, is recognized on the cell surface in the context of a major histocompatibility complex (MHC) molecule.

Exemplary antigen receptors, including CARs, and methods for engineering and introducing such receptors into cells, include those described, for example, in international patent application publication numbers WO200014257, WO2013126726, WO2012/129514, WO2014031687, WO2013/166321, WO2013/071154, WO2013/123061 U.S. patent application publication numbers US2002131960, US2013287748, US20130149337, U.S. Patent Nos.: 6,451,995, 7,446,190, 8,252,592, , 8,339,645, 8,398,282, 7,446,179, 6,410,319, 7,070,995, 7,265,209, 7,354,762, 7,446,191, 8,324,353, and 8,479,118, and European patent application number EP2537416,and/or those described by Sadelain et al., Cancer Discov. 2013 April; 3(4): 388-398; Davila et al. (2013) PLoS ONE 8(4): e61338; Turtle et al., Curr. Opin. Immunol., 2012 October; 24(5): 633-39; Wu et al., Cancer, 2012 March 18(2): 160-75. In some aspects, the antigen receptors include a CAR as described in U.S. Patent No.: 7,446,190, and those described in International Patent Application Publication No.: WO/2014055668 A1. Examples of the CARs include CARs as disclosed in any of the aforementioned publications, such as WO2014031687, U.S. Patent No. 8,339,645, U.S. Patent No. 7,446,179, US 2013/0149337, U.S. Patent No.: 7,446,190, U.S. Patent No.: 8,389,282, Kochenderfer et al., 2013, Nature Reviews Clinical Oncology, 10, 267-276 (2013); Wang et al. (2012) J. Immunother. 35(9): 689-701; and Brentjens et al., Sci Transl Med. 2013 5(177). See also WO2014031687, U.S. Patent No. 8,339,645, U.S. Patent No. 7,446,179, US 2013/0149337, U.S. Patent No.: 7,446,190, and U.S. Patent No.: 8,389,282.

In some instances, the CAR is constructed with a specificity for a particular antigen (or marker or ligand), such as an antigen expressed in a particular cell type to be targeted by adoptive therapy, e.g., a cancer marker, and/or an antigen intended to induce a dampening response, such as an antigen expressed on a normal or non-diseased cell type. Thus, the CAR typically includes in its extracellular portion one or more antigen binding molecules, such as one or more antigen-binding fragment, domain, or portion, or one or more antibody variable domains, and/or antibody molecules. In some instances, the CAR includes an antigen-binding portion or portions of an antibody molecule, such as a single-chain antibody fragment (scFv) derived from the variable heavy (VH) and variable light (VL) chains of a monoclonal antibody (mAb).

In some instances, the antibody or antigen-binding portion thereof is expressed on cells as part of a CAR. Generally, a CAR containing an antibody or antigen-binding fragment that exhibits TCR-like specificity directed against peptide-MHC complexes also may be referred to as a TCR-like CAR. In some instances, the extracellular antigen binding domain specific for an MHC-peptide complex of a TCR-like CAR is linked to one or more intracellular signaling components, in some aspects via linkers and/or transmembrane domain(s). In some instances, such molecules can typically mimic or approximate a signal through a natural antigen receptor, such as a TCR, and, optionally, a signal through such a receptor in combination with a costimulatory receptor.

In some instances, the recombinant receptor, such as a chimeric receptor (e.g. CAR), includes a ligand-binding domain that binds, such as specifically binds, to an antigen (or a ligand). Among the antigens targeted by the chimeric receptors are those expressed in the context of a disease, condition, or cell type to be targeted via the adoptive cell therapy. Among the diseases and conditions are proliferative, neoplastic, and malignant diseases and disorders, including cancers and tumors, including hematologic cancers, cancers of the immune system, such as lymphomas, leukemias, and/or myelomas, such as B, T, and myeloid leukemias, lymphomas, and multiple myelomas.

In some instances, the antigen (or a ligand) is a polypeptide. In some instances, it is a carbohydrate or other molecule. In some instances, the antigen (or a ligand) is selectively expressed or overexpressed on cells of the disease or condition, e.g., the tumor or pathogenic cells, as compared to normal or non-targeted cells or tissues. In other instances, the antigen is expressed on normal cells and/or is expressed on the engineered cells.

In some instances, the CAR contains an antibody or an antigen-binding fragment *(e.g.* scFv) that specifically recognizes an antigen, such as an intact antigen, expressed on the surface of a cell.

In some instances, the antigen (or a ligand) is a tumor antigen or cancer marker. In some instances, the antigen (or a ligand) is αvβ6 integrin (avb6 integrin), B cell maturation antigen (BCMA), B7-H6, carbonic anhydrase 9 (CA9, also known as CAIX or G250), a cancer-testis antigen, cancer/testis antigen 1B (CTAG, also known as NY-ESO-1 and LAGE-2), carcinoembryonic antigen (CEA), a cyclin, cyclin A2, C-C Motif Chemokine Ligand 1 (CCL-1), CD19, CD20, CD22, CD23, CD24, CD30, CD33, CD38, CD44, CD44v6, CD44v7/8, CD123, CD138, CD 171, epidermal growth factor protein (EGFR), truncated epidermal growth factor protein (tEGFR), type III epidermal growth factor receptor mutation (EGFR vIII), epithelial glycoprotein 2 (EPG-2), epithelial glycoprotein 40 (EPG-40), ephrinB2, ephrine receptor A2 (EPHa2), estrogen receptor, Fc receptor like 5 (FCRL5; also known as Fc receptor homolog 5 or FCRH5), fetal acetylcholine receptor (fetal AchR), a folate binding protein (FBP), folate receptor alpha, fetal acetylcholine receptor, ganglioside GD2, O-acetylated GD2 (OGD2), ganglioside GD3, glycoprotein 100 (gp100), Her2/neu (receptor tyrosine kinase erbB2), Her3 (erb-B3), Her4 (erb-B4), erbB dimers, Human high molecular weight-melanoma-associated antigen (HMW-MAA), hepatitis B surface antigen, Human leukocyte antigen A1 (HLA-AI), Human leukocyte antigen A2 (HLA-A2), IL-22 receptor alpha(IL-22Ra), IL-13 receptor alpha 2 (IL-13Ra2), kinase insert domain receptor (kdr), kappa light chain, L1 cell adhesion molecule (L1CAM), CE7 epitope of L1-CAM, Leucine Rich Repeat Containing 8 Family Member A (LRRC8A), Lewis Y, Melanoma-associated antigen (MAGE)-A1, MAGE-A3, MAGE-A6, mesothelin, c-Met, murine cytomegalovirus (CMV), mucin 1 (MUC1), MUC16, natural killer group 2 member D (NKG2D) ligands, melan A (MART-1), neural cell adhesion molecule (NCAM), oncofetal antigen, Preferentially expressed antigen of melanoma (PRAME), progesterone receptor, a prostate specific antigen, prostate stem cell antigen (PSCA), prostate specific membrane antigen (PSMA), Receptor Tyrosine Kinase Like Orphan Receptor 1 (ROR1), survivin, Trophoblast glycoprotein (TPBG also known as 5T4), tumor-associated glycoprotein 72 (TAG72), vascular endothelial growth factor receptor (VEGFR), vascular endothelial growth factor receptor 2 (VEGFR2), Wilms Tumor 1 (WT-1), a pathogen-specific antigen, an antigen associated with a universal tag, and/or biotinylated molecules, and/or molecules expressed by and/or characteristic of or specific for HIV, HCV, HBV, HPV, and/or other pathogens and/or oncogenic versions thereof. Antigens targeted by the receptors in some instances include antigens associated with a B cell malignancy, such as any of a number of known B cell markers. In some instances, the antigen targeted by the receptor is CD20, CD19, CD22, ROR1, CD45, CD21, CD5, CD33, Igkappa, Iglambda, CD79a, CD79b or CD30.

In some instances, the antigen is a pathogen-specific antigen. In some instances, the antigen is a viral antigen (such as a viral antigen from HIV, HCV, HBV, etc.), bacterial antigens, and/or parasitic antigens.

Antigens targeted by the receptors in some instances include antigens associated with a B cell malignancy, such as any of a number of known B cell marker. In some instances, the antigen targeted by the receptor is CD20, CD19, CD22, ROR1, CD45, CD21, CD5, CD33, Igkappa, Iglambda, CD79a, CD79b or CD30. In some instances, the antigen is CD19 and is specifically bound by an anti-CD19 antibody, such as SJ25C1 or an antigen-binding fragment derived from SJ25C1 or FMC63 or an antigen-binding fragment derived from FMC63.

In some instances, the antigen-binding proteins, antibodies and antigen binding fragments thereof specifically recognize an antigen of a full-length antibody. In some instances, the heavy and light chains of an antibody can be full-length or can be an antigen-binding portion (a Fab, F(ab')2, Fv or a single chain Fv fragment (scFv)). In other instances, the antibody heavy chain constant region is chosen from, e.g., IgG1, IgG2, IgG3, IgG4, IgM, IgA1, IgA2, IgD, and IgE, particularly chosen from, e.g., IgG1, IgG2, IgG3, and IgG4, more particularly, IgG1 (e.g., human IgG1). In another instance, the antibody light chain constant region is chosen from, e.g., kappa or lambda, particularly kappa.

Among the disclosed antibodies are antibody fragments. An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')2; diabodies; linear antibodies; variable heavy chain (VH) regions, single-chain antibody molecules such as scFvs and single-domain VH single antibodies; and multispecific antibodies formed from antibody fragments. In particular instances, the antibodies are single-chain antibody fragments comprising a variable heavy chain region and/or a variable light chain region, such as scFvs.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three CDRs. (See, e.g., Kindt et al. Kuby Immunology, 6th ed., W.H. Freeman and Co., page 91 (2007). A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. See, e.g., Portolano et al., J. Immunol. 150:880-887 (1993); Clarkson et al., Nature 352:624-628 (1991).

Single-domain antibodies are antibody fragments comprising all or a portion of the heavy chain variable domain or all or a portion of the light chain variable domain of an antibody. In certain instances, a single-domain antibody is a human single-domain antibody. In some instances, the CAR comprises an antibody heavy chain domain that specifically binds the antigen, such as a cancer marker or cell surface antigen of a cell or disease to be targeted, such as a tumor cell or a cancer cell, such as any of the target antigens described herein or known in the art.

Antibody fragments can be made by various techniques, including but not limited to proteolytic digestion of an intact antibody as well as production by recombinant host cells. In some instances, the antibodies are recombinantly-produced fragments, such as fragments comprising arrangements that do not occur naturally, such as those with two or more antibody regions or chains joined by synthetic linkers, e.g., peptide linkers, and/or that are may not be produced by enzyme digestion of a naturally-occurring intact antibody. In some instances, the antibody fragments are scFvs.

A "humanized" antibody is an antibody in which all or substantially all CDR amino acid residues are derived from non-human CDRs and all or substantially all FR amino acid residues are derived from human FRs. A humanized antibody optionally may include at least a portion of an antibody constant region derived from a human antibody. A "humanized form" of a non-human antibody, refers to a variant of the non-human antibody that has undergone humanization, typically to reduce immunogenicity to humans, while retaining the specificity and affinity of the parental non-human antibody. In some instances, some FR residues in a humanized antibody are substituted with corresponding residues from a non-human antibody (e.g., the antibody from which the CDR residues are derived), e.g., to restore or improve antibody specificity or affinity.

The chimeric receptors, such as CARs, generally include an extracellular antigen binding domain, such as a portion of an antibody molecule (e.g. SJ25C1 or FMC63), generally a variable heavy (VH) chain region and/or variable light (VL) chain region of the antibody, e.g., an scFv antibody fragment.

In some instances, the chimeric antigen receptor includes an extracellular portion containing an antibody or antibody fragment. In some aspects, the chimeric antigen receptor includes an extracellular portion containing the antibody or fragment and an intracellular signaling domain. In some instances, the antibody or fragment includes an scFv. In some instances, the scFv is derived from SJ25C1 and comprises the sequence of amino acids set forth in SEQ ID NO: 28. In some instances, the scFv is derived from FMC63 and comprises the sequence of amino acids set forth in SEQ ID NO: 34.

In some instances, the recombinant receptor such as the CAR, such as the antibody portion thereof, further includes a spacer, which may be or include at least a portion of an immunoglobulin constant region or variant or modified version thereof, such as a hinge region, e.g., an IgG4 hinge region, and/or a CH1/CL and/or Fc region. In some instances, the constant region or portion is of a human IgG, such as IgG4 or IgG1. In some aspects, the portion of the constant region serves as a spacer region between the antigen-recognition component, e.g., scFv, and transmembrane domain. The spacer can be of a length that provides for increased responsiveness of the cell following antigen binding, as compared to in the absence of the spacer. In some examples, the spacer is at or about 12 amino acids in length or is no more than 12 amino acids in length. Exemplary spacers include those having at least about 10 to 229 amino acids, about 10 to 200 amino acids, about 10 to 175 amino acids, about 10 to 150 amino acids, about 10 to 125 amino acids, about 10 to 100 amino acids, about 10 to 75 amino acids, about 10 to 50 amino acids, about 10 to 40 amino acids, about 10 to 30 amino acids, about 10 to 20 amino acids, or about 10 to 15 amino acids, and including any integer between the endpoints of any of the listed ranges. In some instances, a spacer region has about 12 amino acids or less, about 119 amino acids or less, or about 229 amino acids or less. Exemplary spacers include IgG4 hinge alone, IgG4 hinge linked to CH2 and CH3 domains, or IgG4 hinge linked to the CH3 domain. In some instances, the spacer has the sequence set forth in SEQ ID NO: 150, and is encoded by the sequence set forth in SEQ ID NO: 151. In some instances, the spacer has the sequence set forth in SEQ ID NO: 152. In some instances, the spacer has the sequence set forth in SEQ ID NO: 153. Exemplary spacers include, but are not limited to, those described in Hudecek et al. (2013) Clin. Cancer Res., 19: 3153, international patent application publication number WO2014031687, U.S. Patent No. 8,822,647 or published app. No. US2014/0271635.

In some instances, the constant region or portion is of IgD. In some instances, the spacer has the sequence set forth in SEQ ID NO: 154. In some instances, the spacer has a sequence of amino acids that exhibits at least 85%, 86%, 87%, 88%, 89%, 90%, 91%, 92%, 93%, 94%, 95%, 96%, 97%, 98%, 99% or more sequence identity to any of SEQ ID NOS: 1, 3, 4 and 5.

In some instances, the antigen receptor comprises an intracellular domain linked directly or indirectly to the extracellular domain. In some instances, the chimeric antigen receptor includes a transmembrane domain linking the extracellular domain and the intracellular signaling domain. In some instances, the transmembrane domain is fused to the extracellular domain. In some instances, the intracellular signaling domain comprises an ITAM. For example, in some aspects, the antigen recognition domain (e.g. extracellular domain) generally is linked to one or more intracellular signaling components, such as signaling components that mimic activation through an antigen receptor complex, such as a TCR complex, in the case of a CAR, and/or signal via another cell surface receptor. In some instances, the chimeric receptor comprises a transmembrane domain linked or fused between the extracellular domain (e.g. scFv) and intracellular signaling domain. Thus, in some instances, the antigen-binding component (e.g., antibody) is linked to one or more transmembrane and intracellular signaling domains.

In one instance, a transmembrane domain that naturally is associated with one of the domains in the receptor, e.g., CAR, is used. In some instances, the transmembrane domain is selected or modified by amino acid substitution to avoid binding of such domains to the transmembrane domains of the same or different surface membrane proteins to minimize interactions with other members of the receptor complex.

The transmembrane domain in some instances is derived either from a natural or from a synthetic source. Where the source is natural, the domain in some aspects is derived from any membrane-bound or transmembrane protein. Transmembrane regions include those derived from (i.e. comprise at least the transmembrane region(s) of) the alpha, beta or zeta chain of the T-cell receptor, CD28, CD3 epsilon, CD45, CD4, CD5, CD8, CD9, CD 16, CD22, CD33, CD37, CD64, CD80, CD86, CD 134, CD137, CD 154. Alternatively the transmembrane domain in some instances is synthetic. In some aspects, the synthetic transmembrane domain comprises predominantly hydrophobic residues such as leucine and valine. In some aspects, a triplet of phenylalanine, tryptophan and valine will be found at each end of a synthetic transmembrane domain. In some instances, the linkage is by linkers, spacers, and/or transmembrane domain(s). In some aspects, the transmembrane domain contains a transmembrane portion of CD28.

In some instances, the extracellular domain and transmembrane domain can be linked directly or indirectly. In some instances, the extracellular domain and transmembrane are linked by a spacer, such as any described herein. In some instances, the receptor contains extracellular portion of the molecule from which the transmembrane domain is derived, such as a CD28 extracellular portion.

Among the intracellular signaling domains are those that mimic or approximate a signal through a natural antigen receptor, a signal through such a receptor in combination with a costimulatory receptor, and/or a signal through a costimulatory receptor alone. In some instances, a short oligo- or polypeptide linker, for example, a linker of between 2 and 10 amino acids in length, such as one containing glycines and serines, e.g., glycine-serine doublet, is present and forms a linkage between the transmembrane domain and the cytoplasmic signaling domain of the CAR.

T cell activation is in some aspects described as being mediated by two classes of cytoplasmic signaling sequences: those that initiate antigen-dependent primary activation through the TCR (primary cytoplasmic signaling sequences), and those that act in an antigen-independent manner to provide a secondary or co-stimulatory signal (secondary cytoplasmic signaling sequences). In some aspects, the CAR includes one or both of such signaling components.

The receptor, e.g., the CAR, generally includes at least one intracellular signaling component or components. In some aspects, the CAR includes a primary cytoplasmic signaling sequence that regulates primary activation of the TCR complex. Primary cytoplasmic signaling sequences that act in a stimulatory manner may contain signaling motifs which are known as immunoreceptor tyrosine-based activation motifs or ITAMs. Examples of ITAM containing primary cytoplasmic signaling sequences include those derived from CD3 zeta chain, FcR gamma, CD3 gamma, CD3 delta and CD3 epsilon. In some instances, cytoplasmic signaling molecule(s) in the CAR contain(s) a cytoplasmic signaling domain, portion thereof, or sequence derived from CD3 zeta.

In some instances, the receptor includes an intracellular component of a TCR complex, such as a TCR CD3 chain that mediates T-cell activation and cytotoxicity, e.g., CD3 zeta chain. Thus, in some aspects, the antigen-binding portion is linked to one or more cell signaling modules. In some instances, cell signaling modules include CD3 transmembrane domain, CD3 intracellular signaling domains, and/or other CD transmembrane domains. In some instances, the receptor, e.g., CAR, further includes a portion of one or more additional molecules such as Fc receptor γ, CD8, CD4, CD25, or CD16. For example, in some aspects, the CAR or other chimeric receptor includes a chimeric molecule between CD3-zeta (CD3-ζ) or Fc receptor γ and CD8, CD4, CD25 or CD16.

In some instances, upon ligation of the CAR or other chimeric receptor, the cytoplasmic domain or intracellular signaling domain of the receptor activates at least one of the normal effector functions or responses of the immune cell, e.g., T cell engineered to express the CAR. For example, in some contexts, the CAR induces a function of a T cell such as cytolytic activity or T-helper activity, such as secretion of cytokines or other factors. In some instances, a truncated portion of an intracellular signaling domain of an antigen receptor component or costimulatory molecule is used in place of an intact immunostimulatory chain, for example, if it transduces the effector function signal. In some instances, the intracellular signaling domain or domains include the cytoplasmic sequences of the T cell receptor (TCR), and in some aspects also those of co-receptors that in the natural context act in concert with such receptors to initiate signal transduction following antigen receptor engagement, and/or any derivative or variant of such molecules, and/or any synthetic sequence that has the same functional capability.

In the context of a natural TCR, full activation generally requires not only signaling through the TCR, but also a costimulatory signal. Thus, in some instances, to promote full activation, a component for generating secondary or co-stimulatory signal is also included in the CAR. In other instances, the CAR does not include a component for generating a costimulatory signal. In some aspects, an additional CAR is expressed in the same cell and provides the component for generating the secondary or costimulatory signal.

T cell activation is in some aspects described as being mediated by two classes of cytoplasmic signaling sequences: those that initiate antigen-dependent primary activation through the TCR (primary cytoplasmic signaling sequences), and those that act in an antigen-independent manner to provide a secondary or co-stimulatory signal (secondary cytoplasmic signaling sequences). In some aspects, the CAR includes one or both of such signaling components.

In some instances, the chimeric antigen receptor contains an intracellular domain of a T cell costimulatory molecule. In some instances, the CAR includes a signaling domain and/or transmembrane portion of a costimulatory receptor, such as CD28, 4-1BB, OX40, DAP10, and ICOS. In some aspects, the same CAR includes both the activating and costimulatory components. In some instances, the chimeric antigen receptor contains an intracellular domain derived from a T cell costimulatory molecule or a functional variant thereof, such as between the transmembrane domain and intracellular signaling domain. In some aspects, the T cell costimulatory molecule is CD28 or 41BB.

In some instances, the activating domain is included within one CAR, whereas the costimulatory component is provided by another CAR recognizing another antigen. In some instances, the CARs include activating or stimulatory CARs, costimulatory CARs, both expressed on the same cell (see WO2014/055668). In some aspects, the cells include one or more stimulatory or activating CAR and/or a costimulatory CAR. In some instances, the cells further include inhibitory CARs (iCARs, see Fedorov et al., Sci. Transl. Medicine, 5(215) (December, 2013), such as a CAR recognizing an antigen other than the one associated with and/or specific for the disease or condition whereby an activating signal delivered through the disease-targeting CAR is diminished or inhibited by binding of the inhibitory CAR to its ligand, e.g., to reduce off-target effects.

In certain instances, the intracellular signaling domain comprises a CD28 transmembrane and signaling domain linked to a CD3 (e.g., CD3-zeta) intracellular domain. In some instances, the intracellular signaling domain comprises a chimeric CD28 and CD137 (4-1BB, TNFRSF9) co-stimulatory domains, linked to a CD3 zeta intracellular domain.

In some instances, the CAR encompasses one or more, e.g., two or more, costimulatory domains and an activation domain, e.g., primary activation domain, in the cytoplasmic portion. Exemplary CARs include intracellular components of CD3-zeta, CD28, and 4-1BB.

In some instances, the antigen receptor further includes a marker and/or cells expressing the CAR or other antigen receptor further includes a surrogate marker, such as a cell surface marker, which may be used to confirm transduction or engineering of the cell to express the receptor. In some aspects, the marker includes all or part (e.g., truncated form) of CD34, a NGFR, or epidermal growth factor receptor, such as truncated version of such a cell surface receptor (e.g., tEGFR). In some instances, the nucleic acid encoding the marker is operably linked to a polynucleotide encoding for a linker sequence, such as a cleavable linker sequence, e.g., T2A. For example, a marker, and optionally a linker sequence, can be any as disclosed in published patent application No. WO2014031687. For example, the marker can be a truncated EGFR (tEGFR) that is, optionally, linked to a linker sequence, such as a T2A cleavable linker sequence. In instances, the tEGFR contains an amino acid sequence set forth in SEQ ID NO: 155 or 156. In some instances, the tEGFR contains an amino acid sequence with or with about 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or greater than 99% sequence identify to the sequences set forth in SEQ ID NO: 155 or 156.

In some instances, the marker is a molecule, e.g., cell surface protein, not naturally found on T cells or not naturally found on the surface of T cells, or a portion thereof. In some instances, the molecule is a non-self molecule, e.g., non-self protein, i.e., one that is not recognized as "self' by the immune system of the host into which the cells will be adoptively transferred.

In some instances, the marker serves no therapeutic function and/or produces no effect other than to be used as a marker for genetic engineering, e.g., for selecting cells successfully engineered. In other instances, the marker may be a therapeutic molecule or molecule otherwise exerting some desired effect, such as a ligand for a cell to be encountered in vivo, such as a costimulatory or immune checkpoint molecule to enhance and/or dampen responses of the cells upon adoptive transfer and encounter with ligand.

In some cases, CARs are referred to as first, second, and/or third generation CARs. In some aspects, a first generation CAR is one that solely provides a CD3-chain induced signal upon antigen binding; in some aspects, a second-generation CARs is one that provides such a signal and costimulatory signal, such as one including an intracellular signaling domain from a costimulatory receptor such as CD28 or CD137; in some aspects, a third generation CAR is one that includes multiple costimulatory domains of different costimulatory receptors.

In some instances, the chimeric antigen receptor includes an extracellular portion containing the antibody or fragment described herein. In some aspects, the chimeric antigen receptor includes an extracellular portion containing the antibody or fragment described herein and an intracellular signaling domain. In some instances, the antibody or fragment includes an scFv or a single-domain V_{H} antibody and the intracellular domain contains an ITAM. In some aspects, the intracellular signaling domain includes a signaling domain of a zeta chain of a CD3-zeta (CD3ζ) chain. In some instances, the chimeric antigen receptor includes a transmembrane domain disposed between the extracellular domain and the intracellular signaling region.

In some aspects, the transmembrane domain contains a transmembrane portion of CD28. The extracellular domain and transmembrane can be linked directly or indirectly. In some instances, the extracellular domain and transmembrane are linked by a spacer, such as any described herein. In some instances, the chimeric antigen receptor contains an intracellular domain of a T cell costimulatory molecule, such as between the transmembrane domain and intracellular signaling domain. In some aspects, the T cell costimulatory molecule is CD28 or 4-1BB.

For example, in some instances, the CAR contains an antibody, e.g., an antibody fragment, a transmembrane domain that is or contains a transmembrane portion of CD28 or a functional variant thereof, and an intracellular signaling domain containing a signaling portion of CD28 or functional variant thereof and a signaling portion of CD3 zeta or functional variant thereof. In some instances, the CAR contains an antibody, e.g., antibody fragment, a transmembrane domain that is or contains a transmembrane portion of CD28 or a functional variant thereof, and an intracellular signaling domain containing a signaling portion of a 4-1BB or functional variant thereof and a signaling portion of CD3 zeta or functional variant thereof. In some such instances, the receptor further includes a spacer containing a portion of an Ig molecule, such as a human Ig molecule, such as an Ig hinge, e.g. an IgG4 hinge, such as a hinge-only spacer.

In some instances, the transmembrane domain of the receptor, e.g., the CAR is a transmembrane domain of human CD28 or variant thereof, e.g., a 27-amino acid transmembrane domain of a human CD28 (Accession No.: P10747.1), or is a transmembrane domain that comprises the sequence of amino acids set forth in SEQ ID NO: 157 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 157; in some instances, the transmembrane-domain containing portion of the recombinant receptor comprises the sequence of amino acids set forth in SEQ ID NO: 158 or a sequence of amino acids having at least at or about 85%, 86%, 87%, 88%, 89%, 90%, 91%, 92%, 93%, 94%, 95%, 96%, 97%, 98%, 99% or more sequence identity thereto.

In some instances, the chimeric antigen receptor contains an intracellular domain of a T cell costimulatory molecule. In some aspects, the T cell costimulatory molecule is CD28 or 4-1BB.

In some instances, the intracellular signaling region comprises an intracellular costimulatory signaling domain of human CD28 or functional variant or portion thereof, such as a 41 amino acid domain thereof and/or such a domain with an LL to GG substitution at positions 186-187 of a native CD28 protein. In some instances, the intracellular signaling domain can comprise the sequence of amino acids set forth in SEQ ID NO: 159 or 160 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 159 or 160. In some instances, the intracellular region comprises an intracellular costimulatory signaling domain of 4-1BB or functional variant or portion thereof, such as a 42-amino acid cytoplasmic domain of a human 4-1BB (Accession No. Q07011.1) or functional variant or portion thereof, such as the sequence of amino acids set forth in SEQ ID NO: 161 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 161.

In some instances, the intracellular signaling region comprises a human CD3 chain, optionally a CD3 zeta stimulatory signaling domain or functional variant thereof, such as an 112 AA cytoplasmic domain of isoform 3 of human CD3ζ (Accession No.: P20963.2) or a CD3 zeta signaling domain as described in U.S. Patent No.: 7,446,190 or U.S. Patent No. 8,911,993. In some instances, the intracellular signaling region comprises the sequence of amino acids set forth in SEQ ID NO: 162, 163 or 164 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 162, 163 or 164.

In some aspects, the spacer contains only a hinge region of an IgG, such as only a hinge of IgG4 or IgG1, such as the hinge only spacer set forth in SEQ ID NO: 150. In other instances, the spacer is an Ig hinge, e.g., and IgG4 hinge, linked to a C_{H}2 and/or C_{H}3 domains. In some instances, the spacer is an Ig hinge, e.g., an IgG4 hinge, linked to C_{H}2 and C_{H}3 domains, such as set forth in SEQ ID NO: 152. In some instances, the spacer is an Ig hinge, e.g., an IgG4 hinge, linked to a C_{H}3 domain only, such as set forth in SEQ ID NO: 153. In some instances, the spacer is or comprises a glycine-serine rich sequence or other flexible linker such as known flexible linkers.

For example, in some instances, the CAR includes an antibody such as an antibody fragment, including scFvs, a spacer, such as a spacer containing a portion of an immunoglobulin molecule, such as a hinge region and/or one or more constant regions of a heavy chain molecule, such as an Ig-hinge containing spacer, a transmembrane domain containing all or a portion of a CD28-derived transmembrane domain, a CD28-derived intracellular signaling domain, and a CD3 zeta signaling domain. In some instances, the CAR includes an antibody or fragment, such as scFv, a spacer such as any of the Ig-hinge containing spacers, a CD28-derived transmembrane domain, a 4-1BB-derived intracellular signaling domain, and a CD3 zeta-derived signaling domain.

In some instances, nucleic acid molecules encoding such CAR constructs further includes a sequence encoding a T2A ribosomal skip element and/or a tEGFR sequence, e.g., downstream of the sequence encoding the CAR. In some instances, T cells expressing an antigen receptor (e.g. CAR) can also be generated to express a truncated EGFR (EGFRt) as a non-immunogenic selection epitope (e.g. by introduction of a construct encoding the CAR and EGFRt separated by a T2A ribosome switch to express two proteins from the same construct), which then can be used as a marker to detect such cells (see e.g. U.S. Patent No. 8,802,374). In some instances, a single promoter may direct expression of an RNA that contains, in a single open reading frame (ORF), two or three genes (e.g. encoding the molecule involved in modulating a metabolic pathway and encoding the recombinant receptor) separated from one another by sequences encoding a self-cleavage peptide (*e.g.,* 2A sequences) or a protease recognition site (*e.g.,* furin). The ORF thus encodes a single polypeptide, which, either during (in the case of 2A) or after translation, is processed into the individual proteins. In some cases, the peptide, such as T2A, can cause the ribosome to skip (ribosome skipping) synthesis of a peptide bond at the C-terminus of a 2A element, leading to separation between the end of the 2A sequence and the next peptide downstream (*see,* for example, de Felipe. Genetic Vaccines and Ther. 2:13 (2004) and deFelipe et al. Traffic 5:616-626 (2004)). Many 2A elements are known in the art. Examples of 2A sequences that can be used in the methods and nucleic acids disclosed herein, without limitation, 2A sequences from the foot-and-mouth disease virus (F2A, *e.g.,* SEQ ID NO: 131), equine rhinitis A virus (E2A, *e.g.,* SEQ ID NO: 130), Thosea asigna virus (T2A, *e.g.,* SEQ ID NO: 126 or 127), and porcine teschovirus-1 (P2A, *e.g.,* SEQ ID NO: 128 or 129) as described in U.S. Patent Publication No. 20070116690.

The recombinant receptors, such as CARs, expressed by the cells administered to the subject generally recognize or specifically bind to a molecule that is expressed in, associated with, and/or specific for the disease or condition or cells thereof being treated. Upon specific binding to the molecule, e.g., antigen, the receptor generally delivers an immunostimulatory signal, such as an ITAM-transduced signal, into the cell, thereby promoting an immune response targeted to the disease or condition. For example, in some instances, the cells express a CAR that specifically binds to an antigen expressed by a cell or tissue of the disease or condition or associated with the disease or condition. The receptor may be another receptor such as an immunoinhibitory or costimulatory signal-promoting receptor, such as a CCR or iCAR or non-signaling receptor, e.g., for use in depletion or elimination of cells using the antibodies.

### B. Genetically Engineered Cells and Methods of Producing Cells

Among the cells expressing the chimeric antigen receptors are engineered cells. The genetic engineering generally involves introduction of a nucleic acid encoding the recombinant or engineered component into a composition containing the cells, such as by retroviral transduction, transfection, or transformation. Various methods for the introduction of genetically engineered components, e.g., recombinant receptors, e.g., CARs, are well known and may be used. Exemplary methods include those for transfer of nucleic acids encoding the receptors, including via viral, e.g., retroviral or lentiviral, transduction, transposons, and electroporation.

### 1. Vectors and Methods for Genetic Engineering

Also disclosed are one or more polynucleotides (e.g., nucleic acid molecules) encoding chimeric antigen receptors (CARs), vectors for genetically engineering cells to express such CARs and methods for producing the engineered cells. In some instances, the vector contains the nucleic acid encoding the CAR. In some cases, the vector is a viral vector, such as a retroviral vector, e.g., a lentiviral vector or a gammaretroviral vector.

In some instances, recombinant nucleic acids are transferred into cells using recombinant infectious virus particles, such as, e.g., vectors derived from simian virus 40 (SV40), adenoviruses, adeno-associated virus (AAV). In some instances, recombinant nucleic acids are transferred into T cells using recombinant lentiviral vectors or retroviral vectors, such as gamma-retroviral vectors (see, e.g., Koste et al. (2014) Gene Therapy 2014 Apr 3. doi: 10.1038/gt.2014.25; Carlens et al. (2000) Exp Hematol 28(10): 1137-46; Alonso-Camino et al. (2013) Mol Ther Nucl Acids 2, e93; Park et al., Trends Biotechnol. 2011 November 29(11): 550-557.

In some instances, the retroviral vector has a long terminal repeat sequence (LTR), e.g., a retroviral vector derived from the Moloney murine leukemia virus (MoMLV), myeloproliferative sarcoma virus (MPSV), murine embryonic stem cell virus (MESV), murine stem cell virus (MSCV), spleen focus forming virus (SFFV), or adeno-associated virus (AAV). Most retroviral vectors are derived from murine retroviruses. In some instances, the retroviruses include those derived from any avian or mammalian cell source. The retroviruses typically are amphotropic, meaning that they are capable of infecting host cells of several species, including humans. In one instance, the gene to be expressed replaces the retroviral gag, pol and/or env sequences. A number of illustrative retroviral systems have been described (e.g., U.S. Pat. Nos. 5,219,740; 6,207,453; 5,219,740; Miller and Rosman (1989) BioTechniques 7: 980-990; Miller, A. D. (1990) Human Gene Therapy 1: 5-14; Scarpa et al. (1991) Virology 180: 849-852; Burns et al. (1993) Proc. Natl. Acad. Sci. USA 90: 8033-8037; and Boris-Lawrie and Temin (1993) Cur. Opin. Genet. Develop. 3: 102-109.

Methods of lentiviral transduction are known. Exemplary methods are described in, e.g., Wang et al. (2012) J. Immunother. 35(9): 689-701; Cooper et al. (2003) Blood. 101: 1637-1644; Verhoeyen et al. (2009) Methods Mol Biol. 506: 97-114; and Cavalieri et al. (2003) Blood. 102(2): 497-505.

In some instances, recombinant nucleic acids are transferred into T cells via electroporation (see, e.g., Chicaybam et al, (2013) PLoS ONE 8(3): e60298 and Van Tedeloo et al. (2000) Gene Therapy 7(16): 1431-1437). In some instances, recombinant nucleic acids are transferred into T cells via transposition (see, e.g., Manuri et al. (2010) Hum Gene Ther 21(4): 427-437; Sharma et al. (2013) Molec Ther Nucl Acids 2, e74; and Huang et al. (2009) Methods Mol Biol 506: 115-126). Other methods of introducing and expressing genetic material in immune cells include calcium phosphate transfection (e.g., as described in Current Protocols in Molecular Biology, John Wiley & Sons, New York. N.Y.), protoplast fusion, cationic liposome-mediated transfection; tungsten particle-facilitated microparticle bombardment (Johnston, Nature, 346: 776-777 (1990)); and strontium phosphate DNA co-precipitation (Brash et al., Mol. Cell Biol., 7: 2031-2034 (1987)).

Other approaches and vectors for transfer of the nucleic acids encoding the recombinant products are those described, e.g., in international patent application, Publication No.: WO2014055668, and U.S. Patent No. 7,446,190.

In some instances, the cells, e.g., T cells, may be transfected either during or after expansion, e.g. with a T cell receptor (TCR) or a chimeric antigen receptor (CAR). This transfection for the introduction of the gene of the desired receptor can be carried out with any suitable retroviral vector, for example. The genetically modified cell population can then be liberated from the initial stimulus (the anti-CD3/anti-CD28 stimulus, for example) and subsequently be stimulated with a second type of stimulus e.g. via a de novo introduced receptor). This second type of stimulus may include an antigenic stimulus in form of a peptide/MHC molecule, the cognate (cross-linking) ligand of the genetically introduced receptor (e.g. natural ligand of a CAR) or any ligand (such as an antibody) that directly binds within the framework of the new receptor (e.g. by recognizing constant regions within the receptor). See, for example, Cheadle et al, "Chimeric antigen receptors for T-cell based therapy" Methods Mol Biol. 2012; 907: 645-66 or Barrett et al., Chimeric Antigen Receptor Therapy for Cancer Annual Review of Medicine Vol. 65: 333-347 (2014). In some instances, the cells are stimulated with a disclosed anti-idiotype antibody in accord with the disclosed methods.

In some cases, a vector may be used that does not require that the cells, e.g., T cells, are activated. In some such instances, the cells may be selected and/or transduced prior to activation. Thus, the cells may be engineered prior to, or subsequent to culturing of the cells, and in some cases at the same time as or during at least a portion of the culturing.

Among additional nucleic acids, e.g., genes for introduction are those to improve the efficacy of therapy, such as by promoting viability and/or function of transferred cells; genes to provide a genetic marker for selection and/or evaluation of the cells, such as to assess *in vivo* survival or localization; genes to improve safety, for example, by making the cell susceptible to negative selection in vivo as described by Lupton S. D. et al., Mol. and Cell Biol., 11: 6 (1991); and Riddell et al., Human Gene Therapy 3: 319-338 (1992); see also the publications of PCT/US91/08442 and PCT/US94/05601 by Lupton et al. describing the use of bifunctional selectable fusion genes derived from fusing a dominant positive selectable marker with a negative selectable marker. *See,* e.g., Riddell et al., U.S. Patent No. 6,040,177, at columns 14-17.

### 2. Cells and Preparation of Cells for Genetic Engineering

Disclosed herein are cells, including engineered cells that contain a chimeric antigen receptor (CAR). Also disclosed are population of such cells and compositions containing such cells. Among the compositions are input compositions containing cells in which one or more cells is known or likely or will express a recombinant receptor capable of being recognized or bound by a binding molecule present on one or more particles to which the cells are incubated or contacted. Also among the compositions are compositions produced by the disclosed methods, including output compositions in which is contained stimulated or expanded cells, including compositions enriched for cells containing a recombinant receptor bound or recognized by the binding molecule of the particle, such as in which cells expressing the recombinant receptor, e.g. chimeric receptor, make up at least 50, 60, 70, 80, 90, 91, 92, 93, 94, 95, 96, 97, 98, 99, or more percent of the total cells in the composition or cells of a certain type such as T cells or CD8+ or CD4+ cells. Thus, disclosed are genetically engineered cells expressing the recombinant receptors e.g., CARs.

Among the compositions are pharmaceutical compositions and formulations for administration, such as for adoptive cell therapy. Also disclosed are methods for engineering, producing or generating such cells, therapeutic methods for administering the cells and compositions to subjects, e.g., patients, and methods for detecting, selecting, isolating or separating such cells.

In some instances, the nucleic acids are heterologous, i.e., normally not present in a cell or sample obtained from the cell, such as one obtained from another organism or cell, which for example, is not ordinarily found in the cell being engineered and/or an organism from which such cell is derived. In some instances, the nucleic acids are not naturally occurring, such as a nucleic acid not found in nature, including one comprising chimeric combinations of nucleic acids encoding various domains from multiple different cell types.

The cells generally are eukaryotic cells, such as mammalian cells, and typically are human cells. In some instances, the cells are derived from the blood, bone marrow, lymph, or lymphoid organs, are cells of the immune system, such as cells of the innate or adaptive immunity, *e.g.*, myeloid or lymphoid cells, including lymphocytes, typically T cells and/or NK cells. Other exemplary cells include stem cells, such as multipotent and pluripotent stem cells, including induced pluripotent stem cells (iPSCs).

The cells typically are primary cells, such as those isolated directly from a subject and/or isolated from a subject and frozen. In some instances, the cells include one or more subsets of T cells or other cell types, such as whole T cell populations, CD4+ cells, CD8+ cells, and subpopulations thereof, such as those defined by function, activation state, maturity, potential for differentiation, expansion, recirculation, localization, and/or persistence capacities, antigen-specificity, type of antigen receptor, presence in a particular organ or compartment, marker or cytokine secretion profile, and/or degree of differentiation. With reference to the subject to be treated, the cells may be allogeneic and/or autologous. Among the methods include off-the-shelf methods. In some aspects, such as for off-the-shelf technologies, the cells are pluripotent and/or multipotent, such as stem cells, such as induced pluripotent stem cells (iPSCs). In some instances, the methods include isolating cells from the subject, preparing, processing, culturing, and/or engineering them, and re-introducing them into the same subject, before or after cryopreservation.

Among the sub-types and subpopulations of T cells and/or of CD4+ and/or of CD8+ T cells are naive T (T_{N}) cells, effector T cells (T_{EFF}), memory T cells and sub-types thereof, such as stem cell memory T (T_{SCM}), central memory T (T_{CM}), effector memory T (T_{EM}), or terminally differentiated effector memory T cells, tumor-infiltrating lymphocytes (TIL), immature T cells, mature T cells, helper T cells, cytotoxic T cells, mucosa-associated invariant T (MAIT) cells, naturally occurring and adaptive regulatory T (Treg) cells, helper T cells, such as TH1 cells, TH2 cells, TH3 cells, TH17 cells, TH9 cells, TH22 cells, follicular helper T cells, alpha/beta T cells, and delta/gamma T cells.

In some instances, the cells are natural killer (NK) cells. In some instances, the cells are monocytes or granulocytes, e.g., myeloid cells, macrophages, neutrophils, dendritic cells, mast cells, eosinophils, and/or basophils.

In some instances, the cells include one or more nucleic acids introduced via genetic engineering, and thereby express recombinant or genetically engineered products of such nucleic acids. In some instances, the nucleic acids are heterologous, i.e., normally not present in a cell or sample obtained from the cell, such as one obtained from another organism or cell, which for example, is not ordinarily found in the cell being engineered and/or an organism from which such cell is derived. In some instances, the nucleic acids are not naturally occurring, such as a nucleic acid not found in nature, including one comprising chimeric combinations of nucleic acids encoding various domains from multiple different cell types.

In some instances, preparation of the engineered cells includes one or more culture and/or preparation steps. The cells for introduction of the nucleic acid encoding the transgenic receptor such as the CAR, may be isolated from a sample, such as a biological sample, e.g., one obtained from or derived from a subject. In some instances, the subject from which the cell is isolated is one having the disease or condition or in need of a cell therapy or to which cell therapy will be administered. The subject in some instances is a human in need of a particular therapeutic intervention, such as the adoptive cell therapy for which cells are being isolated, processed, and/or engineered.

Accordingly, the cells in some instances are primary cells, e.g., primary human cells. The samples include tissue, fluid, and other samples taken directly from the subject, as well as samples resulting from one or more processing steps, such as separation, centrifugation, genetic engineering (e.g. transduction with viral vector), washing, and/or incubation. The biological sample can be a sample obtained directly from a biological source or a sample that is processed. Biological samples include, but are not limited to, body fluids, such as blood, plasma, serum, cerebrospinal fluid, synovial fluid, urine and sweat, tissue and organ samples, including processed samples derived therefrom.

In some aspects, the sample from which the cells are derived or isolated is blood or a blood-derived sample, or is or is derived from an apheresis or leukapheresis product. Exemplary samples include whole blood, peripheral blood mononuclear cells (PBMCs), leukocytes, bone marrow, thymus, tissue biopsy, tumor, leukemia, lymphoma, lymph node, gut associated lymphoid tissue, mucosa associated lymphoid tissue, spleen, other lymphoid tissues, liver, lung, stomach, intestine, colon, kidney, pancreas, breast, bone, prostate, cervix, testes, ovaries, tonsil, or other organ, and/or cells derived therefrom. Samples include, in the context of cell therapy, e.g., adoptive cell therapy, samples from autologous and allogeneic sources.

In some instances, the cells are derived from cell lines, e.g., T cell lines. The cells in some instances are obtained from a xenogeneic source, for example, from mouse, rat, non-human primate, and pig.

In some instances, isolation of the cells includes one or more preparation and/or non-affinity based cell separation steps. In some examples, cells are washed, centrifuged, and/or incubated in the presence of one or more reagents, for example, to remove unwanted components, enrich for desired components, lyse or remove cells sensitive to particular reagents. In some examples, cells are separated based on one or more property, such as density, adherent properties, size, sensitivity and/or resistance to particular components.

In some examples, cells from the circulating blood of a subject are obtained, e.g., by apheresis or leukapheresis. The samples, in some aspects, contain lymphocytes, including T cells, monocytes, granulocytes, B cells, other nucleated white blood cells, red blood cells, and/or platelets, and in some aspects contains cells other than red blood cells and platelets. In some instances, prior to the selection and/or enrichment of cells, the sample or the cells in the sample can be rested or held prior to further processing steps. In some instances, the sample is maintained at or held at a temperature of from or from about 2° C to 8° C for up to 48 hours, such as for up to12 hours, 24 hours or 36 hours. In certain instances, the cells are not selected and/or enriched prior to contacting the cells with the one or more nucleic acids. In some instances, the sample or the cells can be rested or held prior to contacting or incubating the cells with one or more nucleic acids. In certain instances, the sample is maintained at or held at a temperature of from or from about 2° C to 8° C for up to 48 hours, such as for up to 12 hours, 24 hours or 36 hours prior to contacting or incubating the cells with one or more nucleic acids.

In some instances, the blood cells collected from the subject are washed, e.g., to remove the plasma fraction and to place the cells in an appropriate buffer or media for subsequent processing steps. In some instances, the cells are washed with phosphate buffered saline (PBS). In some instances, the wash solution lacks calcium and/or magnesium and/or many or all divalent cations. In some aspects, a washing step is accomplished a semi-automated "flow-through" centrifuge (for example, the Cobe 2991 cell processor, Baxter) according to the manufacturer's instructions. In some aspects, a washing step is accomplished by tangential flow filtration (TFF) according to the manufacturer's instructions. In some instances, the cells are resuspended in a variety of biocompatible buffers after washing, such as, for example, Ca⁺⁺/Mg⁺⁺ free PBS. In certain instances, components of a blood cell sample are removed and the cells directly resuspended in culture media.

In some instances, the methods include density-based cell separation methods, such as the preparation of white blood cells from peripheral blood by lysing the red blood cells and centrifugation through a Percoll or Ficoll gradient.

In some instances, the isolation methods include the separation of different cell types based on the expression or presence in the cell of one or more specific molecules, such as surface markers, e.g., surface proteins, intracellular markers, or nucleic acid. In some instances, any known method for separation based on such markers may be used. In some instances, the separation is affinity- or immunoaffinity-based separation. For example, the isolation in some aspects includes separation of cells and cell populations based on the cells' expression or expression level of one or more markers, typically cell surface markers, for example, by incubation with an antibody or binding partner that specifically binds to such markers, followed generally by washing steps and separation of cells having bound the antibody or binding partner, from those cells having not bound to the antibody or binding partner.

Such separation steps can be based on positive selection, in which the cells having bound the reagents are retained for further use, and/or negative selection, in which the cells having not bound to the antibody or binding partner are retained. In some examples, both fractions are retained for further use. In some aspects, negative selection can be particularly useful where no antibody is available that specifically identifies a cell type in a heterogeneous population, such that separation is best carried out based on markers expressed by cells other than the desired population.

The separation need not result in 100% enrichment or removal of a particular cell population or cells expressing a particular marker. For example, positive selection of or enrichment for cells of a particular type, such as those expressing a marker, refers to increasing the number or percentage of such cells, but need not result in a complete absence of cells not expressing the marker. Likewise, negative selection, removal, or depletion of cells of a particular type, such as those expressing a marker, refers to decreasing the number or percentage of such cells, but need not result in a complete removal of all such cells.

In some examples, multiple rounds of separation steps are carried out, where the positively or negatively selected fraction from one step is subjected to another separation step, such as a subsequent positive or negative selection. In some examples, a single separation step can deplete cells expressing multiple markers simultaneously, such as by incubating cells with a plurality of antibodies or binding partners, each specific for a marker targeted for negative selection. Likewise, multiple cell types can simultaneously be positively selected by incubating cells with a plurality of antibodies or binding partners expressed on the various cell types.

For example, in some aspects, specific subpopulations of T cells, such as cells positive or expressing high levels of one or more surface markers, e.g., CD28⁺, CD62L⁺, CCR7⁺, CD27⁺, CD127⁺, CD4⁺, CD8⁺, CD45RA⁺, and/or CD45RO⁺ T cells, are isolated by positive or negative selection techniques.

For example, CD3⁺, CD28⁺ T cells can be positively selected using CD3/CD28 conjugated magnetic beads (e.g., DYNABEADS^{®} M-450 CD3/CD28 T Cell Expander). In particular instances, cells are contacted with anti-CD3/anti-CD28 conjugated magnetic beads (e.g., DYNABEADS^{®} M-450 CD3/CD28 T Cell Expander) to expand CD3⁺, CD28⁺ T cells prior to contacting the cells with the one or more nucleic acids. In certain instances, the cells are not contacted with anti-CD3/anti-CD28 conjugated magnetic beads prior to contacting the cells with the one or more nucleic acids.

In some instances, isolation is carried out by enrichment for a particular cell population by positive selection, or depletion of a particular cell population, by negative selection. In some instances, positive or negative selection is accomplished by incubating cells with one or more antibodies or other binding agent that specifically bind to one or more surface markers expressed or expressed (marker⁺) at a relatively higher level (marker^{high}) on the positively or negatively selected cells, respectively.

In some instances, T cells are separated from a PBMC sample by negative selection of markers expressed on non-T cells, such as B cells, monocytes, or other white blood cells, such as CD14. In some aspects, a CD4⁺ or CD8⁺ selection step is used to separate CD4⁺ helper and CD8⁺ cytotoxic T cells. Such CD4⁺ and CD8⁺ populations can be further sorted into sub-populations by positive or negative selection for markers expressed or expressed to a relatively higher degree on one or more naive, memory, and/or effector T cell subpopulations.

In some instances, CD8⁺ cells are further enriched for or depleted of naive, central memory, effector memory, and/or central memory stem cells, such as by positive or negative selection based on surface antigens associated with the respective subpopulation. In some instances, enrichment for central memory T (T_{CM}) cells is carried out to increase efficacy, such as to improve long-term survival, expansion, and/or engraftment following administration, which in some aspects is particularly robust in such sub-populations. *See* Terakura et al. (2012) Blood.1: 72-82; Wang et al. (2012) J Immunother. 35(9): 689-701. In some instances, combining T_{CM}-enriched CD8⁺ T cells and CD4⁺T cells further enhances efficacy.

In instances, memory T cells are present in both CD62L⁺ and CD62L⁻ subsets of CD8⁺ peripheral blood lymphocytes. PBMC can be enriched for or depleted of CD62L⁻CD8⁺ and/or CD62L⁺CD8⁺ fractions, such as using anti-CD8 and anti-CD62L antibodies.

In some instances, the enrichment for central memory T (T_{CM}) cells is based on positive or high surface expression of CD45RO, CD62L, CCR7, CD28, CD3, and/or CD 127; in some aspects, it is based on negative selection for cells expressing or highly expressing CD45RA and/or granzyme B. In some aspects, isolation of a CD8⁺ population enriched for T_{CM} cells is carried out by depletion of cells expressing CD4, CD14, CD45RA, and positive selection or enrichment for cells expressing CD62L. In one aspect, enrichment for central memory T (T_{CM}) cells is carried out starting with a negative fraction of cells selected based on CD4 expression, which is subjected to a negative selection based on expression of CD14 and CD45RA, and a positive selection based on CD62L. Such selections in some aspects are carried out simultaneously and in other aspects are carried out sequentially, in either order. In some aspects, the same CD4 expression-based selection step used in preparing the CD8⁺ cell population or subpopulation, also is used to generate the CD4⁺ cell population or subpopulation, such that both the positive and negative fractions from the CD4-based separation are retained and used in subsequent steps of the methods, optionally following one or more further positive or negative selection steps.

In a particular example, a sample of PBMCs or other white blood cell sample is subjected to selection of CD4⁺ cells, where both the negative and positive fractions are retained. The negative fraction then is subjected to negative selection based on expression of CD14 and CD45RA or CD19, and positive selection based on a marker characteristic of central memory T cells, such as CD62L or CCR7, where the positive and negative selections are carried out in either order.

CD4⁺ T helper cells are sorted into naive, central memory, and effector cells by identifying cell populations that have cell surface antigens. CD4⁺ lymphocytes can be obtained by standard methods. In some instances, naive CD4⁺ T lymphocytes are CD45RO⁻, CD45RA⁺, CD62L⁺, CD4⁺ T cells. In some instances, central memory CD4⁺ cells are CD62L⁺ and CD45RO⁺. In some instances, effector CD4⁺ cells are CD62L⁻ and CD45RO⁻.

In one example, to enrich for CD4⁺ cells by negative selection, a monoclonal antibody cocktail typically includes antibodies to CD14, CD20, CD11b, CD16, HLA-DR, and CD8. In some instances, the antibody or binding partner is bound to a solid support or matrix, such as a magnetic bead or paramagnetic bead, to allow for separation of cells for positive and/or negative selection. For example, in some instances, the cells and cell populations are separated or isolated using immunomagnetic (or affinitymagnetic) separation techniques (reviewed in Methods in Molecular Medicine, vol. 58: Metastasis Research Protocols, Vol. 2: Cell Behavior In Vitro and In Vivo, p 17-25 Edited by: S. A. Brooks and U. Schumacher © Humana Press Inc., Totowa, NJ).

In some aspects, the sample or composition of cells to be separated is incubated with small, magnetizable or magnetically responsive material, such as magnetically responsive particles or microparticles, such as paramagnetic beads (e.g., such as Dynalbeads or MACS beads). The magnetically responsive material, e.g., particle, generally is directly or indirectly attached to a binding partner, e.g., an antibody, that specifically binds to a molecule, e.g., surface marker, present on the cell, cells, or population of cells that it is desired to separate, e.g., that it is desired to negatively or positively select.

In some instances, the magnetic particle or bead comprises a magnetically responsive material bound to a specific binding member, such as an antibody or other binding partner. There are many well-known magnetically responsive materials used in magnetic separation methods. Suitable magnetic particles include those described in Molday, U.S. Pat. No. 4,452,773, and in European Patent Specification EP 452342 B. Colloidal sized particles, such as those described in Owen U.S. Pat. No. 4,795,698, and Liberti et al., U.S. Pat. No. 5,200,084 are other examples.

The incubation generally is carried out under conditions whereby the antibodies or binding partners, or molecules, such as secondary antibodies or other reagents, which specifically bind to such antibodies or binding partners, which are attached to the magnetic particle or bead, specifically bind to cell surface molecules if present on cells within the sample.

In some aspects, the sample is placed in a magnetic field, and those cells having magnetically responsive or magnetizable particles attached thereto will be attracted to the magnet and separated from the unlabeled cells. For positive selection, cells that are attracted to the magnet are retained; for negative selection, cells that are not attracted (unlabeled cells) are retained. In some aspects, a combination of positive and negative selection is performed during the same selection step, where the positive and negative fractions are retained and further processed or subject to further separation steps.

In certain instances, the magnetically responsive particles are coated in primary antibodies or other binding partners, secondary antibodies, lectins, enzymes, or streptavidin. In certain instances, the magnetic particles are attached to cells via a coating of primary antibodies specific for one or more markers. In certain instances, the cells, rather than the beads, are labeled with a primary antibody or binding partner, and then cell-type specific secondary antibody- or other binding partner (e.g., streptavidin)-coated magnetic particles, are added. In certain instances, streptavidin-coated magnetic particles are used in conjunction with biotinylated primary or secondary antibodies.

In some instances, the magnetically responsive particles are left attached to the cells that are to be subsequently incubated, cultured and/or engineered; in some aspects, the particles are left attached to the cells for administration to a patient. In some instances, the magnetizable or magnetically responsive particles are removed from the cells. Methods for removing magnetizable particles from cells are known and include, e.g., the use of competing non-labeled antibodies, and magnetizable particles or antibodies conjugated to cleavable linkers. In some instances, the magnetizable particles are biodegradable.

In some instances, the affinity-based selection is via magnetic-activated cell sorting (MACS) (Miltenyi Biotec, Auburn, CA). Magnetic Activated Cell Sorting (MACS) systems are capable of high-purity selection of cells having magnetized particles attached thereto. In certain instances, MACS operates in a mode wherein the non-target and target species are sequentially eluted after the application of the external magnetic field. That is, the cells attached to magnetized particles are held in place while the unattached species are eluted. Then, after this first elution step is completed, the species that were trapped in the magnetic field and were prevented from being eluted are freed in some manner such that they can be eluted and recovered. In certain instances, the non-target cells are labelled and depleted from the heterogeneous population of cells.

In certain instances, the isolation or separation is carried out using a system, device, or apparatus that carries out one or more of the isolation, cell preparation, separation, processing, incubation, culture, and/or formulation steps of the methods. In some aspects, the system is used to carry out each of these steps in a closed or sterile environment, for example, to minimize error, user handling and/or contamination. In one example, the system is a system as described in International Patent Application, Publication Number WO2009/072003, or US 20110003380 A1. In one example, the system is a system as described in International Publication Number WO2016/073602.

In some instances, the system or apparatus carries out one or more, e.g., all, of the isolation, processing, engineering, and formulation steps in an integrated or self-contained system, and/or in an automated or programmable fashion. In some aspects, the system or apparatus includes a computer and/or computer program in communication with the system or apparatus, which allows a user to program, control, assess the outcome of, and/or adjust various aspects of the processing, isolation, engineering, and formulation steps.

In some aspects, the separation and/or other steps is carried out using CliniMACS system (Miltenyi Biotec), for example, for automated separation of cells on a clinical-scale level in a closed and sterile system. Components can include an integrated microcomputer, magnetic separation unit, peristaltic pump, and various pinch valves. The integrated computer in some aspects controls all components of the instrument and directs the system to perform repeated procedures in a standardized sequence. The magnetic separation unit in some aspects includes a movable permanent magnet and a holder for the selection column. The peristaltic pump controls the flow rate throughout the tubing set and, together with the pinch valves, ensures the controlled flow of buffer through the system and continual suspension of cells.

The CliniMACS system in some aspects uses antibody-coupled magnetizable particles that are supplied in a sterile, non-pyrogenic solution. In some instances, after labelling of cells with magnetic particles the cells are washed to remove excess particles. A cell preparation bag is then connected to the tubing set, which in turn is connected to a bag containing buffer and a cell collection bag. The tubing set consists of pre-assembled sterile tubing, including a pre-column and a separation column, and are for single use only. After initiation of the separation program, the system automatically applies the cell sample onto the separation column. Labelled cells are retained within the column, while unlabeled cells are removed by a series of washing steps. In some instances, the cell populations for use with the methods described herein are unlabeled and are not retained in the column. In some instances, the cell populations for use with the methods described herein are labeled and are retained in the column. In some instances, the cell populations for use with the methods described herein are eluted from the column after removal of the magnetic field, and are collected within the cell collection bag.

In certain instances, separation and/or other steps are carried out using the CliniMACS Prodigy system (Miltenyi Biotec). The CliniMACS Prodigy system in some aspects is equipped with a cell processing unity that permits automated washing and fractionation of cells by centrifugation. The CliniMACS Prodigy system can also include an onboard camera and image recognition software that determines the optimal cell fractionation endpoint by discerning the macroscopic layers of the source cell product. For example, peripheral blood is automatically separated into erythrocytes, white blood cells and plasma layers. The CliniMACS Prodigy system can also include an integrated cell cultivation chamber which accomplishes cell culture protocols such as, e.g., cell differentiation and expansion, antigen loading, and long-term cell culture. Input ports can allow for the sterile removal and replenishment of media and cells can be monitored using an integrated microscope. See, e.g., Klebanoff et al. (2012) J Immunother. 35(9): 651-660, Terakuraet al. (2012) Blood.1: 72-82, and Wang et al. (2012) J Immunother. 35(9): 689-701.

In some instances, a cell population described herein is collected and enriched (or depleted) via flow cytometry, in which cells stained for multiple cell surface markers are carried in a fluidic stream. In some instances, a cell population described herein is collected and enriched (or depleted) via preparative scale (FACS)-sorting. In certain instances, a cell population described herein is collected and enriched (or depleted) by use of microelectromechanical systems (MEMS) chips in combination with a FACS-based detection system (see, e.g., WO 2010/033140, Cho et al. (2010) Lab Chip 10, 1567-1573; and Godin et al. (2008) J Biophoton. 1(5): 355-376. In both cases, cells can be labeled with multiple markers, allowing for the isolation of well-defined T cell subsets at high purity.

In some instances, the antibodies or binding partners are labeled with one or more detectable marker, to facilitate separation for positive and/or negative selection. For example, separation may be based on binding to fluorescently labeled antibodies. In some examples, separation of cells based on binding of antibodies or other binding partners specific for one or more cell surface markers are carried in a fluidic stream, such as by fluorescence-activated cell sorting (FACS), including preparative scale (FACS) and/or microelectromechanical systems (MEMS) chips, e.g., in combination with a flow-cytometric detection system. Such methods allow for positive and negative selection based on multiple markers simultaneously.

In some instances, the preparation methods include steps for freezing, e.g., cryopreserving, the cells, either before or after isolation, incubation, and/or engineering. In some instances, the freeze and subsequent thaw step removes granulocytes and, to some extent, monocytes in the cell population. In some instances, the cells are suspended in a freezing solution, e.g., following a washing step to remove plasma and platelets. Any of a variety of known freezing solutions and parameters in some aspects may be used. One example involves using PBS containing 20% DMSO and 8% human serum albumin (HSA), or other suitable cell freezing media. This is then diluted 1: 1 with media so that the final concentration of DMSO and HSA are 10% and 4%, respectively. The cells are generally then frozen to -80° C. at a rate of 1° per minute and stored in the vapor phase of a liquid nitrogen storage tank.

In some instances, the cells are incubated and/or cultured prior to or in connection with genetic engineering. The incubation steps can include culture, cultivation, stimulation, activation, and/or propagation. The incubation and/or engineering may be carried out in a culture vessel, such as a unit, chamber, well, column, tube, tubing set, valve, vial, culture dish, bag, or other container for culture or cultivating cells. In some instances, the compositions or cells are incubated in the presence of stimulating conditions or a stimulatory agent. Such conditions include those designed to induce proliferation, expansion, activation, and/or survival of cells in the population, to mimic antigen exposure, and/or to prime the cells for genetic engineering, such as for the introduction of a recombinant antigen receptor.

The conditions can include one or more of particular media, temperature, oxygen content, carbon dioxide content, time, agents, e.g., nutrients, amino acids, antibiotics, ions, and/or stimulatory factors, such as cytokines, chemokines, antigens, binding partners, fusion proteins, recombinant soluble receptors, and any other agents designed to activate the cells.

In some instances, the stimulating conditions or agents include one or more agent, e.g., ligand, which is capable of activating an intracellular signaling domain of a TCR complex. In some aspects, the agent turns on or initiates TCR/CD3 intracellular signaling cascade in a T cell. Such agents can include antibodies, such as those specific for a TCR, e.g. anti-CD3. In some instances, the stimulating conditions include one or more agent, e.g. ligand, which is capable of stimulating a costimulatory receptor, e.g., anti-CD28. In some instances, such agents and/or ligands may be, bound to solid support such as a bead, and/or one or more cytokines. Optionally, the expansion method may further comprise the step of adding anti-CD3 and/or anti CD28 antibody to the culture medium (e.g., at a concentration of at least about 0.5 ng/ml). In some instances, the stimulating agents include IL-2, IL-15 and/or IL-7. In some aspects, the IL-2 concentration is at least about 10 units/mL.

In some aspects, incubation is carried out in accordance with techniques such as those described in U.S. Patent No. 6,040,1 77 to Riddell et al., Klebanoff et al.(2012) J Immunother. 35(9): 651-660, Terakura et al. (2012) Blood. 1: 72-82, and/or Wang et al. (2012) J Immunother. 35(9): 689-701.

In some instances, the T cells are expanded by adding to a culture-initiating composition feeder cells, such as non-dividing peripheral blood mononuclear cells (PBMC), (e.g., such that the resulting population of cells contains at least about 5, 10, 20, or 40 or more PBMC feeder cells for each T lymphocyte in the initial population to be expanded); and incubating the culture (e.g. for a time sufficient to expand the numbers of T cells). In some aspects, the non-dividing feeder cells can comprise gamma-irradiated PBMC feeder cells. In some instances, the PBMC are irradiated with gamma rays in the range of about 3000 to 3600 rads to prevent cell division. In some aspects, the feeder cells are added to culture medium prior to the addition of the populations of T cells.

In some instances, the stimulating conditions include temperature suitable for the growth of human T lymphocytes, for example, at least about 25 degrees Celsius, generally at least about 30 degrees, and generally at or about 37 degrees Celsius. Optionally, the incubation may further comprise adding non-dividing EBV-transformed lymphoblastoid cells (LCL) as feeder cells. LCL can be irradiated with gamma rays in the range of about 6000 to 10,000 rads. The LCL feeder cells in some aspects is provided in any suitable amount, such as a ratio of LCL feeder cells to initial T lymphocytes of at least about 10: 1.

### IV. COMPOSITIONS

Also disclosed are compositions including the binding molecules, such as antibodies, as disclosed herein, including pharmaceutical compositions and formulations. The compositions and formulations generally include one or more optional acceptable carriers or excipients.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

In some aspects, the choice of carrier is determined in part by the particular cell, binding molecule, and/or antibody, and/or by the method of administration. Accordingly, there are a variety of suitable formulations. For example, the pharmaceutical composition can contain preservatives. Suitable preservatives may include, for example, methylparaben, propylparaben, sodium benzoate, and benzalkonium chloride. In some aspects, a mixture of two or more preservatives is used. The preservative or mixtures thereof are typically present in an amount of about 0.0001% to about 2% by weight of the total composition. Carriers are described, e.g., by Remington's Pharmaceutical Sciences 16th edition, Osol, A. Ed. (1980). Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG).

Buffering agents in some aspects are included in the compositions. Suitable buffering agents include, for example, citric acid, sodium citrate, phosphoric acid, potassium phosphate, and various other acids and salts. In some aspects, a mixture of two or more buffering agents is used. The buffering agent or mixtures thereof are typically present in an amount of about 0.001% to about 4% by weight of the total composition. Methods for preparing administrable pharmaceutical compositions are known. Exemplary methods are described in more detail in, for example, Remington: The Science and Practice of Pharmacy, Lippincott Williams & Wilkins; 21st ed. (May 1, 2005).

In some aspects, the composition can contain preservatives. Suitable preservatives may include, for example, methylparaben, propylparaben, sodium benzoate, and benzalkonium chloride. In some aspects, a mixture of two or more preservatives is used. The preservative or mixtures thereof are typically present in an amount of about 0.0001% to about 2% by weight of the total composition. Carriers are described, e.g., by Remington's Pharmaceutical Sciences 16th edition, Osol, A. Ed. (1980). Acceptable carriers include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG).

Formulations of the antibodies can include lyophilized formulations and aqueous solutions.

Compositions in some instances are provided as sterile liquid preparations, e.g., isotonic aqueous solutions, suspensions, emulsions, dispersions, or viscous compositions, which may in some aspects be buffered to a selected pH. Liquid preparations are normally easier to prepare than gels, other viscous compositions, and solid compositions. Additionally, liquid compositions are somewhat more convenient to administer, especially by injection. Viscous compositions, on the other hand, can be formulated within the appropriate viscosity range to provide longer contact periods with specific tissues. Liquid or viscous compositions can comprise carriers, which can be a solvent or dispersing medium containing, for example, water, saline, phosphate buffered saline, polyol (for example, glycerol, propylene glycol, liquid polyethylene glycol) and suitable mixtures thereof.

Sterile injectable solutions can be prepared by incorporating the binding molecule in a solvent, such as in admixture with a suitable carrier, diluent, or excipient such as sterile water, physiological saline, glucose, dextrose, or the like. The compositions can also be lyophilized. The compositions can contain auxiliary substances such as wetting, dispersing, or emulsifying agents (e.g., methylcellulose), pH buffering agents, gelling or viscosity enhancing additives, preservatives, flavoring agents, colors, and the like, depending upon the route of administration and the preparation desired. Standard texts may in some aspects be consulted to prepare suitable preparations.

Various additives which enhance the stability and sterility of the compositions, including antimicrobial preservatives, antioxidants, chelating agents, and buffers, can be added. Prevention of the action of microorganisms can be ensured by various antibacterial and antifungal agents, for example, parabens, chlorobutanol, phenol, sorbic acid, and the like. Prolonged absorption of the injectable pharmaceutical form can be brought about by the use of agents delaying absorption, for example, aluminum monostearate and gelatin.

The compositions can also be lyophilized. The compositions can contain auxiliary substances such as wetting, dispersing, or emulsifying agents (e.g., methylcellulose), pH buffering agents, gelling or viscosity enhancing additives, preservatives, colors, and the like. Standard texts may in some aspects be consulted to prepare suitable preparations.

The formulations to be used for in vivo administration are generally sterile. Sterility may be readily accomplished, e.g., by filtration through sterile filtration membranes.

### V. METHODS AND USES OF THE ANTIBODIES

In some instances, disclosed herein are methods involving the use of one or more anti-idiotype antibodies. In some aspects, disclosed herein are methods for measuring or detecting a target antibody, such as a CAR or a cell expressing a CAR, and methods for modifying the activity of the target antibody, such as the activity of a CAR or the activity of a cell expressing a CAR. In certain instances, the one or more anti-idiotype antibodies bind, detect, identify, and/or quantify the CAR and/or cells expressing the CAR. In some instances, the methods disclosed herein provide one or more steps of contacting and/or incubating the one or more anti-idiotype antibodies with a cell or a sample containing or thought to be containing cells that express a chimeric antigen receptor (CAR). In some instances, the anti-idiotype antibody is treated, incubated, and/or contacted with the composition or sample under conditions that allow for the formation of a complex between the anti-idiotype antibody and the target antibody, e.g., the CAR. In some aspects, the complex may be utilized for the purposes of detecting, isolating, and/or measuring the CAR. In some instances, the formation of the complex modifies the activity of the target antibody, e.g., the CAR, such as by stimulating receptor signaling activity, or in some instances, antagonizing the activity of the target antibody, e.g., the CAR, by preventing the association of the CAR with an antigen.

### A. Detection/Isolation Methods

In some instances, there are disclosed methods involving use of one or more of the anti-idiotype antibodies, and/or molecules (such as conjugates and complexes) containing one or more of such anti-idiotype antibodies, for detecting, binding, and/or isolating an antibody, e.g., a target antibody. In certain instances, the methods provide one or more steps of contacting, incubating, and/or exposing the one or more anti-idiotype antibodies to a sample and/or composition. In some instances, the sample and/or composition has, is likely to have, and/or is suspected of having a target antibody and/or antigen binding fragment thereof that is bound by and/or recognized by the one or more anti-idiotype antibodies. In certain instances, the antibody or antigen binding fragment thereof that is bound by or recognized by the one or more anti-idiotype antibodies contains one or more fusion domains and/or is a fusion protein. In certain instances, the target antibody and/or antigen binding fragment thereof is a CAR. In certain instances, the anti-idiotype antibody, binds to and/or recognizes an anti-CD19 antibody (*e.g*., antibody SJ25C1 or FMC63), or an antigen-binding fragment thereof, including a chimeric molecule or conjugate including a CAR, containing such anti-CD19 antibody (e.g., antibody fragment).

The methods in some instances include incubating, treating, and/or contacting a sample and/or a composition containing or suspected of containing the target antibody with the anti-idiotype antibody. In certain instances, the incubating is under conditions permissive for binding of the anti-idiotype antibody to the target antibody present in the composition, for example to form a complex containing the anti-idiotype antibody and the target antibody.

In some instances, the sample and/or composition contains or is suspected of containing the target antibody, e.g., a CAR. In certain instances, the sample and/or composition contains or is suspected of containing cells that express the target antibody, e.g., a CAR. In certain instances, the sample is a biological sample. In particular instances, the sample is a serum sample or a blood sample. In some instances, the biological sample contains one or more immune cells. In some instances, the biological sample is or is derived from a tissue, such as connective tissue, muscle tissue, nervous tissue, or epithelial tissue. In particular instances, the biological sample is or is derived from heart, vasculature, salivary glands, esophagus, stomach, liver, gallbladder, pancreas, intestines, colon, rectum, hypothalamus, pituitary gland, pineal gland, thyroid, parathyroid, adrenal gland, kidney, ureter, bladder, urethra, lymphatic system, skin, muscle, brain, spinal cord, nerves, ovaries, uterus, testes, prostate, pharynx, larynx, trachea, bronchi, lungs, diaphragm, bone, cartilage, ligaments, or tendons. In particular instances, the biological sample is taken, collected, and/or obtained from a human subject. In certain instances, the sample contains cells that are live and/or intact. In some instances, the sample is or contains a homogenate and/or cells that have been disrupted and/or lysed. In some instances, the biological sample contains proteins and/or antibodies that have been isolated from blood, serum, and/or a tissue.

In particular instances, the anti-idiotype antibody forms or is capable of forming a complex with a target antibody, e.g., a CAR. In particular instances, the complex is detected, measured, quantified, and/or assessed, for example, to allow for the detection, identification, measurement, and/or quantification of the target antibody, for example in a composition or a sample. In certain instances, the methods include detecting whether a complex is formed between the anti-idiotype antibody and the target antibody in the sample, and/or detecting the presence or absence or level of such binding. In some instances, the complex contains a detectable label. In particular instances, the anti-idiotype antibody is an immunoconjugate that contains a detectable label. In certain instances, the anti-idiotype antibody contains, is conjugated with, bound to, and/or attached to the detectable label. In some instances, the complex contains an antibody that binds to and/or recognizes the anti-idiotype antibody, e.g., a secondary antibody, that in conjugated with, bound to, and/or attached to a detectable label.

In some instances, methods for detecting, quantifying, detecting, and/or assessing a target antibody, for example in a sample or composition, includes detecting a complex of the target antibody and the anti-idiotype antibody. In some instances, the complex contains a detectable label. In certain instances, the complex is probed and/or contacted with a detectable label. In some instances, the complex is detected by any suitable method or means, such as but not limited to flow cytometry, immunocytochemistry, immunohistochemistry, western blot analysis, and ELISA.

In some instances, the target antibody or antigen-binding fragment is bound to a cell or expressed on the surface of a cell. In particular instances, target antibody, e.g., the CAR is not bound or contained within a cell, for example, in some instances, the target antibody is secreted. In certain instances, the antibody has been detached, removed, and/or lysed from the surface of a cell.

In some instances, the target antibody or antigen-binding fragment is contained in a chimeric antigen receptor (CAR), such as a CAR expressed on the surface of a cell. In some instances, the cell is a stem cell, e.g., an iPSC, or an immune cell. In some instances, the immune cell is a T cell, e.g., a CD4+ T cell, a CD8+ T cell, naive T (T_{N}) cell, effector T cell (T_{EFF}), memory T cell, tumor-infiltrating lymphocyte (TIL), immature T cell, mature T cell, helper T cells, cytotoxic T cell, mucosa-associated invariant T (MAIT) cell, naturally occurring and adaptive regulatory T (Treg) cell, helper T cell, such as a TH1 cell, TH2 cell, TH3 cell, TH17 cell, TH9 cell, TH22 cell, follicular helper T cell, alpha/beta T cell, and/or a delta/gamma T cells. In some instances, the cell is from a tissue, e.g., heart, vasculature, salivary glands, esophagus, stomach, liver, gallbladder, pancreas, intestines, colon, rectum, hypothalamus, pituitary gland, pineal gland, thyroid, parathyroid, adrenal gland, kidney, ureter, bladder, urethra, lymphatic system, skin, muscle, brain, spinal cord, nerves, ovaries, uterus, testes, prostate, pharynx, larynx, trachea, bronchi, lungs, diaphragm, bone, cartilage, ligaments, or tendons.

In some instances, the target antibody is an anti-CD19 antibody. In some instances, the target antibody is or is derived from antibody SJ25C1 or an antigen-binding fragment thereof. In some instances, the target antibody or antigen-binding fragment thereof comprises a heavy chain variable region set forth in SEQ ID NO: 23 and/or a light chain variable region set forth in SEQ ID NO: 24. In some instances, the target antibody is or is derived from antibody FMC63 or an antigen-binding fragment thereof. In some instances, the target antibody or antigen-binding fragment thereof comprises a heavy chain variable region set forth in SEQ ID NO: 30 and/or a light chain variable region set forth in SEQ ID NO: 31.

In some instances, there is disclosed a method of detecting a target antibody, such as antibody SJ25C1 or FMC63, or an antigen-binding fragment thereof (and/or chimeric molecules comprising such antibody, e.g., antibody fragment, such as a CAR), comprising contacting a composition comprising the target antibody or antigen-binding fragment with an anti-idiotype antibody or antigen-binding fragment thereof or an anti-idiotype antibody immunoconjugate described herein, and detecting the anti-idiotype antibody bound to the target antibody or antigen-binding fragment. In some instances, the method further includes detecting whether a complex is formed between the anti-idiotype antibody and the target antibody in the composition, such as detecting the presence or absence or level of such binding. In some instances, the target antibody or antigen-binding fragment is bound to a cell or expressed on the surface of a cell and the detecting comprises detecting cells bound with the anti-idiotype antibody. In some instances, the anti-idiotype antibody or antigen-binding fragment thereof is directly or indirectly labeled for detection. In some instances, the target antibody or antigen-binding fragment thereof comprises a heavy chain variable region set forth in SEQ ID NO: 23 and/or a light chain variable region set forth in SEQ ID NO: 24. In some instances, the target antibody or antigen-binding fragment thereof comprises a heavy chain variable region set forth in SEQ ID NO: 30 and/or a light chain variable region set forth in SEQ ID NO: 31.

In some instances, there is disclosed a method of isolating a target antibody, such as antibody SJ25C1 or FMC63, or an antigen-binding fragment thereof (and/or chimeric molecules comprising such antibody, e.g., antibody fragment, such as a CAR), comprising contacting a composition and/or a sample containing or suspected of containing the target antibody or antigen-binding fragment with an anti-idiotype antibody or antigen-binding fragment thereof or an anti-idiotype antibody immunoconjugate described herein, and isolating complexes comprising the anti-idiotype antibody bound to the target antibody or antigen-binding fragment. In certain instances, the target antibody is isolated with an anti-idiotype antibody or antigen binding fragment thereof is an immunoconjugate, such as an immunoconjugate described in Section I-F. In some instances, the target antibody or antigen-binding fragment is bound to a cell or expressed on the surface of a cell and the isolating comprises isolating cells bound with the anti-idiotype antibody. In some instances, the complexes comprising the anti-idiotype antibody are isolated by affinity-based separation. In some instances, the affinity-based separation is selected from the group consisting of immunoaffinity-based separation, magnetic-based separation, and affinity chromatography. In some instances, the target antibody or antigen-binding fragment thereof comprises a heavy chain variable region set forth in SEQ ID NO: 23 and/or a light chain variable region set forth in SEQ ID NO: 24. In some instances, the target antibody or antigen-binding fragment thereof comprises a heavy chain variable region set forth in SEQ ID NO: 30 and/or a light chain variable region set forth in SEQ ID NO: 31.

In some instances, there is disclosed a method of detecting a cell expressing a CAR comprising a target antibody, such as antibody SJ25C1 or FMC63, or an antigen-binding fragment thereof, comprising contacting the cell expressing the CAR with an anti-idiotype antibody or antigen-binding fragment thereof or an anti-idiotype antibody immunoconjugate described herein, and detecting cells bound with the anti-idiotype antibody. In some instances, the anti-idiotype antibody or antigen-binding fragment thereof is directly or indirectly labeled for detection. In some instances, the target antibody or antigen-binding fragment thereof comprises a heavy chain variable region set forth in SEQ ID NO: 23 and/or a light chain variable region set forth in SEQ ID NO: 24. In some instances, the target antibody or antigen-binding fragment thereof comprises a heavy chain variable region set forth in SEQ ID NO: 30 and/or a light chain variable region set forth in SEQ ID NO: 31.

In certain instances, the methods for detecting a target antibody with an anti-idiotype antibody described herein are used to assess the target antibody in a subject. For example, in some instances, disclosed herein are methods of use for the anti-idiotype antibody for assessing, measuring, and/or quantifying the *in vivo* pharmacokinetics, expansion, and/or persistence of a CAR expressing cells of a therapeutic cell composition. In some instances, the *in vivo* pharmacokinetics, expansion, and/or persistence of the cells, and/or changes in cell phenotypes or functional activity of cells, such as CAR expressing cells administered for immunotherapy, *e.g.* CAR-T cell therapy, in the methods disclosed herein, can be measured with the anti-idiotype antibodies disclosed herein. In some instances, the pharmacokinetics, expansion, and/or persistence of the CAR expressing cells are measured, assessed by detecting the presence and/or amount of cells expressing the CAR in the subject and/or in sample obtained from the subject following the administration of the therapeutic cell composition during and/or after the administration of the therapy with an anti-idiotype antibody disclosed herein.

In some aspects, the anti-idiotype antibody is used with flow cytometry to assess the quantity of cells expressing the recombinant receptor (*e.g*., CAR-expressing cells administered for T cell based therapy) in the blood or serum or organ or tissue sample (*e.g.,* disease site, *e.g.,* tumor sample) of the subject. In some aspects, persistence is quantified as the number of CAR-expressing cells per microliter of the sample, *e.g.,* of blood or serum, or per total number of peripheral blood mononuclear cells (PBMCs) or white blood cells or T cells per microliter of the sample. In certain aspects, expansion is quantified as the increase in the number of CAR-expressing cells per microliter between samples, *e.g.,* of blood or serum, or per total number of peripheral blood mononuclear cells (PBMCs) or white blood cells or T cells per microliter of the samples over time. In some instances, the pharmacokinetics, expansion, and/or persistence are measured or assessed by detecting the amount of CAR expressing cells in the subject and/or in samples collected from the subject at multiple time points. In certain instances, the one or more samples are collected, obtained, and/or taken from the subject within 24 hours, 48 hours, 72 hours, 4 days, 5 days, 6 days, 7 days, 10 days, 14 days, 21 days, 3 weeks, 4 weeks, 5 weeks, 6 weeks, 7 weeks, 8 weeks, 9 weeks, 10 weeks, 11 weeks, 12 weeks, 3 months, 4 months, 6 months, one year, or over one year after the therapeutic cell composition is administered.

In some instances, there is disclosed a method of selecting cells expressing a CAR comprising a target antibody, such as antibody SJ25C1 or FMC63, or an antigen-binding fragment thereof, comprising contacting a population of cells comprising cells expressing the CAR with an anti-idiotype antibody or antigen-binding fragment thereof or an anti-idiotype antibody immunoconjugate described herein, and selecting cells bound with the anti-idiotype antibody. In some instances, the cells bound with the anti-idiotype antibody are selected by affinity-based separation. In some instances, the affinity-based separation is selected from the group consisting of immunoaffinity-based separation, flow cytometry, magnetic-based separation, and affinity chromatography. In some instances, the anti-idiotype antibody or antigen-binding fragment thereof or anti-idiotype antibody immunoconjugate is reversibly bound or immobilized to a support or a stationary phase. In some instances, the target antibody or antigen-binding fragment thereof comprises a heavy chain variable region set forth in SEQ ID NO: 23 and/or a light chain variable region set forth in SEQ ID NO: 24. In some instances, the target antibody or antigen-binding fragment thereof comprises a heavy chain variable region set forth in SEQ ID NO: 30 and/or a light chain variable region set forth in SEQ ID NO: 31.

In some instances, there is disclosed a method of validating a CAR comprising a target antibody, such as antibody SJ25C1 or FMC63, or an antigen-binding fragment thereof, comprising a) incubating a sample comprising T cells transduced with the CAR with an anti-idiotype antibody or antigen-binding fragment thereof targeting the CAR; b) determining the percent of cells bound with the anti-idiotype antibody or antigen-binding fragment thereof; and c) validating the CAR based on the percent of anti-idiotype antibody-bound T cells. In some instances, the anti-idiotype antibody is labeled, and anti-idiotype antibody-bound T cells are assayed by flow cytometry. In some instances, the target antibody or antigen-binding fragment thereof comprises a heavy chain variable region set forth in SEQ ID NO: 23 and/or a light chain variable region set forth in SEQ ID NO: 24. In some instances, the target antibody or antigen-binding fragment thereof comprises a heavy chain variable region set forth in SEQ ID NO: 30 and/or a light chain variable region set forth in SEQ ID NO: 31.

Also disclosed are methods involving use of the disclosed anti-idiotype antibodies, and molecules (such as conjugates and complexes) containing one or more of such anti-idiotype antibodies, for informing treatment decisions in an individual, such as by the detection of CARs recognized by the anti-idiotype antibody, such as CARs comprising a target antibody, such as an anti-CD19 antibody (e.g., antibody SJ25C1 or FMC63), or an antigen-binding fragment thereof. In some instances, the methods are for informing treatment decisions in an individual in association with a therapy comprising administration of CAR T cells, such as anti-CD19 CAR T cells. The methods in some instances include incubating and/or probing a biological sample with the anti-idiotype antibody and/or administering the anti-idiotype antibody to the individual. In certain instances, a biological sample includes a cell or tissue or portion thereof, such as tumor or cancer tissue or biopsy or section thereof. In certain instances, the incubating is under conditions permissive for binding of the anti-idiotype antibody to CARs present in the sample. In some instances, the methods further include detecting whether a complex is formed between the anti-idiotype antibody and CARs in the sample, such as detecting the presence or absence or level of such binding. Such a method may be an in vitro or in vivo method.

In one instance, an anti-idiotype antibody is used to determine whether adjustment to a CAR T cell therapy in an individual is necessary, e.g. where low levels of the CAR T cells in the individual indicate the need to adjust the therapy. In some instances, the target antibody is an anti-CD19 antibody. In some instances, the target antibody is or is derived from antibody SJ25C1 or an antigen-binding fragment thereof. In some instances, the target antibody or antigen-binding fragment thereof comprises a heavy chain variable region set forth in SEQ ID NO: 23 and/or a light chain variable region set forth in SEQ ID NO: 24. In some instances, the target antibody is or is derived from antibody FMC63 or an antigen-binding fragment thereof. In some instances, the target antibody or antigen-binding fragment thereof comprises a heavy chain variable region set forth in SEQ ID NO: 30 and/or a light chain variable region set forth in SEQ ID NO: 31.

In some instances, there is disclosed a method of evaluating a CAR T cell therapy in an individual, wherein the CAR comprises a target antibody, such as antibody SJ25C1 or FMC63, or an antigen-binding fragment thereof, comprising incubating a sample from the individual with an anti-idiotype antibody or antigen-binding fragment thereof targeting the CAR and determining the amount of T cells bound with the anti-idiotype antibody, and determining the potential therapeutic benefit of the therapy based on the amount of anti-idiotype antibody-bound T cells. In some instances, the anti-idiotype antibody is labeled, and anti-idiotype antibody-bound T cells are assayed by flow cytometry. In some instances, the sample is a blood-derived sample, or is or is derived from an apheresis or leukapheresis product. In some instances, the target antibody or antigen-binding fragment thereof comprises a heavy chain variable region set forth in SEQ ID NO: 23 and/or a light chain variable region set forth in SEQ ID NO: 24. In some instances, the target antibody or antigen-binding fragment thereof comprises a heavy chain variable region set forth in SEQ ID NO: 30 and/or a light chain variable region set forth in SEQ ID NO: 31.

In some instances, there is disclosed a method of evaluating a CAR T cell therapy in an individual. In some aspects, the CAR comprises a target antibody, such as an antibody that is or is derived from SJ25C1 or FMC63, such as an antigen-binding fragment of full-length SJ25C1 or FMC63. In some instances, the method includes administering an anti-idiotype antibody or antigen-binding fragment thereof targeting the CAR (e.g., targeting the antibody, e.g., antibody fragment, of the CAR) to the individual. In some aspects, the administration is carried out following initiation of a first dose of the therapy. The method can comprise determining the presence of the anti-idiotype antibody in one or more tissues/organs in the individual. In some aspects, the method includes determining the potential therapeutic benefit of the therapy based on the presence of the anti-idiotype antibody in at least one of the one or more tissues/organs. In some instances, the anti-idiotype antibody is labeled, and presence of the anti-idiotype antibody in the individual is determined by imaging in the individual to detect the label. In some instances, determining the presence of the anti-idiotype antibody in one or more tissues/organs in the individual includes or is carried out by determining a level of the anti-idiotype antibody or binding thereof in the one or more tissues/organs.

In some instances, the method further comprises administering the anti-idiotype antibody to the individual following initiation of a second or subsequent dose of the therapy and/or determining the presence of the anti-idiotype antibody in the one or more tissues/organs in the individual. In some aspects, the method further involves determining the potential therapeutic benefit of the therapy based on the difference in the level of the anti-idiotype antibody in at least one of the one or more tissue/organs in the individual between the first and second anti-idiotype antibody administrations. In some instances, the target antibody or antigen-binding fragment thereof comprises a heavy chain variable region set forth in SEQ ID NO: 23 and/or a light chain variable region set forth in SEQ ID NO: 24. In some instances, the target antibody or antigen-binding fragment thereof comprises a heavy chain variable region set forth in SEQ ID NO: 30 and/or a light chain variable region set forth in SEQ ID NO: 31.

In some instances, there is disclosed a method of adjusting a CAR T cell therapy in an individual, wherein the CAR comprises a target antibody, such as antibody SJ25C1 or FMC63, or an antigen-binding fragment thereof. In some aspects, the method includes incubating or contacting a sample from the individual with an anti-idiotype antibody or antigen-binding fragment thereof targeting the CAR. In some aspects, the method includes determining the amount of T cells bound with or to the anti-idiotype antibody. In some aspects, the method includes adjusting the therapy based on the amount of anti-idiotype antibody-bound T cells. In some instances, the anti-idiotype antibody is labeled directly or indirectly. In some aspects of such instances, the anti-idiotype antibody-bound T cells are imaged in vivo or ex vivo, such as, in some cases, by assaying a sample, from the subject administered the CAR-T cells and anti-idiotype antibody, by flow cytometry. In some instances, the sample is a blood-derived sample, and/or is or is derived from an apheresis or leukapheresis product. In some instances, the target antibody or antigen-binding fragment thereof comprises a heavy chain variable region set forth in SEQ ID NO: 23 and/or a light chain variable region set forth in SEQ ID NO: 24. In some instances, the target antibody or antigen-binding fragment thereof comprises a heavy chain variable region set forth in SEQ ID NO: 30 and/or a light chain variable region set forth in SEQ ID NO: 31.

In some instances, there is disclosed a method of adjusting a CAR T cell therapy in an individual. In some aspects, such a method is carried out for a CAR-T cell therapy where the CAR comprises a target antibody, such as antibody SJ25C1 or FMC63, including an antigen-binding fragment of SJ25C1 or FMC63. The method in some instances comprises administering, to the individual, an anti-idiotype antibody or antigen-binding fragment thereof that targets or binds the CAR following initiation of administration of a first dose of the therapy and determining the presence, absence or level of the anti-idiotype antibody in one or more tissues/organs in the individual. In some aspects, the method includes adjusting the therapy based on the presence, absence or level of the anti-idiotype antibody in at least one of the one or more tissues/organs. In some instances, the anti-idiotype antibody is labeled directly or indirectly, and in some such instances the presence of the anti-idiotype antibody in the individual is determined by imaging in the individual to detect the label. In some instances, determining the presence of the anti-idiotype antibody in one or more tissues/organs in the individual comprises determining a level of or for the anti-idiotype antibody in the one or more tissues/organs or binding thereof. In some instances, the method further comprises administering the anti-idiotype antibody to the individual after initiation of a second or subsequent dose of administration of the therapy and in some aspects determining the presence, absence or level of the anti-idiotype antibody in the one or more tissues/organs in the individual, and/or adjusting the therapy based on the observations such as based on the difference in the level of the anti-idiotype antibody in at least one of the one or more tissue/organs in the individual between the first and second anti-idiotype antibody administrations. In some instances, the target antibody or antigen-binding fragment thereof comprises a heavy chain variable region set forth in SEQ ID NO: 23 and/or a light chain variable region set forth in SEQ ID NO: 24. In some instances, the target antibody or antigen-binding fragment thereof comprises a heavy chain variable region set forth in SEQ ID NO: 30 and/or a light chain variable region set forth in SEQ ID NO: 31.

In some aspects, the therapy is adjusted (i) if the number of cells of the T cell therapy detectable in the blood or other biological sample, after having been detectable, is not detectable or is reduced, optionally reduced compared to a preceding time point after administration of the T cell therapy; (ii) the number of cells of the T cell therapy detectable in the blood or other biological sample is decreased by or more than 1.5-fold, 2.0-fold, 3.0-fold, 4.0-fold, 5.0-fold, 10-fold or more the peak or maximum number cells of the T cell therapy detectable in the blood or the biological sample of the subject after initiation of administration of the T cell therapy, optionally the first, second or subsequent dose; (iii) at a time after a peak or maximum level of the cells of the T cell therapy are detectable in the blood of the subject, the number of cells of or derived from the T cells detectable in the blood from the subject is less than less than 10%, less than 5%, less than 1% or less than 0.1% of total peripheral blood mononuclear cells (PBMCs) in the blood of the subject; and/or (iv) if the number of CD3+ or CD8+ cells of the cell therapy detectable in the blood is less than 20 cells per µL, 15 cells per µL, 10 cells per µL, less than 5 cells per µL or less than per 1 cells per µL. In some instances, the therapy is adjusted by administered one or more additional doses of the CAR-T cell therapy, administered an increased dose of the CAR-T cell therapy, administering an alternative CAR-T cell therapy specific for the same or different antigen, administering one or more immunomodulatory agent or other agent for promoting or increasing expansion or persistence of the CAR-T cells.

Various methods known in the art for detecting specific antibody-antigen binding can be used. Exemplary immunoassays include fluorescence polarization immunoassay (FPIA), fluorescence immunoassay (FIA), enzyme immunoassay (EIA), nephelometric inhibition immunoassay (NIA), enzyme linked immunosorbent assay (ELISA), and radioimmunoassay (RIA). An indicator moiety, or label group, can be attached to the anti-idiotype antibodies and is selected so as to meet the needs of various uses of the method which are often dictated by the availability of assay equipment and compatible immunoassay procedures. Exemplary labels include radionuclides (*e.g*. ¹²⁵I, ¹³¹I, ³⁵S, ³H, or ³²P and/or chromium (⁵¹Cr), cobalt (⁵⁷Co), fluorine (¹⁸F), gadolinium (¹⁵³Gd, ¹⁵⁹Gd), germanium (⁶⁸Ge), holmium (¹⁶⁶Ho), indium (¹¹⁵In, ¹¹³In, ¹¹²In, ¹¹¹In), iodine (¹²⁵I, ¹²³I, ¹²¹I), lanthanium (¹⁴⁰La), lutetium (¹⁷⁷Lu), manganese (⁵⁴Mn), molybdenum (⁹⁹Mo), palladium (¹⁰³Pd), phosphorous (³²P), praseodymium (¹⁴²Pr), promethium (¹⁴⁹Pm), rhenium (186Re, 188Re), rhodium (105Rh), rutheroium (97Ru), samarium (¹⁵³Sm), scandium (⁴⁷Sc), selenium (⁷⁵Se), (⁸⁵Sr), sulphur (³⁵S), technetium (⁹⁹Tc), thallium (²⁰¹Ti), tin (¹¹³Sn, ¹¹⁷Sn), tritium (3H), xenon (¹³³Xe), ytterbium (¹⁶⁹Yb, ¹⁷⁵Yb), yttrium (⁹⁰Y),), enzymes (e.g., alkaline phosphatase, horseradish peroxidase, luciferase, or β-glactosidase), fluorescent moieties or proteins (*e.g*., fluorescein, rhodamine, phycoerythrin, GFP, or BFP), or luminescent moieties (*e.g*., Qdot^{™} nanoparticles supplied by the Quantum Dot Corporation, Palo Alto, Calif.). Various general techniques to be used in performing the various immunoassays noted above are known.

In some instances, anti-idiotype antibodies need not be labeled, and the presence thereof can be detected using a labeled antibody which binds to any of the anti-idiotype antibodies.

The anti-idiotype antibodies disclosed herein can be employed in any known assay method, such as competitive binding assays, direct and indirect sandwich assays, and immunoprecipitation assays. Zola, Monoclonal Antibodies: A Manual of Techniques, pp. 147-158 (CRC Press, Inc. 1987).

The anti-idiotype antibodies can also be used for *in vivo* diagnostic assays, such as *in vivo* imaging. Generally, the anti-idiotype antibody is labeled with a radionuclide (such as ¹¹¹In, ⁹⁹Tc, ¹⁴C, ¹³¹I, ¹²⁵I, or ³H) so that the cells or tissue of interest can be localized *in vivo* following administration to an individual.

In some instances, the anti-idiotype antibody or antigen-binding fragment is immobilized or bound to a solid support, wherein one or more target cells comprising CAR-T cells are contacted with the solid support. In some instances, the solid support is a bead. In some instances, the solid support is the surface of a well or plate, e.g., a cell culture plate. In some instances, the solid support is a resin or matrix present in or contained within a chromatography column, for example, to permit chromatographic isolation or selection of CAR+ T cells. In some instances, the anti-idiotype antibody or antigen-binding fragment is or is capable of being reversibly bound to a solid support. In some instances, the solid support is an affinity chromatography matrix comprising one or more binding sites capable of binding, e.g. reversibly binding, to a binding partner present in the anti-idiotypic antibody. In one exemplary instance, the anti-idiotypic antibody comprises a streptavidin-binding peptide or other streptavidin binding moiety capable of binding to a streptavidin or streptavidin mutein molecule present on or immobilized on the solid support, which, in some cases, can be dissociated in the presence of a competition substance, such as biotin. Exemplary of such systems include those described in U.S. published patent application No. US20150024411.

In some aspects, the anti-ID antibodies disclosed herein can be expressed on the surface of a cell. In some aspects, a cell-expressed anti-ID antibody can be used to induce or stimulate a CAR-expressing cell, such as part of a system for selectively growing CAR cells. In some aspects, the anti-ID antibody or antigen-binding fragment thereof is expressed on an artificial antigen presenting cell (aAPC). The anti-ID-expressing aAPCs can be used as reagents for stimulation or expansion of CAR T-cell populations.

Methods for preparing or generating aAPCs are known, see e.g. U.S. Pat. Nos. 6,225,042, 6,355,479, 6,362,001, 6,790,662; 7,754,482; U.S. Patent Application Publication Nos. 2009/0017000 and 2009/0004142; and International Publication No. WO2007/103009. Various aAPCs are known in the art, see e.g., U.S. Patent No. 8,722,400, published application No. US2014/0212446; Butler and Hirano (2014) Immunol Rev., 257(1):10. 1111/imr. 12129; Suhoshki et al. (2007) Mol. Ther., 15:981-988).

aAPCs include features of natural APCs, including expression of an MHC molecule, stimulatory and costimulatory molecule(s), Fc receptor, adhesion molecule(s) and/or the ability to produce or secrete cytokines (e.g. IL-2). Normally, an aAPC is a cell line that lacks expression of one or more of the above, and is generated by introduction (e.g. by transfection or transduction) of one or more of the missing elements necessary for stimulation of a cell, e.g. a CAR-T cell.

In some instances, cells selected to become aAPCs have deficiencies in intracellular antigen-processing, intracellular peptide trafficking, and/or intracellular MHC Class I or Class II molecule-peptide loading. In some aspects, such cells also lack the ability to express an MHC Class I or Class II molecule and/or molecules involve in or related to antigen processing. Exemplary aAPCs either constitute or are derived from a transporter associated with antigen processing (TAP)-deficient cell line, such as an insect cell line. An exemplary cell line is a *Drosophila* cell line, such as a Schneider 2 cell line (see, e.g. Schneider, J. Embryol. Exp. Morph. 1972 Vol 27, pp. 353-365). Illustrative methods for the preparation, growth, and culture of Schneider 2 cells, are provided in U.S. Pat. Nos. 6,225,042, 6,355,479, and 6,362,001.

In some instances, the cell is a K652 cell or a K562-derived cell. In some instances, the cell is the cell line available at ATCC No. CCL-243.

In some aspects, the aAPC is further engineered to express a further molecule to enhance, potentiate or augment stimulation of a CAR-expressing T cell. In some instances, the further molecule is an immune stimulatory ligand, a co-stimulatory ligand, a cytokine or an adhesion molecule. In some instances, the co-stimulatory ligand specifically binds with at least one co-stimulatory molecule present on a T cell. In some instances, the aAPC is generated, e.g. by transduction or transfection, to express one or more of a co-stimulatory signal (e.g. CD7, B7-1 (CD80), B7-2 (CD86), PD-L1, PD-L2, 4-1BBL, OX40L, ICOS-L, ICAM, CD30L, CD40, CD70, CD83, HLA-G, MICA, MICB, HVEM, lymphotoxin beta receptor, ILT3, ILT4, 3/TR6 or a ligand of B7-H3; or an antibody that specifically binds to CD27, CD28, 4-1BB, OX40, CD30, CD40, PD-1, ICOS, LFA-1, CD2, CD7, LIGHT, NKG2C, B7-H3, Toll ligand receptor or a ligand of CD83), a cell adhesion molecule (e.g. ICAM-1 or LFA-3) and/or a cytokine (e.g. IL-2, IL-4, IL-6, IL-7, IL-10, IL-12, IL-15, IL-21, interferon-alpha (IFNα), interferon-beta (IFNβ), interferon-gamma (IPNγ), tumor necrosis factor-alpha (TNFα), tumor necrosis factor-beta (TNFβ), granulocyte macrophage colony stimulating factor (GM-CSF), and granulocyte colony stimulating factor (GCSF)). In some cases, an aAPC does not normally express an MHC molecule, but can be engineered to express an MHC molecule or, in some cases, is or can be induced to express an MHC molecule, such as by stimulation with cytokines. In some cases, aAPCs also can be loaded with a stimulatory or co-stimulatory ligand, which can include, for example, an anti-CD3 antibody, an anti-CD28 antibody or an anti-CD2 antibodyIn some instances, the aAPC expresses a molecule capable of mediating a primary signal in the cell, such as mediated via the T cell receptor/CD3 complex on a T cell. In some instances, the aAPCs comprise a stimulatory ligand that specifically binds with a TCR/CD3 complex such that a primary signal is transduced.

In some instances, because the anti-ID is capable of delivering a signal through the CAR, the aAPC does not express a stimulatory ligand that specifically binds with a TCR/CD3 complex. In some instances, the aAPC does not express a costimulatory molecule.

In some instances, the anti-ID is expressed as a single chain fragment (scFv) for expression on the surface of the cell. The nucleic acid encoding the scFV can be fused to DNA sequences encoding a transmembrane domain. Transmembrane regions of particular use include those derived from the transmembrane region(s) of the alpha, beta or zeta chain of the T-cell receptor, CD28, CD3 epsilon, CD45, CD4, CD5, CD8, CD9, CD16, CD22, CD33, CD37, CD64, CD80, CD86, CD134, CD137, CD154. Alternatively the transmembrane domain may be synthetic, in which case it will comprise predominantly hydrophobic residues such as leucine and valine. In some cases, a triplet of phenylalanine, tryptophan and valine will be found at each end of a synthetic transmembrane domain. Exemplary transmembrane domain include those derived from CD8 or CD28.

### B. Use in Cell Stimulation

In some instances, the disclosed anti-idiotype antibodies or antigen-binding fragments thereof are agonists and/or exhibit specific activity to stimulate cells expressing a target antibody including conjugates or chimeric receptors containing the same, such as an anti-CD19 antibody (*e.g*., antibody SJ25C1 or FMC63), or an antigen-binding fragment thereof. In some instances, disclosed are methods involving use of the disclosed anti-idiotype antibodies, and molecules (such as conjugates and complexes) containing one or more of such anti-idiotype antibodies, for stimulation or activation of CAR-expressing or other chimeric receptor-expressing cells, such as T cells. In some aspects, the CAR or other receptor comprises the target antibody, such as an anti-CD19 antibody (*e.g*., antibody SJ25C1 or FMC63), or an antigen-binding fragment thereof.

In some instances, the methods can be used in connection with methods of preparing genetically engineered T cells, such as in methods of expanding genetically engineered T cells or other cells into which a nucleic acid molecule encoding the chimeric receptor such as the CAR comprising the target antibody has been introduced, e.g., by transfection, transduction, or a non-viral means of nucleic acid transfer, such as transposon-based approaches. In some aspects, the target antibody is an anti-CD19 antibody (*e.g*., antibody SJ25C1 or FMC63), or an antigen-binding fragment thereof. In particular instances, the target antibody is or contains a CAR, e.g., an anti-CD19 CAR. In particular instances, the anti-CD19 CAR contains an scFv that is from and/or is derived from an anti-CD19 antibody such as antibody SJ25C1 or FMC63.

The methods in some instances include incubating a sample comprising T cells transduced with a CAR with the anti-idiotype antibody. In certain instances, the methods further include detecting whether the CAR T cells are activated or stimulated, such as by assessing the viability, proliferation, and/or expression of activation markers in the CAR T cells. In some instances, the target antibody is an anti-CD19 antibody. In some instances, the target antibody is or is derived from antibody SJ25C1 or FMC63 or an antigen-binding fragment thereof. In some instances, the target antibody or antigen-binding fragment thereof comprises a heavy chain variable region set forth in SEQ ID NO: 23 and/or a light chain variable region set forth in SEQ ID NO: 24. In some instances, the target antibody or antigen-binding fragment thereof comprises a heavy chain variable region set forth in SEQ ID NO: 30 and/or a light chain variable region set forth in SEQ ID NO: 31.

In some instances, there is disclosed a method of simulating cells, comprising incubating an input composition comprising cells expressing a CAR comprising a target antibody, such as antibody SJ25C1 or FMC63, or an antigen-binding fragment thereof, with an anti-idiotype antibody or antigen-binding fragment thereof described herein, thereby generating an output composition comprising stimulated cells. In some instances, the incubation is performed under conditions in which the anti-idiotype antibody or antigen-binding fragment thereof binds to the CAR, thereby inducing or modulating a signal in one or more cells in the input composition. In some instances, the cells comprise T cells. In some instances, the T cells comprise CD4+ and/or CD8+ T cells.

In some instances, disclosed herein is a method of stimulating or expanding cells that express a CAR, by incubating an input composition containing cells expressing a CAR with an anti-ID antibody that binds to and/or recognizes the CAR. In some instances, binding between the anti-ID antibody and the CAR induces expansion of the cells expressing the CAR, thereby producing an output composition comprising expanded cells.

In some instances, anti-idiotype antibody is contacted to or incubated with an input composition of one or more cells to generate an output composition. In certain instances, the input cells and/or the input composition is a composition and/or a plurality of cells that are, or are desired to be, treated, incubated, or contacted under conditions that will produce one or more changes to at least a portion of the cells of the input composition, thereby converting the input composition into an output composition. In some instances, the input cells are a composition of immune cells, for example, a composition of T cells that contain cells expressing a CAR. In particular instances, at least a portion of the cells in the input composition are activated, expanded, and/or enriched in the generated output composition by practice of the disclosed methods.

In certain instances, the anti-idiotype antibody expands or enriches the CAR expressing cells of an input composition. In some instances, the input composition comprises eukaryotic cells, such as mammalian cells. In certain instances, the input composition contains human cells. In some instances, the input composition contains cells that are derived from the blood, bone marrow, lymph, or lymphoid organs. In particular instances, the input composition contains cells of the immune system, i.e., cells of innate or adaptive immunity, e.g., myeloid or lymphoid cells, including lymphocytes, typically T cells and/or NK cells. In some instances, the input composition contains stem cells, such as multipotent and pluripotent stem cells, including induced pluripotent stem cells (iPSCs). In particular instances, the input composition contains CD3⁺ cells. In certain instances, the input composition contains CD4⁺ cells. In some instances, the input composition contains CD8⁺ cells. In some instances, the input composition is a composition of CD4+ cells. In particular instances, the input composition is a composition of CD8+ cells.

In some instances, the methods and agents are capable of stimulating T cells deficient in or that have downregulated one or more natural signaling molecules such as one or more costimulatory receptors or antigen receptors or cytokine receptors but that express the chimeric receptor, e.g., the CAR, recognized by the anti-Id antibody. In some instances, cells of the input composition are low or negative for surface expression of CD28 or other costimulatory molecule or other signaling molecule. Thus in some instances, the disclosed agents and methods have certain advantages compared to certain other activation or stimulatory agents or methods that which may require or depend upon surface expression of CD28 or other endogenous signaling molecule, to provide the desired signal and/or the full extent of such signal, e.g., to provide costimulatory signal and/or to achieve full activation. In some instances, the disclosed agents and methods are advantageous in such regards compared to anti-CD3/anti-CD28 reagents (e.g. beads); in some aspects, the disclosed anti-ID antibodies are advantageous in being able to stimulate or achieve a desired effect such as activation or proliferation of cells that are low or negative for CD28 or other natural signaling molecule. In some aspects, signaling through the CAR by stimulation with an anti-ID antibody results in both a primary and secondary (costimulatory) signal via the CAR using only the single reagent. In some instances, the input composition comprises CD3+ cells that express low levels of CD28 or other endogenous signaling molecule. In some instances, the input composition comprises CD3+ cells that are CD28 negative or are negative for other endogenous signaling molecule. In some instances, the anti-ID antibody stimulates activation and/or expansion of cells expressing low levels of CD28 or cells that are CD28 negative. In some instances, the cells are contacted with anti-idiotype antibody or antigen-binding fragment that is immobilized or bound to a solid support. In some instances, the solid support is a bead. In some instances, the solid support is the surface of a well or plate, e.g., a cell culture plate. In some examples, the anti-ID antibody is soluble. In certain instances, the cells are not contacted with anti-CD3/anti-CD28 conjugated reagents prior to contacting the cells with the anti-idiotype antibody or antigen-binding fragment.

In certain instances, the anti-idiotype antibody is applied to, contacted to, or incubated with an input composition of cells that have been transduced or transfected with a nucleotide encoding a CAR. In particular instances, incubating, treating, and/or contacting input cells with the anti-idiotype antibody results in an expansion and/or enrichment of cells expressing the CAR. In particular instances, incubating, treating, and/or contacting input cells with the anti-idiotype antibody does not result in an expansion and/or enrichment of cells that do not express the CAR. In particular instances, incubating, treating, and/or contacting input cells with the anti-idiotype antibody results in an expansion and/or enrichment of cells that do not express the CAR that is at least 50%, at least 75%, at least 85%, at least 90%, at least 95%, at least 99%, at least 99.9% or at least 99.99% less than the expansion and/or enrichment of cells that express the CAR. In some instances, the anti-idiotype antibodies disclosed herein are used to expand CAR expressing cells of an input composition that experienced a low transduction and/or transfection efficiency, and/or that contains a low amount CAR expressing cells. In certain instances, the anti-idiotype antibody selectively expands and/or enriches cells that express a CAR.

Some instances contemplate that the anti-idiotype antibody is more effective for expanding and/or enriching cells of an input composition with a low transduction or transfection efficiency and/or have a low amount of cells that express the CAR than by expanding and/or enriching the cells by polyclonal stimulation, e.g., anti-CD3 and/or anti-CD28 antibody stimulation. In particular instances, polyclonal stimulation results in expansion of cells that express and cells that do not express the CAR in the input composition, and therefore, in some instances, may fail to enrich CAR expressing cells, particular when the input composition has a low number of CAR expressing cells. In contrast, in some instances, incubation with an anti-idiotype antibody results in a selective expansion of CAR expressing cells and will therefore, in certain instances, result in selective expansion and/or enrichment of the CAR expressing cells. In some instances, incubating, contacting, and/or treating input cells with the anti-idiotype antibody results in a greater enrichment and/or expansion of CAR expressing cells than by polyclonal stimulation.

In particular instances, the anti-idiotype antibody is incubated with, applied to, and/or contacted with input cells that were transfected and/or transduced with a lower amount of viral particles, ratio of copies of the viral vector particles to cells, and/or infectious units (IU), than input cells that are expanded and/or enriched by polyclonal stimulation. For example, in some instances, the input composition that is incubated with the anti-idiotype antibody is generated from cells that were transduced with or with at least 0.5, 1, 2, 3, 4, 5, 10, 15, 20, 30, 40, 50, or 60 fewer IU per cell than the input composition that is expanded and/or enriched by polyclonal stimulation. In some instances, the input composition that is incubated with the anti-idiotype antibody is generated from cells that were transduced with a titer of viral vector particles with or with at least 1 × 10⁵ IU/mL, 5 × 10⁵ IU/mL, 1 × 10⁶ IU/mL, 5 x 10⁶ IU/mL, 6 x 10⁶ IU/mL, 7 x 10⁶ IU/mL, 8 × 10⁶ IU/mL, 9 x 10⁶ IU/mL, or 1 × 10⁷ IU/mL less than the input composition that is expanded and/or enriched by polyclonal stimulation.

In particular instances, transducing cells with a high IU/cell will lead to high transduction efficiency but, in some instances, may also lead to transfected cells with a high vector copy number (VCN), which can present safety risks and may not meet regulatory standards. In particular instances, lowering the IU/cell that cells are transduced with will reduce transduction efficiency but will lower VCN. In particular instances, increasing the IU/cell that cells are transduced with will increase transduction efficiency but will also increase VCN.

In some instances, an input composition contains a population of cells that have been transduced or transfected, or cells that are derived from cells that have been transduced or transfected, with one or more nucleic acids encoding a CAR, that is bound by or recognized by the anti-idiotype antibody. In some instances, the input composition contains less than 80%, less than 75%, less than 70%, less than 65%, less than 60%, less than 55%, less than 50%, less than 45%, less than 40%, less than 35%, less than 30%, less than 25%, less than 20%, less than 15%, less than 10%, less than 5%, or less than 1% of the cells are CAR expressing cells. In particular instances, the cells from the input composition have been transfected or transduced as described in Section III. In certain instances, the input cells contains a population of cells that have been transduced or transfected, or cells that are derived from cells that have been transduced or transfected, with one or more nucleic acids encoding an anti-CD19 CAR, such as an anti-CD19 CAR that contains an scFv that is from and/or is derived from an anti-CD19 antibody such as antibody SJ25C1 or FMC63.

In particular instances, the incubation, contacting, or treatment of cells from the input composition with the anti-idiotype antibody is performed under conditions for stimulation, expansion, and/or activation of cells which conditions can include one or more of particular media, temperature, oxygen content, carbon dioxide content, time, agents, e.g., nutrients, amino acids, antibiotics, ions, and/or stimulatory factors, such as cytokines, chemokines, antigens, binding partners, fusion proteins, recombinant soluble receptors, and any other agents designed to activate the cells.

In some instances, the cells of the input composition have been transfected or transduced with one nucleic acid comprising a gene encoding a CAR and the cells are contacted, incubated, or treated with the anti-idiotype antibody that binds to or recognizes the recombinant receptor. In some instances, the cells of the input composition are treated, incubated, or contacted with the anti-idiotype antibody after the cells transduced or transfected nucleic acid encoding a CAR. In particular instances, the cells of the input composition are treated, incubated, or contacted with the anti-idiotype antibody immediately, within about 1 minute, within about 5 minutes, within about 30 minutes, within about 1 hour, within about 2 hours, within about 4 hours, within about 6 hours, within about 8 hours, within about 12 hours, within about 24 hours, within about 2 days, within about 3 days, within about 4 days, within about 5 days, within about 6 days, within about 1 week, within about 2 weeks, within about 3 weeks, within about 4 weeks, within about 5 weeks, or within about 6 weeks after the cells of the input composition have been transduced or transfected.

In some instances, cells of the input composition are treated, incubated, and/or contacted with soluble anti-idiotype antibody, contacted with an antibody that is not crosslinked and/or contacted with an antibody that is not bound or attached to a solid support

In some instances, the methods result in proliferation, activation, stimulation, cytokine release, or other functional outcome such as upregulation of an activation marker or cytokine release or production, of cells expressing the chimeric receptor such as the CAR recognized by the anti-Id antibody. In some aspects, such proliferation or other functional response or readout is induced in such cells to a degree that is similar to or greater than that induced by incubation of the cells with an agent and/or conditions that stimulates proliferation of T cells, such as anti-CD3/CD28 beads and/or crosslinked anti-CD3. In some aspects, the methods do not involve crosslinking of the anti-idiotype antibody. In some aspects of any of the instances, the anti-idiotype agents are capable of inducing the specified proliferation or functional outcome or degree thereof, without crosslinking of the anti-idiotype antibody. In some aspects, anti-idiotype agents herein are advantageous in their ability to stimulate or cause a particular functional outcome of T cells or other immune cells expressing the target receptor, without the need to crosslink the anti-Id antibody or use a secondary agent. In some aspects, the result is achieved with soluble or plate-bound form of the anti-idiotype antibody. In some aspects, the result is achieved with the anti-idiotype antibody coupled to a bead.

. In particular instances, the cells of the input composition are treated, incubated, and/or contacted with between 10 pg/ml and 100 µg/ml, between 1 pg/ml and 1 ng/ml, between 1ng/ml and 1 µg/l between 100 ng/ml and 1.0 µg/ml, between 1 ng/ml and 100 ng/ml, between 10 ng/ml and 1.0 µg/ml, between 100 ng/ml and 10 pg/ml, between 250 ng/ml and 10 µg/ml, between 250 pg/ml and 1 ng/ml, between 1 µg/ml and 10 µg/ml, between 250 ng and about 2.5 µg/ml, or between 1 µg/ml and 10 µg/ml.

In some instances, the anti-idiotype antibody or antigen-binding fragment thereof is immobilized to a solid support, which optionally comprises or is conjugated to a reagent comprising a plurality of binding sites capable of reversibly binding to the anti-idiotype antibody or antigen-binding fragment thereof. In some instances, the solid support is a surface of a plate or a well. In some instances, the anti-idiotype antibody or antigen-binding fragment thereof is immobilized to a soluble reagent, which optionally is or comprises a plurality of binding sites capable of reversibly binding to the anti-idiotype antibody or antigen-binding fragment thereof. In some instances, the reagent comprises a streptavidin mutein. In one exemplary instance, the anti-idiotypic antibody comprises a streptavidin-binding peptide or other streptavidin binding moiety capable of binding to a streptavidin or streptavidin mutein molecule present on or immobilized on the soluble reagent, which, in some cases, can be dissociated in the presence of a competition substance, such as biotin. Exemplary of such systems include those described in PCT published patent application No. WO2015/158868.

In particular instances, the cells of the input composition are treated, incubated, and/or contacted with anti-idiotype antibody that is attached, bound, coated, and/or conjugated to a solid surface or support, e.g., a plate or a well. In certain instances, the anti-idiotype antibody has been attached, bound, coated, and/or conjugated to the solid surface or support by incubating the solid surface or support with a concentration of the anti-idiotype antibody. In particular instances, the solid surface or support is incubated with between 10 ng/ml and 100 µg/ml, between 100 ng/ml and 1.0 µg/ml, between 250 ng/ml and 10 µg/ml, between 250 ng/ml and 1µg/ml, between 1 µg/ml and 10 µg/ml, between 250 ng and 2.5 µg/ml, or between 1 µg/ml and 10 µg/ml the anti-idiotype antibody. In some instances, the solid surface or support is incubated with between 250 ng/ml and 10 µg/ml. In certain instances, the solid surface or support is incubated with or with about 0.25 µg/ml , 0.5 µg/ml, 1.0 µg/ml, 1.25 µg/ml, 2 µg/ml, 2.5 µg/ml, 5 µg/ml or 10 µg/ml the anti-idiotype antibody.

In some instances, the incubation is for at least or about at least 5 minutes, 10 minutes, 30 minutes, 60 minutes, 2 hours, 6 hours, 12 hours, 24 hours, 36, 48 hours, 72 hours or 96 hours. In some instances, the input composition comprises less than or less than about 60%, less than or less than about 50%, less than or less than about 40%, less than or less than about 30%, less than or less than about 20% or less than or less than about 10% CAR-expressing cells as a percentage of the total cells in the composition. In some instances, the number of CAR-expressing cells in the output composition is increased by greater than 1.2-fold, 1.5-fold, 2.0-fold, 3.0-fold, 4.0-fold, 5.0-fold, 10-fold or more compared to the number of CAR-expressing cells in the input composition; and/or the percentage of CAR-expressing in the output composition compared to the total cells in the composition is increased by greater than 10 %, 20 %, 40 %, 50 %, 60 %, 70 %, 80 % or more. In some instances, prior to incubating, the cells are not selected or enriched for CAR-expressing cells. In some instances, the target antibody or antigen-binding fragment thereof comprises a heavy chain variable region set forth in SEQ ID NO: 23 and/or a light chain variable region set forth in SEQ ID NO: 24. In some instances, the target antibody or antigen-binding fragment thereof comprises a heavy chain variable region set forth in SEQ ID NO: 30 and/or a light chain variable region set forth in SEQ ID NO: 31.

In certain instances, the anti-idiotype antibody are contacted or incubated with cells from the input composition, e.g. comprising cells that express a CAR, for an amount of time to expand one or more cells of the input composition, such as to expand cells of the input composition that express the recombinant receptor. In particular instances, the cells from the input composition are contacted, incubated, or treated with the anti-idiotype antibody for at least about 12 hours, at least about 24 hours, at least about 2 days, at least about 3 days, at least about 4 days, at least about 5 days, at least about 6 days, at least about 7 days, at least about 8 days, at least about 9 days, at least about 10 days, at least about 11 days, at least about 12 days, at least about 13 days, at least about14 days, at least about 3 weeks, or at least about 4 weeks. In particular instances, the cells from the input composition are contacted, incubated, or treated with the anti-idiotype antibody for less than about 1 day, less than about 2 days, less than about 3 days, less than about 4 days, less than about 5 days, less than about 6 days, or less than or about 12 days. In some instances, the cells from the input composition are contacted, incubated, or treated with the anti-idiotype antibody for between about 1 day and about 14 days, between about 3 days, and 7 days, or for between 4 days and 6 days.

In particular instances, cells from an input composition, e.g. comprising cells that express a CAR, are incubated, contacted, or treated with anti-idiotype antibody at temperatures greater than room temperature to expand the cells of the input composition that express the recombinant receptor. In some instances, the treatment, incubation, or contacting is performed at a temperature greater than about 25 °C, such as generally greater than or greater than about 32 °C, 35 °C or 37 °C. In some instances, the treatment, contacting, or incubation is performed at a temperature of at or about 37 °C ± 2 °C, such as at a temperature of at or about 37 °C.

In some instances, at least 50%, at least 55%, at least 60%, at least 65%, at least 70%, at least 75%, at least 80%, at least 85%, at least 90%, at least 95%, at least 97%, at least 99%, at least 99.9%, about 100%, or 100% of the cells of the output composition express the CAR.

In particular instances, the number of cells that express the CAR in the output composition that was incubated, treated, and/or contacted with the anti-idiotype antibody is at least 1%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 95%, at least 100%, at least 2-fold, at least 3-fold, at least 4-fold, at least 5-fold, at least 6-fold, at least 7-fold, at least 8-fold, at least 9-fold, at least 10-fold, at least 15-fold, at least 20-fold, at least 25-fold, at least 50-fold, or at least 100-fold greater than the number of cells that express the CAR in the input composition.

In particular instances, the percentage of cells that express the CAR in the output composition that was incubated, treated, and/or contacted with the anti-idiotype antibody is at least 1%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 95%, at least 100%, at least 2-fold, at least 3-fold, at least 4-fold, at least 5-fold, at least 6-fold, at least 7-fold, at least 8-fold, at least 9-fold, at least 10-fold, at least 15-fold, at least 20-fold, at least 25-fold, at least 50-fold, or at least 100-fold greater than the number of cells that express the CAR in the input composition.

In some instances, the number of cells that express the CAR in the output composition that was incubated, treated, and/or contacted with the anti-idiotype antibody is at least 1%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 95%, at least 100%, at least 2-fold, at least 3-fold, at least 4-fold, at least 5-fold, at least 6-fold, at least 7-fold, at least 8-fold, at least 9-fold, at least 10-fold, at least 15-fold, at least 20-fold, at least 25-fold, at least 50-fold, or at least 100-fold greater than the number of cells of an output composition that received polyclonal stimulation, incubation with anti-CD3 and anti-CD28 antibodies.

In certain instances, the percentage of cells that express the CAR in the output composition that was incubated, treated, and/or contacted with the anti-idiotype antibody is at least 1%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 95%, at least 100%, at least 2-fold, at least 3-fold, at least 4-fold, at least 5-fold, at least 6-fold, at least 7-fold, at least 8-fold, at least 9-fold, at least 10-fold, at least 15-fold, at least 20-fold, at least 25-fold, at least 50-fold, or at least 100-fold greater than the number of cells of an output composition that received polyclonal stimulation, incubation with anti-CD3 and anti-CD28 antibodies.

In some instances, the cells that express the CAR in the output composition that was incubated, treated, and/or contacted with the anti-idiotype antibody contain at least a 1%, at least a 5%, at least a 10%, at least a 15%, at least a 20%, at least a 25%, at least a 30%, at least a 35%, at least a 40%, at least a 45%, at least a 50%, at least a 55%, at least a 60%, at least a 65%, at least 70%, at least 75%, at least 80%, at least 85%, at least 95%, or at least a 99% lower VCN than cells of an output composition that received polyclonal stimulation, e.g., incubation with anti-CD3 and anti-CD28 antibodies. In some instances, the average VCN of CAR expressing cells of the output no more than at or about 10, 5, 4, 2.5, 1.5, or 1.

In some instances, such methods can be used as part of the manufacturing, analytic, and/or quality control methods, e.g., in association with the generation of cell therapies expressing recombinant polypeptides containing an antibody or fragment thereof recognized by the anti-idiotype antibody, such as the CAR T cells, for testing purpose, including to test expression and/or potency of the engineered receptor, e.g., in cells engineered for use in therapy in an individual. In certain instances, the cell compositions may be tested at any stage in the process of generating CAR expressing T cells. In particular instances, a sample of cells may be collected from a cell composition at any stage of the process and stored, e.g., by cryofreezing and/or cyropreservation, for later testing and/or analysis. The compositions tested may be pharmaceutical compositions e.g., including those containing the cells and a pharmaceutically acceptable recipient and/or cryopreservative agent.

In some instances, the anti-idiotype antibody stimulates cells expressing a target antibody, e.g., a CAR, *in vivo.* Particular instances contemplate that CAR-T cell therapies are effective in the treatment of cancer and other diseases and disorders. However, in certain contexts, available approaches to CAR-T cell therapy may not always be entirely satisfactory. For example, in some instances, the exposure and persistence of CAR expressing cells in the subject is reduced or declines over time. Yet, observations indicate that, in some cases, increased exposure of the CAR expressing cells may improve efficacy and therapeutic outcomes in CAR-T cell therapy. Thus, in some instances, the anti-idiotype antibody is administered to boost, augment and/or increase persistence and/or expansion of CAR expressing cells.

In certain instances, the anti-idiotype antibody is administered to a subject, such as a subject who has previously been administered a therapeutic cell composition containing CAR expressing cells. In some instances, administering the anti-idiotype antibody to a subject promotes re-expansion of the CAR expressing cells in the subject, which, in some cases, may reach or exceed the initial peak level of expansion prior to the administration of the anti-idiotype antibody. In some instances, the anti-idiotype antibody is administered to modulate expansion and/or persistence of the CAR expressing cells at times when the levels of the CAR expressing cells have declined or are not detectable. In some instances, CAR expressing cells that re re-expanded by the anti-idiotype antibody exhibit increased potency in a subject to which it is administered, for example, as compared to the potency prior to administration of the anti-idiotype antibody.

In certain instances, administration of the anti-idiotype antibody increases or enhances persistence of the CAR expressing cells in the subject. In some instances, the CAR expressing cells are detectable in the subject at or at least 7 days, 14 days, 21 days, 28 days, 35 days, 42 days, 49 days, 56 days, 63 days, 2 months, 3 months, 4 months, 5 months, 6 months, or more than 6 months following the administration of the anti-idiotype antibody. In some aspects, increased exposure of the subject to the cells includes expansion and/or increased expansion of the cells.

In some instances, the CAR-expressing cells expand in the subject following administration of the anti-idiotype antibody. In particular instances, administering the anti-idiotype antibody results in a maximum concentration in the blood or serum or other bodily fluid or organ or tissue of the subject, of at least 100, 500, 1000, 1500, 2000, 5000, 10,000 or 15,000 copies of a nucleic acid encoding the CAR per microgram of DNA, or at least 0.1, 0.2, 0.3, 0.4, 0.5, 0.6, 0.7, 0.8, or 0.9 CAR-expressing cells per microliter. In some instances, the cells expressing the CAR are detected as at least 10, 20, 30, 40, 50, or 60 % of total PBMCs in the blood of the subject, and/or at such a level for at least 1, 2, 3, 4, 5, 6, 7, 8, 9, 10, 11, 12, 24, 36, 48, or 52 weeks following the administration of the anti-idiotype antibody or for 1, 2, 3, 4, or 5, or more years following administration of the anti-idiotype antibody. In some aspects, administering the anti-idiotype antibody results in at least a 2-fold, at least a 4-fold, at least a 10-fold, or at least a 20-fold increase in copies of nucleic acid encoding the recombinant receptor, *e.g.,* CAR, per microgram of DNA, *e.g.,* in the serum, plasma, blood or tissue, e.g., tumor sample, of the subject. In particular instances, administering the anti-idiotype antibody results in at least a 2-fold, at least a 4-fold, at least a 10-fold, or at least a 20-fold increase in the number of circulating CAR expressing cells in the subject.

In some aspects, at least about 1 × 10², at least about 1 × 10³, at least about 1 × 10⁴, at least about 1 × 10⁵, or at least about 1 × 10⁶ or at least about 5 × 10⁶ or at least about 1 × 10⁷ or at least about 5 × 10⁷ or at least about 1 × 10⁸ CAR-expressing cells and/or at least 10, 25, 50, 100, 200, 300, 400, or 500, or 1000 CAR expressing cells per microliter, e.g., at least 10 per microliter, are detectable or are present in the subject or fluid, plasma, serum, tissue, or compartment thereof, such as in the blood, e.g., peripheral blood, or disease site following administration of the anti-idiotype antibody. In some instances, such a number or concentration of cells is detectable in the subject for at least about 20 days, at least about 40 days, or at least about 60 days, or at least about 3, 4, 5, 6, 7, 8, 9, 10, 11, or 12 months, or at least 2 or 3 years, following administration of the anti-idiotype antibody.

Various delivery systems are known and can be used to administer the anti-idiotype antibody. In certain instances, the anti-idiotype antibody is administered by encapsulation in and/or attachment to liposomes, microparticles, and microcapsules. Methods of administering the anti-idiotype antibody include but are not limited to intradermal, intramuscular, intraperitoneal, intravenous, subcutaneous, intranasal, epidural, and oral routes. The anti-idiotype antibody may be administered by any convenient route, for example by infusion, by bolus injection, by absorption through epithelial or mucocutaneous linings (e.g., oral, rectal and intestinal mucosa, etc.), and may be administered together with other biologically active agents. Administration can be systemic or local. Pulmonary administration can also be employed, e.g., by use of an inhaler or nebulizer, and formulation with an aerosolizing agent. In certain instances, the anti-idiotype antibody is delivered in a vesicle, in particular a liposome (Langer, 1990, Science 249:1527-1533), for example a cationic liposome (WO 98140052).

In some instances, there is disclosed a method of producing a cell composition, comprising introducing into cells a nucleic acid molecule encoding a CAR, thereby generating an input composition, and incubating the input composition with an anti-idiotype antibody or antigen-binding fragment thereof specific for the antigen-binding domain of the CAR, thereby producing the cell composition. In some instances, the CAR comprises a target antibody or antigen-binding fragment thereof that specifically binds to CD 19. In some instances, the target antibody is antibody SJ25C1 or FMC63 or an antigen-binding fragment thereof. In some instances, the anti-idiotype antibody or antigen-binding fragment thereof is an anti-idiotype antibody or antigen-binding fragment thereof described herein. In some instances, the anti-idiotype antibody or antigen-binding fragment thereof is an agonist of the CAR. In some instances, the introducing comprises introducing the nucleic acid molecule into the cells by viral transduction, transposition, electroporation, or chemical transfection. In some instances, the introducing comprises introducing the nucleic acid molecule in the cells by transduction with a retroviral vector comprising the nucleic acid molecule, by transduction with a lentiviral vector comprising the nucleic acid molecule, by transposition with a transposon comprising the nucleic acid molecule, or by electroporation or transfection of a vector comprising the nucleic acid molecule.

In some instances, the method further comprises a step of stimulating or activating the cells prior to introducing the nucleic acid molecule encoding the CAR. In some instances, activating the cells comprises contacting the cells with an agonist of CD3 and optionally an agonist of CD28. In some instances, activating the cells comprising contacting the cells with a reagent comprising agonistic anti-CD3 and anti-CD28 antibodies. In some such instances, during at least a portion of the contacting with an anti-CD3/anti-CD28 and/or during at least a portion of introducing the nucleic acid encoding the CAR, the method includes incubating or contacting the cells with the anti-idiotypic antibody or antigen-binding In some instances, the incubation is performed under conditions in which the anti-idiotype antibody or antigen-binding fragment thereof binds to the CAR, thereby inducing or modulating a signal in one or more cells in the input composition. In some instances, the cells comprise T cells.

In some such instances, the T cells comprise CD4+ and/or CD8+ T cells. In some instances, the anti-idiotype antibody or antigen-binding fragment thereof is immobilized to a solid support, which optionally comprises or is conjugated to a reagent comprising a plurality of binding sites capable of reversibly binding to the anti-idiotype antibody or antigen-binding fragment thereof. In some instances, the anti-idiotype antibody or antigen-binding fragment thereof is immobilized to a soluble reagent, which optionally is or comprises a plurality of binding sites capable of reversibly binding to the anti-idiotype antibody or antigen-binding fragment thereof. In some instances, the reagent comprises a streptavidin mutein. In one exemplary instance, the anti-idiotypic antibody comprises a streptavidin-binding peptide or other streptavidin binding moiety capable of binding to a streptavidin or streptavidin mutein molecule present on or immobilized on the soluble reagent, which, in some cases, can be dissociated in the presence of a competition substance, such as biotin. Exemplary of such systems include those described in PCT published patent application No. WO2015/158868. In some instances, the incubation is for at least or about at least 5 minutes, 10 minutes, 30 minutes, 60 minutes, 2 hours, 6 hours, 12 hours, 24 hours, 36, 48 hours, 72 hours or 96 hours. In some instances, the input composition comprises less than or less than about 60%, less than or less than about 50%, less than or less than about 40%, less than or less than about 30%, less than or less than about 20% or less than or less than about 10% CAR-expressing cells as a percentage of the total cells in the composition. In some instances, the number of CAR-expressing cells in the output composition is increased by greater than 1.2-fold, 1.5-fold, 2.0-fold, 3.0-fold, 4.0-fold, 5.0-fold, 10-fold or more compared to the number of CAR-expressing cells in the input composition; and/or the percentage of CAR-expressing in the output composition compared to the total cells in the composition is increased by greater than 10 %, 20 %, 40 %, 50 %, 60 %, 70 %, 80 % or more. In some instances, prior to incubating, the cells are not selected or enriched for CAR-expressing cells. In some instances, the target antibody or antigen-binding fragment thereof comprises a heavy chain variable region set forth in SEQ ID NO: 23 and/or a light chain variable region set forth in SEQ ID NO: 24. In some instances, the target antibody or antigen-binding fragment thereof comprises a heavy chain variable region set forth in SEQ ID NO: 30 and/or a light chain variable region set forth in SEQ ID NO: 31.

In some instances, there is disclosed a method of monitoring activity of a CAR comprising a target antibody, such as antibody SJ25C1 or FMC63, or an antigen-binding fragment thereof, including the steps of incubating a sample comprising T cells transduced with the CAR with an agonistic anti-idiotype antibody or antigen-binding fragment thereof that targets or binds the CAR; and/ordetermining the presence, absence or amount of activation, stimulation and/or expansion of the CAR T cells, thereby monitoring the activity of the CAR-T cells. In some instances, such methods can be used for validating the CAR, in which case the method can include c) validating the CAR based on the level of activation, stimulation and/or expansion of CAR-T cells.

In some instances, activation, stimulation and/or expansion of CAR T cells is assessed by determining the viability, proliferation, and/or expression of T cell activation markers in the CAR T cells following a period of incubation with the anti-idiotype antibody. In some instances, viability of CAR T cells is assessed by calculating the percent of living versus total T cells transduced with the CAR following incubation with the anti-idiotype antibody. In some instances, proliferation of CAR T cells is assessed by dye dilution of a dye used to stain the CAR T cells prior to incubation with the anti-idiotype antibody. In some instances, expression of T cell activation markers is assessed by flow cytometry with staining for antibodies recognizing the T cell activation markers. In some instances, the T cell activation markers are selected from the group consisting of CD25, CD26, CD27, CD28, CD30, CD69, CD71, CD134, CD137, and CD154. In some instances, the period of incubation is from about 1 to about 10 days (such as about any of 1, 2, 3, 4, 5, 6, 7, 8, 9, or 10 days, including any ranges between these values). In some instances, the target antibody or antigen-binding fragment thereof comprises a heavy chain variable region set forth in SEQ ID NO: 23 and/or a light chain variable region set forth in SEQ ID NO: 24. In some instances, the target antibody or antigen-binding fragment thereof comprises a heavy chain variable region set forth in SEQ ID NO: 30 and/or a light chain variable region set forth in SEQ ID NO: 31.

In some instances, there is disclosed a method of monitoring a preparation of CAR T cells, wherein the CAR comprises a target antibody, such as antibody SJ25C1 or FMC63, or an antigen-binding fragment thereof, comprising a) incubating a portion of the preparation with an agonistic anti-idiotype antibody or antigen-binding fragment thereof that targets or binds the CAR; and b) determining the presence, absence or amount of activation, stimulation and/or expansion of the CAR T cells. In some instances, the preparation of CAR-T cells can be cells produced or manufactured under particular conditions desirable to be tested. In some instances, the monitoring is carried out in connection with a release assay, such as for validating the cells prior to administration to a subject. In some aspects, the method further includes c) validating the preparation based on the level of activation of the CAR T cells. In some instances, activation of CAR T cells in the preparation is assessed by determining the viability, proliferation, and/or expression of T cell activation markers in the CAR T cells following a period of incubation with the anti-idiotype antibody. In some instances, viability of CAR T cells is assessed by calculating the percent of living versus total T cells transduced with the CAR following incubation with the anti-idiotype antibody. In some instances, proliferation of CAR T cells is assessed by dye dilution of a dye used to stain the CAR T cells prior to incubation with the anti-idiotype antibody. In some instances, expression of T cell activation markers is assessed by flow cytometry with staining for antibodies recognizing the T cell activation markers. In some instances, the T cell activation markers are selected from the group consisting of CD25, CD26, CD27, CD28, CD30, CD69, CD71, CD134, CD137, and CD154. In some instances, the period of incubation is from about 1 to about 10 days (such as about any of 1, 2, 3, 4, 5, 6, 7, 8, 9, or 10 days, including any ranges between these values). In some instances, the target antibody or antigen-binding fragment thereof comprises a heavy chain variable region set forth in SEQ ID NO: 23 and/or a light chain variable region set forth in SEQ ID NO: 24. In some instances, the target antibody or antigen-binding fragment thereof comprises a heavy chain variable region set forth in SEQ ID NO: 30 and/or a light chain variable region set forth in SEQ ID NO: 31.

### C. Use in Cell Inactivation/Depletion

In some instances, the disclosed anti-idiotype antibodies or antigen-binding fragments thereof are antagonists and/or exhibit specific activity to inhibit, ablate, and/or deplete (for example, kill via antibody-dependent cell-mediated cytotoxicity, ADCC) cells expressing a target antibody, such as an anti-CD19 antibody (*e.g*., antibody SJ25C1 or FMC63), or an antigen-binding fragment thereof. Also disclosed are methods involving use of the disclosed anti-idiotype antibodies, and molecules (such as conjugates and complexes) containing one or more of such anti-idiotype antibodies, for inactivation, ablation, and/or depletion of CAR T cells, wherein the CAR comprises a target antibody, such as an anti-CD19 antibody (e.g., antibody SJ25C1 or FMC63), or an antigen-binding fragment thereof.

The methods in some instances include treating, contacting, and/or incubating a composition and/or a sample comprising T cells transduced with a CAR with the anti-idiotype antibody. In certain instances, the methods further include detecting whether the CAR T cells are inactivated, such as by assessing the viability, proliferation, and/or expression of activation markers in the CAR T cells. In some instances, the methods are in association with a therapy comprising administration of CAR T cells. The methods in some instances include administering the anti-idiotype antibody to an individual. In one instance, an anti-idiotype antibody or conjugate is used to ablate and/or deplete (such as kill) CAR T cells in an individual. In some instances, the target antibody is an anti-CD19 antibody. In some instances, the target antibody is or is derived from antibody SJ25C1 or FMC63 or an antigen-binding fragment thereof. In some instances, the target antibody or antigen-binding fragment thereof comprises a heavy chain variable region set forth in SEQ ID NO: 23 and/or a light chain variable region set forth in SEQ ID NO: 24. In some instances, the target antibody or antigen-binding fragment thereof comprises a heavy chain variable region set forth in SEQ ID NO: 30 and/or a light chain variable region set forth in SEQ ID NO: 31.

In some instances, the anti-idiotype antibody is administered to deplete, reduce, and/or decrease the number of CAR expressing cells in a subject. In particular instances, administration of the anti-idiotype antibody depletes, reduces, and/or decreases the amount of CAR expressing cells, e.g., circulating CAR-T cells, by at least 25%, at least 50%, at least 60%, at least 70%, at least 80%, at least 90%, at least 95%, at least 99%, at least 99.9%, 100% or about 100%. In certain instances, the depletion, reduction, and/or decrease is in relation to an amount of CAR expressing cells in the subject prior to the administration of the anti-idiotype antibody. In particular instances, the depletion, reduction, and/or decrease is in relation to an amount of CAR expressing cells in a subject that is not administered the anti-idiotype antibody. In some instances, CAR expressing cells are not detectable in the subject following administration of the anti-idiotype antibody. In particular instances, the anti-idiotype antibody is a human or humanized antibody.

In some instances, there is disclosed a method of inactivating CAR T cells, wherein the CAR comprises a target antibody, such as antibody SJ25C1 or FMC63, or an antigen-binding fragment thereof, comprising incubating a sample comprising the CAR T cells with an antagonistic anti-idiotype antibody or antigen-binding fragment thereof targeting the CAR, thereby inactivating the CAR T cells in the sample. In some instances, the anti-idiotype antibody is used in an amount sufficient to attenuate the activation of the CAR T cells in the sample. In some instances, the anti-idiotype antibody is used in an amount sufficient to substantially inactivate the CAR T cells in the sample. In some instances, incubation with the anti-idiotype antibody results in ablation and/or depletion of CAR T cells in the sample. In some instances, the anti-idiotype antibody is used in an amount sufficient to result in clearance of the CAR T cells in the sample. In some instances, the target antibody or antigen-binding fragment thereof comprises a heavy chain variable region set forth in SEQ ID NO: 23 and/or a light chain variable region set forth in SEQ ID NO: 24. In some instances, the target antibody or antigen-binding fragment thereof comprises a heavy chain variable region set forth in SEQ ID NO: 30 and/or a light chain variable region set forth in SEQ ID NO: 31.

In some instances, the anti-idiotype antibody is administered to deplete, reduce, and/or decrease the activity of the CAR and/or the CAR expressing cells in a subject. In particular instances, administration of the anti-idiotype antibody reduces and/or decreases stimulation and/or activation of the CAR and/or the CAR expressing cell by at least 25%, at least 50%, at least 60%, at least 70%, at least 80%, at least 90%, at least 95%, at least 99%, at least 99.9%, 100% or about 100%. In certain instances, the reduction and/or decrease is in relation to the stimulation and/or activity of the CAR and/or CAR expressing cells in the subject prior to the administration of the anti-idiotype antibody. In particular instances, the reduction and/or decrease is in relation to stimulation and/or activity of the CAR and/or CAR expressing cells in a subject that is not administered the anti-idiotype antibody. In some instances, the activity and/or stimulation refers to one or more aspects of CAR receptor or CAR T cell activity and may be assessed by any suitable known means, including by any means disclosed herein. In some instances, activity and/or stimulation of the CAR and/or CAR expressing cells are not detectable in the subject following administration of the anti-idiotype antibody. In particular instances, the anti-idiotype antibody is a human or humanized antibody.

In some instances, the anti-idiotype antibody is administered to prevent, reduce, and/or decrease the binding and/or the ability of the CAR and/or the CAR expressing cells to bind to the antigen. In particular instances, administration of the anti-idiotype antibody reduces and/or decreases antigen binding of the CAR and/or the CAR expressing cell by at least 25%, at least 50%, at least 60%, at least 70%, at least 80%, at least 90%, at least 95%, at least 99%, at least 99.9%, 100% or about 100%. In certain instances, the reduction and/or decrease is in relation to the antigen binding and/or the ability of the CAR and/or CAR expressing cells to bind to the antigen in the subject prior to the administration of the anti-idiotype antibody. In particular instances, the reduction, and/or decrease is in relation to antigen binding and/or the ability to bind the antigen of the CAR and/or CAR expressing cells in a subject that is not administered the anti-idiotype antibody. In particular instances, the anti-idiotype antibody is a human or humanized antibody.

In some instances, there is disclosed a method of ablating and/or depleting (such as killing) CAR T cells, wherein the CAR comprises a target antibody, such as antibody SJ25C1 or FMC63, or an antigen-binding fragment thereof, comprising incubating a sample comprising the CAR T cells with an anti-idiotype antibody or antigen-binding fragment thereof targeting the CAR, thereby ablating and/or depleting CAR T cells in the sample. In some instances, the ablating and/or depleting is by antibody-dependent cell-mediated cytotoxicity (ADCC). In some instances, the anti-idiotype antibody is used in an amount sufficient to result in ablation and/or depletion of substantially all of the CAR T cells in the sample. In some instances, the target antibody or antigen-binding fragment thereof comprises a heavy chain variable region set forth in SEQ ID NO: 23 and/or a light chain variable region set forth in SEQ ID NO: 24. In some instances, the target antibody or antigen-binding fragment thereof comprises a heavy chain variable region set forth in SEQ ID NO: 30 and/or a light chain variable region set forth in SEQ ID NO: 31.

In some instances, there is disclosed a method of adjusting a CAR T cell therapy in an individual, wherein the CAR comprises a target antibody, such as antibody SJ25C1 or FMC63, or an antigen-binding fragment thereof, comprising administering an antagonistic anti-idiotype antibody or antigen-binding fragment thereof targeting the CAR to the individual, thereby inactivating the CAR T cells. In some instances, the anti-idiotype antibody is administered in an amount sufficient to attenuate the activation of the CAR T cells in the individual. In some instances, the anti-idiotype antibody is administered in an amount sufficient to substantially inactivate the CAR T cells in the individual. In some instances, administration of the anti-idiotype antibody results in ablation and/or depletion of CAR T cells in the individual. In some instances, the anti-idiotype antibody is administered in an amount sufficient to result in clearance of the CAR T cells in the individual. In some instances, the target antibody or antigen-binding fragment thereof comprises a heavy chain variable region set forth in SEQ ID NO: 23 and/or a light chain variable region set forth in SEQ ID NO: 24. In some instances, the target antibody or antigen-binding fragment thereof comprises a heavy chain variable region set forth in SEQ ID NO: 30 and/or a light chain variable region set forth in SEQ ID NO: 31.

In some instances, there is disclosed a method of adjusting a CAR T cell therapy in an individual, wherein the CAR comprises a target antibody, such as antibody SJ25C1 or FMC63, or an antigen-binding fragment thereof, comprising administering an anti-idiotype antibody immunoconjugate targeting the CAR to the individual, wherein the anti-idiotype antibody immunoconjugate comprises a cytotoxic agent. In some instances, the anti-idiotype antibody immunoconjugate is administered in an amount sufficient to attenuate the CAR T cell therapy in the individual. In some instances, the anti-idiotype antibody immunoconjugate is administered in an amount sufficient to substantially stop the CAR T cell therapy in the individual. In some instances, the anti-idiotype antibody immunoconjugate is administered in an amount sufficient to result in clearance of the CAR T cells in the individual. In some instances, the cytotoxic agent is selected from the group consisting of chemotherapeutic agents or drugs, growth inhibitory agents, toxins (*e.g.*, protein toxins, enzymatically active toxins of bacterial, fungal, plant, or animal origin, or fragments thereof), and radioactive isotopes. In some instances, the target antibody or antigen-binding fragment thereof comprises a heavy chain variable region set forth in SEQ ID NO: 23 and/or a light chain variable region set forth in SEQ ID NO: 24. In some instances, the target antibody or antigen-binding fragment thereof comprises a heavy chain variable region set forth in SEQ ID NO: 30 and/or a light chain variable region set forth in SEQ ID NO: 31.

### D. Use in Binding Assay or Method

Disclosed herein are methods for assessing the presence or absence of a molecule in a sample that binds to a chimeric antigen receptor (CAR), such as the extracellular domain of a CAR or to a portion thereof containing the antigen-binding domain. In some instances, the methods can be used to assess the presence or absence of a humoral response or antibody response in a subject to an administered cell therapy comprising a chimeric antigen receptor (CAR). In some instances, the chimeric antigen receptor comprises a target antibody that is antibody FMC63 or an antigen-binding fragment thereof. In some instances, the chimeric antigen receptor comprises a target antibody that is antibody SJ25C1 or an antigen-binding fragment thereof. In some instances, an anti-idiotype antibody or antigen-binding fragment thereof specific to the extracellular domain of the CAR, such as any described herein, can be used as a positive control in the method.

In particular instances, the method includes contacting a sample with an anti-idiotype antibody or antigen-binding fragment thereof specific to the extracellular domain of the CAR at a concentration of between 10 ng/ml and 100 µg/ml, between 100 ng/ml and 1.0 µg/ml, between 250 ng/ml and 10 µg/ml, between 250 ng/ml and 1µg/ml, between 1 µg/ml and 10 µg/ml, between 250 ng and 2.5 µg/ml, or between 1 µg/ml and 10 µg/ml of the anti-idiotype antibody. In some instances, the concentration of the anti-idiotype antibody between 250 ng/ml and 10 µg/ml. In certain instances, the concentration of the anti-idiotype antibody is about 0.1 µg/ml, 0.25 µg/ml , 0.5 µg/ml, 1.0 µg/ml, 1.25 µg/ml, 2 µg/ml, 2.5 µg/ml, or 5 µg/ml of the anti-idiotype antibody.

In some aspects, adoptive cell therapy may be associated with development of an immune response in the subject to the cells and/or construct administered. For example, in some cases, exposure to a chimeric receptor may be limited by host immune responses against the recombinant receptors expressed by the administered cells, which may prematurely eliminate the cells. It is observed that even in certain subjects having B cell malignancies, who often are immunocompromised, immune responses can be detected that are specific for regions of receptors expressed by cells administered in adoptive cell therapy. For example, subjects, e.g. human subjects, administered cells genetically engineered with a CAR can develop a specific immune response to an immunogenic region of the chimeric region, including regions that may contain non-human sequences (e.g. murine scFv) and or to a region containing the junction between two domains or portions of the chimeric receptor, e.g. the transmembrane and costimulatory domain of the CAR.

In some instances, there are disclosed methods that involve contacting or incubating a binding reagent with a sample from a subject having been administered a cell therapy comprising cell engineered with a chimeric antigen receptor in which the binding reagent is a protein that includes the extracellular domain of the CAR or a portion thereof containing the target antibody or the antigen-binding fragment thereof. In some instances, the methods further include detecting whether a complex is formed between the binding reagent and a molecule, e.g. binding molecule, such as an antibody, present in the sample, and/or detecting the presence or absence or level of such binding. In certain instances, the contacting or incubating is under conditions permissive for binding of the binding reagent to a molecule present in the sample from the subject. In certain aspects, the method can be further carried out on a positive control sample containing an anti-idiotypic antibody or antigen-binding fragment thereof specific for the CAR, such as any as described. In some instances, determining the presence, absence or level of binding of the molecule to the binding reagent can include comparison of the binding or detection to the binding or detection of the positive control sample to the binding reagent.

In some instances, the cell therapy is or comprises genetically engineered cells expressing an anti-CD19 CAR comprising a target antibody that is the antibody SJ25C1 or an antigen-binding fragment thereof, wherein the binding reagent comprises the extracellular domain of the CAR or a portion thereof comprising the SJ25C1 antibody or the antigen-binding fragment thereof. In some instances, the positive control includes an anti-iditoypic antibody as described in subsection I.A.

In some instances, the cell therapy is or comprises genetically engineered cells expressing an anti-CD19 CAR comprising a target antibody that is the antibody FMC63 or an antigen-binding fragment thereof, wherein the binding reagent comprise the extracellular domain of the CAR or a portion thereof comprising theFMC63 antibody or the antigen-binding fragment thereof. In some instances, the positive control includes an anti-iditoypic antibody as described in subsection I.B.

In some instances, the methods include detecting whether a complex is formed between the binding reagent anda molecule, e.g. binding molecule, such as an antibody, present in the sample, and/or detecting the presence or absence or level of such binding. In certain instances, the contacting or incubating is under conditions permissive for binding of the binding reagent to a molecule present in the sample from the subject. In some aspects, the complex is detected by an immunoassay, optionally a sandwich or bridge assay. For examples, the immunoassay is an enzyme-linked immunosorbent assay (ELISA), chemiluminescent, electrochemiluminescent, surface plasmon resonance (SPR)-based biosensor (e.g. , BIAcore), flow cytometry, or Western blot. In some instances, the immunoassay is or or includes meso scale discovery.

In some aspects, the immunoassay is a sandwich assay or a bridge assay. In a sandwich or bridge assay, the binding reagent is a first binding reagent and detecting the presence or absence of a molecule or a complex comprising a molecule includes contacting the complex formed between the first binding reagent and molecule with a second binding reagent in which the second binding reagent is an agent that is able to bind to the same or similar molecule as the first binding reagent. In some instances, the second binding reagent comprises the extracellular domain of the CAR or a portion thereof. In some aspects, the extracellular domain of the CAR or portion thereof of the first binding agent and the second binding agent is the same or substantially the same.

In some instances, the binding reagent, such as the first and/or second binding reagent, is detectably labeled or is capable of producing a detectable signal. The binding reagent, such as the first and/or second binding reagent, is linked, directly or indirectly, to a detectable label. In some instances, the detectable label is or includes a fluorescent label, a chemiluminescent label, an electroluminescent label, a colorimetric label, a bioluminescent label or a radiolabel. In some instances, the binding reagent, such as the first and/or second binding reagent is linked, directly or indirectly, to a SULFO-Tag.In some instances, at least one of the first and second binding reagent is detectably labeled or is capable of producing a detectable signal and the other of the first and second binding reagent is attached or immobilized to a solid support. In some aspects, the first binding reagent is attached or immobilized to a solid support or capable of being attached or immobilized to a solid support. Methods for directly or indirectly attaching an binding reagentto a solid support are well known in the art. Methods of attachment generally include non-specific adsorption of the binding reagent to the solid support or covalent attachment of the binding reagent, typically through a free amine group, to a chemically reactive group on the solid support, such as an activated carboxyl, hydroxyl, or aldehyde group. Methods of attachment also include indirect attachment of the binding reagent to the solid support such as by coating the solid support with a capture reagent, such as streptavidin, and adding affinity labeled binding reagents, such as biotin-labeled reagents, to the solid support so that the interaction between the affinity label (e.g., biotin) and capture reagent (e.g., streptavidin) link the binding reagent to the solid support. In some instances, the first binding reagent is linked, directly or indirectly, to a biotin. In some examples, the first soluble reagent is bound to a solid support coated with streptavidin. In some instances, the second binding reagent is linked, directly or indirectly, to a detectably label, optionally a SULFO-Tag.

In particular instances, the sample is contacted with a first binding reagent that is attached, bound, coated, and/or conjugated to a solid surface or support, e.g., a plate or a well. In certain instances, the first binding reagent has been attached, bound, coated, and/or conjugated to the solid surface or support by indirect attachment of the binding reagent to the solid support such as by coating the solid support with a capture reagent, such as streptavidin, and adding affinity labeled binding reagents, such as biotin-labeled reagents, to the solid support so that the interaction between the affinity label (e.g., biotin) and capture reagent (e.g., streptavidin) link the binding reagent to the solid support. In some instances, the sample is contacted with a second binding reagent that is linked, directly or indirectly, to a SULFO-Tag. In particular instances, the first and/or second binding reagent is used at a concentration of between 10 ng/ml and 100 µg/ml, between 100 ng/ml and 1.0 µg/ml, between 250 ng/ml and 10 µg/ml, between 250 ng/ml and 1µg/ml, between 1 µg/ml and 10 µg/ml, between 250 ng and 2.5 µg/ml, or between 1 µg/ml and 10 µg/ml the anti-idiotype antibody. In some instances, the solid surface or support is incubated with between 250 ng/ml and 10 µg/ml. In certain instances, the solid surface or support is incubated with or with about 0.25 µg/ml , 0.5 µg/ml, 1.0 µg/ml, 1.25 µg/ml, 2 µg/ml, 2.5 µg/ml, 5 µg/ml or 10 µg/ml.

In some instances, the sample from a subject having been administered a cell therapy comprising cell engineered with a chimeric antigen receptor is or comprises any bodily fluid sample from the subject. In some aspects, the sample is or comprises whole blood, serum or plasma. In some instances, the sample is obtained from the subject within or about within 1 hour to 1 year after initiation of administration of the cell therapy or dose of cells, such as within or about within 6 hours, 12 hours, 24 hours, one week, two weeks, three weeks, one month, two months, three months, four months, five months, six months, seven months, eight months, nine months, ten months, eleven months or twelve months. In some aspects, the sample is obtained from the subject from or from about 1 month to 6 months of initiation of administration of the cell therapy, such as 2 months to 6 months or 2 months to 4 months, for example, about or approximately 2 months, 3 months, 4 months, 5 months or 6 months after initiation of administration of the cell therapy.

### VI. ARTICLES OF MANUFACTURE

Also disclosed are articles of manufacture or kits containing the disclosed anti-idiotype antibodies and/or compositions. In some instances, disclosed are articles of manufacture comprising an anti-idiotype antibody or an antigen-binding fragment thereof. In some cases, the anti-idiotype antibody binds an anti-CD19 antibody or antigen-binding fragment thereof, or a chimeric antigen receptor comprising an anti-CD19 antibody or antigen-binding fragment thereof. In some examples, the anti-CD19 antibody is SJ25C1or FMC63. In some aspects, a conjugate containing the anti-idiotype antibodies described herein are disclosed in the articles of manufacture or kits.

In some instances, the kit or article of manufacture includes the anti-idiotype antibody or antigen binding fragment thereof and a binding reagent containing the extracellular domain, or portion of an extracellular domain, of a chimeric antigen receptor (CAR) to which the anti-idiotype antibody binds, such as specifically binds. In some instances, the extracellular domain of the CAR is or includes the anti-CD19 antibody (e.g., FMC63 or SJ25C1) or an antigen-binding fragment thereof.

In some instances, the binding reagent is a first binding reagent and the kit or article of manufacture additionally includes a second binding reagent. In such examples, the second binding reagent is an agent that is able to bind to the same or similar molecule as the first binding reagent. In some instances, the second binding reagent comprises the extracellular domain of the CAR or a portion thereof. In some aspects, the extracellular domain of the CAR or portion thereof of the first binding agent and the second binding agent is the same or substantially the same

In some instances, the binding reagent, or at least one of the first and second binding reagents, is attached to a label (e.g. detectable label) such as a label described herein. In some instances, at least one of the first and second binding reagent is attached to a solid support or capable of being attached to a solid support, such as a solid support described herein. In some aspects, one of the first and second binding reagent is detectably labeled or is capable of producing a detectable signal and the other of the first and seconding binding reagent is attached or immobilized to the solid support. In some instances, the binding reagents are provided as a kit or as part of a system as described elsewhere herein for use in connection with an immunoassay (e.g. sandwich or bridge assay). In some instances, the first binding reagent is bound to a solid support, optionally a streptavidin coated solid support. In some instances, the second soluble protein is linked directly or indirectly to a detectable label, such as SULFO-Tag.

In some instances, the kit further comprises an anti-idiotype antibody or antigen-binding fragment. In some aspects, the anti-idiotype anibody binds an anti-CD19 antibody or antigen-binding fragment thereof, or a chimeric antigen receptor comprising an anti-CD19 antibody or antigen-binding fragment thereof. In some examples, the anti-CD19 antibody is SJ25C 1 or FMC63. In some instances, the anti-idiotype antibody or antigen-binding fragment thereof is described as a positive control sample. In some examples, the positive control sample forms a complex with the first and second soluble proteins or reagents which contains regions of the extracellular domain of a chimeric antigen receptor (CAR) comprising the CD19 antibody or an antigen-binding fragment thereof.

In some instances, the kit or article of manufacturer comprises reagents or components for carrying out any of the disclosed methods. In some instances, the article of manufacture or kit comprises one or more reagent or other materials desirable from a commercial, therapeutic, and user standpoint including secondary antibodies, affinity labels, capture reagents, buffers, diluents, signal detection agents, filters, needles, syringes, capillary tubes, and package inserts with instructions for use.

In some instances, the kits can be disclosed as articles of manufacture that include packing materials for the packaging of the antibodies or compositions thereof or the one or more additional reagents, e.g. binding reagents, or components. For example,the kits can contain containers, bottles, tubes, vial and any packaging material suitable for separating or organizing the components of the kit.

In some instances, the kit includes one or more containers. Suitable containers include, for example, bottles, vials (e.g., dual chamber vials), syringes (such as single or dual chamber syringes) and test tubes. The one or more containers may be formed from a variety of materials such as glass or plastic. The one or more containers hold a composition comprising an antibody or other reagents, e.g. binding reagents, for use in the methods. The article of manufacture or kit herein may comprise the antibodies or reagents in separate containers or in the same container. In some instances, the one or more containers holding the composition may be a single-use vial or a multi-use vial, which, in some cases, may allow for repeat use of the reconstituted composition.

In some instances, the article of manufacture or kit may further comprise a second container comprising a suitable diluent. The article of manufacture or kit may further include other materials desirable from a commercial, therapeutic, and user standpoint, including other buffers, diluents, filters, needles, syringes, therapeutic agents and/or package inserts with instructions for use.

The articles of manufacture may include a container and a label or package insert on or associated with the container. Suitable containers include, for example, bottles, vials, syringes, IV solution bags, etc. The containers may be formed from a variety of materials such as glass or plastic. The container in some instances holds a composition containing an anti-idiotype antibody as disclosed herein which is by itself or is combined with another composition effective for treating, preventing and/or diagnosing a disease or condition. In some instances, the container has a sterile access port. Exemplary containers include an intravenous solution bags, vials, including those with stoppers pierceable by a needle for injection. The article of manufacture may include a first container with a composition contained therein, wherein the composition includes the anti-idiotype antibody. Alternatively, or additionally, the article of manufacture may further include another or the same container comprising an acceptable buffer. It may further include other materials such as other buffers, diluents, filters, needles, and/or syringes.

In some instances, the article of manufacture or kit comprises a solid support, including a solid support formed of glass (e.g., controlled pore glass), polysaccharides (e.g., agarose), polyacrylamides, polystyrene, polyvinyl alcohol, nitrocellulose, cellulose, nylon, silicones and other material well known in the art that is used in a solid support for direct or indirect attachment of a binding reagent as described. Solid supports included in the articles of manufacture or kits disclosed herein include, but are not limited to, a bead, column (e.g., chromatography column, etc.), an array (e.g., microarray, nanoarray, etc.), an assay plate, a cartridge, a stick, a filter, a strip or any other solid support described herein.

In some instances, the article of manufacture or kit may further comprise a second container comprising a suitable diluent. The article of manufacture or kit may further include other materials desirable from a commercial, therapeutic, and user standpoint, including other buffers, diluents, filters, needles, syringes, therapeutic agents and/or package inserts with instructions for use.

In some instances, the kit can, optionally, include instructions. Instructions typically include a tangible expression describing the antibodies and, optionally, other components included in the kit, e.g. binding reagent, and methods for using the antibodies and/or other components in or in conjunction with any of the uses or methods as described. In some instances, the instructions are provided as a label or a package insert, which is on or associated with the container. In some instances, the instructions may indicate directions for reconstitution and/or use of the composition.

In some instances, instructions are provided for using the anti-idiotype antibody to detect an SJ25C1 antibody or antigen-binding fragment thereof or a chimeric antigen receptor comprising the SJ25C1 antibody or antigen-binding fragment thereof, such as in accord with or conjunction with any of the methods or assays as described. In some examples, instructions are provided for using the anti-idiotype antibody to select or enrich, from a population of cells, engineered cells expressing a chimeric antigen receptor (CAR) comprising the antibody SJ25C1 or an antigen-binding fragment thereof. In some examples, instructions are provided for using the anti-idiotype antibody to stimulate an input composition comprising cells expressing a chimeric antigen receptor comprising the SJ25C1 antibody or antigen-binding fragment thereof.

In some instances, instructions are provided for using the anti-idiotype antibody to detect an FMC63 antibody or antigen-binding fragment thereof or a chimeric antigen receptor comprising the FMC63 antibody or antigen-binding fragment thereof, such as in accord with or conjunction with any of the methods or assays as described. In some aspects, instructions are provided for using the anti-idiotype antibody to to select or enrich, from a population of cells, engineered cells expressing a chimeric antigen receptor (CAR) comprising the antibody FMC63 or an antigen-binding fragment thereof. In some instances, instructions are provided for using the anti-idiotype antibody to stimulate an input composition comprising cells expressing a chimeric antigen receptor comprising the FMC63 antibody or antigen-binding fragment thereof.

In some instances, instructions are provided for use of the kit provided to detect a molecule that binds to a chimeric antigen receptor of the cell therapy, such as an antibody, e.g. an antibody produced by a humoral immune response to the chimeric antigen receptor (CAR). In some instances, the instructions are provided for contacting a binding reagent with a sample from a subject having been administered a cell therapy comprising cells engineered with a CAR comprising a target antibody that is the anti-CD19 antibody (e.g., FMC63 or SJ25C1) or an antigen-binding fragment thereof, wherein the binding reagent comprises the extracellular domain of the CAR or a portion of the extracellular domain comprising the target antibody or the antigen-binding fragment thereof. In some aspects, the instructions also specify detecting the presence or absence of a complex comprising the binding reagents and a molecule from the sample that binds to both the first and the second binding reagent, optionally wherein the molecule is or comprises an antibody. In some aspects, the CD19 antibody is SJ25C 1 or FMC63. In some further instances, instructions for using the binding reagents and the positive control sample are provided.

### VII. DEFINITIONS

Unless defined otherwise, all terms of art, notations and other technical and scientific terms or terminology used herein are intended to have the same meaning as is commonly understood by one of ordinary skill in the art to which the claimed subject matter pertains. In some cases, terms with commonly understood meanings are defined herein for clarity and/or for ready reference, and the inclusion of such definitions herein should not necessarily be construed to represent a substantial difference over what is generally understood in the art.

As used herein, reference to a "corresponding form" of an antibody means that when comparing a property or activity of two antibodies, the property is compared using the same form of the antibody. For example, if it is stated that an antibody has greater activity compared to the activity of the corresponding form of a first antibody, that means that a particular form, such as a scFv of that antibody, has greater activity compared to the scFv form of the first antibody.

As used herein, recitation that nucleotides or amino acid positions "correspond to" nucleotides or amino acid positions in a disclosed sequence, such as set forth in the Sequence listing, refers to nucleotides or amino acid positions identified upon alignment with the disclosed sequence to maximize identity using a standard alignment algorithm, such as the GAP algorithm. By aligning the sequences, one skilled in the art can identify corresponding residues, for example, using conserved and identical amino acid residues as guides. In general, to identify corresponding positions, the sequences of amino acids are aligned so that the highest order match is obtained (see, e.g. : Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New. Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991; Carrillo et al. (1988) SIAM J Applied Math 48: 1073).

"Effector functions" refer to those biological activities attributable to the Fc region of an antibody, which vary with the antibody isotype. Examples of antibody effector functions include: C1q binding and complement dependent cytotoxicity (CDC); Fc receptor binding; antibody-dependent cell-mediated cytotoxicity (ADCC); phagocytosis; down regulation of cell surface receptors (e.g. B cell receptor); and B cell activation.

The term "Fc region" herein is used to define a C-terminal region of an immunoglobulin heavy chain that contains at least a portion of the constant region. The term includes native sequence Fc regions and variant Fc regions. In one instance, a human IgG heavy chain Fc region extends from Cys226, or from Pro230, to the carboxyl-terminus of the heavy chain. However, the C-terminal lysine (Lys447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

The terms "full length antibody," "intact antibody," and "whole antibody" are used herein interchangeably to refer to an antibody having a structure substantially similar to a native antibody structure or having heavy chains that contain an Fc region as defined herein.

An "isolated" antibody is one which has been separated from a component of its natural environment. In some instances, an antibody is purified to greater than 95% or 99% purity as determined by, for example, electrophoretic (e.g., SDS-PAGE, isoelectric focusing (IEF), capillary electrophoresis) or chromatographic (e.g., ion exchange or reverse phase HPLC). For review of methods for assessment of antibody purity, see, e.g., Flatman et al., J. Chromatogr. B 848: 79-87 (2007).

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

"Isolated nucleic acid encoding an anti-idiotype antibody" refers to one or more nucleic acid molecules encoding antibody heavy and light chains (or fragments thereof), including such nucleic acid molecule(s) in a single vector or separate vectors, and such nucleic acid molecule(s) present at one or more locations in a host cell.

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

As used herein, "percent (%) amino acid sequence identity" and "percent identity" when used with respect to an amino acid sequence (reference polypeptide sequence) is defined as the percentage of amino acid residues in a candidate sequence (e.g., the subject antibody or fragment) that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared.

An amino acid substitution may include replacement of one amino acid in a polypeptide with another amino acid. The substitution may be a conservative amino acid substitution or a non-conservative amino acid substitution . Amino acid substitutions may be introduced into a binding molecule, e.g., antibody, of interest and the products screened for a desired activity, e.g., retained/improved antigen binding, decreased immunogenicity, or improved ADCC or CDC.

Amino acids generally can be grouped according to the following common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

In some instances, conservative substitutions can involve the exchange of a member of one of these classes for another member of the same class. In some instances, non-conservative amino acid substitutions can involve exchanging a member of one of these classes for another class.

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

The term "package insert" is used to refer to instructions customarily included in commercial packages of therapeutic products, that contain information about the indications, usage, dosage, administration, combination therapy, contraindications and/or warnings concerning the use of such therapeutic products.

As used herein, the singular forms "a," "an," and "the" include plural referents unless the context clearly dictates otherwise. For example, "a" or "an" means "at least one" or "one or more." It is understood that aspects and variations described herein include "consisting" and/or "consisting essentially of" aspects and variations.

Throughout this disclosure, various aspects of the claimed subject matter are presented in a range format. It should be understood that the description in range format is merely for convenience and brevity and should not be construed as an inflexible limitation on the scope of the claimed subject matter. Accordingly, the description of a range should be considered to have specifically disclosed all the possible sub-ranges as well as individual numerical values within that range. For example, where a range of values is provided, it is understood that each intervening value, between the upper and lower limit of that range and any other stated or intervening value in that stated range is encompassed within the claimed subject matter. The upper and lower limits of these smaller ranges may independently be included in the smaller ranges, and are also encompassed within the claimed subject matter, subject to any specifically excluded limit in the stated range. Where the stated range includes one or both of the limits, ranges excluding either or both of those included limits are also included in the claimed subject matter. This applies regardless of the breadth of the range.

The term "about" as used herein refers to the usual error range for the respective value readily known to the skilled person in this technical field. Reference to "about" a value or parameter herein includes (and describes) instances that are directed to that value or parameter *per se.* For example, description referring to "about X" includes description of "X".

The terms "polypeptide" and "protein" are used interchangeably to refer to a polymer of amino acid residues, and are not limited to a minimum length. Polypeptides, including the provided antibodies and antibody chains and other peptides, e.g., linkers, may include amino acid residues including natural and/or non-natural amino acid residues. The terms also include post-expression modifications of the polypeptide, for example, glycosylation, sialylation, acetylation, phosphorylation, and the like. In some aspects, the polypeptides may contain modifications with respect to a native or natural sequence, as long as the protein maintains the desired activity. These modifications may be deliberate, as through site-directed mutagenesis, or may be accidental, such as through mutations of hosts which produce the proteins or errors due to PCR amplification.

As used herein, a composition refers to any mixture of two or more products, substances, or compounds, including cells. It may be a solution, a suspension, liquid, powder, a paste, aqueous, non-aqueous or any combination thereof.

As used herein, a statement that a cell or population of cells is "positive" for a particular marker refers to the detectable presence on or in the cell of a particular marker, typically a surface marker. When referring to a surface marker, the term refers to the presence of surface expression as detected by flow cytometry, for example, by staining with an antibody that specifically binds to the marker and detecting said antibody, wherein the staining is detectable by flow cytometry at a level substantially above the staining detected carrying out the same procedure with an isotype-matched control under otherwise identical conditions and/or at a level substantially similar to that for cell known to be positive for the marker, and/or at a level substantially higher than that for a cell known to be negative for the marker.

As used herein, a statement that a cell or population of cells is "negative" for a particular marker refers to the absence of substantial detectable presence on or in the cell of a particular marker, typically a surface marker. When referring to a surface marker, the term refers to the absence of surface expression as detected by flow cytometry, for example, by staining with an antibody that specifically binds to the marker and detecting said antibody, wherein the staining is not detected by flow cytometry at a level substantially above the staining detected carrying out the same procedure with an isotype-matched control under otherwise identical conditions, and/or at a level substantially lower than that for cell known to be positive for the marker, and/or at a level substantially similar as compared to that for a cell known to be negative for the marker.

### IX. EXAMPLES

The following examples are included for illustrative purposes only and are not intended to limit the scope of the invention.

### Example 1 Generation of anti-idiotype antibodies against an SJ25C1 variable region-derived antibody

This example describes the generation of anti-idiotype antibodies (anti-IDs) recognizing the scFv portion of an exemplary anti-CD19 chimeric antigen receptor (CAR) containing an anti-CD19 scFv with variable heavy and variable light chain regions derived from SJ25C1 (an antibody (in this case an scFv) having a variable region sequence of SEQ ID NO: 23 and 24 separated by a linker set forth in SEQ ID NO: 25), a human CD28-derived extracellular portion, a human CD28-derived transmembrane domain, a human CD28-derived intracellular signaling domain and a human CD3 zeta-derived signaling domain.

### A. Hybridoma Generation and Antibody Screening

Mice were immunized with an extracellular domain (ECD) portion of the CAR (containing an anti-CD19 scFv with variable regions derived from SJ25C1). The ECD portion contained the sequence
EVKLQQSGAELVRPGSSVKISCKASGYAFSSYWMNWVKQRPGQGLEWIGQIYPGD GDTNYNGKFKGQATLTADKSSSTAYMQLSGLTSEDSAVYFCARKTISSVVDFYFDYWGQGTTV TVSSGGGGSGGGGSGGGGSDIELTQSPKFMSTSVGDRVSVTCKASQNVGTNVAWYQQKPGQSP KPLIYSATYRNSGVPDRFTGSGSGTDFTLTITNVQSKDLADYFCQQYNRYPYTSGGGTKLEIKR (SEQ ID NO: 28) and an extracellular portion from CD28 (SEQ ID NO: 27).

Serum isolated from immunized mice was tested by ELISA for its ability to bind to the recombinant soluble ECD portion by detection with a secondary antibody. Hybridoma fusion clones were generated and further characterized by ELISA for binding to the ECD and five (5) positive clones were selected. Each selected hybridoma clone was expanded and antibody purified. To permit detection of the antibody in subsequent assays, each antibody was conjugated with Alexa647.

The antibodies were assessed for the ability to specifically bind to T cells engineered with the anti-CD19 (SJ25C1-derived) CAR, (assessed by comparison with binding to mock-transduced control T cells). T cells were isolated by immunoaffinity-based enrichment from human subjects and cells were activated and transduced with a viral vector encoding the anti-CD19 (SJ25C1-derived) CAR. A series of two fold serial dilutions (starting from 20 µg/mL and diluted to 0.0195 µg/mL) of each of the anti-idiotype antibodies was individually used to detect surface expression of the CAR by flow cytometry. As a positive control, cells were also were assessed for surface expression of the CAR using similar concentrations of a goat-anti-mouse ("GAM") antibody that was able to detect the murine variable region portion of the ECD portion of the CAR. A no antibody control also was tested. The dose-response curve for percent of cells positive for the Alexa647 signal in T cells transduced with the CAR was similar for each of the anti-idiotype antibodies tested and comparable to that for the positive control GAM antibody, none of which recognized mock T cells transduced with the empty vector. The staining index, which is the difference between the positive and background (mock) peak means and the spread of the background peak, of the anti-idiotype antibodies and the GAM antibody was also determined and found to be comparable between the anti-idiotype antibodies and the positive control GAM antibody. Anti-idiotype antibody clone A-1 (anti-ID A-1) was selected for further characterization.

### B. Functional Activity

Erk1/2 phosphorylation in Jurkat cells engineered to express the CAR described above was assessed by flow cytometry following stimulation with the anti-ID A-1 antibody or an anti-CD3 antibody, in each case in the presence or absence of a cross-linker antibody. Following incubation, the cells were fixed with formaldehyde, permeabilized, incubated with an antibody specific for phosphorylated Erk1/2 and analyzed by flow cytometry. As shown in FIG. 1, following the incubation of the cells in the presence of the anti-ID A-1 antibody, an increase in Erk1/2 phosphorylation was observed at a level similar to that observed for stimulation with the anti-CD3 antibody in the presence of a crosslinker. Both antibodies were observed to induce similar degrees of Erk1/2 phosphorylation even in the absence of cross-linking.

Western blotting further was used to compare Erk phosphorylation in CAR-expressing Jurkat cells or parental Jurkat cells not expressing the CAR, following stimulation with anti-ID A-1, an isotype control, or anti-CD3 or in the absence of stimulus, in the presence or absence of a cross-linker antibody. The results indicated that stimulation with the anti-idiotype antibody specifically increased Erk1/2 phosphorylation in Jurkat cells transduced with the anti-CD19 (SJ25C1-derived) CAR, but not in the parental Jurkat cells (in which only background signal was observed, similar to unstimulated and isotype control-stimulated cells), to a degree similar to that induced by the anti-CD3 antibody. In contrast, the anti-CD3 antibody induced phosphorylation in a non-specific manner, i.e., in both the CAR-expressing and parental Jurkat cell.

### C. Sequence Identification

Sequences of the anti-ID A-1 antibody were determined. Total RNA was extracted from hybridoma cells containing the hybridoma clone expressing anti-ID A-1 and cDNA using isotype-specific anti-sense primers or universal primers using the PrimeScript^{™} 1^{st} Strand cDNA Synthesis Kit (Takara, Cat. No. 6110A). RACE PCR was performed to amplify the variable (heavy and light chains) and constant regions of the antibody, which were then separately cloned into a cloning vector and sequenced. Table 2 sets forth the corresponding SEQ ID NOS of the nucleotide or amino acid sequence of the antibody.

**Table 2. anti-ID A-1 sequence**

| | Light Chain | | | Heavy Chain | | |
|---|---|---|---|---|---|---|
| | Full | Variable | Constant | Full | Variable | Constant |
| Nucleotide | SEQ ID NO: 21 | SEQ ID NO: 19 | SEQ ID NO: 20 | SEQ ID NO: 17 | SEQ ID NO: 15 | SEQ ID NO: 16 |
| Amino acid | SEQ ID NO: 7 | SEQ ID NO: 5 | SEQ ID NO: 6 | SEQ ID NO: 3 | SEQ ID NO: 1 | SEQ ID NO: 2 |

### Example 2 Generation of anti-idiotype antibodies against an FMC63-derived antibody

This example describes the generation of anti-idiotype antibodies recognizing the binding domain (scFv) portion of an exemplary anti-CD19 chimeric antigen receptor (CAR) containing an anti-CD19 scFv with VH and VL domains derived from FMC63 (an antibody containing the variable heavy chain (VH) and variable light (VL) light chain sequences set forth in SEQ ID NOs: 30 and 31, respectively). The scFv is set forth in SEQ ID NO:34 and contains the VH and VL regions separated by a linker set forth in SEQ ID NO: 33.

### A. Hybridoma Generation and Antibody Screening

Mice were immunized with a soluble protein containing the scFv portion of this CAR (SEQ ID NO:34), fused to a human IgG1 Fc domain (SEQ ID NO:32; the protein lacked the hinge region). The soluble protein reagent used for immunization is set forth in SEQ ID NO:35.

Serum isolated from immunized mice was tested by ELISA for ability to bind to the scFv-Fc portion by detection with a secondary antibody. Clones were counter-screened against a peptide containing just the Fc domain (SEQ ID NO: 32), to select for anti-idiotype antibodies that did not cross react with the Fc portion of the scFv-Fc used to immunize the mice. Clones were also counter-screened against a construct containing an scFv derived from another CD19 antibody, SJ25C1 (variable region sequences of SEQ ID NO: 23 and 24 separated by linker set forth in SEQ ID NO: 25), fused to the hingeless Fc domain (SEQ ID NO: 32) to further select for anti-idiotype antibodies that did not cross react with a different anti-CD19 antibody.

Hybridoma fusion clones were generated and 12 candidate clones were further characterized by flow cytometry. Peripheral blood mononuclear cells were isolated from human subjects and cells were activated and transduced with a viral vector encoding the anti-CD19 CAR. For flow cytometry, about 1 × 10⁶ cells per 100 µL were incubated with 10 µL of biotin-conjugated anti-idiotype antibody followed by staining with a PE-conjugated streptavidin. As a positive control for surface expression of the CAR, cells were stained with an anti-EGFR antibody to verify expression of the EGFRt transduction marker, which was a surrogate of CAR expression. As a mock control, PBMCs were transduced with an empty vector that did not express the CAR. Cells were gated on CD3⁺ CD4⁺/CD8⁺ PBMCs and fluorescence signal was determined. The results showed that the candidate anti-idiotype antibodies showed specific binding for the CAR on the surface of the PBMCs. To confirm the antibodies were specific for the scFv derived from the anti-CD19 antibody derived from FMC63, similar experiments were performed on cells transduced with an anti-CD19 CAR derived from SJ25C1 as described in Example 1. None of the candidate anti-idiotype antibodies against a FMC63-derived antibody exhibited specific binding for cells expressing a different anti-CD19 CAR containing an scFv derived from SJ25C1. Anti-idiotype antibody clones B-1 (anti-ID B-1) and B-2 (anti-ID B-2) were selected for further characterization.

### B. Sequence Identification

Sequences of the anti-ID B-1 and B-2 antibodies were determined. Total RNA was extracted from hybridoma cells containing the hybridoma clones expressing anti-ID B-1 or anti-ID B-2 and cDNA was generated using isotype-specific anti-sense primers or universal primers using the PrimeScript^{™} 1^{st} Strand cDNA Synthesis Kit (Takara, Cat. No. 6110A). RACE PCR was performed to amplify the variable (heavy and light chains) and constant regions of the antibodies, which were then separately cloned into a cloning vector and sequenced. Table 3 sets forth the corresponding SEQ ID NOS of the nucleotide or amino acid sequences of the antibodies.

**Table 3. anti-ID B-1 and B-2 sequences**

| | Light Chain | | | Heavy Chain | | |
|---|---|---|---|---|---|---|
| | Full (SEQ ID NO) | Variable (SEQ ID NO) | Constant (SEQ ID NO) | Full (SEQ ID NO) | Variable (SEQ ID NO) | Constant (SEQ ID NO) |
| Anti-ID B-1 Nucleotide | 56 | 54 | 55 | 52 | 50 | 51 |
| Anti-ID B-1 Amino acid | 42 | 40 | 41 | 38 | 36 | 37 |
| Anti-ID B-2 Nucleotide | 76 | 75 | 55 | 73 | 71 | 72 |
| Anti-ID B-2 Amino acid | 63 | 62 | 41 | 60 | 58 | 59 |

### Example 3 Effect of plate-bound anti-idiotype antibody-on T cell stimulation

This example describes results following the incubation of CAR T cells in the presence of the SJ25C1-specific anti-idiotype antibody (anti-ID A-1) described in Example 1 or in the presence of the FMC63-derived scFv-specific anti-idiotype antibody (anti-ID B-1) described in Example 2.

Peripheral blood mononuclear cells were isolated from human subjects and cells were activated and transduced with a viral vector encoding an anti-CD19 CAR having a binding domain including an scFv with VH and VL domains derived from FMC63 or derived from SJ25C1. Such vectors were introduced into cells to generate T cells engineered to express a FMC63-derived scFv-containing CAR and T cells engineered to express a SJ25C1-derived scFv-containing CAR, respectively. In the case of the FMC63-derived CAR, the CAR-encoding construct further included a sequence that encoded a truncated EGFR (EGFRt), which served as a surrogate marker for transduction and for CAR expression; the EGFRt-coding region was separated from the CAR-encoding sequence by a T2A skip sequence. The engineered cells were assessed in various assays.

For proliferation studies, engineered T cells were labeled with 50 nM carboxyfluorescein succinimidyl ester (CFSE) or CELL TRACE VIOLET (CTV) dye. Where relevant, expression of the surrogate EGFRt marker was detected by staining with an anti-EGFR antibody. Cells were seeded at a fixed number of cells per well in wells previously coated with 10, 5, 2.5, or 1.25 µg/ml of OKT3 (anti-CD3 antibody), or of anti-idiotype antibodies recognizing SJ25C1 and FMC63 (anti-ID A-1and anti-ID B-1, respectively) in sodium carbonate/bicarbonate buffer (pH 9.0) overnight. Cells seeded in wells with no antibody coating were included as negative controls. The cells were cultured for 4 days and assessed for viability, proliferation, and expression of CD69 and CD25.

Proliferation of anti-CD19 (SJ25C1 or FMC63) CAR-expressing T cells was assessed by dye dilution using flow cytometry. The percent of CD3⁺/CFSE^{low} cells observed following stimulation of anti-CD19 (SJ25C1) CAR-expressing T cells with anti-ID A-1 is shown in FIG. 2A. The percent of CD3+/EGFR+/CFSE^{low} cells observed following stimulation of anti-CD19 (FMC63) CAR-expressing T cells with anti-ID B-1 is shown in FIG. 2B. Incubation in the presence of the SJ25C1-derived scFv-specific anti-ID A-1 antibody was observed to lead to proliferation of anti-CD19 (SJ25C1) CAR-expressing T cells at levels comparable to stimulation with the anti-CD3 antibody (FIG. 2A). In contrast, proliferation observed in anti-CD19 (SJ25C1) CAR-expressing T cells incubated in the presence of the other anti-Id specific for a different anti-CD19 CAR binding domain, the FMC63-derived scFv-specific anti -ID B-1) was similar to that observed in cells in the absence of any stimulatory agent. Similarly, incubation in the presence of the FMC63- derived scFv-specific anti-idiotype antibody anti-ID B-1 was observed to result in proliferation of anti-CD19 (FMC63-derived binding domain) CAR-expressing T cells at levels comparable to stimulation with the anti-CD3 antibody. By contrast, cells incubated in the presence of another anti-Id recognizing a different anti-CD19 CAR (the SJ25C1-derived scFv-specific anti-ID A-1 antibody) was similar to that observed for cells incubated in the absence of stimulatory agents (FIG. 2B). These results demonstrated that each of the anti-ID A-1 and anti-ID B-1 was able to both stimulate and induce proliferation of T cells expressing a CAR containing the respective scFv for which the antibody was specific, and did not induce proliferation of T cells expressing a different CAR directed to the same antigen but not containing the specific binding domain to which the antibody was specific. The results are consistent with the ability of the anti-ID A-1 and anti-ID B-1 to specifically recognize their respective targets and not CARs containing other binding domains against the same antigen.

Results of another assay confirmed the ability of exemplary anti-IDs assessed in this assay to provide a CAR-spec signal to T cells in an anti-ID concentration-specific manner, consistent with the utility of anti-IDs to tune the amount of signal received via the CD19-specific CAR -expressing T cells. In this assay, mock transduced and CAR transduced T cells were labeled with CELL TRACE VIOLET (CTV). Prior to seeding, wells were coated with 0.25, 0.5, or 1 µg/ml of an exemplary anti-idiotype antibody specific for the binding domain of the anti-CD19 CAR. The cells were cultured for 4 days and proliferation was assessed by assessing degree of dye dilution via flow cytometry. FIG. 2C shows an anti-idiotype concentration-dependent induction of proliferation of CD8+ CAR T cells. These results demonstrate the ability to tune the amount of plate-bound exemplary anti-idiotype antibodies provided herein, for example, to tune the amount of signal and/or provide controlled level of stimulation and/or optimize degree of stimulation, of CAR-expressing T cells in various contexts.

To further probe the stimulatory capacity of anti-ID A-1 and anti-ID B-1, T cells transduced with a target anti-CD19 CAR (SJ25C1- or FMC63-derived, respectively) were assessed using flow cytometry for expression of two markers of T cell activation, CD69 and CD25, following stimulation using plate-bound antibodies at concentrations including 1.25, 2.5, 5, and 10 µg/ml. Transduced T cells were gated for EGFR as a surrogate for CAR expression, and CD4+ or CD8+ EGFR+ cells were assessed for CD69 or CD25 expression.

As shown in FIG. 3 for T cells transduced with an SJ25C1-derived anti-CD19 CAR, anti-ID A-1 induced greater expression of CD25 in both CD4⁺ and CD8⁺ T cells than in cells stimulated with the anti-CD3 antibody. Anti-ID A-1 also induced the expression of CD69 in CD4⁺ and CD8⁺ T cells at levels that were similar or slightly less than when cells were stimulated with the anti-CD3 antibody. Expression of CD69 and CD25 was not observed in unstimulated cells or in cells treated with the FMC63-derived scFv-specific anti-ID B-1. As shown in FIG. 4 for T cells transduced with an FMC63-derived anti-CD19 CAR, anti-ID B-1 induced greater expression of CD25 in both CD4⁺/EGFR+ T cells and CD8⁺/EGFR+ T cells than in cells stimulated with the anti-CD3 antibody. Compared to stimulation with anti-CD3 antibody, the expression of CD69 also was induced at greater levels with the anti-ID B-1 antibody in CD4+/EGFR+ T cells and was induced at levels that were similar or slightly higher in CD8+/EGFRT+ T cells. The expression of CD69 was similar or slightly higher at greater antibody concentrations in CD8⁺/EGFR+ T cells. Expression of CD69 and CD25 was not observed in unstimulated cells or in cells treated with the SJ25C1-specific anti-ID A-1 antibody. These results indicate that both anti-ID A-1 and anti-ID B-1 were able to specifically stimulate T cells expressing a CAR containing their target scFv.

### Example 4 Analysis of transgene product-specific host immune responses

A bridge anti-therapeutic antibody (ATA) assay was developed to detect the presence or absence of antibodies, in serum of treated subjects, recognizing administered anti-CD19 CARs. Certain anti-ID antibodies described in Examples 1-3 were used as positive controls and to validate the ability of the assay to detect the presence of such antibodies.

A biotinylated human Fc-fusion protein (biotinylated ECD fusion protein) was used, containing an scFv portion of the CAR with variable regions derived from FMC63 (set forth in SEQ ID NO:34). The biotinylated ECD fusion protein was added to streptavidin-coated wells; the plates were incubated under conditions to allow binding of the fusion protein, and washed. To the biotinylated fusion protein-coated wells, various concentrations of anti-ID B-1 or anti-ID B-2 were added to the wells, and allowed to incubate under conditions to allow specific binding of the antibody. After washing, a sulfo-tagged version of the ECD fusion protein (sulfo-ECD fusion protein) containing the FMC63-derived Fc-scFv fusion. The wells were washed and an electrochemiluminescence (ECL) signal was read on a Meso Scale Discovery (MSD) Sector Imager.

As shown in FIG. 5, the ECL signal increased as the concentration of the anti-ID B-1 and anti-ID B-2 were increased, indicating that the assay could be used to assess presence or absence and levels of antibody in serum samples, with either of the exemplary anti-ID antibodies used as a positive control.

In some embodiments, the ATA assay, using anti-ID B-1 and/or anti-ID B-2 antibody as a positive control, is used to assess the presence or absence of ATA antibodies against CAR in samples from subjects having received infusion of a dose of a cell therapy containing anti-CD19 (FMC63) CAR-expressing T cells. Such ATA may in some contexts potentially be indicative of a host humoral immune response against administered CAR. In an exemplary assay, plasma samples are obtained from subjects at various time-points, such as prior to infusion and/or on days 14 and day 28, and, in some cases, at 3, 6 and/or 12 months, after initiation of administration of the cell therapy. Samples derived from such plasma samples are used alongside control samples containing the anti-ID antibodies, in an assay such as described above.

A similar bridge assay also was generated to assess samples from subjects having received infusion of a dose of a cell therapy containing anti-CD19 (SJ25C1) CAR-expressing T cells. In this assay, the anti-ID A-1 antibody was used as a positive control. The assay was observed to have a sensitivity of less than 100 ng/mL as determined based on the positive control anti-ID antibody and >4- to 5-fold signal over plasma background.

### Example 5 Conjugation of Anti-Idiotype Antibody to Beads

Either anti-ID B-1 or Anti-ID-B1 as described in Example 2 was covalently coupled to the surface of commercially available tosyl-activated magnetic beads (ThermoFisher, Waltham MA) that are superparamagnetic, non-porous, monodisperse, tosylactivated beads. The beads covalently bind primary amino and sulfhydryl groups. Conjugation was performed using beads having a diameter of approximately 2.8 µm (designated M-280) or 4.5 µm (designated M-450).

200 µg anti-ID antibody was added to approximately 1 mL of the tocyl-activated beads (e.g. approximately 4×10⁹ tocyl-activated beads having a diameter of 2.8 µm or about 4×10⁸ tocylactivated beads having a diameter of 4.5 µm) and covalent coupling was performed by overnight incubation at 37°C in phosphate buffered solution (PBS) containing 0.1% human serum albumin (HSA). Beads were washed and resuspended in 1 mL PBS with 0.1% HSA. After conjugation, the bead concentration was determined using a Cellometer.

To assess stability of the anti-ID conjugated beads, the beads were pelleted, the supernatant was removed and loaded on a 4-12% Bis-Tris SDS-PAGE gel and the gel was stained with Coomasie blue. As a control for total protein conjugated on the beads, the pelleted beads were boiled in 4X (lithium dodecyl sulfate) LDS sample buffer at about 70° C for 20 minutes and approximately 12.5 µL or 25 µL boiled supernatant also was run on SDS-PAGE gel and assessed by Coomasie blue. Approximately 2.5 µg or 5.0 µg anti-ID antibody that had not been conjugated to the beads (positive control) or 5 µL 0.1% HSA (negative control) also were assessed by SDS-PAGE and Coomasie staining. No anti-ID antibody was detected in supernatant from conjugated samples that had not been boiled indicating that the conjugation was stable, whereas anti-ID antibody was detected in supernatant from conjugated samples that were boiled.

### Example 6 Assessment of stimulation of T cells cultured with anti-idiotype antibody-conjugated beads

The FMC63-derived scFv-specific anti-ID B-1- conjugated beads were incubated with T cells. CD3-purified T cells were isolated by immunoaffinity-based enrichment from leukapheresis samples from healthy donors. Isolated cells were transduced with a viral vector encoding an anti-CD19 CAR having an scFv derived from FMC63. The viral vector construct further encoded a truncated EGFR (EGFRt), which served as a surrogate marker for CAR expression; the EGFRt-coding region was separated from the CAR sequence by a T2A skip sequence. After transductions, cells were expanded in culture and frozen by cryopreservation.

For T cells stimulation studies, thawed CD4+ or CD8+ CAR-expressing cells were separately seeded at approximately 50,000 total cells per well. In some cases, the culture media was additionally supplemented with cytokines as follows: for CD4+ cells, approximately 1200 IU/mL recombinant IL-7, 20 IU/mL recombinant IL-15 and 100 IU/mL recombinant IL-2; for CD8+ cells, approximately 200 IU/mL recombinant IL-2 and 20 IU/mL recombinant IL-15. Anti-ID B-1 conjugated beads were added to cells at a 1:1 or 1:5 cell:bead ratio and incubated up to 14 days with a 50% media exchange every 2-3 days. As a positive control, cells were cultured with anti-CD3/anti-CD28 magnetic beads at a 3:1 cell:bead ratio in the presence or absence of the indicated cytokines.

At various times of culture, CD4+ or CD8+ transduced cells (as detected by anti-EGFR for expression of the surrogate marker) were assessed for expansion, PD-1 expression and viability.

As shown in FIG.6 and 7, expansion of CD4+ and CD8+ T cells, respectively, was observed when cells were cultured with anti-ID conjugated beads at either the 1:1 or 1:5 cell:bead ratio, particularly in the presence of cytokines. The extent of expansion was greater than when cells were cultured with the control anti-CD3/anti-CD28 magnetic beads.

Surface expression of PD-1 on the CD4+ cell subset was assessed by flow cytometry at days 3, 7, 10 and 14 of culture. As shown in FIG. 8, PD-1 expression was high on cells cultured with anti-CD3/anti-CD28 magnetic beads, however, the levels of PD-1 was substantially lower or undetectable in cells that were cultured with anti-ID conjugated beads.

As shown in FIG. 9, the percent viability of CD4+ and CD8+ T cells cultured with either the 1:1 or 1:5 ratio of anti-ID conjugated beads or the control anti-CD3/anti-CD28 conjugated beads remained consistently high during the period of culture when the cells were additionally cultured in the presence of cytokines. Under all conditions, however, viability of cells decreased in the absence of added cytokines, with the greatest loss in cell viability occurring at later days of cell culture.

### Example 7 Assessment of cytokine production of T cells cultured with anti-idiotype antibody-conjugated beads

T cells engineered with an anti-CD19 CAR having an scFv derived from FMC63 were generated substantially as described in Example 6. Thawed CD8+ T cells were cultured with anti-ID B-1 conjugated beads for 4 hours in the presence of Golgi inhibitor. Intracellular cytokine levels of TNFα, IFNγ, and IL-2 was determined by flow cytometry in either the CAR⁺ T cell subset (as determined by positive surface staining for the surrogate EGFRt transduction marker with anti-EGFR) or the CAR⁻ T cell subset (as determined by negative surface staining for EGFRt with anti-EGFR). As a comparison, CAR+ T cells (EGFR+) also were cultured with CD19-expressing target cells (K562 cells transduced to express CD19, K562-CD19) at an effector:T cell ratio of 1:2.

As shown in FIG. 10A, intracellular cytokine levels of TNFα, IFNγ and IL2 cytokines were induced in CAR+ T cells (EGFRt⁺), but not in CAR- T cells (EGFR⁻), when the cells were cultured in the presence of the anti-ID B-1 conjugated beads. In this study, the extent of stimulation observed in the presence of anti-ID conjugated beads was similar to stimulation of CAR+ T cells using antigen-expressing K562-CD19 cells, which is an alternative CAR-specific stimulation reagent (FIG. 10B). These results demonstrated that anti-ID conjugated to beads are agonistic and specifically stimulate T cells expressing a CAR having an antigen-binding domain recognized by the anti-ID antibody. Further, the bead reagent provides for a better CAR-specific stimulation reagent compared to cell lines, which require cell culture and are prone to lot to lot variability.

### Example 8 Assessment of Expansion after Serial Restimulation

The ability of cells to expand *ex vivo* following repeated stimulations may be a potential surrogate for capacity of CAR+ T cells to persist (e.g. following initial activation) and/or is indicative of function in vivo (Zhao et al. (2015) Cancer Cell, 28:415-28). CAR+ T cells were generated as described above and thawed CD4+ or CD8+ CAR-expressing T cells were plated separately at 50,000 CAR+ cells/well. Anti-ID B-1 conjugated beads were added to cells at a 1:1 or 1:5 cell:bead ratio in the presence or absence of cytokines as described in Example 6. As a control, anti-CD3/anti-CD28 magnetic beads were added to cells at a 3:1 cell:bead ratio in the presence or absence of the cytokines. Cells were harvested every 3-4 days and counted, and restimulated with new target cells using the same culture conditions after resetting cell number to initial seeding density for each round. A total of 4 rounds of stimulation during a 14 day culture period were carried out. For each round of stimulation, the number of doublings was determined.

As shown in FIG. 11, continued cell expansion of CAR-expressing (EGFR+) CD4+ cells was observed after restimulation with anti-ID conjugated beads, although the degree of expansion was greater when the cells were cultured in the presence of cytokines. Also, the extent of expansion was greater than when cells were cultured with anti-CD3/anti-CD28 beads. For CD8+ T cells, similar expansion kinetics was observed when cells were cultured in the presence of either anti-ID conjugated beads or anti-CD3/anti-CD28 beads, although expansion was somewhat greater when cells were cultured with anti-ID conjugated beads, particularly in the absence of added recombinant cytokines.

### Example 9 Further Analysis of CAR-Specific Cell Expansion Using Anti-idiotype antibody-Conjugated Beads

Similar studies as those described in Examples 7 and 8 were performed, except using CAR-expressing T cells generated from two different patient donors. CD3-purified T cells were isolated from peripheral blood mononuclear cells (PBMCs) of two donor patients, transduced with a viral vector encoding an anti-CD19 CAR having an scFv derived from FMC63, expanded in culture, frozen, and thawed.

Thawed CD4+, CD8+ or co-cultures of CD4/CD8 (1:1 ratio) were seeded in wells of a 6-well plate at approximately 5 × 10⁶ total cells/well in culture media that was additionally supplemented with cytokines as follows: for CD4+ cells or CD4+/CD8+ co-cultures media was supplemented with approximately 1200 IU/mL recombinant IL-7, 20 IU/mL recombinant IL-15 and 100 IU/mL recombinant IL-2; for CD8+ cells media was supplemented with 200 IU/mL recombinant IL-2 and 20 IU/mL recombinant IL-15. Anti-ID B-1 conjugated beads were added to cells at a 1:1 cell:bead ratio and incubated up to 9 days with a 50% media exchange every 2-3 days.

At various times of culture, the number of CD4+ or CD8+ transduced cells (as detected by anti-EGFR for expression of the surrogate marker) present in the culture for each condition was assessed and fold expansion or frequency of the CAR-expressing cells as a percent of total cells was determined. Expression of PD-1 and CD25 and cell viability also was determined.

As shown in FIG. 12A, over 60-fold expansion of CAR-expressing (EGFRt+) CD4+ T cells was observed when CD4+ cells were cultured alone with anti-ID conjugated beads. For CD8+ T cells, substantially higher expansion of CAR-expressing (EGFRt+) CD8+ T cells occurred in the presence of anti-ID conjugated beads when the CD8+ T cells were co-cultured with CD4+ cells. As shown in FIG. 12B, the frequency of CAR-expressing (EGFRt+) CD4+ or CD8+ increased during the 9 days of culture in the presence of the anti-ID conjugated beads with >90% of transduced cells (EGFRt+) present in the culture at day 9. Viability of CD4+ and CD8+ cells also remained close to 100% during the culture, with somewhat greater viability of CD8+ T cells observed when co-cultured with CD4+ T cells (FIG. 12C). Similar results were observed for both donors.

Surface expression of PD-1 and CD25 on transduced (EGFRt+) CD4+ or CD8+ cells was assessed by flow cytometry at days 5, 7 and 9 of culture with the anti-ID conjugated beads. As shown in FIG. 13A, PD-1 expression on both CD4+ and CD8+ T cells decreased substantially over time of cultured with anti-ID conjugated beads. As shown in FIG. 13B, CD25 expression also was decreased after 9 days of culture in the presence of anti-ID conjugated beads. Similar results were observed for both donors.

### Example 10 Comparison of Cytokine Levels and Phenotype After Culture with Anti-Idiotype Antibody Conjugated Beads

CD3-purified T cells were isolated from peripheral blood mononuclear cells (PBMCs) of two donor patients, transduced with a viral vector encoding an anti-CD19 CAR having an scFv derived from FMC63 and expanded in culture either with anti-CD3 and CD28 antibody coated beads. After expansion, the expanded T cells were frozen by cryopreservation. For the studies, frozen T cells were thawed and CD4+ and CD8+ T cells assessed for intracellular cytokine levels or surface phenotype (d=0) or thawed CD4+, CD8+, or CD4+/CD8+ co-culture T cells were cultured for an additional 9 days in the presence of anti-ID B-1 conjugated beads prior to assessing intracellular cytokine levels and surface marker phenotype following stimulation by PMA/ionomycin or CD19 transduced K562 cells (d=9).

For assessment of intracellular cytokine levels, Golgi inhibitor was added for 4 hours and then TNFα, IFNγ, and IL-2 was assessed by flow cytometry. For all conditions, the extent of intracellular cytokine expression was substantially greater when cells were stimulated with PMA/ionomycin as compared to CAR-specific stimulation with CD19 transduced K562 cells. As shown in FIG. 14A, the levels of TNFα and IL-2 cytokines were similar in CD4+ or CD8+ cells immediately after thaw compared to corresponding CD4+ or CD8+ cells, or co-cultures of CD4/CD8 T cells, further cultured in the presence of anti-ID conjugated beads for 9 days. Increased levels of IFNγ was observed in thawed CD4+, CD8+ or CD4/CD8 co-culture T cells that were further cultured in the presence of anti-ID conjugated beads for 9 days compared to the level οf IFNγ in CD4+ or CD8+ T cells immediately after thaw. These results demonstrated that T cell function was maintained following 9 day expansion with anti-ID conjugated beads. Similar results were obtained in cells from the two donors.

Surface expression of the activation marker CD25, surface expression of inhibitory receptors PD-1 and LAG-3 and nuclear expression of the proliferation marker Ki-67 were also assessed in CD4+ or CD8+ cells immediately after thaw (d=0) or in CD4+ or CD8+ T cells expanded alone or as a CD4/CD8 co-culture for an additional 9 days in the presence of the anti-ID conjugated beads (d=9). As shown in FIG. 14B, reduced expression of CD25, but not Ki-67, was observed in cells that were further cultured in the presence of anti-ID conjugated beads for 9 days compared to T cells immediately after thaw. The reduced expression of CD25 was substantially greater in CD8+ cells than in CD4+ cells. In addition, decreased expression of PD-1 and LAG-3 also was observed in both CD4+ and CD8+ cells cultured alone or as a CD4/CD8 co-culture after incubation for 9 days with anti-ID conjugated beads compared to expression in cells immediately after thaw. This result demonstrated that after incubation with the anti-ID conjugated beads, the previously frozen transduced cells retained functional ability as evident by the high percentage of cells that were positive for the marker Ki-67, indicative of cell proliferation, but also exhibited a different activation state characterized by the low surface expression for the CD25 activation marker and the inhibitory receptor markers PD-1 and LAG-3.

### Example 11 Detection of CAR-Expressing Cells With Anti-Idiotype Antibodies.

T cell compositions were generated containing CAR-T cells expressing an anti-CD19 CAR containing an anti-CD19 scFv with variable regions derived from the FMC63 antibody, an immunoglobulin spacer, a transmembrane domain derived from CD28, a costimulatory region derived from 4-1BB, and a CD3-zeta intracellular signaling domain. The viral construct encoding the CAR further encoded an EGFRt surrogate marker that was separated from CAR sequence by a T2A skip sequence.

The anti-CD19 CAR expressing cells were spiked in to a sample of cells containing healthy human peripheral blood mononuclear cells (PBMCs). The resulting cell compositions were incubated with different concentrations of the anti-CD19 FMC63 scFv-specific anti-ID B1 or anti-ID B2 antibodies. As a control, samples of each condition were also incubated with a concentration of anti-EGFR antibody, capable of detecting the EGFRt surrogate marker on the transduced cells. Control samples included those only containing the PBMCs without the addition of added CAR-expressing T cells. Geometric mean fluorescence intensity (MFI) was quantified in positively labeled cells to assess antibody staining.

As shown in FIG. 15A, both anti-idiotype antibodies detected the T cells with the anti-CD19 CAR having an scFv with variable regions derived from FMC63, in a concentration-dependent manner. As shown in FIG. 15B, the anti-ID B1 and anti-ID B2 antibodies detected positive cells in the compositions that contained CAR expressing cells and not in compositions that contained only PBMCs. Staining for EGFRt indicated that the ratio of cells expressing the transgene to PBMCs was consistent across all conditions. The results confirmed the ability of the anti-ID B1 and anti-ID B2 antibodies to specifically detect anti-CD19 FMC63 scFv CAR-expressing cells, even among samples containing human PBMCs that did not express a CAR. The results are consistent with an interpretation that the anti-idiotype antibodies may be used to detect CAR expressing cells in samples from subjects having been administered the CAR-expressing cells recognized by the antibodies, such as peripheral blood samples collected from human subjects, for example to measure expansion, trafficking, and/or persistence of such cells over time in various tissues and/or fluids of the subject.

### SEQUENCES

| # | SEQUENCE | ANNOTATION |
|---|---|---|
| 1 | QVQLQQPGSELVRPGGSVKLSCKASDYTFTSYWMHWVRQRPGQ GLEWIGNIYPGSGGTNYDEKFKRKATLTVDTSSSTAYMQLRSLTS EDSAVYYCTREVTTVAYYYSMDYWGQGTSVTVSS | anti-ID VH |
| 2 | AKTTPPSVYPLAPGSAAQTNSMVTLGCLVKGYFPEPVTVTWNSGS LSSGVHTFPAVLQSDLYTLSSSVTVPSSTWPSETVTCNVAHPASST KVDKKIVPRDCGCKPCICTVPEVSSVFIFPPKPKDVLTITLTPKVTC VVVDISKDDPEVQFSWFVDDVEVHTAQTQPREEQFNSTFRSVSEL PIMHQDWLNGKEFKCRVNSAAFPAPIEKTISKTKGRPKAPQVYTIP PPKEQMAKDKVSLTCMITDFFPEDITVEWQWNGQPAENYKNTQPI MDTDGSYFVYSKLNVQKSNWEAGNTFTCSVLHEGLHNHHTEKSL SHSPGK | anti-ID CH |
| 3 | QVQLQQPGSELVRPGGSVKLSCKASDYTFTSYWMHWVRQRPGQ GLEWIGNIYPGSGGTNYDEKFKRKATLTVDTSSSTAYMQLRSLTS EDSAVYYCTREVTTVAYYYSMDYWGQGTSVTVSSAKTTPPSVYP LAPGSAAQTNSMVTLGCLVKGYFPEPVTVTWNSGSLSSGVHTFPA VLQSDLYTLSSSVTVPSSTWPSETVTCNVAHPASSTKVDKKIVPRD CGCKPCICTVPEVSSVFIFPPKPKDVLTITLTPKVTCVVVDISKDDP EVQFSWFVDDVEVHTAQTQPREEQFNSTFRSVSELPIMHQDWLN GKEFKCRVNSAAFPAPIEKTISKTKGRPKAPQVYTIPPPKEQMAKD KVSLTCMITDFFPEDITVEWQWNGQPAENYKNTQPIMDTDGSYFV YSKLNVQKSNWEAGNTFTCSVLHEGLHNHHTEKSLSHSPGK | anti-ID heavy chain |
| 4 | MGWSSIILFLVATASGVHS | anti-ID HC signal sequence |
| 5 | DIQMTQTTSSLSASLGDRVTISCRASQDISNYLNWYQQKPDGTVK LLIYYTSRLHSGVPSRFSGSGSGTDYSLTISNLEQEDIATYFCQQGK TVPFTFGSGTKLEIK | anti-ID VL |
| 6 | RADAAPTVSIFPPSSEQLTSGGASVVCFLNNFYPKDINVKWKIDGS ERQNGVLNSWTDQDSKDSTYSMSSTLTLTKDEYERHNNYTCEAT HKTSTSPIVKSFNRNEC | anti-ID CL |
| 7 | DIQMTQTTSSLSASLGDRVTISCRASQDISNYLNWYQQKPDGTVK LLIYYTSRLHSGVPSRFSGSGSGTDYSLTISNLEQEDIATYFCQQGK TVPFTFGSGTKLEIKRADAAPTVSIFPPSSEQLTSGGASVVCFLNNF YPKDINVKWKIDGSERQNGVLNSWTDQDSKDSTYSMSSTLTLTK DEYERHNNYTCEATHKTSTSPIVKSFNRNEC | anti-ID light chain |
| 8 | MMSSAQFLGLLLLCFQGTRC | anti-ID LC signal sequence |
| 9 | SYWMH | anti-ID HC-CDR1 |
| 10 | NIYPGSGGTNYDEKFKR | anti-ID HC-CDR2 |
| 11 | EVTTVAYYYSMDY | anti-ID HC-CDR3 |
| 12 | RASQDISNYLN | anti-ID LC-CDR1 |
| 13 | YTSRLHS | anti-ID LC-CDR2 |
| 14 | QQGKTVPFT | anti-ID LC-CDR3 |
| 15 | CAGGTCCAACTGCAACAACCTGGGTCTGAGCTGGTGAGGCCTG | anti-ID VH |
| | GAGGTTCAGTGAAGCTGTCCTGCAAGGCTTCTGACTACACTTTC ACCAGCTACTGGATGCACTGGGTGAGGCAGAGGCCTGGACAA GGCCTTGAGTGGATTGGAAATATTTATCCTGGTAGTGGTGGTA CTAACTACGATGAGAAGTTCAAGAGGAAGGCCACACTGACTGT AGACACATCCTCCAGCACAGCCTACATGCAGCTCCGCAGCCTG ACATCTGAGGACTCTGCGGTCTATTACTGTACAAGAGAGGTTA CTACAGTAGCTTATTACTATTCTATGGACTACTGGGGTCAAGG AACCTCAGTCACCGTCTCCTCA | |
| 16 | GCCAAAACGACACCCCCATCTGTCTATCCACTGGCCCCTGGAT CTGCTGCCCAAACTAACTCCATGGTGACCCTGGGATGCCTGGT CAAGGGCTATTTCCCTGAGCCAGTGACAGTGACCTGGAACTCT GGATCCCTGTCCAGCGGTGTGCACACCTTCCCAGCTGTCCTGCA GTCTGACCTCTACACTCTGAGCAGCTCAGTGACTGTCCCCTCCA GCACCTGGCCCAGCGAGACCGTCACCTGCAACGTTGCCCACCC GGCCAGCAGCACCAAGGTGGACAAGAAAATTGTGCCCAGGGA TTGTGGTTGTAAGCCTTGCATATGTACAGTCCCAGAAGTATCAT CTGTCTTCATCTTCCCCCCAAAGCCCAAGGATGTGCTCACCATT ACTCTGACTCCTAAGGTCACGTGTGTTGTGGTAGACATCAGCA AGGATGATCCCGAGGTCCAGTTCAGCTGGTTTGTAGATGATGT GGAGGTGCACACAGCTCAGACGCAACCCCGGGAGGAGCAGTT CAACAGCACTTTCCGCTCAGTCAGTGAACTTCCCATCATGCACC AGGACTGGCTCAATGGCAAGGAGTTCAAATGCAGGGTCAACA GTGCAGCTTTCCCTGCCCCCATCGAGAAAACCATCTCCAAAAC CAAAGGCAGACCGAAGGCTCCACAGGTGTACACCATTCCACCT CCCAAGGAGCAGATGGCCAAGGATAAAGTCAGTCTGACCTGC ATGATAACAGACTTCTTCCCTGAAGACATTACTGTGGAGTGGC AGTGGAATGGGCAGCCAGCGGAGAACTACAAGAACACTCAGC CCATCATGGACACAGATGGCTCTTACTTCGTCTACAGCAAGCT CAATGTGCAGAAGAGCAACTGGGAGGCAGGAAATACTTTCAC CTGCTCTGTGTTACATGAGGGCCTGCACAACCACCATACTGAG AAGAGCCTCTCCCACTCTCCTGGTAAATGA | anti-ID CH |
| 17 | CAGGTCCAACTGCAACAACCTGGGTCTGAGCTGGTGAGGCCTG GAGGTTCAGTGAAGCTGTCCTGCAAGGCTTCTGACTACACTTTC ACCAGCTACTGGATGCACTGGGTGAGGCAGAGGCCTGGACAA GGCCTTGAGTGGATTGGAAATATTTATCCTGGTAGTGGTGGTA CTAACTACGATGAGAAGTTCAAGAGGAAGGCCACACTGACTGT AGACACATCCTCCAGCACAGCCTACATGCAGCTCCGCAGCCTG ACATCTGAGGACTCTGCGGTCTATTACTGTACAAGAGAGGTTA CTACAGTAGCTTATTACTATTCTATGGACTACTGGGGTCAAGG AACCTCAGTCACCGTCTCCTCAGCCAAAACGACACCCCCATCT GTCTATCCACTGGCCCCTGGATCTGCTGCCCAAACTAACTCCAT GGTGACCCTGGGATGCCTGGTCAAGGGCTATTTCCCTGAGCCA GTGACAGTGACCTGGAACTCTGGATCCCTGTCCAGCGGTGTGC ACACCTTCCCAGCTGTCCTGCAGTCTGACCTCTACACTCTGAGC AGCTCAGTGACTGTCCCCTCCAGCACCTGGCCCAGCGAGACCG TCACCTGCAACGTTGCCCACCCGGCCAGCAGCACCAAGGTGGA CAAGAAAATTGTGCCCAGGGATTGTGGTTGTAAGCCTTGCATA TGTACAGTCCCAGAAGTATCATCTGTCTTCATCTTCCCCCCAAA GCCCAAGGATGTGCTCACCATTACTCTGACTCCTAAGGTCACG TGTGTTGTGGTAGACATCAGCAAGGATGATCCCGAGGTCCAGT TCAGCTGGTTTGTAGATGATGTGGAGGTGCACACAGCTCAGAC | anti-ID heavy chain |
| | GCAACCCCGGGAGGAGCAGTTCAACAGCACTTTCCGCTCAGTC AGTGAACTTCCCATCATGCACCAGGACTGGCTCAATGGCAAGG AGTTCAAATGCAGGGTCAACAGTGCAGCTTTCCCTGCCCCCAT CGAGAAAACCATCTCCAAAACCAAAGGCAGACCGAAGGCTCC ACAGGTGTACACCATTCCACCTCCCAAGGAGCAGATGGCCAAG GATAAAGTCAGTCTGACCTGCATGATAACAGACTTCTTCCCTG AAGACATTACTGTGGAGTGGCAGTGGAATGGGCAGCCAGCGG AGAACTACAAGAACACTCAGCCCATCATGGACACAGATGGCTC TTACTTCGTCTACAGCAAGCTCAATGTGCAGAAGAGCAACTGG GAGGCAGGAAATACTTTCACCTGCTCTGTGTTACATGAGGGCC TGCACAACCACCATACTGAGAAGAGCCTCTCCCACTCTCCTGG TAAATGA | |
| 18 | ATGGGATGGAGCTCTATCATCCTCTTCTTGGTAGCAACAGCCTC AGGTGTCCACTCC | anti-ID HC signal sequence |
| 19 | GATATCCAGATGACACAGACTACATCCTCCCTGTCTGCCTCTCT GGGAGACAGAGTCACCATCAGTTGCAGGGCAAGTCAGGACAT TAGCAATTATTTAAACTGGTATCAGCAGAAACCAGATGGAACT GTTAAACTCCTGATCTACTACACATCAAGATTACACTCAGGAG TCCCATCAAGGTTCAGTGGCAGTGGGTCTGGAACAGATTATTC TCTCACCATTAGCAACCTGGAGCAAGAAGATATTGCCACTTAC TTTTGTCAGCAGGGTAAAACGGTTCCATTCACGTTCGGCTCGG GGACAAAGTTGGAAATAAAA | anti-ID VL |
| 20 | CGGGCTGATGCTGCACCAACTGTATCCATCTTCCCACCATCCAG TGAGCAGTTAACATCTGGAGGTGCCTCAGTCGTGTGCTTCTTGA ACAACTTCTACCCCAAAGACATCAATGTCAAGTGGAAGATTGA TGGCAGTGAACGACAAAATGGCGTCCTGAACAGTTGGACTGAT CAGGACAGCAAAGACAGCACCTACAGCATGAGCAGCACCCTC ACGTTGACCAAGGACGAGTATGAACGACATAACAACTATACCT GTGAGGCCACTCACAAGACATCAACTTCACCCATTGTCAAGAG CTTCAACAGGAATGAGTGTTAG | anti-ID CL |
| 21 | GATATCCAGATGACACAGACTACATCCTCCCTGTCTGCCTCTCT GGGAGACAGAGTCACCATCAGTTGCAGGGCAAGTCAGGACAT TAGCAATTATTTAAACTGGTATCAGCAGAAACCAGATGGAACT GTTAAACTCCTGATCTACTACACATCAAGATTACACTCAGGAG TCCCATCAAGGTTCAGTGGCAGTGGGTCTGGAACAGATTATTC TCTCACCATTAGCAACCTGGAGCAAGAAGATATTGCCACTTAC TTTTGTCAGCAGGGTAAAACGGTTCCATTCACGTTCGGCTCGG GGACAAAGTTGGAAATAAAACGGGCTGATGCTGCACCAACTGT ATCCATCTTCCCACCATCCAGTGAGCAGTTAACATCTGGAGGT GCCTCAGTCGTGTGCTTCTTGAACAACTTCTACCCCAAAGACAT CAATGTCAAGTGGAAGATTGATGGCAGTGAACGACAAAATGG CGTCCTGAACAGTTGGACTGATCAGGACAGCAAAGACAGCACC TACAGCATGAGCAGCACCCTCACGTTGACCAAGGACGAGTATG AACGACATAACAACTATACCTGTGAGGCCACTCACAAGACATC AACTTCACCCATTGTCAAGAGCTTCAACAGGAATGAGTGTTAG | anti-ID light chain |
| 22 | ATGATGTCCTCTGCTCAGTTCCTTGGTCTCCTGTTGCTCTGTTTT CAAGGTACCAGATGT | anti-ID LC signal sequence |
| 23 | EVKLQQSGAELVRPGSSVKISCKASGYAFSSYWMNWVKQRPGQG LEWIGQIYPGDGDTNYNGKFKGQATLTADKSSSTAYMQLSGLTSE DSAVYFCARKTISSVVDFYFDYWGQGTTVTVSS | SJ25C1 VH |
| 24 | DIELTQSPKFMSTSVGDRVSVTCKASQNVGTNVAWYQQKPGQSP | SJ25C1 VL |
| | KPLIYSATYRNSGVPDRFTGSGSGTDFTLTITNVQSKDLADYFCQQ YNRYPYTSGGGTKLEIKR | |
| 25 | GGGGSGGGGSGGGGS | Linker |
| 26 | GGGGS | 4GS linker |
| 27 | IEVMYPPPYLDNEKSNGTIIHVKGKHLCPSPLFPGPSKP | An extracellular portion of CD28 |
| 28 | EVKLQQSGAELVRPGSSVKISCKASGYAFSSYWMNWVKQRPGQG LEWIGQIYPGDGDTNYNGKFKGQATLTADKSSSTAYMQLSGLTSE DSAVYFCARKTISSVVDFYFDYWGQGTTVTVSSGGGGSGGGGSG GGGSDIELTQSPKFMSTSVGDRVSVTCKASQNVGTNVAWYQQKP GQSPKPLIYSATYRNSGVPDRFTGSGSGTDFTLTITNVQSKDLADY FCQQYNRYPYTSGGGTKLEIKR | SJ25C1 scFv |
| 29 | GGGS | 3GS linker |
| 30 | EVKLQESGPGLVAPSQSLSVTCTVSGVSLPDYGVSWIRQPPRKGL EWLGVIWGSETTYYNSALKSRLTIIKDNSKSQVFLKMNSLQTDDT AIYYCAKHYYYGGSYAMDYWGQGTSVTVSS | FMC63 VH |
| 31 | DIQMTQTTSSLSASLGDRVTISCRASQDISKYLNWYQQKPDGTVK LLIYHTSRLHSGVPSRFSGSGSGTDYSLTISNLEQEDIATYFCQQGN TLPYTFGGGTKLEIT | FMC63 VL |
| 32 | PCPAPELLGGPSVFLFPPKPKDTLMISRTPEVTCVVVDVSHEDPEV KFNWYVDGVEVHNAKTKPREEQYNSTYRVVSVLTVLHQDWLNG KEYKCKVSNKALPAPIEKTISKAKGQPREPQVYTLPPSREEMTKN QVSLTCLVKGFYPSDIAVEWESNGQPENNYKTTPPVLDSDGSFFL YSKLTVDKSRWQQGNVFSCSVMHEALHNHYTQKSLSLSPGK | IgG1 Fc lacking hinge |
| 33 | GSTSGSGKPGSGEGSTKG | Linker |
| 34 | DIQMTQTTSSLSASLGDRVTISCRASQDISKYLNWYQQKPDGTVK LLIYHTSRLHSGVPSRFSGSGSGTDYSLTISNLEQEDIATYFCQQGN TLPYTFGGGTKLEITGSTSGSGKPGSGEGSTKGEVKLQESGPGLVA PSQSLSVTCTVSGVSLPDYGVSWIRQPPRKGLEWLGVIWGSETTY YNSALKSRLTIIKDNSKSQVFLKMNSLQTDDTAIYYCAKHYYYGG SYAMDYWGQGTSVTVSS | FMC63 scFv |
| 35 | DIQMTQTTSSLSASLGDRVTISCRASQDISKYLNWYQQKPDGTVK LLIYHTSRLHSGVPSRFSGSGSGTDYSLTISNLEQEDIATYFCQQGN TLPYTFGGGTKLEITGSTSGSGKPGSGEGSTKGEVKLQESGPGLVA PSQSLSVTCTVSGVSLPDYGVSWIRQPPRKGLEWLGVIWGSETTY YNSALKSRLTIIKDNSKSQVFLKMNSLQTDDTAIYYCAKHYYYGG SYAMDYWGQGTSVTVSSPCPAPELLGGPSVFLFPPKPKDTLMISR TPEVTCVVVDVSHEDPEVKFNWYVDGVEVHNAKTKPREEQYNS TYRVVSVLTVLHQDWLNGKEYKCKVSNKALPAPIEKTISKAKGQ PREPQVYTLPPSREEMTKNQVSLTCLVKGFYPSDIAVEWESNGQP ENNYKTTPPVLDSDGSFFLYSKLTVDKSRWQQGNVFSCSVMHEA LHNHYTQKSLSLSPGK | FMC63 reagent |
| 36 | EVQLQQSGPELVKPGASVKMSCKASGYTFTDYYMKWVKQCHGK SLEWIGDINPNNGGTDYNQNFKGKATLTVDKSSSTAYMQLNSLTS EDSAVYYCAREGNNYGSRDAMDYWGQGTSVTVSS | anti-ID B-1 VH |
| 37 | AKTTAPSVYPLAPVCGDTTGSSVTLGCLVKGYFPEPVTLTWNSGS LSSGVHTFPAVLQSDLYTLSSSVTVTSSTWPSQSITCNVAHPASST KVDKKIEPRGPTIKPCPPCKCPAPNLLGGPSVFIFPPKIKDVLMISLS PIVTCVVVDVSEDDPDVQISWFVNNVEVHTAQTQTHREDYNSTL RVVSALPIQHQDWMSGKEFKCKVNNKDLPAPIERTISKPKGSVRA PQVYVLPPPEEEMTKKQVTLTCMVTDFMPEDIYVEWTNNGKTEL | anti-ID B-1 CH |
| | NYKNTEPVLDSDGSYFMYSKLRVEKKNWVERNSYSCSVVHEGL HNHHTTKSFSRTPGK | |
| 38 | EVQLQQSGPELVKPGASVKMSCKASGYTFTDYYMKWVKQCHGK SLEWIGDINPNNGGTDYNQNFKGKATLTVDKSSSTAYMQLNSLTS EDSAVYYCAREGNNYGSRDAMDYWGQGTSVTVSSAKTTAPSVY PLAPVCGDTTGSSVTLGCLVKGYFPEPVTLTWNSGSLSSGVHTFP AVLQSDLYTLSSSVTVTSSTWPSQSITCNVAHPASSTKVDKKIEPR GPTIKPCPPCKCPAPNLLGGPSVFIFPPKIKDVLMISLSPIVTCVVVD VSEDDPDVQISWFVNNVEVHTAQTQTHREDYNSTLRVVSALPIQH QDWMSGKEFKCKVNNKDLPAPIERTISKPKGSVRAPQVYVLPPPE EEMTKKQVTLTCMVTDFMPEDIYVEWTNNGKTELNYKNTEPVL DSDGSYFMYSKLRVEKKNWVERNSYSCSVVHEGLHNHHTTKSFS RTPGK | anti-ID B-1 heavy chain |
| 39 | MGWSWIFLFLLSGTAGVLS | anti-ID B-1 HC signal sequence |
| 40 | QIVLTQSPALMSASPGEKVTMTCSASSGVIYMYWYQQKPRSSPKP WIYLTSNLASGVPARFSGSGSGTSYSLTISSMEAEDAATYYCQQW SSNPLTFGAGTKLELK | anti-ID B-1 VL |
| 41 | RADAAPTVSIFPPSSEQLTSGGASVVCFLNNFYPKDINVKWKIDGS ERQNGVLNSWTDQDSKDSTYSMSSTLTLTKDEYERHNSYTCEAT HKTSTSPIVKSFNRNEC | anti-ID B-1 CL |
| 42 | QIVLTQSPALMSASPGEKVTMTCSASSGVIYMYWYQQKPRSSPKP WIYLTSNLASGVPARFSGSGSGTSYSLTISSMEAEDAATYYCQQW SSNPLTFGAGTKLELKRADAAPTVSIFPPSSEQLTSGGASVVCFLN NFYPKDINVKWKIDGSERQNGVLNSWTDQDSKDSTYSMSSTLTL TKDEYERHNSYTCEATHKTSTSPIVKSFNRNEC | anti-ID B-1 light chain |
| 43 | MDFQVQIFSFLLMSASVIMSRG | anti-ID B-1 LC signal sequence |
| 44 | DYYMK | anti-ID B-1 HC-CDR1 |
| 45 | DINPNNGGTDYNQNFKG | anti-ID B-1 HC-CDR2 |
| 46 | EGNNYGSRDAMDY | anti-ID B-1 HC-CDR3 |
| 47 | SASSGVIYMY | anti-ID B-1 LC-CDR1 |
| 48 | LTSNLAS | anti-ID B-1 LC-CDR2 |
| 49 | QQWSSNPLT | anti-ID B-1 LC-CDR3 |
| 50 | GAGGTCCAGCTGCAACAATCTGGACCTGAGCTGGTGAAGCCTG GGGCTTCAGTGAAGATGTCCTGTAAGGCTTCTGGATACACATT CACTGACTACTACATGAAGTGGGTGAAGCAGTGTCATGGAAAG AGCCTTGAGTGGATTGGAGATATTAATCCTAACAATGGTGGTA CTGACTACAACCAGAACTTTAAGGGCAAGGCCACATTGACTGT AGACAAATCCTCCAGCACAGCCTACATGCAGCTCAACAGCCTG ACATCTGAGGACTCTGCAGTCTATTACTGTGCAAGAGAGGGGA ATAACTACGGTAGTAGAGATGCTATGGACTACTGGGGTCAAGG AACGTCAGTCACCGTCTCCTCA | anti-ID B-1 VH |
| 51 | GCCAAAACAACAGCCCCATCGGTCTATCCACTGGCCCCTGTGT GTGGAGATACAACTGGCTCCTCGGTGACTCTAGGATGCCTGGT | anti-ID B-1 CH |
| | CAAGGGTTATTTCCCTGAGCCAGTGACCTTGACCTGGAACTCT GGATCCCTGTCCAGTGGTGTGCACACCTTCCCAGCTGTCCTGCA GTCTGACCTCTACACCCTCAGCAGCTCAGTGACTGTAACCTCG AGCACCTGGCCCAGCCAGTCCATCACCTGCAATGTGGCCCACC CGGCAAGCAGCACCAAGGTGGACAAGAAAATTGAGCCCAGAG GGCCCACAATCAAGCCCTGTCCTCCATGCAAATGCCCAGCACC TAACCTCTTGGGTGGACCATCCGTCTTCATCTTCCCTCCAAAGA TCAAGGATGTACTCATGATCTCCCTGAGCCCCATAGTCACATGT GTGGTGGTGGATGTGAGCGAGGATGACCCAGATGTCCAGATCA GCTGGTTTGTGAACAACGTGGAAGTACACACAGCTCAGACACA AACCCATAGAGAGGATTACAACAGTACTCTCCGGGTGGTCAGT GCCCTCCCCATCCAGCACCAGGACTGGATGAGTGGCAAGGAGT TCAAATGCAAGGTCAACAACAAAGACCTCCCAGCGCCCATCGA GAGAACCATCTCAAAACCCAAAGGGTCAGTAAGAGCTCCACA GGTATATGTCTTGCCTCCACCAGAAGAAGAGATGACTAAGAAA CAGGTCACTCTGACCTGCATGGTCACAGACTTCATGCCTGAAG ACATTTACGTGGAGTGGACCAACAACGGGAAAACAGAGCTAA ACTACAAGAACACTGAACCAGTCCTGGACTCTGATGGTTCTTA CTTCATGTACAGCAAGCTGAGAGTGGAAAAGAAGAACTGGGT GGAAAGAAATAGCTACTCCTGTTCAGTGGTCCACGAGGGTCTG CACAATCACCACACGACTAAGAGCTTCTCCCGGACTCCGGGTA AA | |
| 52 | GAGGTCCAGCTGCAACAATCTGGACCTGAGCTGGTGAAGCCTG GGGCTTCAGTGAAGATGTCCTGTAAGGCTTCTGGATACACATT CACTGACTACTACATGAAGTGGGTGAAGCAGTGTCATGGAAAG AGCCTTGAGTGGATTGGAGATATTAATCCTAACAATGGTGGTA CTGACTACAACCAGAACTTTAAGGGCAAGGCCACATTGACTGT AGACAAATCCTCCAGCACAGCCTACATGCAGCTCAACAGCCTG ACATCTGAGGACTCTGCAGTCTATTACTGTGCAAGAGAGGGGA ATAACTACGGTAGTAGAGATGCTATGGACTACTGGGGTCAAGG AACGTCAGTCACCGTCTCCTCAGCCAAAACAACAGCCCCATCG GTCTATCCACTGGCCCCTGTGTGTGGAGATACAACTGGCTCCTC GGTGACTCTAGGATGCCTGGTCAAGGGTTATTTCCCTGAGCCA GTGACCTTGACCTGGAACTCTGGATCCCTGTCCAGTGGTGTGC ACACCTTCCCAGCTGTCCTGCAGTCTGACCTCTACACCCTCAGC AGCTCAGTGACTGTAACCTCGAGCACCTGGCCCAGCCAGTCCA TCACCTGCAATGTGGCCCACCCGGCAAGCAGCACCAAGGTGGA CAAGAAAATTGAGCCCAGAGGGCCCACAATCAAGCCCTGTCCT CCATGCAAATGCCCAGCACCTAACCTCTTGGGTGGACCATCCG TCTTCATCTTCCCTCCAAAGATCAAGGATGTACTCATGATCTCC CTGAGCCCCATAGTCACATGTGTGGTGGTGGATGTGAGCGAGG ATGACCCAGATGTCCAGATCAGCTGGTTTGTGAACAACGTGGA AGTACACACAGCTCAGACACAAACCCATAGAGAGGATTACAA CAGTACTCTCCGGGTGGTCAGTGCCCTCCCCATCCAGCACCAG GACTGGATGAGTGGCAAGGAGTTCAAATGCAAGGTCAACAAC AAAGACCTCCCAGCGCCCATCGAGAGAACCATCTCAAAACCCA AAGGGTCAGTAAGAGCTCCACAGGTATATGTCTTGCCTCCACC AGAAGAAGAGATGACTAAGAAACAGGTCACTCTGACCTGCAT GGTCACAGACTTCATGCCTGAAGACATTTACGTGGAGTGGACC AACAACGGGAAAACAGAGCTAAACTACAAGAACACTGAACCA GTCCTGGACTCTGATGGTTCTTACTTCATGTACAGCAAGCTGAG AGTGGAAAAGAAGAACTGGGTGGAAAGAAATAGCTACTCCTG | anti-ID B-1 heavy chain |
| | TTCAGTGGTCCACGAGGGTCTGCACAATCACCACACGACTAAG AGCTTCTCCCGGACTCCGGGTAAA | |
| 53 | ATGGGATGGAGCTGGATCTTTCTCTTCCTCTTGTCAGGAACTGC AGGTGTCCTCTCT | anti-ID B-1 HC signal sequence |
| 54 | CAAATTGTTCTCACCCAGTCTCCAGCACTCATGTCTGCATCTCC AGGGGAGAAGGTCACCATGACCTGCAGTGCCAGCTCAGGTGTA ATTTACATGTACTGGTACCAACAGAAGCCAAGATCCTCCCCCA AACCCTGGATTTATCTCACATCCAACCTGGCTTCTGGAGTCCCT GCTCGCTTCAGTGGCAGTGGGTCTGGGACCTCTTACTCTCTCAC AATCAGCAGCATGGAGGCTGAAGATGCTGCCACTTATTACTGC CAGCAGTGGAGTAGTAACCCGCTCACGTTCGGTGCTGGCACCA AGCTGGAGCTGAAA | anti-ID B-1 VL |
| 55 | CGGGCTGATGCTGCACCAACTGTATCCATCTTCCCACCATCCAG TGAGCAGTTAACATCTGGAGGTGCCTCAGTCGTGTGCTTCTTGA ACAACTTCTACCCCAAAGACATCAATGTCAAGTGGAAGATTGA TGGCAGTGAACGACAAAATGGCGTCCTGAACAGTTGGACTGAT CAGGACAGCAAAGACAGCACCTACAGCATGAGCAGCACCCTC ACGTTGACCAAGGACGAGTATGAACGACATAACAGCTATACCT GTGAGGCCACTCACAAGACATCAACTTCACCCATTGTCAAGAG CTTCAACAGGAATGAGTGT | anti-ID B-1 CL |
| 56 | CAAATTGTTCTCACCCAGTCTCCAGCACTCATGTCTGCATCTCC AGGGGAGAAGGTCACCATGACCTGCAGTGCCAGCTCAGGTGTA ATTTACATGTACTGGTACCAACAGAAGCCAAGATCCTCCCCCA AACCCTGGATTTATCTCACATCCAACCTGGCTTCTGGAGTCCCT GCTCGCTTCAGTGGCAGTGGGTCTGGGACCTCTTACTCTCTCAC AATCAGCAGCATGGAGGCTGAAGATGCTGCCACTTATTACTGC CAGCAGTGGAGTAGTAACCCGCTCACGTTCGGTGCTGGCACCA AGCTGGAGCTGAAACGGGCTGATGCTGCACCAACTGTATCCAT CTTCCCACCATCCAGTGAGCAGTTAACATCTGGAGGTGCCTCA GTCGTGTGCTTCTTGAACAACTTCTACCCCAAAGACATCAATGT CAAGTGGAAGATTGATGGCAGTGAACGACAAAATGGCGTCCT GAACAGTTGGACTGATCAGGACAGCAAAGACAGCACCTACAG CATGAGCAGCACCCTCACGTTGACCAAGGACGAGTATGAACGA CATAACAGCTATACCTGTGAGGCCACTCACAAGACATCAACTT CACCCATTGTCAAGAGCTTCAACAGGAATGAGTGT | anti-ID B-1 light chain |
| 57 | ATGGATTTTCAAGTGCAGATTTTCAGCTTCCTGCTAATGAGTGC CTCAGTCATAATGTCCAGGGGA | anti-ID B-1 LC signal sequence |
| 58 | QVQLQQPGAELVRPGASVKLSCKTSGYSFTRYWMNWVKQRPGQ GLEWIGMIHPSDSETRLNQKFKDKATLTVDNSSSTAYMQLSSPTS EDSAVYYCASIYYEEAWGQGTLVTVSA | anti-ID B-2 VH |
| 59 | AKTTPPSVYPLAPGSAAQTNSMVTLGCLVKGYFPEPVTVTWNSGS LSSGVHTFPAVLQSDLYTLSSSVTVPSSTWPSETVTCNVAHPASST KVDKKIVPRDCGCKPCICTVPEVSSVFIFPPKPKDVLTITLTPKVTC VVVDISKDDPEVQFSWFVDDVEVHTAQTQPREEQFNSTFRSVSEL PIMHQDWLNGKEFKCRVNSAAFPAPIEKTISKTKGRPKAPQVYTIP PPKEQMAKDKVSLTCMITDFFPEDITVEWQWNGQPAENYKNTQPI MDTDGSYFVYSKLNVQKSNWEAGNTFTCSVLHEGLHNHHTEKSL SHSPGK | anti-ID B-2 CH |
| 60 | QVQLQQPGAELVRPGASVKLSCKTSGYSFTRYWMNWVKQRPGQ GLEWIGMIHPSDSETRLNQKFKDKATLTVDNSSSTAYMQLSSPTS EDSAVYYCASIYYEEAWGQGTLVTVSAAKTTPPSVYPLAPGSAA | anti-ID B-2 heavy chain |
| | QTNSMVTLGCLVKGYFPEPVTVTWNSGSLSSGVHTFPAVLQSDL YTLSSSVTVPSSTWPSETVTCNVAHPASSTKVDKKIVPRDCGCKPC ICTVPEVSSVFIFPPKPKDVLTITLTPKVTCVVVDISKDDPEVQFSW FVDDVEVHTAQTQPREEQFNSTFRSVSELPIMHQDWLNGKEFKCR VNSAAFPAPIEKTISKTKGRPKAPQVYTIPPPKEQMAKDKVSLTCM ITDFFPEDITVEWQWNGQPAENYKNTQPIMDTDGSYFVYSKLNVQ KSNWEAGNTFTCSVLHEGLHNHHTEKSLSHSPGK | |
| 61 | MGWSSIILFLVATATGVHS | anti-ID B-2 HC signal sequence |
| 62 | DIQMTQSPASLSASVGETVTITCRASGNIHNYLAWYQQKQGKSPQ LLVYNAKTLADSVPSRFSGSGSGTQYSLKINSLQPEDFGSYYCQHF WSTPYTFGGGTKLEIK | anti-ID B-2 VL |
| 63 | DIQMTQSPASLSASVGETVTITCRASGNIHNYLAWYQQKQGKSPQ LLVYNAKTLADSVPSRFSGSGSGTQYSLKINSLQPEDFGSYYCQHF WSTPYTFGGGTKLEIKRADAAPTVSIFPPSSEQLTSGGASVVCFLN NFYPKDINVKWKIDGSERQNGVLNSWTDQDSKDSTYSMSSTLTL TKDEYERHNSYTCEATHKTSTSPIVKSFNRNEC | anti-ID B-2 light chain |
| 64 | MSVLTQVLALLLLWLTGARC | anti-ID B-2 LC signal sequence |
| 65 | RYWMN | anti-ID B-2 HC-CDR1 |
| 66 | MIHPSDSETRLNQKFKD | anti-ID B-2 HC-CDR2 |
| 67 | IYYEEA | anti-ID B-2 HC-CDR3 |
| 68 | RASGNIHNYLA | anti-ID B-2 LC-CDR1 |
| 69 | NAKTLAD | anti-ID B-2 LC-CDR2 |
| 70 | QHFWSTPYT | anti-ID B-2 LC-CDR3 |
| 71 | CAGGTCCAACTGCAGCAGCCTGGGGCTGAGCTGGTGAGGCCTG GAGCTTCAGTGAAGCTGTCCTGCAAGACTTCTGGCTACTCCTTC ACCAGGTACTGGATGAACTGGGTGAAGCAGAGGCCTGGACAA GGCCTTGAGTGGATTGGCATGATTCATCCTTCCGATAGTGAAA CTAGGTTAAATCAGAAGTTCAAGGACAAGGCCACATTGACTGT AGACAATTCCTCCAGCACAGCCTACATGCAACTCAGCAGCCCG ACATCTGAGGACTCTGCGGTCTATTACTGTGCAAGCATCTACTA TGAAGAGGCCTGGGGCCAAGGGACTCTGGTCACTGTCTCTGCA | anti-ID B-2 VH |
| 72 | GCCAAAACGACACCCCCATCTGTCTATCCACTGGCCCCTGGAT CTGCTGCCCAAACTAACTCCATGGTGACCCTGGGATGCCTGGT CAAGGGCTATTTCCCTGAGCCAGTGACAGTGACCTGGAACTCT GGATCCCTGTCCAGCGGTGTGCACACCTTCCCAGCTGTCCTGCA GTCTGACCTCTACACTCTGAGCAGCTCAGTGACTGTCCCCTCCA GCACCTGGCCCAGCGAGACCGTCACCTGCAACGTTGCCCACCC GGCCAGCAGCACCAAGGTGGACAAGAAAATTGTGCCCAGGGA TTGTGGTTGTAAGCCTTGCATATGTACAGTCCCAGAAGTATCAT CTGTCTTCATCTTCCCCCCAAAGCCCAAGGATGTGCTCACCATT ACTCTGACTCCTAAGGTCACGTGTGTTGTGGTAGACATCAGCA AGGATGATCCCGAGGTCCAGTTCAGCTGGTTTGTAGATGATGT GGAGGTGCACACAGCTCAGACGCAACCCCGGGAGGAGCAGTT | anti-ID B-2 CH |
| | CAACAGCACTTTCCGCTCAGTCAGTGAACTTCCCATCATGCACC AGGACTGGCTCAATGGCAAGGAGTTCAAATGCAGGGTCAACA GTGCAGCTTTCCCTGCCCCCATCGAGAAAACCATCTCCAAAAC CAAAGGCAGACCGAAGGCTCCACAGGTGTACACCATTCCACCT CCCAAGGAGCAGATGGCCAAGGATAAAGTCAGTCTGACCTGC ATGATAACAGACTTCTTCCCTGAAGACATTACTGTGGAGTGGC AGTGGAATGGGCAGCCAGCGGAGAACTACAAGAACACTCAGC CCATCATGGACACAGATGGCTCTTACTTCGTCTACAGCAAGCT CAATGTGCAGAAGAGCAACTGGGAGGCAGGAAATACTTTCAC CTGCTCTGTGTTACATGAGGGCCTGCACAACCACCATACTGAG AAGAGCCTCTCCCACTCTCCTGGTAAA | |
| 73 | CAGGTCCAACTGCAGCAGCCTGGGGCTGAGCTGGTGAGGCCTG GAGCTTCAGTGAAGCTGTCCTGCAAGACTTCTGGCTACTCCTTC ACCAGGTACTGGATGAACTGGGTGAAGCAGAGGCCTGGACAA GGCCTTGAGTGGATTGGCATGATTCATCCTTCCGATAGTGAAA CTAGGTTAAATCAGAAGTTCAAGGACAAGGCCACATTGACTGT AGACAATTCCTCCAGCACAGCCTACATGCAACTCAGCAGCCCG ACATCTGAGGACTCTGCGGTCTATTACTGTGCAAGCATCTACTA TGAAGAGGCCTGGGGCCAAGGGACTCTGGTCACTGTCTCTGCA GCCAAAACGACACCCCCATCTGTCTATCCACTGGCCCCTGGAT CTGCTGCCCAAACTAACTCCATGGTGACCCTGGGATGCCTGGT CAAGGGCTATTTCCCTGAGCCAGTGACAGTGACCTGGAACTCT GGATCCCTGTCCAGCGGTGTGCACACCTTCCCAGCTGTCCTGCA GTCTGACCTCTACACTCTGAGCAGCTCAGTGACTGTCCCCTCCA GCACCTGGCCCAGCGAGACCGTCACCTGCAACGTTGCCCACCC GGCCAGCAGCACCAAGGTGGACAAGAAAATTGTGCCCAGGGA TTGTGGTTGTAAGCCTTGCATATGTACAGTCCCAGAAGTATCAT CTGTCTTCATCTTCCCCCCAAAGCCCAAGGATGTGCTCACCATT ACTCTGACTCCTAAGGTCACGTGTGTTGTGGTAGACATCAGCA AGGATGATCCCGAGGTCCAGTTCAGCTGGTTTGTAGATGATGT GGAGGTGCACACAGCTCAGACGCAACCCCGGGAGGAGCAGTT CAACAGCACTTTCCGCTCAGTCAGTGAACTTCCCATCATGCACC AGGACTGGCTCAATGGCAAGGAGTTCAAATGCAGGGTCAACA GTGCAGCTTTCCCTGCCCCCATCGAGAAAACCATCTCCAAAAC CAAAGGCAGACCGAAGGCTCCACAGGTGTACACCATTCCACCT CCCAAGGAGCAGATGGCCAAGGATAAAGTCAGTCTGACCTGC ATGATAACAGACTTCTTCCCTGAAGACATTACTGTGGAGTGGC AGTGGAATGGGCAGCCAGCGGAGAACTACAAGAACACTCAGC CCATCATGGACACAGATGGCTCTTACTTCGTCTACAGCAAGCT CAATGTGCAGAAGAGCAACTGGGAGGCAGGAAATACTTTCAC CTGCTCTGTGTTACATGAGGGCCTGCACAACCACCATACTGAG AAGAGCCTCTCCCACTCTCCTGGTAAA | anti-ID B-2 heavy chain |
| 74 | ATGGGATGGAGCTCTATCATCCTCTTCTTGGTAGCAACAGCTAC AGGTGTCCACTCC | anti-ID B-2 HC signal sequence |
| 75 | GACATCCAGATGACTCAGTCTCCAGCCTCCCTATCTGCATCTGT GGGAGAAACTGTCACCATCACATGTCGAGCAAGTGGGAATATT CACAATTATTTAGCATGGTATCAGCAGAAACAGGGAAAATCTC CTCAGCTCCTGGTCTATAATGCAAAAACCTTAGCAGATAGTGT GCCATCAAGGTTCAGTGGCAGTGGATCAGGAACACAATATTCT CTCAAGATCAACAGCCTGCAGCCTGAAGATTTTGGGAGTTATT ACTGTCAACATTTTTGGAGTACTCCGTACACGTTCGGAGGGGG | anti-ID B-2 VL |
| | GACCAAGCTGGAAATAAAA | |
| 76 | GACATCCAGATGACTCAGTCTCCAGCCTCCCTATCTGCATCTGT GGGAGAAACTGTCACCATCACATGTCGAGCAAGTGGGAATATT CACAATTATTTAGCATGGTATCAGCAGAAACAGGGAAAATCTC CTCAGCTCCTGGTCTATAATGCAAAAACCTTAGCAGATAGTGT GCCATCAAGGTTCAGTGGCAGTGGATCAGGAACACAATATTCT CTCAAGATCAACAGCCTGCAGCCTGAAGATTTTGGGAGTTATT ACTGTCAACATTTTTGGAGTACTCCGTACACGTTCGGAGGGGG GACCAAGCTGGAAATAAAACGGGCTGATGCTGCACCAACTGTA TCCATCTTCCCACCATCCAGTGAGCAGTTAACATCTGGAGGTG CCTCAGTCGTGTGCTTCTTGAACAACTTCTACCCCAAAGACATC AATGTCAAGTGGAAGATTGATGGCAGTGAACGACAAAATGGC GTCCTGAACAGTTGGACTGATCAGGACAGCAAAGACAGCACCT ACAGCATGAGCAGCACCCTCACGTTGACCAAGGACGAGTATGA ACGACATAACAGCTATACCTGTGAGGCCACTCACAAGACATCA ACTTCACCCATTGTCAAGAGCTTCAACAGGAATGAGTGT | anti-ID B-2 light chain |
| 77 | ATGAGTGTGCTCACTCAGGTCCTGGCGTTGCTGCTGCTGTGGCT TACAGGTGCCAGATGT | anti-ID B-2 LC signal sequence |
| 78 | DYTFTSY | anti-ID HC-CDR1 |
| 79 | DYTFTSYWMH | anti-ID HC-CDR1 |
| 80 | TSYWMH | anti-ID HC-CDR1 |
| 81 | YPGSGG | anti-ID HC-CDR2 |
| 82 | NIYPGSGGTN | anti-ID HC-CDR2 |
| 83 | WIGNIYPGSGGTN | anti-ID HC-CDR2 |
| 84 | TREVTTVAYYYSMD | anti-ID HC-CDR3 |
| 85 | SNYLNWY | anti-ID LC-CDR1 |
| 86 | LLIYYTSRLH | anti-ID LC-CDR2 |
| 87 | QQGKTVPF | anti-ID LC-CDR3 |
| 88 | GYTFTDY | anti-ID B-1 HC-CDR1 |
| 89 | GYTFTDYYMK | anti-ID B-1 HC-CDR1 |
| 90 | TDYYMK | anti-ID B-1 HC-CDR1 |
| 91 | NPNNGG | anti-ID B-1 HC-CDR2 |
| 92 | DINPNNGGTD | anti-ID B-1 HC-CDR2 |
| 93 | WIGDINPNNGGTD | anti-ID B-1 HC-CDR2 |
| 94 | AREGNNYGSRDAMD | anti-ID B-1 HC-CDR3 |
| 95 | IYMYWY | anti-ID B-1 LC-CDR1 |
| 96 | PWIYLTSNLA | anti-ID B-1 LC-CDR2 |
| 97 | QQWSSNPL | anti-ID B-1 LC-CDR3 |
| 98 | GYSFTRY | anti-ID B-2 HC-CDR1 |
| 99 | GYSFTRYWMN | anti-ID B-2 HC-CDR1 |
| 100 | TRYWMN | anti-ID B-2 HC-CDR1 |
| 101 | HPSDSE | anti-ID B-2 HC-CDR2 |
| 102 | MIHPSDSETR | anti-ID B-2 HC-CDR2 |
| 103 | WIGMIHPSDSETR | anti-ID B-2 HC-CDR2 |
| 104 | ASIYYEE | anti-ID B-2 HC-CDR3 |
| 105 | HNYLAWY | anti-ID B-2 LC-CDR1 |
| 106 | LLVYNAKTLA | anti-ID B-2 LC-CDR2 |
| 107 | QHFWSTPY | anti-ID B-2 LC-CDR3 |
| 108 | GYX₃FX₅X₆YX₈MX₁₀ | HC-CDR1 Consensus |
| | X₃ = T or S; | |
| | X₅ = T or S; | |
| | X₆ = D or R; | |
| | X₈ = Y or W; | |
| | X₁₀ = KorN | |
| 109 | WIGX₄IX₆PX₈X₉X₁₀X₁₁TX₁₃Xₗ₄NQX₁₇FKX₂₀ | HC-CDR2 Consensus |
| | X₄ = D or M; | |
| | X₆ = N or H; | |
| | X₈ = NorS; | |
| | X₉ = NorD; | |
| | X₁₀ = G or S; | |
| | X₁₁ = G or E; | |
| | X₁₃ = D or R; | |
| | X₁₄ = Y or L; | |
| | X₁₇ = N or K; | |
| | X₂₀ = G or D | |
| 110 | AX₂X₃X₄X₅X₆ X₇X₈ X₉ X₁₀X₁₁ X₁₂ X₁₃ X₁₄ X₁₅ | HC-CDR3 Consensus |
| | X₂ = R or S; | |
| | X₃ = E or I; | |
| | X₄ = G or Y; | |
| | X₅ = N or Y; | |
| | X₆ = N or E; | |
| | X₇ = Y or null; | |
| | X₈ = G or null; | |
| | X₉ = S or null; | |
| | X₁₀ = R or null; | |
| | X₁₁ = D or null; | |
| | X₁₂ = A or null; | |
| | X₁₃ = M or null; | |
| | X₁₄ = D or E; | |
| | X₁₅ = Y or A | |
| 111 | X₁AX₃X₄X₅X₆ X₇X₈ YX₁₀X₁₁WY | LC-CDR1 Consensus |
| | X₁ = S or R; | |
| | X₃ = S or R; | |
| | X₄ = S or G; | |
| | X₅ = G or N; | |
| | X₆ = V or I; | |
| | X₇ = I or H; | |
| | X₈ = N or null; | |
| | X₁₀ = M or L; | |
| | X₁₁ = Y or A | |
| 112 | X₁X₂X₃YX₅X₆ X₇X₈ LAX₁₁ | LC-CDR2 Consensus |
| | X₁ = P or L; | |
| | X₂ = W or L; | |
| | X₃ = IorV; | |
| | X₅ = L or N; | |
| | X₆ = T or A; | |
| | X₇ = S or K; | |
| | X₈ = N or T; | |
| | X₁₁ = S or D | |
| 113 | QX₂X₃X₄X₅X₆PX₈T | LC-CDR3 Consensus |
| | X₂ = Q or H; | |
| | X₃ =W or F; | |
| | X₄ = S or W; | |
| | X₅ = S or W; | |
| | X₆ = N or T; | |
| | X₈ = LorY | |
| 114 | SYWMN | SJ25C1 HC-CDR1 |
| 115 | QIYPGDGDTNYNGKFKG | SJ25C1 HC-CDR2 |
| 116 | KTISSVVDFYFDY | SJ25C1 HC-CDR3 |
| 117 | KASQNVGTNVA | SJ25C1 LC-CDR1 |
| 118 | SATYRNS | SJ25C1 LC-CDR2 |
| 119 | QQYNRYPYT | SJ25C1 LC-CDR3 |
| 120 | DYGVS | FMC63 HC-CDR1 |
| 121 | VIWGSETTYYNSALKS | FMC63 HC-CDR2 |
| 122 | HYYYGGSYAMDY | FMC63 HC-CDR3 |
| 123 | RASQDISKYLN | FMC63 LC-CDR1 |
| 124 | HTSRLHS | FMC63 LC-CDR2 |
| 125 | QQGNTLPYT | FMC63 LC-CDR3 |
| 126 | LEGGGEGRGSLLTCGDVEENPGPR | T2A |
| 127 | EGRGSLLTCGDVEENPGP | T2A |
| 128 | GSGATNFSLLKQAGDVEENPGP | P2A |
| 129 | ATNFSLLKQAGDVEENPGP | P2A |
| 130 | QCTNYALLKLAGDVESNPGP | E2A |
| 131 | VKQTLNFDLLKLAGDVESNPGP | F2A |
| 132 | QVQLKQSGPGLVQPSQSLSITCTVS DYTFTSY GVHWVRQSPGKGLEWLGVI YPGSGG TDYNTPFTSRLSINKDNSKSQVFFKMNSLQSNDTAIYYCAR EVTTVAYYYSMDY WGQGTLVTVSA TLVTVSAASTKGPSVFPLAPSSKSTSGGTAALGCLVKDYFPEPVTV SWNSGALTSGVHTFPAVLQSSGLYSLSSVVTVPSSSLGTQTYICNV NHKPSNTKVDKKVEPKSCDKTHTCPPCPAPELLGGPSVFLFPPKPK DTLMISRTPEVTCVVVDVSHEDPEVKFNWYVDGVEVHNAKTKPR EEQYNSTYRVVSVLTVLHQDWLNGKEYKCKVSNKALPAPIEKTIS | Exemplary humanized anti-SJ25C1 heavy chain |
| | KAKGQPREPQVYTLPPSRDELTKNQVSLTCLVKGFYPSDIAVEWE SNGQPENNYKTTPPVLDSDGSFFLYSKLTVDKSRWQQGNVFSCSV MHEALHNHYTQKSLSLSPGK | |
| 133 | DILLTQSPVILSVSPGERVSFSC RASQDISNYLN WYQQRTNGSPRLLIK YTSRLHS GIPSRFSGSGSGTDFTLSINSVESEDIADYYC QQGKTVPFT FGAGTKLELKRTV AAPSVFIFPPSDEQLKSGTASVVCLLNNFYPREAKVQWKVDNALQ SGNSQESVTEQDSKDSTYSLSSTLTLSKADYEKHKVYACEVTHQG LSSPVTKSFNRGEC | Exemplary humanized anti-SJ25C1 light chain |
| 134 | QVQLKQSGPGLVQPSQSLSITCTVSGFSL TSYWMH WVRQSPGKGLE WIGNIYPGSGGTN YNTPFTSRLSINKDNSKSQVFFKMNSLQSNDTAIYYC TREVTTVAYYYSMD YWGQGTLVTVSA TLVTVSAASTKGPSVFPLAPSSKSTSGGTAALGCLVKDYFPEPVTV SWNSGALTSGVHTFPAVLQSSGLYSLSSVVTVPSSSLGTQTYICNV NHKPSNTKVDKKVEPKSCDKTHTCPPCPAPELLGGPSVFLFPPKPK DTLMISRTPEVTCVVVDVSHEDPEVKFNWYVDGVEVHNAKTKPR EEQYNSTYRVVSVLTVLHQDWLNGKEYKCKVSNKALPAPIEKTIS KAKGQPREPQVYTLPPSRDELTKNQVSLTCLVKGFYPSDIAVEWE SNGQPENNYKTTPPVLDSDGSFFLYSKLTVDKSRWQQGNVFSCSV MHEALHNHYTQKSLSLSPGK | Exemplary humanized anti-SJ25C1 heavy chain |
| 135 | DILLTQSPVILSVSPGERVSFSCRASQSI SNYLNWY QQRTNGSPR LLIYYTSRLH SGIPSRFSGSGSGTDFTLSINSVESEDIADYYC QQGKTVPF TFGAGTKLELKRTV AAPSVFIFPP SDEQLKSGTASVVCLLNNFYPREAKVQWKVDNALQSGNSQESVT EQDSKDSTYSLSSTLTLSKADYEKHKVYACEVTHQGLSSPVTKSF NRGEC | Exemplary humanized anti-SJ25C1 light chain |
| 136 | QVQLKQSGPGLVQPSQSLSITCTVSGFSLT SYWMH WVRQSPGKGLEWLG NIYPGSGGTNYDEKFKR RLSINKDNSKSQVFFKMNSLQSNDTAIYYCAR EVTTVAYYYSMDY WGQGTLVTVSA TLVTVSAASTKGPSVFPLAPSSKSTSGGTAALGCLVKDYFPEPVTV SWNSGALTSGVHTFPAVLQSSGLYSLSSVVTVPSSSLGTQTYICNV NHKPSNTKVDKKVEPKSCDKTHTCPPCPAPELLGGPSVFLFPPKPK DTLMISRTPEVTCVVVDVSHEDPEVKFNWYVDGVEVHNAKTKPR EEQYNSTYRVVSVLTVLHQDWLNGKEYKCKVSNKALPAPIEKTIS KAKGQPREPQVYTLPPSRDELTKNQVSLTCLVKGFYPSDIAVEWE SNGQPENNYKTTPPVLDSDGSFFLYSKLTVDKSRWQQGNVFSCSV MHEALHNHYTQKSLSLSPGK | Exemplary humanized anti-SJ25C1 heavy chain |
| 137 | DILLTQSPVILSVSPGERVSFSC RASQDISNYLN WYQQRTNGSPRLLIK YTSRLHS GIPSRFSGSGSGTDFTLSINSVESEDIADYYC QQGKTVPFT FGAGTKLELKRTV AAPSVFIFPP SDEQLKSGTASVVCLLNNFYPREAKVQWKVDNALQSGNSQESVT EQDSKDSTYSLSSTLTLSKADYEKHKVYACEVTHQGLSSPVTKSF NRGEC | Exemplary humanized anti-SJ25C1 light chain |
| 138 | QVQLKQSGPGLVQPSQSLSITCTVS GYTFTDY GVHWVRQSPGKGLEWLGVI NPNNGG TDYNTPFTSRLSINKDNSKSQVFFKMNSLQSNDTAIYYCAR EGNNYGSRDAMDY WGQGTLVTVSA | Exemplary humanized anti-FMC63 heavy chain |
| | TLVTVSAASTKGPSVFPLAPSSKSTSGGTAALGCLVKDYFPEPVTV SWNSGALTSGVHTFPAVLQSSGLYSLSSVVTVPSSSLGTQTYICNV NHKPSNTKVDKKVEPKSCDKTHTCPPCPAPELLGGPSVFLFPPKPK DTLMISRTPEVTCVVVDVSHEDPEVKFNWYVDGVEVHNAKTKPR EEQYNSTYRVVSVLTVLHQDWLNGKEYKCKVSNKALPAPIEKTIS KAKGQPREPQVYTLPPSRDELTKNQVSLTCLVKGFYPSDIAVEWE SNGQPENNYKTTPPVLDSDGSFFLYSKLTVDKSRWQQGNVFSCSV MHEALHNHYTQKSLSLSPGK | |
| 139 | DILLTQSPVILSVSPGERVSFSC SASSGVIYMY WYQQRTNGSPRLLIK LTSNLAS GIPSRFSGSGSGTDFTLSINSVESEDIADYYC QQWSSNPLT FGAGTKLELKRTV AAPSVFIFPPSDEQLKSGTASVVCLLNNFYPREAKVQWKVDNALQ SGNSQESVTEQDSKDSTYSLSSTLTLSKADYEKHKVYACEVTHQG LSSPVTKSFNRGEC | Exemplary humanized anti-FMC63 light chain |
| 140 | QVQLKQSGPGLVQPSQSLSITCTVSGFSLT DYYMK WVRQSPGKGLEWLG DINPNNGGTDYNQNFKG RLSINKDNSKSQVFFKMNSLQSNDTAIYYCAR EGNNYGSRDAMDY WGQGTLVTVSA TLVTVSAASTKGPSVFPLAPSSKSTSGGTAALGCLVKDYFPEPVTV SWNSGALTSGVHTFPAVLQSSGLYSLSSVVTVPSSSLGTQTYICNV NHKPSNTKVDKKVEPKSCDKTHTCPPCPAPELLGGPSVFLFPPKPK DTLMISRTPEVTCVVVDVSHEDPEVKFNWYVDGVEVHNAKTKPR EEQYNSTYRVVSVLTVLHQDWLNGKEYKCKVSNKALPAPIEKTIS KAKGQPREPQVYTLPPSRDELTKNQVSLTCLVKGFYPSDIAVEWE SNGQPENNYKTTPPVLDSDGSFFLYSKLTVDKSRWQQGNVFSCSV MHEALHNHYTQKSLSLSPGK | Exemplary humanized anti-FMC63 heavy chain |
| 141 | DILLTQSPVILSVSPGERVSFSC SASSGVIYMY WYQQRTNGSPRLLIK LTSNLAS GIPSRFSGSGSGTDFTLSINSVESEDIADYYC QQWSSNPLT FGAGTKLELKRTV AAPSVFIFPP SDEQLKSGTASVVCLLNNFYPREAKVQWKVDNALQSGNSQESVT EQDSKDSTYSLSSTLTLSKADYEKHKVYACEVTHQGLSSPVTKSF NRGEC | Exemplary humanized anti-FMC63 light chain |
| 142 | QVQLKQSGPGLVQPSQSLSITCTVSGFSL TDYYMK WVRQSPGKGLE WIGDINPNNGGTD YNTPFTSRLSINKDNSKSQVFFKMNSLQSNDTAIYYC AREGNNYGSRDAMD YWGQGTLVTVSA TLVTVSAASTKGPSVFPLAPSSKSTSGGTAALGCLVKDYFPEPVTV SWNSGALTSGVHTFPAVLQSSGLYSLSSVVTVPSSSLGTQTYICNV NHKPSNTKVDKKVEPKSCDKTHTCPPCPAPELLGGPSVFLFPPKPK DTLMISRTPEVTCVVVDVSHEDPEVKFNWYVDGVEVHNAKTKPR EEQYNSTYRVVSVLTVLHQDWLNGKEYKCKVSNKALPAPIEKTIS KAKGQPREPQVYTLPPSRDELTKNQVSLTCLVKGFYPSDIAVEWE SNGQPENNYKTTPPVLDSDGSFFLYSKLTVDKSRWQQGNVFSCSV MHEALHNHYTQKSLSLSPGK | Exemplary humanized anti-FMC63 heavy chain |
| 143 | DILLTQSPVILSVSPGERVSFSCRASQSI IYMYWY QQRTNGSPR PWIYLTSNLA SGIPSRFSGSGSGTDFTLSINSVESEDIADYYC QQWSSNPL TFGAGTKLELKRTV AAPSVFIFPP SDEQLKSGTASVVCLLNNFYPREAKVQWKVDNALQSGNSQESVT EQDSKDSTYSLSSTLTLSKADYEKHKVYACEVTHQGLSSPVTKSF | Exemplary humanized anti-FMC63 light chain |
| | NRGEC | |
| 144 | QVQLKQSGPGLVQPSQSLSITCTVS GYSFTRY GVHWVRQSPGKGLEWLGVI HPSDSE TDYNTPFTSRLSINKDNSKSQVFFKMNSLQSNDTAIYYCAR IYYEEA WGQGTLVTVSA TLVTVSAASTKGPSVFPLAPSSKSTSGGTAALGCLVKDYFPEPVTV SWNSGALTSGVHTFPAVLQSSGLYSLSSVVTVPSSSLGTQTYICNV NHKPSNTKVDKKVEPKSCDKTHTCPPCPAPELLGGPSVFLFPPKPK DTLMISRTPEVTCVVVDVSHEDPEVKFNWYVDGVEVHNAKTKPR EEQYNSTYRVVSVLTVLHQDWLNGKEYKCKVSNKALPAPIEKTIS KAKGQPREPQVYTLPPSRDELTKNQVSLTCLVKGFYPSDIAVEWE SNGQPENNYKTTPPVLDSDGSFFLYSKLTVDKSRWQQGNVFSCSV MHEALHNHYTQKSLSLSPGK | Exemplary humanized anti-FMC63 heavy chain |
| 145 | DILLTQSPVILSVSPGERVSFSC RASGNIHNYLA WYQQRTNGSPRLLIK NAKTLAD GIPSRFSGSGSGTDFTLSINSVESEDIADYYC QHFWSTPYT FGAGTKLELKRTV AAPSVFIFPPSDEQLKSGTASVVCLLNNFYPREAKVQWKVDNALQ SGNSQESVTEQDSKDSTYSLSSTLTLSKADYEKHKVYACEVTHQG LSSPVTKSFNRGEC | Exemplary humanized anti-FMC63 light chain |
| 146 | QVQLKQSGPGLVQPSQSLSITCTVSGFSLT RYWMN WVRQSPGKGLEWLG MIHPSDSETRLNQKFKD RLSINKDNSKSQVFFKMNSLQSNDTAIYYCAR IYYEEA WGQGTLVTVSA TLVTVSAASTKGPSVFPLAPSSKSTSGGTAALGCLVKDYFPEPVTV SWNSGALTSGVHTFPAVLQSSGLYSLSSVVTVPSSSLGTQTYICNV NHKPSNTKVDKKVEPKSCDKTHTCPPCPAPELLGGPSVFLFPPKPK DTLMISRTPEVTCVVVDVSHEDPEVKFNWYVDGVEVHNAKTKPR EEQYNSTYRVVSVLTVLHQDWLNGKEYKCKVSNKALPAPIEKTIS KAKGQPREPQVYTLPPSRDELTKNQVSLTCLVKGFYPSDIAVEWE SNGQPENNYKTTPPVLDSDGSFFLYSKLTVDKSRWQQGNVFSCSV MHEALHNHYTQKSLSLSPGK | Exemplary humanized anti-FMC63 heavy chain |
| 147 | DILLTQSPVILSVSPGERVSFSC RASGNIHNYLA WYQQRTNGSPRLLIK NAKTLAD GIPSRFSGSGSGTDFTLSINSVESEDIADYYC QHFWSTPYT FGAGTKLELKRTV AAPSVFIFPP SDEQLKSGTASVVCLLNNFYPREAKVQWKVDNALQSGNSQESVT EQDSKDSTYSLSSTLTLSKADYEKHKVYACEVTHQGLSSPVTKSF NRGEC | Exemplary humanized anti-FMC63 light chain |
| 148 | QVQLKQSGPGLVQPSQSLSITCTVSGFSL TRYWMN WVRQSPGKGLE WIGMIHPSDSETR YNTPFTSRLSINKDNSKSQVFFKMNSLQSNDTAIYYC ASIYYEE YWGQGTLVTVSA TLVTVSAASTKGPSVFPLAPSSKSTSGGTAALGCLVKDYFPEPVTV SWNSGALTSGVHTFPAVLQSSGLYSLSSVVTVPSSSLGTQTYICNV NHKPSNTKVDKKVEPKSCDKTHTCPPCPAPELLGGPSVFLFPPKPK DTLMISRTPEVTCVVVDVSHEDPEVKFNWYVDGVEVHNAKTKPR EEQYNSTYRVVSVLTVLHQDWLNGKEYKCKVSNKALPAPIEKTIS KAKGQPREPQVYTLPPSRDELTKNQVSLTCLVKGFYPSDIAVEWE SNGQPENNYKTTPPVLDSDGSFFLYSKLTVDKSRWQQGNVFSCSV MHEALHNHYTQKSLSLSPGK | Exemplary humanized anti-FMC63 heavy chain |
| 149 | DILLTQSPVILSVSPGERVSFSCRASQSI HNYLAWY QQRTNGSPR LLVYNAKTLA SGIPSRFSGSGSGTDFTLSINSVESEDIADYYC QHFWSTPY TFGAGTKLELKRTV AAPSVFIFPPSDEQLKSGTASVVCLLNNFYPREAKVQWKVDNALQ SGNSQESVTEQDSKDSTYSLSSTLTLSKADYEKHKVYACEVTHQG LSSPVTKSFNRGEC | Exemplary humanized anti-FMC63 light chain |
| 150 | ESKYGPPCPPCP | spacer (IgG4hinge) (aa) Homo sapiens |
| 151 | GAATCTAAGTACGGACCGCCCTGCCCCCCTTGCCCT | spacer (IgG4hinge) (nt) Homo sapiens |
| 152 | ESKYGPPCPPCPGQPREPQVYTLPPSQEEMTKNQVSLTCLVKGFYP SDIAVEWESNGQPENNYKTTPPVLDSDGSFFLYSRLTVDKSRWQE GNVFSCSVMHEALHNHYTQKSLSLSLGK | Hinge-CH3 spacer Homo sapiens |
| 153 | ESKYGPPCPPCPAPEFLGGPSVFLFPPKPKDTLMISRTPEVTCVVVD VSQEDPEVQFNWYVDGVEVHNAKTKPREEQFNSTYRVVSVLTVL HQDWLNGKEYKCKVSNKGLPSSIEKTISKAKGQPREPQVYTLPPS QEEMTKNQVSLTCLVKGFYPSDIAVEWESNGQPENNYKTTPPVL DSDGSFFLYSRLTVDKSRWQEGNVFSCSVMHEALHNHYTQKSLS LSLGK | Hinge-CH2-CH3 spacer Homo sapiens |
| 154 | RWPESPKAQASSVPTAQPQAEGSLAKATTAPATTRNTGRGGEEK KKEKEKEEQEERETKTPECPSHTQPLGVYLLTPAVQDLWLRDKAT FTCFVVGSDLKDAHLTWEVAGKVPTGGVEEGLLERHSNGSQSQH SRLTLPRSLWNAGTSVTCTLNHPSLPPQRLMALREPAAQAPVKLS LNLLASSDPPEAASWLLCEVSGFSPPNILLMWLEDQREVNTSGFAP ARPPPQPGSTTFWAWSVLRVPAPPSPQPATYTCVVSHEDSRTLLN ASRSLEVSYVTDH | IgD-hinge-Fc Homo sapiens |
| 155 | RKVCNGIGIGEFKDSLSINATNIKHFKNCTSISGDLHILPVAFRGDS FTHTPPLDPQELDILKTVKEITGFLLIQAWPENRTDLHAFENLEIIR GRTKQHGQFSLAVVSLNITSLGLRSLKEISDGDVIISGNKNLCYAN TINWKKLFGTSGQKTKIISNRGENSCKATGQVCHALCSPEGCWGP EPRDCVSCRNVSRGRECVDKCNLLEGEPREFVENSECIQCHPECLP QAMNITCTGRGPDNCIQCAHYIDGPHCVKTCPAGVMGENNTLVW KYADAGHVCHLCHPNCTYGCTGPGLEGCPTNGPKIPSIATGMVG ALLLLLVVALGIGLFM | tEGFR artificial |
| 156 | MLLLVTSLLLCELPHPAFLLIPRKVCNGIGIGEFKDSLSINATNIKHF KNCTSISGDLHILPVAFRGDSFTHTPPLDPQELDILKTVKEITGFLLI QAWPENRTDLHAFENLEIIRGRTKQHGQFSLAVVSLNITSLGLRSL KEISDGDVIISGNKNLCYANTINWKKLFGTSGQKTKIISNRGENSC KATGQVCHALCSPEGCWGPEPRDCVSCRNVSRGRECVDKCNLLE GEPREFVENSECIQCHPECLPQAMNITCTGRGPDNCIQCAHYIDGP HCVKTCPAGVMGENNTLVWKYADAGHVCHLCHPNCTYGCTGP GLEGCPTNGPKIPSIATGMVGALLLLLVVALGIGLFM | tEGFR artificial |
| 157 | FWVLVVVGGVLACYSLLVTVAFIIFWV | CD28 (amino acids 153-179 of Accession No. P10747) Homo sapiens |
| 158 | IEVMYPPPYLDNEKSNGTIIHVKGKHLCPSPLFPGPSKP FWVLVVVGGVLACYSLLVTVAFIIFWV | CD28 (amino acids 114-179 of Accession No. P10747) Homo sapiens |
| 159 | RSKRSRLLHSDYMNMTPRRPGPTRKHYQPYAPPRDFAAYRS | CD28 (amino acids 180-220 of P10747) Homo sapiens |
| 160 | RSKRSRGGHSDYMNMTPRRPGPTRKHYQPYAPPRDFAAYRS | CD28 (LL to GG) Homo sapiens |
| 161 | KRGRKKLLYIFKQPFMRPVQTTQEEDGCSCRFPEEEEGGCEL | 4-1BB (amino acids 214-255 of Q07011.1) Homo sapiens |
| 162 | RVKFSRSADAPAYQQGQNQLYNELNLGRREEYDVLDKRRGRDPE MGGKPRRKNPQEGLYNELQKDKMAEAYSEIGMKGERRRGKGHD GLYQGLSTATKDTYDALHMQALPPR | CD3 zeta Homo sapiens |
| 163 | RVKFSRSAEPPAYQQGQNQLYNELNLGRREEYDVLDKRRGRDPE MGGKPRRKNPQEGLYNELQKDKMAEAYSEIGMKGERRRGKGHD GLYQGLSTATKDTYDALHMQALPPR | CD3 zeta Homo sapiens |
| 164 | RVKFSRSADAPAYKQGQNQLYNELNLGRREEYDVLDKRRGRDPE MGGKPRRKNPQEGLYNELQKDKMAEAYSEIGMKGERRRGKGHD GLYQGLSTATKDTYDALHMQALPPR | CD3 zeta Homo sapiens |

## Claims

1. An anti-idiotype antibody or antigen-binding fragment thereof comprising:
a CDR-H1 comprising the amino acid sequence of SEQ ID NO: 89 or 90, a CDR-H2 comprising the amino acid sequence of SEQ ID NO: 91, 92 or 93, and a CDR-H3 comprising the amino acid sequence of SEQ ID NO: 94; and
a CDR-L1 comprising the amino acid sequence of SEQ ID NO: 95, a CDR-L2 comprising the amino acid sequence of SEQ ID NO: 96, and a CDR-L3 comprising the amino acid sequence of SEQ ID NO: 97, wherein:
(i) the anti-idiotype antibody or antigen-binding fragment thereof specifically binds to a target antibody or an antigen-binding fragment thereof, wherein the antibody or antigen-binding fragment thereof comprises the heavy chain variable (VH) region sequence set forth in SEQ ID NO: 30 and the light chain variable (VL) region sequence set forth in SEQ ID NO: 31; and
(ii) the anti-idiotype antibody or antigen-binding fragment thereof is an agonist antibody of a chimeric antigen receptor (CAR) comprising the target antibody or antigen-binding fragment.

2. The anti-idiotype antibody or antigen-binding fragment of claim 1, wherein the antibody or antigen-binding fragment comprises:
a light chain variable (VL) region comprising at least 90% sequence identity to the VL region amino acid sequence set forth in SEQ ID NO: 40; and
a heavy chain variable (VH) region comprising at least 90% sequence identity to the VH region amino acid sequence set forth in SEQ ID NO: 36.

3. The anti-idiotype antibody or antigen-binding fragment thereof of claim 1 or claim 2, wherein:
the VH region of the antibody or fragment comprises the amino acid sequence of SEQ ID NO: 36; and
the VL region of the antibody or fragment comprises the amino acid sequence of SEQ ID NO: 40.

4. The anti-idiotype antibody or antigen-binding fragment thereof of any one of claims 1-3, which is humanized.

5. The anti-idiotype antibody or antigen-binding fragment thereof of any one of claims 1-4, which is an antigen-binding fragment, optionally wherein the antigen-binding fragment is selected from among fragment antigen binding (Fab) fragments, F(ab')₂ fragments, Fab' fragments, Fv fragments, or a single chain variable fragment (scFv).

6. The anti-idiotype antibody or antigen-binding fragment of any one of claims 1-4, which is a full-length antibody.

7. The anti-idiotype antibody or antigen-binding fragment thereof of any of claims 1-6, wherein the antibody or fragment specifically binds to a single chain variable fragment (scFv) comprised in the extracellular portion of a chimeric antigen receptor, wherein the scFv comprises a heavy chain variable region set forth in SEQ ID NO: 30 and a light chain variable region set forth in SEQ ID NO: 31; and/or comprises the sequence of amino acids set forth in SEQ ID NO: 34.

8. A conjugate, comprising the anti-idiotype antibody or antigen-binding fragment of any one of claims 1-7 and a heterologous molecule or moiety, optionally wherein the heterologous molecule or moiety is:
i) a label, optionally wherein the label is selected from a fluorescent dye, a fluorescent protein, a radioisotope, a chromophore, a metal ion, a gold particle, a silver particle, a magnetic particle, a polypeptide, an enzyme, a streptavidin, a biotin, a luminescent compound or an oligonucleotide; or
ii) a protein, peptide, nucleic acid or small molecule, which optionally is or comprises a toxin or a Strep-Tag.

9. A nucleic acid molecule encoding the heavy chain and light chain of the anti-idiotype antibody or antigen-binding fragment thereof of any one of claims 1-7.

10. A vector, comprising the nucleic acid molecule of claim 9.

11. A cell, comprising the anti-idiotype antibody or antigen-binding fragment thereof of any one of claims 1-7 or the nucleic acid molecule of claim 9.

12. A method of producing an anti-idiotype antibody or antigen-binding fragment thereof, comprising expressing the heavy and light chain encoded by the nucleic acid molecule of claim 9 or the vector of claim 10 in a suitable host cell and recovering or isolating the antibody.

13. A composition comprising the anti-idiotype antibody or antigen-binding fragment thereof of any one of claims 1-7, conjugate of claim 8, or the cell of claim 11, optionally further comprising a pharmaceutically acceptable excipient.

14. A method of detecting a target antibody or antigen-binding fragment thereof, comprising:
(a) contacting a composition comprising a target antibody or an antigen-binding fragment thereof with the anti-idiotype antibody or antigen-binding fragment thereof of any one of claims 1-7 or the conjugate of claim 8 that specifically binds to the target antibody or antigen-binding fragment thereof, wherein the target antibody or antigen-binding fragment thereof comprises the heavy chain variable (VH) region sequence set forth in SEQ ID NO: 30 and the light chain variable (VL) region sequence set forth in SEQ ID NO: 31; and
(b) detecting the anti-idiotype antibody bound to the target antibody or antigen-binding fragment,
optionally wherein the target antibody or antigen-binding fragment is expressed on the surface of a cell, wherein the cell expresses on its surface a CAR comprising the target antibody or antigen-binding fragment, and detecting in (b) comprises detecting cells bound with the anti-idiotype antibody.

15. An in vitro or ex vivo method of stimulating cells, comprising incubating an input composition comprising cells expressing a chimeric antigen receptor (CAR) comprising a target antibody or an antigen-binding fragment thereof with the anti-idiotype antibody or antigen-binding fragment thereof of any one of claims 1-7 or the conjugate of claim 8 that specifically binds to the target antibody or antigen-binding fragment thereof, thereby generating an output composition comprising stimulated cells, wherein the antibody or antigen-binding fragment thereof comprises the heavy chain variable (VH) region sequence set forth in SEQ ID NO: 30 and the light chain variable (VL) region sequence set forth in SEQ ID NO: 31.

16. The method of claim 15, wherein the anti-idiotype antibody or antigen-binding fragment thereof is immobilized to a solid support, which optionally comprises or is conjugated to a reagent comprising a plurality of binding sites capable of reversibly binding to the anti-idiotype antibody or antigen-binding fragment thereof,
optionally wherein the reagent comprises a streptavidin mutein; or
wherein the anti-idiotype antibody or antigen-binding fragment thereof is immobilized to a soluble reagent, which optionally is or comprises a plurality of binding sites capable of reversibly binding to the anti-idiotype antibody or antigen-binding fragment thereof,
optionally wherein the reagent comprises a streptavidin mutein.

17. The method of claim 15 or claim 16, wherein the incubation is for at least 5 minutes, 10 minutes, 30 minutes, 60 minutes, 2 hours, 6 hours, 12 hours, 24 hours, 36, 48 hours, 72 hours or 96 hours, and/or
wherein the input composition comprises less than 60%, less than 50%, less than 40%, less than 30%, less than 20% or less than 10% CAR-expressing cells as a percentage of the total cells in the composition.

18. The method of any one of claims 15-17, wherein:
the number of CAR-expressing cells in the output composition is increased by greater than 1.2-fold, 1.5-fold, 2.0-fold, 3.0-fold, 4.0-fold, 5.0-fold, 10-fold or more compared to the number of CAR-expressing cells in the input composition; and/or
the percentage of CAR-expressing in the output composition compared to the total cells in the composition is increased by greater than 10 %, 20 %, 40 %, 50 %, 60 %, 70 %, 80 % or more; and/or
prior to the introducing and/or incubating the cells are not selected or enriched for CAR-expressing cells.

## Patentansprüche

1. Antiidiotypischer Antikörper oder antigenbindendes Fragment davon, umfassend:
eine CDR-H1, umfassend die Aminosäuresequenz von SEQ ID NO: 89 oder 90, eine CDR-H2, umfassend die Aminosäuresequenz von SEQ ID NO: 91, 92 oder 93, und eine CDR-H3, umfassend die Aminosäuresequenz von SEQ ID NO: 94; und
eine CDR-L1, umfassend die Aminosäuresequenz von SEQ ID NO: 95, eine CDR-L2, umfassend die Aminosäuresequenz von SEQ ID NO: 96, und eine CDR-L3, umfassend die Aminosäuresequenz von SEQ ID NO: 97, wobei:
(i) der antiidiotypische Antikörper oder das antigenbindende Fragment davon spezifisch an einen Zielantikörper oder an ein antigenbindendes Fragment davon bindet, wobei der Antikörper oder das antigenbindende Fragment davon die in SEQ ID NO: 30 dargelegte Sequenz der variablen Region der schweren Kette (VH) und die in SEQ ID NO: 31 dargelegte Sequenz der variablen Region der leichten Kette (VL) umfasst; und
(ii) der antiidiotypische Antikörper oder das antigenbindende Fragment davon ein agonistischer Antikörper eines chimären Antigenrezeptors (CAR) ist, umfassend den Zielantikörper oder das antigenbindende Fragment.

2. Antiidiotypischer Antikörper oder antigenbindendes Fragment davon nach Anspruch 1, wobei der Antikörper oder antigenbindendes Fragment davon umfasst:
eine variable Region der leichten Kette (VL), umfassend mindestens 90 % Sequenzidentität zu der in SEQ ID NO: 40 dargelegten VL-Region-Aminosäuresequenz; und
eine variable Region der schweren Kette (VH), umfassend mindestens 90 % Sequenzidentität zu der in SEQ ID NO: 36 dargelegten VH-Region-Aminosäuresequenz.

3. Antiidiotypischer Antikörper oder antigenbindendes Fragment davon nach Anspruch 1 oder 2, wobei:
die VH-Region des Antikörpers oder Fragments die Aminosäuresequenz von SEQ ID NO: 36 umfasst; und
die VL-Region des Antikörpers oder Fragments die Aminosäuresequenz von SEQ ID NO: 40 umfasst.

4. Antiidiotypischer Antikörper oder antigenbindendes Fragment davon nach einem der Ansprüche 1-3, der/das humanisiert ist.

5. Antiidiotypischer Antikörper oder antigenbindendes Fragment davon nach einem der Ansprüche 1-4, bei dem es sich um ein antigenbindendes Fragment handelt, optional wobei das antigenbindende Fragment ausgewählt ist aus Fab-Fragmenten, F(ab')₂-Fragmenten, Fab'-Fragmenten, Fv-Fragmenten oder einem einkettigen variablen Fragment (scFv).

6. Antiidiotypischer Antikörper oder antigenbindendes Fragment nach einem der Ansprüche 1-4, der/das ein Volllängen-Antikörper ist.

7. Antiidiotypischer Antikörper oder antigenbindendes Fragment davon nach einem der Ansprüche 1-6, wobei der Antikörper oder das Fragment spezifisch an ein einkettiges variables Fragment (scFv) bindet, umfassend in dem extrazellulären Abschnitt eines chimären Antigenrezeptors, wobei das scFv eine variable Region der schweren Kette umfasst, dargelegt in SEQ ID NO: 30, und eine variable Region der leichten Kette, dargelegt in SEQ ID NO: 31; und/oder die Sequenz von Aminosäuren umfasst, die dargelegt ist in SEQ ID NO: 34.

8. Konjugat, umfassend den antiidiotypischen Antikörper oder das antigenbindende Fragment davon nach einem der Ansprüche 1-7 und ein heterologes Molekül oder einen Molekülteil, optional wobei das heterologe Molekül oder der Molekülteil ist:
i) ein Marker, optional wobei der Marker ausgewählt ist aus einem fluoreszierenden Farbstoff, einem fluoreszierenden Protein, einem Radioisotop, einem Chromophor, einem Metallion, einem Goldpartikel, einem Silberpartikel, einem magnetischen Partikel, einem Polypeptid, einem Enzym, einem Streptavidin, einem Biotin, einer lumineszierenden Verbindung oder einem Oligonukleotid; oder
ii) ein Protein, ein Peptid, eine Nukleinsäure oder ein Kleinmolekül, das/die optional ein Toxin oder ein Strep-Tag ist.

9. Nukleinsäuremolekül, das für die schwere Kette oder die leichte Kette des antiidiotypischen Antikörpers oder des antigenbindenden Fragments davon nach einem der Ansprüche 1-7 kodiert.

10. Vektor, umfassend das Nukleinsäuremolekül nach Anspruch 9.

11. Zelle, umfassend den antiidiotypischen Antikörper oder das antigenbindende Fragment davon nach einem der Ansprüche 1-7 oder Nukleinsäuremolekül nach Anspruch 9.

12. Verfahren zum Erzeugen eines antiidiotypischen Antikörpers oder eines antigenbindenden Fragments davon, umfassend das Exprimieren der schweren und der leichten Kette, für die durch das Nukleinsäuremolekül nach Anspruch 9 kodiert wird, oder des Vektors nach Anspruch 10 in einer geeigneten Wirtszelle und zum Gewinnen oder Isolieren des Antikörpers.

13. Zusammensetzung, umfassend den antiidiotypischen Antikörper oder das antigenbindende Fragment davon nach einem der Ansprüche 1-7, das Konjugat nach Anspruch 8 oder die Zelle nach Anspruch 11, optional ferner umfassend einen pharmazeutisch annehmbaren Hilfsstoff.

14. Verfahren zum Nachweisen eines Zielantikörpers oder eines antigenbindenden Fragments davon, umfassend:
(a) Inberührungbringen einer Zusammensetzung, umfassend einen Zielantikörper oder ein antigenbindendes Fragment davon mit dem antiidiotypischen Antikörper oder dem antigenbindenden Fragment davon nach einem der Ansprüche 1-7 oder dem Konjugat nach Anspruch 8, der/das spezifisch an den Zielantikörper oder an das antigenbindende Fragment davon bindet, wobei der Zielantikörper oder das antigenbindende Fragment davon die in SEQ ID NO: 30 dargelegte Sequenz der variablen Region der schweren Kette (VH) und die in SEQ ID NO: 31 dargelegte Sequenz der variablen Region der leichten Kette (VL) umfasst; und
(b) Nachweisen des mit dem Zielantikörper oder dem antigenbindenden Fragment verbundenen antiidiotypischen Antikörpers,
optional wobei der Zielantikörper oder das antigenbindende Fragment auf der Oberfläche einer Zelle exprimiert wird, wobei die Zelle auf ihrer Oberfläche einen CAR exprimiert, umfassend den Zielantikörper oder das antigenbindende Fragment, und das Nachweisen in (b) das Nachweisen von innerhalb des antiidiotypischen Antikörpers gebundenen Zellen umfasst.

15. In-Vitro- oder Ex-Vivo-Verfahren zum Stimulieren von Zellen, umfassend das Inkubieren einer Eingabezusammensetzung, umfassend Zellen, die einen chimären Antigenrezeptor (CAR) exprimieren, umfassend einen Zielantikörper oder ein antigenbindendes Fragment davon, mit dem antiidiotypischen Antikörper oder dem antigenbindenden Fragment davon nach einem der Ansprüche 1-7 oder dem Konjugat nach Anspruch 8, das spezifisch an den Zielantikörper oder das antigenbindende Fragment davon bindet, wodurch eine Ausgabezusammensetzung erzeugt wird, umfassend stimulierte Zellen, wobei der Antikörper oder das antigenbindende Fragment davon die in SEQ ID NO: 30 dargelegte Sequenz der variablen Region der schweren Kette (VH) und die in SEQ ID NO: 31 dargelegte Sequenz der variablen Region der leichten Kette (VL) umfasst.

16. Verfahren nach Anspruch 15, wobei der antiidiotypische Antikörper oder das antigenbindende Fragment davon auf einem festen Träger immobilisiert ist, das optional ein Reagens umfasst oder daran konjugiert ist, umfassend eine Vielzahl von Bindungsstellen, die in der Lage sind, reversibel an den antiidiotypischen Antikörper oder das antigenbindende Fragment davon zu binden,
optional wobei das Reagens ein Streptavidin-Mutein umfasst; oder
wobei der antiidiotypische Antikörper oder das antigenbindende Fragment davon auf einem löslichen Reagens immobilisiert ist, das optional eine Vielzahl von Bindungsstellen umfasst oder daran konjugiert ist, die in der Lage sind, reversibel an den antiidiotypischen Antikörper oder das antigenbindende Fragment davon zu binden,
optional wobei das Reagens ein Streptavidin-Mutein umfasst.

17. Verfahren nach Anspruch 15 oder 16, wobei die Inkubierung mindestens 5 Minuten, 10 Minuten, 30 Minuten, 60 Minuten, 2 Stunden, 6 Stunden, 12 Stunden, 24 Stunden, 36, 48 Stunden, 72 Stunden oder 96 Stunden andauert und/oder
wobei die Eingabezusammensetzung weniger als 60 %, weniger als 50 %, weniger als 40 %, weniger als 30 %, weniger als 20 % oder weniger als 10 % CAR exprimierende Zellen umfasst, ausgedrückt als ein Prozentsatz der Gesamtzellen in der Zusammensetzung.

18. Verfahren nach einem der Ansprüche 15-17, wobei:
die Anzahl von CAR exprimierenden Zellen in der Ausgabezusammensetzung im Vergleich zu der Anzahl von CAR exprimierenden Zellen in der Eingabezusammensetzung um mehr als das 1,2fache, das 1,5fache, das 2,0fache, das 3,0fache, das 4,0fache, das 5,0fache, das 10fache oder mehr gesteigert ist; und/oder
der Prozentsatz von CAR-Expression in der Ausgabezusammensetzung im Vergleich zu den Gesamtzellen in der Zusammensetzung um mehr als 10 %, 20 %, 40 %, 50 %, 60 %, 70 %, 80 % oder mehr gesteigert ist; und/oder vor dem Einführen und/oder dem Inkubieren die Zellen nicht in Bezug auf CAR exprimierende Zellen ausgewählt oder angereichert werden.

## Revendications

1. Anticorps anti-idiotype ou fragment de liaison à un antigène de celui-ci comprenant :
une CDR-H1 comprenant la séquence d'acides aminés de SEQ ID NO: 89 ou 90, une CDR-H2 comprenant la séquence d'acides aminés de SEQ ID NO: 91, 92 ou 93, et une CDR-H3 comprenant la séquence d'acides aminés de SEQ ID NO: 94 ; et
une CDR-L1 comprenant la séquence d'acides aminés de SEQ ID NO: 95, une CDR-L2 comprenant la séquence d'acides aminés de SEQ ID NO: 96, et une CDR-L3 comprenant la séquence d'acides aminés de SEQ ID NO: 97, dans lequel :
(i) l'anticorps anti-idiotype ou un fragment de liaison à un antigène de celui-ci se lie spécifiquement à un anticorps cible ou à un fragment de liaison à un antigène de celui-ci, dans lequel l'anticorps ou le fragment de liaison à un antigène de celui-ci comprend la séquence de région variable de chaîne lourde (VH) présentée dans SEQ ID NO: 30 et la séquence de région variable de chaîne légère (VL) présentée dans SEQ ID NO: 31 ; et
(ii) l'anticorps anti-idiotype ou un fragment de liaison à un antigène de celui-ci est un anticorps agoniste d'un récepteur antigénique chimérique (CAR) comprenant l'anticorps cible ou le fragment de liaison à un antigène.

2. Anticorps anti-idiotype ou fragment de liaison à un antigène selon la revendication 1, dans lequel l'anticorps ou le fragment de liaison à un antigène comprend :
une région variable de chaîne légère (VL) comprenant au moins 90 % d'identité de séquence avec la séquence d'acides aminés de la région VL présentée dans SEQ ID NO: 40 ; et
une région variable de chaîne lourde (VH) comprenant au moins 90 % d'identité de séquence avec la séquence d'acides aminés de la région VH présentée dans SEQ ID NO: 36.

3. Anticorps anti-idiotype ou fragment de liaison à un antigène de celui-ci selon la revendication 1 ou la revendication 2, dans lequel :
la région VH de l'anticorps ou du fragment comprend la séquence d'acides aminés de SEQ ID NO: 36 ; et
la région VL de l'anticorps ou du fragment comprend la séquence d'acides aminés de SEQ ID NO: 40.

4. Anticorps anti-idiotype ou fragment de liaison à un antigène de celui-ci selon l'une quelconque des revendications 1 à 3, qui est humanisé.

5. Anticorps anti-idiotype ou fragment de liaison à un antigène de celui-ci selon l'une quelconque des revendications 1 à 4, qui est un fragment de liaison à un antigène, éventuellement dans lequel le fragment de liaison à un antigène est choisi parmi des fragments de liaison à un antigène (Fab), des fragments F(ab')₂, des fragments Fab', des fragments Fv, ou un fragment variable à chaîne unique (scFv).

6. Anticorps anti-idiotype ou fragment de liaison à un antigène selon l'une quelconque des revendications 1 à 4, qui est un anticorps de pleine longueur.

7. Anticorps anti-idiotype ou fragment de liaison à un antigène de celui-ci selon l'une quelconque des revendications 1 à 6, dans lequel l'anticorps ou le fragment se lie spécifiquement à un fragment variable à chaîne unique (scFv) compris dans la partie extracellulaire d'un récepteur antigénique chimérique, dans lequel le scFv comprend une région variable de chaîne lourde présentée dans SEQ ID NO: 30 et une région variable de chaîne légère présentée dans SEQ ID NO: 31 ; et/ou comprend la séquence d'acides aminés présentée dans SEQ ID NO: 34.

8. Conjugué, comprenant l'anticorps anti-idiotype ou le fragment de liaison à un antigène selon l'une quelconque des revendications 1 à 7 et une molécule ou un fragment hétérologue, éventuellement dans lequel la molécule ou le fragment hétérologue est :
i) un marqueur, éventuellement dans lequel le marqueur est choisi parmi un colorant fluorescent, une protéine fluorescente, un radio-isotope, un chromophore, un ion métallique, une particule d'or, une particule d'argent, une particule magnétique, un polypeptide, une enzyme, une streptavidine, une biotine, un composé luminescent ou un oligonucléotide ; ou
ii) une protéine, un peptide, un acide nucléique ou une petite molécule, qui est ou comprend éventuellement une toxine ou un Strep-Tag.

9. Molécule d'acide nucléique codant pour la chaîne lourde et la chaîne légère de l'anticorps anti-idiotype ou le fragment de liaison à un antigène de celui-ci selon l'une quelconque des revendications 1 à 7.

10. Vecteur, comprenant la molécule d'acide nucléique selon la revendication 9.

11. Cellule, comprenant l'anticorps anti-idiotype ou un fragment de liaison à un antigène de celui-ci selon l'une quelconque des revendications 1 à 7 ou la molécule d'acide nucléique selon la revendication 9.

12. Procédé de production d'un anticorps anti-idiotype ou d'un fragment de liaison à un antigène de celui-ci, comprenant l'expression de la chaîne lourde et légère codée par la molécule d'acide nucléique de la revendication 9 ou le vecteur de la revendication 10 dans une cellule hôte appropriée et la récupération ou l'isolement de l'anticorps.

13. Composition comprenant l'anticorps anti-idiotype ou un fragment de liaison à un antigène de celui-ci selon l'une quelconque des revendications 1 à 7, le conjugué selon la revendication 8, ou la cellule selon la revendication 11, comprenant éventuellement en outre un excipient pharmaceutiquement acceptable.

14. Procédé de détection d'un anticorps cible ou d'un fragment de liaison à un antigène de celui-ci, comprenant :
(a) la mise en contact d'une composition comprenant un anticorps cible ou un fragment de liaison à un antigène de celui-ci avec l'anticorps anti-idiotype ou le fragment de liaison à un antigène de celui-ci selon l'une quelconque des revendications 1 à 7 ou le conjugué selon la revendication 8 qui se lie spécifiquement à l'anticorps cible ou au fragment de liaison à un antigène de celui-ci, dans lequel l'anticorps cible ou le fragment de liaison à un antigène de celui-ci comprend la séquence de région variable de chaîne lourde (VH) présentée dans SEQ ID NO: 30 et la séquence de région variable de chaîne légère (VL) présentée dans SEQ ID NO: 31 ; et
(b) la détection de l'anticorps anti-idiotype lié à l'anticorps cible ou au fragment de liaison à un antigène,
éventuellement, dans lequel l'anticorps cible ou le fragment de liaison à un antigène est exprimé sur la surface d'une cellule, la cellule exprimant sur sa surface un CAR comprenant l'anticorps cible ou le fragment de liaison à un antigène, et la détection dans (b) comprend la détection de cellules liées à l'anticorps anti-idiotype.

15. Procédé *in vitro* ou ex *vivo* de stimulation de cellules, comprenant l'incubation d'une composition d'entrée comprenant des cellules exprimant un récepteur antigénique chimérique (CAR) comprenant un anticorps cible ou un fragment de liaison à un antigène de celui-ci avec l'anticorps anti-idiotype ou le fragment de liaison à un antigène de celui-ci selon l'une quelconque des revendications 1 à 7 ou le conjugué selon la revendication 8 qui se lie spécifiquement à l'anticorps cible ou au fragment de liaison à un antigène de celui-ci, générant ainsi une composition de sortie comprenant des cellules stimulées, dans lequel l'anticorps ou le fragment de liaison à un antigène de celui-ci comprend la séquence de région variable de chaîne lourde (VH) présentée dans SEQ ID NO: 30 et la séquence de région variable de chaîne légère (VL) présentée dans SEQ ID NO: 31.

16. Procédé selon la revendication 15, dans lequel l'anticorps anti-idiotype ou un fragment de liaison à un antigène de celui-ci est immobilisé sur un support solide, qui comprend ou est conjugué éventuellement à un réactif comprenant une pluralité de sites de liaison capables de se lier de manière réversible à l'anticorps anti-idiotype ou au fragment de liaison à un antigène de celui-ci,
éventuellement, dans lequel le réactif comprend une mutéine de streptavidine ; ou
dans lequel l'anticorps anti-idiotype ou un fragment de liaison à un antigène de celui-ci est immobilisé sur un réactif soluble, qui est ou comprend éventuellement une pluralité de sites de liaison capables de se lier de manière réversible à l'anticorps anti-idiotype ou au fragment de liaison à un antigène de celui-ci,
éventuellement, dans lequel le réactif comprend une mutéine de streptavidine.

17. Procédé selon la revendication 15 ou la revendication 16, dans lequel l'incubation dure au moins 5 minutes, 10 minutes, 30 minutes, 60 minutes, 2 heures, 6 heures, 12 heures, 24 heures, 36 heures, 48 heures, 72 heures ou 96 heures, et/ou
dans lequel la composition d'entrée comprend moins de 60 %, moins de 50 %, moins de 40 %, moins de 30 %, moins de 20 % ou moins de 10 % de cellules exprimant un CAR en pourcentage des cellules totales dans la composition.

18. Procédé selon l'une quelconque des revendications 15 à 17, dans lequel :
le nombre de cellules exprimant un CAR dans la composition de sortie est augmenté de plus de 1,2 fois, 1,5 fois, 2,0 fois, 3,0 fois, 4,0 fois, 5,0 fois, 10 fois ou plus par rapport au nombre de cellules exprimant un CAR dans la composition d'entrée ; et/ou
le pourcentage d'expression de CAR dans la composition de sortie par rapport aux cellules totales dans la composition est augmenté de plus de 10 %, 20 %, 40 %, 50 %, 60 %, 70 %, 80 % ou plus ; et/ou
avant l'introduction et/ou l'incubation, les cellules ne sont pas sélectionnées ou enrichies pour des cellules exprimant un CAR.
